(19) Europäisches Patentamt
European Patent Office
Office européen des brevets

(11) EP 4 691 496 A1

(12) **EUROPEAN PATENT APPLICATION**
published in accordance with Art. 153(4) EPC

(43) Date of publication:
**11.02.2026 Bulletin 2026/07**

(21) Application number: **24778276.6**

(22) Date of filing: **29.03.2024**

(51) International Patent Classification (IPC):
***A61K 47/68*** (2017.01) ***C07K 19/00*** (2006.01)
***A61K 38/16*** (2006.01) ***A61P 25/28*** (2006.01)
***A61P 3/10*** (2006.01)

(52) Cooperative Patent Classification (CPC):
**A61K 38/16; A61K 47/68; A61P 3/10; A61P 25/28; C07K 19/00**

(86) International application number:
**PCT/CN2024/085034**

(87) International publication number:
**WO 2024/199491 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.03.2023 CN 202310331480**
**21.06.2023 PCT/CN2023/101978**
**27.03.2024 CN 202410366079**

(71) Applicants:
- **Guangzhou Innogen Pharmaceutical Group Co., Ltd.**
**Guangzhou, Guangdong 510700 (CN)**
- **Shanghai Innogen Pharmaceutical Technology Co., Ltd.**
**Pudong New Area, Shanghai 201203 (CN)**
- **Shanghai Innogen Biomedical Engineering Co., Ltd.**
**Shanghai 201908 (CN)**

(72) Inventor: **WANG, Qinghua**
**Shanghai 201203 (CN)**

(74) Representative: **FRKelly**
**Waterways House**
**Grand Canal Quay**
**Dublin D02 PD39 (IE)**

Remarks:
The complete document including Reference Table(s) and the Sequence Listing(s) can be downloaded from the EPO website

# (54) PHARMACEUTICAL PREPARATION CONTAINING GLP-1 FUSION PROTEIN AND USE THEREOF

(57) The present invention relates to a pharmaceutical preparation containing a GLP-1 fusion protein and its application. The pharmaceutical preparation of the present invention comprises: a GLP-1 fusion protein; a buffer; wherein the pH value of the pharmaceutical preparation is in the range of about 6.0 to about 7.0. The pharmaceutical preparation of the present invention is long-acting, stable, has few side effects, has a simple preparation process, a clear route, is safe and reliable, has low adverse reactions, and is easy to use.

EP 4 691 496 A1

## Description

Technical field

The present invention belongs to the technical field of pharmaceutical preparations, and specifically relates to pharmaceutical preparations containing GLP-1 fusion protein and their applications.

Glucagon-like peptide-1 (GLP-1), also known as incretin, is secreted by L cells in the small intestine. It exerts multi-organ targeted regulatory effects, including promoting insulin secretion, inhibiting glucagon release, slowing gastric emptying, reducing appetite, and playing an important role in the regulation of nutrient intake and absorption. The biological effects of GLP-1 are primarily mediated through the activation of the GLP-1 receptor (GLP-1R). GLP-1R is a G protein-coupled membrane protein primarily expressed in pancreatic beta cells, with varying degrees of expression in other tissues and cells such as the lungs, heart, kidneys, gastrointestinal tract, and brain. Upon binding to the receptor, GLP-1 activates adenylate cyclase (AC), thereby stimulating the generation of the second messenger cyclic adenosine monophosphate (cAMP) and interacting with protein kinase A (PKA) and cAMP-regulated guanine nucleotide exchange factors (camp GEFs) of the Epac family ( Leech, C.A., et al., Expression of cAMP-regulated guanine nucleotide exchange factors in pancreatic beta-cells. Biochem Biophys Res Commun, 2000. 278(1): p. 44-7 ) .

The natural half-life of GLP-1 in the human body is only 1-2 minutes, primarily due to rapid enzymatic inactivation, including by dipeptidyl peptidase-IV (DPP-IV), and/or renal clearance . Therefore, scientists have developed various long-acting GLP-1 analogs resistant to degradation. For example, human GLP-1 analogs have been modified through amino acid substitutions and/or N-terminal modifications, including fatty acylation and N-acetylation , to extend their circulating half-life. Albumin-conjugated GLP-1 (albiglutide) also exhibits an extended half-life. **In** recent years, various GLP-1 receptor agonists and analogs have been widely used for the treatment of metabolic disorders related to glucose and lipid metabolism, particularly type 2 diabetes mellitus (T2DM) and obesity. GLP-1 receptor agonists play a significant role in diabetes treatment and also have preventive and therapeutic effects on cardiovascular diseases and neurological disorders. Additionally, GLP-1 can bind to GLP-1 receptors in organs such as the kidneys and skin, influencing tissue metabolism and related diseases.

The GLP-1 fusion protein disclosed in US Patent US8658174 comprises a GLP-1 peptide fused with an IgG/Fc domain and can be used for the treatment of diabetes.

The use of genetic engineering recombinant protein technology to produce fusion proteins for therapeutic purposes aims to express the properties of natural polypeptides. The process involves cell engineering steps of transcription, translation and post-translational modification. The process directly affects the physicochemical characteristics, conformation, half-life in vivo, biological activity and production yield of the drug. Therefore, there is still a need for improved GLP-1 fusion proteins, such as those suitable for large-scale production, with higher yield and activity and longer half-life for clinical treatment, which is of great significance.

At present, there is an urgent need for a fusion protein preparation that can ensure long-term storage and is easy to use. Since the usual protein freeze-drying method is very time-consuming and energy-consuming, and the freeze-dried powder is troublesome to use, which is not conducive to patients' home use, the research and development of long-acting, stable, low-side effect, simple preparation process, clear route, safe and reliable, low adverse reaction, and easy-to-use GLP-1 analog preparation products is of great significance.

Invention content

The purpose of the present invention is to provide a pharmaceutical preparation containing an improved GLP-1 receptor agonist fusion protein, which has many advantages: long-term, stable, low side effects, safe and reliable preparation process, low adverse reactions, and easy to use.

In the pharmaceutical preparation containing GLP-1 fusion protein provided by the present invention, the GLP-1 fusion protein is an improved GLP-1 fusion protein with the characteristics of increased yield and activity, and/or prolonged half-life. The fusion protein can be obtained by a variety of ways or methods, such as selecting amino acid replacement, hydroxylation, oxidation, etc. at a specific protein position, such as by hydroxylating K34 of the GLP-1 polypeptide and/or reducing the oxidation of the GLP-1 polypeptide. In addition, the amino acid replacement at a specific position of the GLP-1 fusion protein in the pharmaceutical preparation provided by the present invention significantly prolongs the half-life of the improved fusion protein, and at the same time has good preventive and therapeutic effects in human diseases and animal disease models.

In one aspect, the present invention provides a pharmaceutical preparation comprising:

(a) a GLP-1 fusion protein, the GLP-1 fusion protein comprising a GLP-1 polypeptide and an immunoglobulin Fc domain, wherein the GLP-1 polypeptide is covalently linked to the immunoglobulin Fc domain, wherein,

the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G;
the immunoglobulin Fc domain comprises or is an IgG2-Fc domain, and the IgG2-Fc domain comprises one or more amino acid substitutions selected from the following group: C222S, A330S and P331S;
and

(b) a buffer;

wherein, the pH value of the pharmaceutical preparation is in the range of about 6.0 to about 7.0.

In another aspect, the present invention provides a pharmaceutical preparation comprising:

(a) a GLP-1 fusion protein, the fusion protein comprising a GLP-1 polypeptide and an immunoglobulin Fc domain, wherein the GLP-1 polypeptide is covalently linked to the immunoglobulin Fc domain, wherein,

the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G;
the immunoglobulin Fc domain comprises or is an IgG2-Fc domain, the IgG2-Fc domain comprises one or more amino acid substitutions selected from the following group: C222S, A330S and P331S;
and

(b) a buffer, the buffer being selected from the following group: phosphate buffer, citrate buffer, borate buffer, histidine buffer and acetate buffer.

In some embodiments, the buffer is a phosphate buffer. In some embodiments, the phosphate buffer comprises phosphate dibasic (e.g., disodium hydrogen phosphate, dipotassium hydrogen phosphate, etc.), dihydrogen phosphate (e.g., sodium dihydrogen phosphate, potassium dihydrogen phosphate, etc.), or a combination thereof. In some embodiments, the phosphate buffer is a disodium hydrogen phosphate/sodium dihydrogen phosphate buffer. In some embodiments, the phosphate buffer is prepared from disodium hydrogen phosphate hydrate and sodium dihydrogen phosphate hydrate. In some embodiments, the phosphate buffer is prepared from disodium hydrogen phosphate dodecahydrate and sodium dihydrogen phosphate monohydrate. In some embodiments, the concentration of the phosphate buffer in the pharmaceutical preparation is about 5 mM to about 15 mM (e.g., about 10 mM).

In some embodiments, the pharmaceutical preparation provided by the present invention further comprises carbohydrates. In some embodiments, the carbohydrates are selected from the group consisting of mannitol, sorbitol, maltitol, erythritol, arabitol, xylitol, sucrose, lactose, trehalose, dextran, and combinations thereof. In some embodiments, the carbohydrates are selected from one or more of mannitol, sucrose, and sorbitol, for example, the carbohydrates are mannitol. In some embodiments, the concentration of the carbohydrates in the pharmaceutical preparation is 1-10% (w/v), for example, about 4.6% (w/v).

In some embodiments, the pharmaceutical preparation provided by the present invention further comprises a surfactant. In some embodiments, the surfactant is selected from the following group: polysorbate (e.g., polysorbate 80), polyoxyethylene castor oil derivatives, poloxamer (e.g., poloxamer 188), lecithin, polyethylene glycol 15-hydroxystearate, cyclodextrins and combinations thereof. In some embodiments, the surfactant is polysorbate 80. In some embodiments, the concentration of the surfactant in the pharmaceutical preparation is 0.01% (w/v) - 0.04% (w/v), for example, about 0.02% (w/v). In some embodiments, the pharmaceutical preparation comprises the GLP-1 fusion protein, phosphate buffer, carbohydrates and surfactant.

In certain embodiments, the pharmaceutical preparation comprises the GLP-1 fusion protein, a phosphate buffer (e.g., disodium hydrogen phosphate/sodium dihydrogen phosphate buffer), mannitol, and polysorbate.

In certain embodiments, the concentration of the GLP-1 fusion protein in the pharmaceutical preparation is 0.2-20 mg/mL (e.g., 1-5 mg/mL).

In certain embodiments, the pharmaceutical preparation provided by the present invention comprises: (a) a GLP-1 fusion protein, wherein the GLP-1 fusion protein has an amino acid sequence as shown in SEQ ID NO: 7; (b) disodium hydrogen phosphate/sodium dihydrogen phosphate buffer; (c) mannitol; and (d) polysorbate 80.

In certain embodiments, the pharmaceutical preparation provided by the present invention comprises: (a) 0.2-20 mg/mL GLP-1 fusion protein, wherein the GLP-1 fusion protein has an amino acid sequence as shown in SEQ ID NO: 7; (b) 5-15 mM disodium hydrogen phosphate/sodium dihydrogen phosphate buffer; (c) 1-10% (w/v) mannitol; and (d) 0.01% (w/v) - 0.04% (w/v) polysorbate 80.

In certain embodiments, the pharmaceutical preparation provided by the present invention comprises: (a) about

2 mg/mL, about 4 mg/mL or about 6 mg/mL GLP-1 fusion protein, wherein the amino acid sequence of the GLP-1 fusion protein is as shown in SEQ ID NO: 7; (b) about 10 mM disodium hydrogen phosphate/sodium dihydrogen phosphate buffer; (c) about 4.6% (w/v) mannitol; and (d) about 0.02% (w/v) polysorbate 80.

In certain embodiments, the pharmaceutical preparation provided by the present invention comprises: (a) about 5.3 mg/mL, about 6.67 mg/mL, about 10 mg/mL, about 12 mg/mL, about 13.3 mg/mL or about 20 mg/mL of GLP-1 fusion protein, wherein the amino acid sequence of the GLP-1 fusion protein is as shown in SEQ ID NO: 7; (b) about 10 mM disodium hydrogen phosphate/sodium dihydrogen phosphate buffer; (c) about 4.6% (w/v) mannitol; and (d) about 0.02% (w/v) polysorbate 80.

In certain embodiments, the unit dose of the pharmaceutical preparation provided by the present invention is about 0.5 mL, and comprises: (a) about 1 mg, about 2 mg or about 3 mg of GLP-1 fusion protein, wherein the amino acid sequence of the GLP-1 fusion protein is as shown in SEQ ID NO: 7; (b) about 0.58 mg of disodium hydrogen phosphate dodecahydrate; (c) about 0.465 mg of sodium dihydrogen phosphate monohydrate; (d) about 23.2 mg of mannitol; and (e) about 0.1 mg of polysorbate 80, and the rest is water for injection.

In certain embodiments, the unit dose of the pharmaceutical preparation provided by the present invention is about 0.75 mL, and comprises: (a) about 4 mg, about 5 mg, about 7.5 mg, about 9 mg, about 10 mg or about 15 mg of GLP-1 fusion protein, wherein the amino acid sequence of the GLP-1 fusion protein is as shown in SEQ ID NO: 7; (b) about 0.87 mg of disodium hydrogen phosphate dodecahydrate; (c) about 0.6975 mg of sodium dihydrogen phosphate monohydrate; (d) about 34.8 mg of mannitol; and (e) about 0.15 mg of polysorbate 80, and the rest is water for injection.

In certain embodiments, the pharmaceutical preparation provided by the present invention has a pH value in the range of 6.4-7.0.

In certain embodiments, the pharmaceutical preparation provided by the present invention is isotonic. In certain embodiments, the pharmaceutical preparation provided by the present invention is a liquid preparation (e.g., an injection). In certain embodiments, the pharmaceutical preparation is administered by intravenous injection, subcutaneous injection or intramuscular injection.

In certain embodiments, the pharmaceutical preparation provided by the present invention remains stable for at least 3 months at 25±2°C, or remains stable for at least 2 weeks at 40±2°C.

In certain embodiments, after the pharmaceutical preparation provided by the present invention is placed for at least 1 month, the pH value changes by no more than about 10% (e.g., no more than about 5%, no more than about 4%, no more than about 3%, no more than about 2%, no more than about 1%).

In certain embodiments, after the pharmaceutical preparation provided by the present invention is placed for at least 1 month, the content of high molecular weight (HMW) derivatives or low molecular weight (LMW) derivatives does not exceed about 10% (e.g., no more than about 5%, no more than about 4%, no more than about 3%, no more than about 2%, no more than about 1%, etc.). In certain embodiments, the content of the HMW derivatives and/or LMW derivatives is determined by size exclusion chromatography (SEC) or reversed phase liquid chromatography (RP-LC).

In certain embodiments, after the pharmaceutical preparation provided by the present invention is placed for at least 1 month, the purity changes by no more than about 5% (e.g., no more than about 3%, no more than about 2%, no more than about 1%, etc.). In certain embodiments, the purity of the pharmaceutical preparations provided herein is determined by SDS-capillary electrophoresis (eg, non-reducing SDS-capillary electrophoresis).

In certain embodiments, the isoelectric point of the pharmaceutical preparation provided by the present invention does not change by more than about 5% (e.g., no more than about 4%, no more than about 3%, no more than about 2%, no more than about 1%, etc.) after being placed for at least 1 month. In certain embodiments, the isoelectric point of the pharmaceutical preparation is determined by capillary focusing isoelectric electrophoresis (cIEF).

In certain embodiments, the GLP-1 polypeptide in the GLP-1 fusion protein has a certain level of hydroxylation at lysine 34 (K34) relative to native human GLP-1. In certain embodiments, the hydroxylation level is between 10% and 100%, for example, greater than or equal to 10%, or greater than or equal to 15%, or greater than or equal to 20%, or greater than or equal to 26%, greater than or equal to 27%, greater than or equal to 28%, greater than or equal to 29%, or greater than or equal to 30%, or greater than or equal to 35%, or greater than or equal to 40%, or greater than or equal to 45%, or greater than or equal to 50%, or greater than or equal to 55%, or greater than or equal to 60%, or greater than or equal to 65%, or greater than or equal to 70%, or greater than or equal to 75%, or greater than or equal to 80%, or greater than or equal to 85%, or greater than or equal to 90%, or greater than or equal to 95%.

In certain embodiments, the GLP-1 polypeptide in the GLP-1 fusion protein has a reduced degree of oxidation at tryptophan 31 (W31) relative to native human GLP-1. In certain embodiments, the GLP-1 polypeptide in the GLP-1 fusion protein is substantially unoxidized at W31 relative to native human GLP-1. In certain embodiments, the GLP-1 polypeptide in the GLP-1 fusion protein has an oxidation level of less than 0.5% (e.g., less than 0.4%, less than 0.3%, less than 0.2%, or less than 0.1%) or undetectable on W31 relative to native human GLP-1.

In certain embodiments, the GLP-1 polypeptide in the GLP-1 fusion protein has at least 90% sequence identity compared to the amino acid sequence shown in SEQ ID NO: 1, SEQ ID NO: 2, or SEQ ID NO: 3, and comprises one or more amino acid substitutions selected from the group consisting of A8G, G22E, and R36G relative to native human GLP-1. In

certain embodiments, the amino acid sequence of the GLP-1 polypeptide in the GLP-1 fusion protein is shown in SEQ ID NO: 3.

In certain embodiments, the IgG2-Fc domain in the GLP-1 fusion protein is an Fc domain from human IgG2. In certain embodiments, the IgG2-Fc domain has a reduced degree of oxidation at methionine 253 (M253) corresponding to SEQ ID NO: 7. In certain embodiments, the IgG2-Fc domain has a certain level of oxidation at M253 corresponding to SEQ ID NO: 7. In certain embodiments, the level of oxidation at M253 corresponding to SEQ ID NO: 7 of the IgG2-Fc domain is less than or equal to about 15%, less than or equal to about 10%, less than or equal to about 9%, less than or equal to about 8%, less than or equal to about 7%, less than or equal to about 6%, less than or equal to about 5%, less than or equal to about 4%, less than or equal to about 3%, less than or equal to about 2%, less than or equal to about 1%, less than or equal to about 0.5%, or undetectable.

In certain embodiments, the IgG2-Fc domain in the GLP-1 fusion protein has at least 90% sequence identity compared to the amino acid sequence shown in SEQ ID NO: 5 or SEQ ID NO: 6, and comprises one or more amino acid substitutions selected from the group consisting of C222S, A330S, and P331S. In certain embodiments, the amino acid sequence of the IgG2-Fc domain in the GLP-1 fusion protein is as shown in SEQ ID NO: 6.

In certain embodiments, the GLP-1 fusion protein in the pharmaceutical preparation provided by the present invention includes a GLP-1 polypeptide as shown in SEQ ID NO: 3, and includes an immunoglobulin Fc domain as shown in SEQ ID NO: 6.

In certain embodiments, in the GLP-1 fusion protein in the pharmaceutical preparation provided by the present invention, the GLP-1 polypeptide is covalently linked to the immunoglobulin Fc domain via a linker. In certain embodiments, in the GLP-1 fusion protein in the pharmaceutical preparation provided by the present invention, the amino acid sequence of the GLP-1 polypeptide is as shown in SEQ ID NO: 3, the amino acid sequence of the immunoglobulin Fc domain is as shown in SEQ ID NO: 6, and the amino acid sequence of the linker is as shown in SEQ **ID** NO: 9.

In certain embodiments, the GLP-1 fusion protein in the pharmaceutical preparation provided by the present invention has an amino acid sequence as shown in SEQ ID NO: 7 or an amino acid sequence having at least 80% sequence identity with SEQ ID NO: 7. In another aspect, the present invention also provides the use of a pharmaceutical preparation comprising a GLP-1 fusion protein as described herein in the preparation of a drug for treating or preventing a disease.

In another aspect, the present invention also provides the use of a pharmaceutical preparation comprising a GLP-1 fusion protein as described herein and an additional therapeutic agent in the preparation of a drug for treating or preventing a disease.

In another aspect, the present invention also provides a pharmaceutical combination comprising a pharmaceutical preparation comprising a GLP-1 fusion protein as described herein and an additional therapeutic agent.

In certain embodiments, the disease is selected from the group consisting of metabolic diseases associated with disorders of glucose metabolism and/or lipid metabolism, complications of metabolic diseases, and neurological diseases and other related diseases. In certain embodiments, the additional therapeutic agent is selected from the group consisting of insulin, metformin, sulfonylurea drugs (e.g., glimepiride, glibenclamide, gliclazide, gliquidone), $\alpha$-glucosidase inhibitors (e.g., acarbose) and $\gamma$-aminobutyric acid. Other features and advantages of the present invention will be apparent from the detailed description below in the embodiments. While the detailed description and specific examples are given by way of illustration only of the preferred embodiment of the invention, various changes and modifications within the spirit and scope of the invention will be apparent to those skilled in the art.

BRIEF DESCRIPTION OF THE DRAWINGS:

Figure 1 is a schematic diagram of the pKN012-GLP1-IgG2/Fc vector.

Figure 2 shows the mass spectrum of the enzymatic cleavage peptide segment 27-34: EFIAWLVK (+16Da) modified with GLP-1 fusion protein YN-011 after Lys-C digestion. A: Primary mass spectrum of EFIAWLVK; B: Primary mass spectrum of EFIAWLVK (+16 Da) modification; C: Secondary mass spectrum of EFIAWLVK; D: Secondary mass spectrum of EFIAWLVK (+16 Da) modification.

Figure 3A shows the UV detection data for the modified peptide 27-34 (aa21-28) with EFIAWLVK (+16 Da) modification, the unmodified peptide 54-79 (aa48-73) without the +16 Da modification, and the unmodified peptide 224-240 (aa218-234) after Lys-C digestion.

Figure 3B is an enlarged view of the inset in Figure 3A.

Figure 4 depicts the average plasma concentration-time profiles following single and multiple subcutaneous injections of 1 mg, 2 mg, 3 mg, and 4 mg of YN-011 in T2DM subjects. Figures 4A and 4B represent the average plasma concentration-time profiles for single subcutaneous administration, while Figures 4C and 4D represent the average plasma concentration-time profiles for multiple subcutaneous administration.

Figure 5 presents the schematic diagram of a Phase IIa double-blind, placebo-controlled study conducted in T2DM subjects to assess the efficacy and safety of YN-011 administered subcutaneously at dose levels of 1 mg, 2 mg, 3 mg, and 4 mg. The filled triangles represent the administration of YN-011 at the specified dose levels, filled circles indicate the oral glucose tolerance test (OGTT) measurements, empty triangles represent adaptive dosing, and pentagrams represent safety evaluations.

Figure 6 displays the impact of multiple doses of YN-011 on fasting blood glucose levels in T2DM subjects. (At each time point, YN-011 demonstrated a significant difference compared to the placebo at dose levels of 3 mg and 4 mg, with a p-value of < 0.05.)

Figure 7 displays the impact of multiple doses of YN-011 on HbA1c levels in T2DM subjects. (At each time point, YN-011 demonstrated a significant difference compared to the placebo at dose levels of 1 mg, 3 mg, and 4 mg, with a p-value of < 0.05.)

Figure 8 represents the change in body weight (BW) of obese rhesus monkeys following repeated subcutaneous injections of YN-011.

Figure 9 illustrates the results after 48 hours of TNF-α stimulation on SH-SY5Y neuronal cells at different concentrations (*P<0.05 TNF-α (20 ng/ml) vs control; **P<0.01 TNF-α (40, 60, 80, 100 ng/ml) vs control).

Figure 10 demonstrates the effects of YN-011 and GABA on inhibiting TNF-α-induced reduction in SH-SY5Y cell viability (**P<0.01 TNF-α vs control; #P<0.05 YN-011 (10 nM or 100 nM) or GABA (100 μM) + TNF-α vs TNF-α; ##P<0.01 YN-011 (500 nM) + TNF-α vs TNF-α; n = 6).

Figure 11 demonstrates a significant increase in SH-SY5Y cell viability with the combined use of YN-011 and GABA (**P<0.01 TNF-α vs control, #P<0.05 TNF-α + GABA + YN-011 vs TNF-α + YN-011; n = 6).

Figure 12 demonstrates the reduction of TNF-α-induced apoptosis in SH-SY5Y neuronal cells by YN-011 (**P<0.01 TNF-α vs control, #P<0.05 10 nM YN-011 + TNF-α vs TNF-α, ##P<0.01 100 nM or 500 nM YN-011 + TNF-α vs TNF-α; n = 3).

Figure 13 illustrates the reduction of TNF-α-induced apoptosis in SH-SY5Y neuronal cells by GABA (**P<0.01 TNF-α vs control, #P<0.05 10 μM or 100 μM GABA + TNF-α vs TNF-α; n = 3).

Figure 14 demonstrates the reduction of TNF-α-induced apoptosis in SH-SY5Y neuronal cells by the combined use of YN-011 and GABA (**P<0.01 TNF-α vs control, #P<0.05 GABA or YN-011 + TNF-α vs GABA + YN-011 + TNF-α; n = 3).

Figure 15 is a live cell staining image that demonstrates the damage promoted by TNF-α in SH-SY5Y neuronal cells (**: significant; ***: highly significant).

Figure 16 is a live cell staining image that demonstrates the protective effect of YN-011 against TNF-α-induced damage in neuronal cells (** P < 0.01 TNF-α vs control; # P < 0.05 10 nM YN-011 + TNF-α vs TNF-α; ## P < 0.01 100 nM or 500 nM YN-011 + TNF-α vs TNF-α; n = 3).

Figure 17 is a live cell staining image that demonstrates the protective effect of GABA against TNF-α-induced damage in neuronal cells (** P < 0.01 TNF-α vs control; # P < 0.05 10 μM GABA + TNF-α vs TNF-α; ## P < 0.01 100 μM GABA + TNF-α vs TNF-α; n = 3).

Figure 18 is a live cell staining image that demonstrates the protective effect of the combined use of YN-011 and GABA against TNF-α-induced damage in neuronal cells (** P < 0.01 TNF-α vs control; # P < 0.05 GABA or YN-011 + TNF-α vs GABA + YN-011 + TNF-α; n = 3).

Figure 19 demonstrates the reduction of apoptosis in TNF-α-induced neuronal cells by YN-011 (** P < 0.01 TNF-α vs control; # P < 0.05 100 nM or 500 nM YN-011 + TNF-α vs TNF-α; n = 3).

Figure 20 illustrates the reduction of apoptosis in TNF-α-induced neuronal cells by GABA (** P < 0.01 TNF-α vs control; # P < 0.05 100 μM GABA + TNF-α vs TNF-α; n = 3).

Figure 21 demonstrates the reduction of apoptosis in TNF-α-induced neuronal cells by the combined use of YN-011 and GABA (** P < 0.01 TNF-α vs control; # P < 0.05 100 nM YN-011 + TNF-α vs 100 μM GABA + 100 nM YN-011 + TNF-α; ## P < 0.01 100 μM GABA + TNF-α vs 100 μM GABA + 100 nM YN-011 + TNF-α; n = 3).

Figure 22 illustrates the reduction of apoptosis in TNF-α-induced neuronal cells by YN-011 (** P < 0.01 TNF-α vs control; # P < 0.05 10 nM, 100 nM, or 500 nM YN-011 + TNF-α vs TNF-α; n = 3).

Figure 23 demonstrates the reduction of apoptosis in TNF-α-induced neuronal cells by GABA (** P < 0.01 TNF-α vs control; # P < 0.05 100 μM GABA + TNF-α vs TNF-α; n = 3).

Figure 24 illustrates the reduction of apoptosis in TNF-α-induced neuronal cells by the combined use of YN-011 and GABA (** P < 0.01 TNF-α vs control; # P < 0.05 TNF-α + 100 μM GABA + 100 nM YN-011 vs TNF-α + 100 μM GABA; ## P < 0.05 TNF-α + 100 μM GABA + 100 nM YN-011 vs TNF-α + 100 nM YN-011; n = 3).

Figure 25 demonstrates the reduction of inflammatory cytokine mRNA expression induced by Aβ1-42 oligomers in HMC3 microglial cells by GABA (* P < 0.05, ** P < 0.01 Aβ1-42 oligomers vs control; # P < 0.05 GABA + Aβ1-42 oligomers vs Aβ1-42 oligomers; n = 3).

Figure 26 demonstrates the reduction of inflammatory cytokine mRNA expression induced by Aβ1-42 oligomers in HMC3 microglial cells by YN-011 (* P < 0.05, ** P < 0.01 Aβ1-42 oligomers vs control; # P < 0.05, ## P < 0.01 YN-011 +

Aβ1-42 oligomers vs Aβ1-42 oligomers; n = 3).

Figure 27 demonstrates the reduction of inflammatory cytokine mRNA expression induced by Aβ1-42 oligomers in HMC3 microglial cells by the combined use of YN-011 and GABA (* $P < 0.05$, ** $P < 0.01$ Aβ1-42 oligomers vs control; # $P < 0.05$, GABA + YN-011 + Aβ1-42 oligomers vs GABA + Aβ1-42 oligomers or YN-011 + Aβ1-42 oligomers; n = 3).

Figure 28 illustrates the reduction of inflammatory cytokine expression induced by Aβ1-42 oligomers in HMC3 microglial cells by GABA (** $P < 0.01$ Aβ1-42 oligomers vs control; # $P < 0.05$ GABA + Aβ1-42 oligomers vs Aβ1-42 oligomers; n = 3).

Figure 29 demonstrates the reduction of inflammatory cytokine expression induced by Aβ 1-42 oligomers in HMC3 microglial cells by YN-011 (** $P < 0.01$ Aβ1-42 oligomers vs control; # $P < 0.05$ YN-011 + Aβ1-42 oligomers vs Aβ1-42 oligomers; n = 3).

Figure 30 demonstrates the reduction of inflammatory cytokine expression induced by Aβ 1-42 oligomers in HMC3 microglial cells by the combination of YN-011 and GABA (** $P < 0.01$ Aβ1-42 oligomers vs control; # $P < 0.05$ GABA + YN-011 + Aβ1-42 oligomers vs GABA + Aβ1-42 oligomers or YN-011 + Aβ1-42 oligomers; n = 3).

DETAILED DESCRIPTION OF THE DISCLOSURE:

The following is a detailed description to assist those skilled in the art in practicing the present invention. Unless otherwise defined, all technical and scientific terms used herein have the same meaning as commonly understood by a person skilled in the art to which the invention belongs. The terminology used in the present invention is for the purpose of describing specific embodiments only and is not intended to limit the invention. All publications, patent applications, patents, figures, and other references mentioned herein are incorporated by reference in their entirety into the present invention.

I. Definitions

Unless otherwise indicated, the terms defined in this document and the terminology used should be understood as dictionary definitions, definitions in incorporated documents, and/or the generally known meanings of the defined terms.

All references, patents, and patent applications cited in this document are incorporated by reference in their entirety for the respective subject matter they are cited for, and in certain cases, may encompass the entire content of the referenced document.

All features disclosed in this specification may be combined in any manner. Each feature disclosed in this specification may be replaced by alternative features serving the same, equivalent, or similar purpose. Therefore, unless otherwise indicated, each feature disclosed is merely an example of a series of equivalent or similar features.

The terms "peptide," "polypeptide," and "protein" as used herein refer to amino acid chains comprising two or more naturally or non-naturally occurring amino acid residues, whether or not post-translationally modified (e.g., glycosylated or phosphorylated). The polypeptides disclosed in the present invention may include, for example, 3 to 3500 naturally or non-naturally occurring amino acid residues. The protein referred to can be a single peptide chain or a multi-subunit protein (e.g., composed of two or more polypeptides). The terms "peptide," "polypeptide," and "protein" as described herein can be used interchangeably and may contain naturally occurring amino acids as well as non-naturally occurring amino acids or analogs or mimetics of amino acids. The peptides, polypeptides, or proteins described in this application can be obtained by any methods known in the art, including but not limited to natural isolation, recombinant expression, chemical synthesis, etc.

The term "amino acid" as used herein refers to organic compounds that contain an amino group (-NH2), a carboxyl group (-COOH), and a specific side chain characteristic of each amino acid. The names of amino acids in this application are also represented by standard single-letter or three-letter codes, summarized as follows:

| Name | Three-letter code | Single-letter code |
|---|---|---|
| Alanine | Ala | A |
| Arginine | Arg | R |
| Asparagine | Asn | N |
| Aspartic acid | Asp | D |
| Cysteine | Cys | C |
| Glutamate | Glu | E |
| Glutamine | Gln | Q |

(continued)

| Name | Three-letter code | Single-letter code |
|---|---|---|
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | I |
| Tryptophan | Trp | W |
| Tyrosine | Tyr | Y |
| Valine | Val | V |

As used herein, the term "GLP-1 polypeptide" includes GLP-1 receptor agonist polypeptides having lysine at amino acid residue position 34 or corresponding to amino acid residue position 34, such as the polypeptides shown in SEQ **ID** NO: 1 or SEQ ID NO: 2. Specifically, it includes but is not limited to GLP-1 (7-37), GLP-1 (7-36) amide (also interchangeably referred to herein as GLP-1 (7-36) amide, GLP-1 (7-36 amide), GLP-1 (7-36)), DPP-IV resistant GLP-1, and other GLP-1 analogs having lysine at amino acid residue position 34 or corresponding to lysine at amino acid residue position 34. For example, the GLP-1 polypeptide may include a GLP-1 polypeptide from liraglutide (VICTOZA® from Novo Nordisk), semaglutide (OZEMPIC® from Novo Nordisk), albiglutide (SYNCRIA® from GlaxoSmithKline), tapoglutide (Roche), dulaglutide (TRULICITY® from Eli Lilly) or LY2428757 (Eli Lilly), and may also include GLP-1 polypeptides disclosed in WO2021163972A1, CN111217915A, WO2011056713A2 and WO2000034332A1, each of which is incorporated herein by reference. For example, the above polypeptide may be a GLP-1 analog comprising a "KG" amino acid motif sequence.

The terms "polynucleotide" or "oligonucleotide" as used in this document refer to two or more covalently linked nucleotides. Unless otherwise specified in context, this term generally includes, but is not limited to, deoxyribonucleic acid (DNA) and ribonucleic acid (RNA), which can be single-stranded (ss) or double-stranded (ds). For example, the polynucleotide molecules or oligonucleotides of the present invention can be composed of single-stranded and double-stranded DNA, DNA with a mixture of single-stranded and double-stranded regions, single-stranded and double-stranded RNA, and RNA. The mixture of single-stranded and double-stranded regions may contain hybrid molecules comprising both DNA and RNA, which can be either single-stranded or, more typically, a mixture of single-stranded and double-stranded regions. Furthermore, the polynucleotide molecules can be composed of a three-stranded region containing RNA or DNA or both RNA and DNA. The term "oligonucleotide" as used in this document generally refers to polynucleotides with a length of no more than 200 base pairs and can be single-stranded or double-stranded. The sequences provided in this document can be DNA sequences or RNA sequences. However, it should be understood that the provided sequences include both DNA and RNA, as well as complementary RNA and DNA sequences, unless otherwise specified in context. For example, the sequence 5'-GAATCC-3' should be understood to include 5'-GAAUCC-3', 5'-GGATTC-3', and 5'-GGAUUC-3'.

The term "sequence identity" or "sequence similarity" as used in this document refers to the percentage of sequence similarity between two peptide sequences or two nucleotide sequences. To determine the percentage of sequence identity between two amino acid sequences or two nucleotide sequences, the sequences are aligned for optimal comparison purposes (e.g., introducing gaps in the sequence of the first amino acid or nucleotide sequence to align it optimally with the second amino acid or nucleotide sequence), and then the amino acid residues or nucleotides at corresponding positions are compared. In other words, the percentage (%) sequence identity of an amino acid sequence (or nucleic acid sequence) can be calculated by dividing the number of identical amino acid residues (or bases) that are the same as the reference sequence being compared by the total number of amino acid residues (or bases) in the candidate or reference sequence (taking the shorter one as a reference). When a position in the first sequence is occupied by an amino acid residue or nucleotide that is the same as the corresponding position in the second sequence, the residue is considered identical at that position. The percentage of similarity between two sequences is a function of the number of

shared identical positions in the sequence (i.e., similarity percentage = number of identical overlapping positions / total number of positions × 100%). In one embodiment, the lengths of the two sequences are the same. Mathematical algorithms can also be used to determine the percentage of sequence similarity between two sequences. One preferred, non-limiting example of a mathematical algorithm used for comparing two sequences is the Karlin-Altschul algorithm, which was later modified as the Karlin-Altschul algorithm. This algorithm is incorporated into the NBLAST and XBLAST programs, which can be used with the NBLAST nucleotide program parameter set to perform BLAST nucleotide searches, e.g., score = 100, word length = 12, to obtain nucleotide sequences homologous to a specific polynucleotide molecule. BLAST protein searches can be performed using the XBLAST program parameter set, e.g., score = 50, word length = 3, to obtain amino acid sequences homologous to the protein molecules described in this document. Gapped BLAST can be used to obtain gapped alignments for comparison purposes. Alternatively, PSI-BLAST can be used to perform iterative searches to detect distant relationships between molecules (Id.). When using BLAST, Gapped BLAST, and PSI-Blast programs, the default parameters of each program can be used (e.g., XBLAST and NBLAST) (see, e.g., the NCBI website). Another preferred, non-limiting example of a mathematical algorithm used for sequence comparison is the algorithm proposed by Myers and Miller, which is incorporated into the ALIGN program (version 2.0), a part of the GCG sequence alignment software package. When comparing amino acid sequences using the ALIGN program, the PAM120 weight residue table, a gap length penalty of 12, and a gap penalty of 4 can be used. The percentage of sequence identity between two sequences can be determined using techniques similar to those described above, with or without allowing gaps. When calculating the percentage of identity, typically only exact matches are considered.

In the present invention, "conservative amino acid substitution" refers to the substitution of one amino acid residue with another amino acid residue without eliminating the essential properties of the protein. Appropriate conservative amino acid substitutions can be performed by replacing amino acids with similar hydrophobicity, polarity, and R-chain length. Examples of conservative substitutions include substituting one non-polar (hydrophobic) residue with another non-polar residue (e.g., alanine, isoleucine, valine, leucine, or methionine), substituting one polar (hydrophilic) residue with another polar residue (e.g., between arginine and lysine), between glutamine and asparagine, between glycine and serine, substituting one basic residue with another basic residue (e.g., lysine, arginine, or histidine), or substituting one acidic residue with another acidic residue (e.g., aspartic acid or glutamic acid). The phrase "conservative substitution" also includes the use of chemically derived residues or non-natural amino acids in place of non-derived residues, provided that the peptide exhibits the necessary activity.

In the present invention, the term "fusion protein" refers to a protein that contains two or more peptides that form different functional domains. For example, the GLP-1 fusion protein described in this article includes the GLP-1 peptide and the immunoglobulin Fc domain.

In the present invention, the term "linker" refers to any chemical moiety that is capable of covalently connecting one portion to another portion. For example, the linker can be a sequence of 1, 2, 3, 4, or 5 amino acid residues, or an artificial amino acid sequence with a length ranging from 5 to 15, 20, 30, 50, or more amino acid residues, connected by peptide bonds and used to link one or more peptides. The linker may or may not have a secondary structure. Linker sequences are known in the art, for example, see Holliger et al., Proc. Natl. Acad. Sci. USA 90:6444-6448 (1993); Poljak et al., Structure 2:1121-1123 (1994).

In the present invention, the term "CH2" refers to the constant domain 2 of the immunoglobulin heavy chain. Similarly, the term "CH3" refers to the constant domain 3, which is another structural domain of the immunoglobulin heavy chain.

In the present invention, the term "hinge region" refers to the flexible region between the antigen-binding fragment (Fab) and the crystallizable fragment (Fc) in the context of immunoglobulins, such as IgG.

The term "vector" as used in this document refers to a vehicle capable of operatively inserting genetic elements, allowing the genetic elements to be expressed, producing proteins, RNA, or DNA encoded by the genetic elements, or replicating the genetic elements. Vectors can be used to transform, transduce, or transfect host cells, enabling the carried genetic elements to be expressed within the host cells. Examples of vectors include plasmids, phages, cosmids, artificial chromosomes such as yeast artificial chromosomes (YACs), bacterial artificial chromosomes (BACs), or P1-derived artificial chromosomes (PACs), bacteriophages such as lambda phage or M13 phage, and animal viruses, among others. Vectors may contain various regulatory elements for expression control, including promoter sequences, transcription initiation sequences, enhancer sequences, selection elements, and reporter genes. Additionally, vectors may contain replication origin sites. Vectors may also include components that facilitate their entry into cells, including but not limited to viral particles, liposomes, or protein coats. Vectors can be expression vectors or cloning vectors.

The terms "DPPIV" and "DPP-IV" refer to dipeptidyl peptidase-IV, which is an enzyme that can inactivate native GLP-1.

The term "hydroxylation level" refers to the percentage of residues at amino acid positions in a peptide sample that are modified by hydroxylation. For example, a hydroxylation level of 20% means that 20% (by mole fraction) of peptide molecules are hydroxylated at specific amino acid positions. Modulators can be used to increase or decrease the hydroxylation level of expressed proteins. For instance, when present in the expression system, minoxidil and $Zn^{2+}$ (e.g.,

from ZnSO4) can inhibit hydroxylation and reduce the hydroxylation level. The hydroxylation level can be measured using methods described in this embodiment or by mass spectrometry, as described by Hou et al ( Hou, Y., et al., Nutrient Optimization Reduces Phosphorylation and Hydroxylation Level on an Fc-Fusion Protein in a CHO Fed-Batch Process. Biotechnol J, 2019. 14(3): p. e1700706. ) , or as further described in this document.

The term "oxidation level" refers to the percentage of residues at amino acid positions in a peptide sample that are modified by oxidation. For example, an oxidation level of 2% means that 2% (by mole fraction) of peptide molecules are oxidized at specific amino acid positions. The oxidation level can be measured using methods described in this embodiment or by mass spectrometry methods as described in WO2002046227A2, or according to the protein oxidation assay method by Bettinger et al ( Bettinger, J.Q., et al., Quantitative Analysis of in Vivo Methionine Oxidation of the Human Proteome. J Proteome Res, 2020. 19(2): p. 624-633. ) , or as described further in this document.

In the present invention, the term "pharmaceutical grade" refers to the chemical purity or proportion of a drug, biologic molecule, or reagent that meets the requirements for pharmaceutical production.

In the present invention, the term "treatment" refers to the administration of an effective amount of a compound, composition, or formulation to a subject, which can consist of a single administration or optionally include a series of procedures. As known in the art, "treatment" refers to a method employed to achieve beneficial or desired results, including clinical outcomes. Beneficial or desired clinical outcomes may include, but are not limited to, alleviation or improvement of one or more symptoms or conditions, reduction in the severity of a disease, stabilization (i.e., no worsening) of a disease state, prevention of disease progression, reversal of a disease, improvement or mitigation of a disease, and relief of symptoms associated with a disease (whether partial, complete, or temporary). Beneficial or desired clinical outcomes may include improved fasting blood glucose levels and/or HbA1c levels, weight loss, improved liver lipid content, as well as improved cognitive function, motor coordination, and so on.

In the present invention, the term "subject," also referred to as a "patient," as used in this document, includes all animals, including mammals, and preferably refers to humans. The term "subject" may also include livestock animals such as cattle, pigs, sheep, poultry, and horses; or rodents such as rats and mice; or primates such as apes, monkeys, chimpanzees, gorillas, orangutans, and baboons; or domesticated animals such as dogs and cats.

In the present invention, the term "pharmaceutically acceptable carrier" refers to any carrier, agent, or excipient that is biologically or otherwise acceptable. Unless the carrier, agent, or excipient is incompatible with the active ingredient, its use in a therapeutic formulation is acceptable. The use of such pharmaceutically acceptable carriers is well known in the art, for example, various ingredients that may be included in a pharmaceutical preparation are described in Allen, L.V., Jr. et al. (Allen, L.V., Jr., Remington: The Science and Practice of Pharmacy: from the past into the future. Int J Pharm Compd, 2012. 16(5): p. 358-62.). In the present invention, the term "therapeutically effective dose" refers to any dose that produces the desired effects in a subject, such as relieving symptoms, slowing disease progression, preventing disease onset, etc. This dose can be administered multiple times to achieve the desired effect over a period of time. In the context of this document, the term can refer to a dose that lowers blood glucose levels in the subject.

In the present invention, the term "co-administration" refers to the administration of two or more substances (such as compounds, compositions, etc.) to a subject, where these substances have biological activity. The specific dosing regimen will depend on the pharmacokinetics of the two or more substances in the presence of each other.

In understanding the scope of the present invention, the terms "contain" and its derivatives, as used in this document, are open-ended terms that specify the presence of the stated features, elements, components, moieties, integers, and/or steps, but do not exclude the presence of other unstated features, elements, components, groups, integers, and/or steps. The same applies to similar terms with similar meanings, such as the terms "include," "have," and their derivatives.

The terms "comprised of," "consisting of," and their derivatives, as used in this document, are closed-ended terms that specify the presence of the stated features, elements, components, groups, integers, and/or steps, and further exclude the presence of other unstated features, elements, components, groups, integers, and/or steps.

The numerical ranges enumerated in this document, through endpoint listing, include all numbers and fractions contained within that range (e.g., 1 to 5 includes 1, 1.5, 2, 2.75, 3, 3.90, 4, and 5). It should also be understood that all numbers and fractions are assumed to be modified by the term "about."

In addition, degree terms such as "substantially," "approximately," and "approximately" used in this document indicate a reasonable amount of deviation from the modifying term that would not significantly alter the final result. If such deviation does not negate the meaning of the modifier, these degree terms should be interpreted as including a deviation of at least ± 5% from the modifier. More specifically, the term "about" refers to a deviation of approximately ± 0.1-25%, ± 1-20%, ± 1-15%, ± 1-10%, such as up to 10% or up to 5% from the reference value.

As used in this specification and the appended claims, the singular forms "a," "an," and "one" include references to the plural, unless otherwise specified. Therefore, for example, a composition containing "a compound" includes a mixture of two or more compounds. It should also be noted that, unless otherwise specified, the term "or" is generally used in its inclusive sense, meaning "and/or."

Furthermore, the definitions and embodiments described in specific sections are intended to be applicable to

other embodiments described in this document, as understood by those skilled in the art. For example, in the following paragraphs, different aspects of the invention are defined in more detail. Each aspect defined in this way can be combined with any other aspect or multiple aspects, unless explicitly stated otherwise. Specifically, any feature indicated as preferred or advantageous can be combined with any other feature indicated as preferred or advantageous.

While methods and materials similar or equivalent to those described in this document can be used in the practice or testing of the present invention, specific methods and materials are also described in this embodiment.

II. Pharmaceutical preparation

The pharmaceutical preparation provided by the present invention comprises a GLP-1 fusion protein and a buffer. In one aspect, the present invention provides a pharmaceutical preparation comprising:

(a) a GLP-1 fusion protein, wherein the GLP-1 fusion protein comprises a GLP-1 polypeptide and an immunoglobulin Fc domain, wherein the GLP-1 polypeptide is covalently linked to the immunoglobulin Fc domain, wherein,

the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G;
the immunoglobulin Fc domain comprises or is an IgG2-Fc domain, and the IgG2-Fc domain comprises one or more amino acid substitutions selected from the following group: C222S, A330S and P331S;
and

(b) a buffer;

wherein, the pH value of the pharmaceutical preparation is in the range of about 6.0 to about 7.0.

In the present invention, the buffer can be any suitable buffer. In certain embodiments, the buffer is a pH regulator. In certain embodiments, the buffer is selected from the group consisting of a phosphate buffer, a citrate buffer, a borate buffer, a histidine buffer, and an acetate buffer.

In certain embodiments, the pH value of the pharmaceutical preparation provided by the present invention is about 6.0, about 6.1, about 6.2, about 6.3, about 6.4, about 6.5, about 6.6, about 6.7, about 6.8, about 6.9, about 7.0, or any value in the range between any two of the above values. In certain embodiments, the pharmaceutical preparation provided by the present invention has a pH value in the range of about 6.4 to about 7.0. In certain embodiments, the pharmaceutical preparation provided by the present invention has a pH value in the range of about 6.5 to about 7.0. In certain embodiments, the pharmaceutical preparation provided by the present invention has a pH value in the range of 6.4 to 7.0. In certain embodiments, the pharmaceutical preparation provided by the present invention has a pH value in the range of 6.5 to 7.0. In certain embodiments, the pharmaceutical preparation provided by the present invention has a pH value in the range of 6.4 to 7.0. In certain embodiments, the pharmaceutical preparation provided by the present invention has a pH value in the range of 6.5 to 7.0. In certain embodiments, the pharmaceutical preparation provided by the present invention has a pH value in the range of about 6.4. In certain embodiments, the pharmaceutical preparation provided by the present invention has a pH value in the range of about 6.5. In certain embodiments, the pH value of the pharmaceutical preparation provided by the present invention is about 6.6. In certain embodiments, the pH value of the pharmaceutical preparation provided by the present invention is about 6.7. In certain embodiments, the pH value of the pharmaceutical preparation provided by the present invention is 6.4. In certain embodiments, the pH value of the pharmaceutical preparation provided by the present invention is 6.5. In certain embodiments, the pH value of the pharmaceutical preparation provided by the present invention is 6.6. In certain embodiments, the pH value of the pharmaceutical preparation provided by the present invention is 6.7.

In another aspect, the present invention also provides a pharmaceutical preparation comprising:

(a) a GLP-1 fusion protein, the fusion protein comprising a GLP-1 polypeptide and an immunoglobulin Fc domain, wherein the GLP-1 polypeptide is covalently linked to the immunoglobulin Fc domain, wherein,

the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G;
the immunoglobulin Fc domain comprises or is an IgG2-Fc domain, the IgG2-Fc domain comprises one or more amino acid substitutions selected from the following group: C222S, A330S and P331S;
and

(b) a buffer, the buffer being selected from the following group: phosphate buffer, citrate buffer, borate buffer, histidine buffer and acetate buffer.

In some embodiments, the buffer is a phosphate buffer. Various phosphate buffers are known in the art. In some embodiments, the phosphate buffer comprises phosphate dibasic, dihydrogen phosphate, or a combination thereof. In some embodiments, the phosphate buffer comprises phosphate dibasic or a hydrate thereof. In some embodiments, the phosphate buffer comprises dihydrogen phosphate or a hydrate thereof.

Exemplary phosphate dibasic include, for example, disodium hydrogen phosphate, dipotassium hydrogen phosphate, diammonium hydrogen phosphate, dicalcium hydrogen phosphate, and the like. Exemplary dihydrogen phosphate include, for example, sodium dihydrogen phosphate, potassium dihydrogen phosphate, ammonium dihydrogen phosphate, calcium dihydrogen phosphate, and the like.

In some embodiments, the phosphate buffer is disodium hydrogen phosphate/sodium dihydrogen phosphate buffer. In some embodiments, the phosphate buffer is prepared from disodium hydrogen phosphate and sodium dihydrogen phosphate. In some embodiments, the phosphate buffer is prepared from disodium hydrogen phosphate hydrate and sodium dihydrogen phosphate hydrate.

There are many kinds of disodium hydrogen phosphate hydrates known in the prior art, such as disodium hydrogen phosphate monohydrate, disodium hydrogen phosphate dihydrate, disodium hydrogen phosphate heptahydrate, disodium hydrogen phosphate dodecahydrate, etc. There are also many kinds of sodium dihydrogen phosphate hydrates known in the prior art, such as sodium dihydrogen phosphate monohydrate, sodium dihydrogen phosphate dihydrate, etc. In some embodiments, the phosphate buffer is prepared from disodium hydrogen phosphate dodecahydrate and sodium dihydrogen phosphate monohydrate. In some embodiments, the disodium hydrogen phosphate dodecahydrate and sodium dihydrogen phosphate monohydrate used to prepare the phosphate buffer meet the standards of the Chinese Pharmacopoeia (e.g., 2020 edition, Part IV).

In certain embodiments, the concentration of the phosphate buffer in the pharmaceutical preparation is about 5 mM to about 15 mM (e.g., about 5 mM, about 6 mM, about 7 mM, about 8 mM, about 9 mM, about 10 mM, about 11 mM, about 12 mM, about 13 mM, about 14 mM, about 15 mM, or any value between any two numerical ranges above). In certain embodiments, the concentration of the phosphate buffer in the pharmaceutical preparation is about 10 mM. In certain embodiments, the concentration of the phosphate buffer in the pharmaceutical preparation is 10 mM.

"mM" stands for volume molar concentration, which is a commonly used concentration unit in the art, indicating the molar amount of a substance per unit volume, in units of "mmol/L". For example, "the concentration of the phosphate buffer in the pharmaceutical preparation is 10 mM" means that there is 10 mmol of phosphate in each liter of the pharmaceutical preparation, that is, the concentration of the phosphate in the pharmaceutical preparation is 10 mmol/L.

In certain embodiments, the buffer may be a disodium hydrogen phosphate/sodium dihydrogen phosphate buffer having a concentration of about 5 mM to about 15 mM (e.g., about 5 mM, about 6 mM, about 7 mM, about 8 mM, about 9 mM, about 10 mM, about 11 mM, about 12 mM, about 13 mM, about 14 mM, about 15 mM, or any value between any two of the above ranges). In certain embodiments, the buffer may be a disodium hydrogen phosphate/sodium dihydrogen phosphate buffer having a concentration of about 10 mM. In certain embodiments, the buffer may be a disodium hydrogen phosphate/sodium dihydrogen phosphate buffer having a concentration of 10 mM. In certain embodiments, the buffer may be a disodium hydrogen phosphate/sodium dihydrogen phosphate buffer having a concentration of about 6.65 mM. In certain embodiments, the buffer may be a disodium hydrogen phosphate/sodium dihydrogen phosphate buffer having a concentration of 6.65 mM.

In certain embodiments, the unit dose of the pharmaceutical preparation provided by the present invention is about 0.5 mL, and contains about 0.6 mg of disodium hydrogen phosphate dodecahydrate and about 0.47 mg of sodium dihydrogen phosphate monohydrate. In certain embodiments, the unit dose of the pharmaceutical preparation provided by the present invention is about 0.5 mL/tube, and contains about 0.6 mg of disodium hydrogen phosphate dodecahydrate and about 0.47 mg of sodium dihydrogen phosphate monohydrate. In certain embodiments, the unit dose of the pharmaceutical preparation provided by the present invention is about 0.5 mL, and contains about 0.58 mg of disodium hydrogen phosphate dodecahydrate and about 0.465 mg of sodium dihydrogen phosphate monohydrate. In certain embodiments, the unit dose of the pharmaceutical preparation provided by the present invention is about 0.5 mL/tube, and contains about 0.58 mg of disodium hydrogen phosphate dodecahydrate and about 0.465 mg of sodium dihydrogen phosphate monohydrate.

In certain embodiments, the unit dose of the pharmaceutical preparation provided by the present invention is about 0.75 mL, and contains about 0.9 mg of disodium hydrogen phosphate dodecahydrate and about 0.7 mg of sodium dihydrogen phosphate monohydrate. In certain embodiments, the unit dose of the pharmaceutical preparation provided by the present invention is about 0.75 mL/tube, and contains about 0.9 mg of disodium hydrogen phosphate dodecahydrate and about 0.7 mg of sodium dihydrogen phosphate monohydrate. In certain embodiments, the unit dose of the pharmaceutical preparation provided by the present invention is about 0.75 mL, and contains about 0.87 mg of disodium hydrogen phosphate dodecahydrate and about 0.6975 mg of sodium dihydrogen phosphate monohydrate. In certain

embodiments, the unit dose of the pharmaceutical preparation provided by the present invention is about 0.75 mL/tube, and contains about 0.87 mg of disodium hydrogen phosphate dodecahydrate and about 0.6975 mg of sodium dihydrogen phosphate monohydrate.

In certain embodiments, the pharmaceutical preparation further includes an osmotic pressure regulator (e.g., Carbohydrates). In certain embodiments, the pharmaceutical preparation further includes carbohydrates. In some embodiments, the carbohydrates are selected from the group consisting of mannitol, sorbitol, maltitol, erythritol, arabitol, xylitol, sucrose, lactose, trehalose, dextran, and combinations thereof. In some embodiments, the carbohydrates are selected from one or more of mannitol, sucrose, and sorbitol. In some embodiments, the carbohydrates are mannitol. In some embodiments, the carbohydrates are sucrose. In some embodiments, the carbohydrates are sorbitol.

In some embodiments, the concentration of the carbohydrates in the pharmaceutical preparation is 1-10% (w/v), for example, about 1% (w/v), about 2% (w/v), about 3% (w/v), about 4% (w/v), about 5% (w/v), about 6% (w/v), about 7% (w/v), about 8% (w/v), about 9% (w/v), about 10% (w/v), or any value between any two of the above numerical ranges. In some embodiments, the concentration of the carbohydrate in the pharmaceutical preparation is about 4.6% (w/v) (e.g., 4.64% (w/v)). In some embodiments, the concentration of the carbohydrate in the pharmaceutical preparation is 4.64% (w/v). In some embodiments, the concentration of the carbohydrate in the pharmaceutical preparation is 4.6% (w/v). In some embodiments, the concentration of the carbohydrate in the pharmaceutical preparation is about 3.1% (w/v) (e.g., 3.09% (w/v)). In some embodiments, the concentration of the carbohydrate in the pharmaceutical preparation is 3.1% (w/v).

"w/v" represents mass concentration, which is a commonly used concentration unit in the art, indicating the mass of a substance per unit volume, and the unit is "kg/L", "kg/m3" or "g/mL". For example, "the concentration of the carbohydrate in the pharmaceutical preparation is 4.6% (w/v)" means that there is 0.046g of carbohydrate in each milliliter of the pharmaceutical preparation, that is, the concentration of the carbohydrate in the pharmaceutical preparation is 0.046 g/mL.

In certain embodiments, the pharmaceutical preparations provided herein comprise mannitol at a concentration of 1-10% (w/v) (e.g., about 1% (w/v), about 2% (w/v), about 3% (w/v), about 4% (w/v), about 5% (w/v), about 6% (w/v), about 7% (w/v), about 8% (w/v), about 9% (w/v), about 10% (w/v) or any numerical value between any two numerical ranges above). In certain embodiments, the pharmaceutical preparations provided herein comprise mannitol at a concentration of about 4.6% (w/v) (e.g., 4.64% (w/v)). In certain embodiments, the pharmaceutical preparations provided herein comprise mannitol at a concentration of 4.64% (w/v). In certain embodiments, the pharmaceutical preparations provided herein comprise mannitol at a concentration of 4.6% (w/v). In certain embodiments, the pharmaceutical preparations provided herein comprise mannitol at a concentration of about 3.1% (w/v) (e.g., 3.09% (w/v)). In certain embodiments, the pharmaceutical preparations provided herein comprise mannitol at a concentration of 3.1 % (w/v).

In certain embodiments, the unit dose of the pharmaceutical preparations provided herein is about 0.5 mL and comprises about 23 mg of mannitol. In certain embodiments, the unit dose of the pharmaceutical preparations provided herein is about 0.5 mL/tube and comprises about 23 mg of mannitol. In certain embodiments, the unit dose of the pharmaceutical preparations provided herein is about 0.5 mL and comprises about 23.2 mg of mannitol. In certain embodiments, the unit dose of the pharmaceutical preparations provided herein is about 0.5 mL/tube and comprises about 23.2 mg of mannitol.

In certain embodiments, the unit dose of the pharmaceutical preparations provided herein is about 0.75 mL and comprises about 35 mg of mannitol. In certain embodiments, the unit dose of the pharmaceutical preparations provided herein is about 0.75 mL/tube and comprises about 35 mg of mannitol. In certain embodiments, the unit dose of the pharmaceutical preparation provided by the present invention is about 0.75 mL and contains about 34.8 mg of mannitol. In certain embodiments, the unit dose of the pharmaceutical preparation provided by the present invention is about 0.75 mL/tube and contains about 34.8 mg of mannitol.

In certain embodiments, the pharmaceutical preparation further comprises a protein stabilizer (e.g., a surfactant). In certain embodiments, the pharmaceutical preparation further comprises a surfactant, such as an ionic surfactant, a non-ionic surfactant. In certain embodiments, the surfactant is selected from the group consisting of polysorbate (e.g., polysorbate 80), polyoxyethylene castor oil derivatives, poloxamers (e.g., poloxamer 188), lecithin, polyethylene glycol 15-hydroxystearate, cyclodextrins, and combinations thereof. In certain embodiments, the surfactant is polysorbate 80. In this application, "polysorbate 80" is also referred to as "Tween 80" or "Tween-80".

In certain embodiments, the concentration of the surfactant in the pharmaceutical preparation is 0.01% (w/v) - 0.04% (w/v), for example, about 0.01% (w/v), about 0.02% (w/v), about 0.03% (w/v), about 0.04% (w/v), or any value between any two of the above numerical ranges. In certain embodiments, the concentration of the surfactant in the pharmaceutical preparation is about 0.02% (w/v). In certain embodiments, the concentration of the surfactant in the pharmaceutical preparation is 0.02% (w/v). In certain embodiments, the concentration of the surfactant in the pharmaceutical preparation is about 0.01% (w/v) (e.g., 0.013% (w/v)). In certain embodiments, the concentration of the surfactant in the pharmaceutical preparation is 0.01% (w/v).

In certain embodiments, the pharmaceutical preparations provided herein contain polysorbate 80 at a concentration of 0.01% (w/v) - 0.04% (w/v) (e.g., about 0.01% (w/v), about 0.02% (w/v), about 0.03% (w/v), about 0.04% (w/v), or any value between any two numerical ranges above). In certain embodiments, the pharmaceutical preparations provided herein contain polysorbate 80 at a concentration of about 0.02% (w/v). In certain embodiments, the pharmaceutical preparations provided herein contain polysorbate 80 at a concentration of 0.02% (w/v). In certain embodiments, the pharmaceutical preparations provided herein contain polysorbate 80 at a concentration of about 0.01% (w/v) (e.g., 0.013% (w/v)). In certain embodiments, the pharmaceutical preparations provided herein contain polysorbate 80 at a concentration of 0.01% (w/v).

In certain embodiments, the unit dose of the pharmaceutical preparation provided by the present invention is about 0.5 mL and contains about 0.1 mg of polysorbate 80. In certain embodiments, the unit dose of the pharmaceutical preparation provided by the present invention is about 0.5 mL/tube and contains about 0.1 mg of polysorbate 80.

In certain embodiments, the unit dose of the pharmaceutical preparation provided by the present invention is about 0.75 mL and contains about 0.15 mg of polysorbate 80. In certain embodiments, the unit dose of the pharmaceutical preparation provided by the present invention is about 0.75 mL/tube and contains about 0.15 mg of polysorbate 80.

In certain embodiments, the pharmaceutical preparation provided by the present invention comprises a GLP-1 fusion protein, a phosphate buffer, a carbohydrate, and a surfactant. In certain embodiments, the pharmaceutical preparation provided by the present invention comprises a GLP-1 fusion protein, a phosphate buffer (e.g., disodium hydrogen phosphate/sodium dihydrogen phosphate buffer), mannitol, and a polysorbate (e.g., polysorbate 80). In certain embodiments, the pharmaceutical preparation provided by the present invention comprises GLP-1 fusion protein, disodium hydrogen phosphate/sodium dihydrogen phosphate buffer, mannitol and polysorbate 80.

In certain embodiments, the concentration of the GLP-1 fusion protein in the pharmaceutical preparation is 0.2-25 mg/mL, for example, about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 10.5 mg/mL, about 11 mg/mL, about 11.5 mg/mL, about 12 mg/mL, about 12.5 mg/mL, about 13 mg/mL, about 13.5 mg/mL, about 14 mg/mL, about 14.5 mg/mL, about 15 mg/mL, about 15.5 mg/mL, about 16 mg/mL, about 16.5 mg/mL, about 17 mg/mL, about 17.5 mg/mL, about 18 mg/mL, about 18.5 mg/mL, about 19 mg/mL, about 19.5 mg/mL, about 20 mg/mL, about 20.5 mg/mL, about 21 mg/mL, about 21.5 mg/mL, about 22 mg/mL, about 22.5 mg/mL, about 23 mg/mL, about 23.5 mg/mL, about 24 mg/mL, about 24.5 mg/mL, about 25 mg/mL or any value between any two of the above numerical ranges. In certain embodiments, the concentration of the GLP-1 fusion protein in the pharmaceutical preparation is 0.2-20 mg/mL. In certain embodiments, the concentration of the GLP-1 fusion protein in the pharmaceutical preparation is 1-5 mg/mL.

In certain embodiments, the concentration of the GLP-1 fusion protein in the pharmaceutical preparation is about 2 mg/mL. In certain embodiments, the concentration of the GLP-1 fusion protein in the pharmaceutical preparation is 2 mg/mL. In certain embodiments, the concentration of the GLP-1 fusion protein in the pharmaceutical preparation is about 3 mg/mL. In certain embodiments, the concentration of the GLP-1 fusion protein in the pharmaceutical preparation is 3 mg/mL. In certain embodiments, the concentration of the GLP-1 fusion protein in the pharmaceutical preparation is about 4 mg/mL. In certain embodiments, the concentration of the GLP-1 fusion protein in the pharmaceutical preparation is 4 mg/mL. In certain embodiments, the concentration of the GLP-1 fusion protein in the pharmaceutical preparation is about 6 mg/mL. In certain embodiments, the concentration of the GLP-1 fusion protein in the pharmaceutical preparation is 6 mg/mL.

In certain embodiments, the concentration of the GLP-1 fusion protein in the pharmaceutical preparation is about 5.3 mg/mL. In certain embodiments, the concentration of the GLP-1 fusion protein in the pharmaceutical preparation is 5.3 mg/mL. In certain embodiments, the concentration of the GLP-1 fusion protein in the pharmaceutical preparation is about 6.67 mg/mL. In certain embodiments, the concentration of the GLP-1 fusion protein in the pharmaceutical preparation is 6.67 mg/mL. In certain embodiments, the concentration of the GLP-1 fusion protein in the pharmaceutical preparation is about 10 mg/mL. In certain embodiments, the concentration of the GLP-1 fusion protein in the pharmaceutical preparation is 10 mg/mL. In certain embodiments, the concentration of the GLP-1 fusion protein in the pharmaceutical preparation is about 12 mg/mL. In certain embodiments, the concentration of the GLP-1 fusion protein in the pharmaceutical preparation is 12 mg/mL. In certain embodiments, the concentration of the GLP-1 fusion protein in the pharmaceutical preparation is about 13.3 mg/mL. In certain embodiments, the concentration of the GLP-1 fusion protein in the pharmaceutical preparation is 13.3 mg/mL. In certain embodiments, the concentration of the GLP-1 fusion protein in the pharmaceutical preparation is about 20 mg/mL. In certain embodiments, the concentration of the GLP-1 fusion protein in the pharmaceutical preparation is 20 mg/mL.

In certain embodiments, the pharmaceutical preparations provided herein comprise 0.2-20 mg/mL of a GLP-1 fusion protein, wherein the GLP-1 fusion protein has an amino acid sequence as shown in SEQ ID NO: 7. In certain embodiments, the pharmaceutical preparations provided herein comprise about 2 mg/mL, about 4 mg/mL, or about 6 mg/mL of a GLP-1 fusion protein, wherein the amino acid sequence of the GLP-1 fusion protein is as shown in SEQ ID NO: 7. In certain embodiments, the pharmaceutical preparations provided herein comprise 2 mg/mL, 4 mg/mL, or 6 mg/mL of a

GLP-1 fusion protein, wherein the amino acid sequence of the GLP-1 fusion protein is as shown in SEQ ID NO: 7.

In certain embodiments, the pharmaceutical preparation provided by the present invention comprises about 5.3 mg/mL, about 6.67 mg/mL, about 10 mg/mL, about 12 mg/mL, about 13.3 mg/mL or about 20 mg/mL of GLP-1 fusion protein, wherein the amino acid sequence of the GLP-1 fusion protein is as shown in SEQ ID NO: 7. In certain embodiments, the pharmaceutical preparation provided by the present invention comprises 5.3 mg/mL, 6.67 mg/mL, 10 mg/mL, 12 mg/mL, 13.3 mg/mL or 20 mg/mL of GLP-1 fusion protein, wherein the amino acid sequence of the GLP-1 fusion protein is as shown in SEQ ID NO: 7.

In certain embodiments, the unit dose of the pharmaceutical preparation provided by the present invention is about 0.5 mL, and comprises about 1 mg, about 2 mg or about 3 mg of GLP-1 fusion protein, wherein the amino acid sequence of the GLP-1 fusion protein is as shown in SEQ ID NO: 7. In certain embodiments, the unit dose of the pharmaceutical preparation provided by the present invention is about 0.5 mL/vial, and contains about 1 mg, about 2 mg or about 3 mg of GLP-1 fusion protein, wherein the amino acid sequence of the GLP-1 fusion protein is as shown in SEQ ID NO: 7.

In certain embodiments, the unit dose of the pharmaceutical preparation provided by the present invention is about 0.75 mL, and contains about 4 mg, about 5 mg, about 7.5 mg, about 9 mg, about 10 mg or about 15 mg of GLP-1 fusion protein, wherein the amino acid sequence of the GLP-1 fusion protein is as shown in SEQ ID NO: 7. In certain embodiments, the unit dose of the pharmaceutical preparation provided by the present invention is about 0.75 mL/vial, and contains about 4 mg, about 5 mg, about 7.5 mg, about 9 mg, about 10 mg or about 15 mg of GLP-1 fusion protein, wherein the amino acid sequence of the GLP-1 fusion protein is as shown in SEQ ID NO: 7.

In certain embodiments, the pharmaceutical preparation provided by the present invention comprises:

(a) GLP-1 fusion protein, wherein the GLP-1 fusion protein has an amino acid sequence as shown in SEQ ID NO: 7;
(b) disodium hydrogen phosphate/sodium dihydrogen phosphate buffer;
(c) mannitol; and
(d) polysorbate 80.

In certain embodiments, the pharmaceutical preparation provided by the present invention comprises:

(a) 0.2-20 mg/mL GLP-1 fusion protein, wherein the GLP-1 fusion protein has an amino acid sequence as shown in SEQ ID NO: 7;
(b) 5-15 mM disodium hydrogen phosphate/sodium dihydrogen phosphate buffer;
(c) 1-10% (w/v) mannitol; and
(d) 0.01% (w/v) - 0.04% (w/v) polysorbate 80.

In certain embodiments, the pharmaceutical preparation provided by the present invention comprises:

(a) about 2 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 4 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 6 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 5.3 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 6.67 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 10 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 12 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 13.3 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

In certain embodiments, the pharmaceutical preparation provided by the present invention comprises:

(a) about 20 mg/mL GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

The pharmaceutical preparation provided by the present invention can be prepared, packaged and/or sold in batches as a unit dose and/or multiple unit doses. In certain embodiments, the unit dose of the pharmaceutical preparation provided by the present invention is about 0.5 mL and comprises:

(a) about 1 mg of GLP-1 fusion protein, wherein the amino acid sequence of the GLP-1 fusion protein is as shown in SEQ ID NO: 7;
(b) about 0.58 mg of disodium hydrogen phosphate dodecahydrate;
(c) about 0.465 mg of sodium dihydrogen phosphate monohydrate;
(d) about 23.2 mg of mannitol; and
(e) about 0.1 mg of polysorbate 80, and the rest is water for injection.

In certain embodiments, the unit dose of the pharmaceutical preparation provided by the present invention is about 0.5 mL and comprises:

(a) about 2 mg of GLP-1 fusion protein, wherein the amino acid sequence of the GLP-1 fusion protein is as shown in SEQ ID NO: 7;
(b) about 0.58 mg of disodium hydrogen phosphate dodecahydrate;
(c) about 0.465 mg of sodium dihydrogen phosphate monohydrate;
(d) about 23.2 mg of mannitol; and
(e) about 0.1 mg of polysorbate 80, and the rest is water for injection.

In certain embodiments, the unit dose of the pharmaceutical preparation provided by the present invention is about 0.5 mL and comprises:

(a) about 3 mg of GLP-1 fusion protein, wherein the amino acid sequence of the GLP-1 fusion protein is as shown in SEQ ID NO: 7;
(b) about 0.58 mg of disodium hydrogen phosphate dodecahydrate;
(c) about 0.465 mg of sodium dihydrogen phosphate monohydrate;
(d) about 23.2 mg of mannitol; and
(e) about 0.1 mg of polysorbate 80, and the rest is water for injection.

In certain embodiments, the unit dose of the pharmaceutical preparation provided by the present invention is about 0.75 mL and comprises:

(a) about 4 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80, and the rest is water for injection.

In certain embodiments, the unit dose of the pharmaceutical preparation provided by the present invention is about 0.75 mL and comprises:

(a) about 5 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80, and the rest is water for injection.

In certain embodiments, the unit dose of the pharmaceutical preparation provided by the present invention is about 0.75 mL and comprises:

(a) about 7.5 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80, and the rest is water for injection.

In certain embodiments, the unit dose of the pharmaceutical preparation provided by the present invention is about 0.75 mL and comprises:

(a) about 9 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and

(e) about 0.15 mg of polysorbate 80, and the rest is water for injection.

In certain embodiments, the unit dose of the pharmaceutical preparation provided by the present invention is about 0.75 mL and comprises:

(a) about 10 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80, and the rest is water for injection.

In certain embodiments, the unit dose of the pharmaceutical preparation provided by the present invention is about 0.75 mL and comprises:

(a) about 15 mg of GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80, and the rest is water for injection.

The pharmaceutical preparation provided by the present invention has better stability. In certain embodiments, the pharmaceutical preparations provided herein are stable for at least 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months, etc., at 2°C-8°C. In certain embodiments, the pharmaceutical preparations provided herein are stable for at least 24 months at 2°C-8°C. In certain embodiments, the pharmaceutical preparations provided herein are stable for at least 36 months at 2°C-8°C.

In certain embodiments, the pharmaceutical preparation provided by the present invention remains stable for at least 1 month, 2 months, 3 months, 4 months, 5 months, 6 months, 7 months, 8 months, 9 months, 10 months, 11 months or 12 months at 25±2°C. In certain embodiments, the pharmaceutical preparation provided by the present invention remains stable for at least 6 months at 25±2°C. In certain embodiments, the pharmaceutical preparation provided by the present invention remains stable for at least 3 months at 25±2°C.

In certain embodiments, the pharmaceutical preparation provided by the present invention remains stable for at least 1 week, 2 weeks, 3 weeks, 4 weeks or 1 month at 40±2°C. In certain embodiments, the pharmaceutical preparation provided by the present invention remains stable for at least 2 weeks at 40±2°C.

The stability of the pharmaceutical preparation provided by the present invention can be evaluated by a variety of indicators, such as changes in pH, changes in GLP-1 fusion protein concentration, content of high molecular weight (HMW) derivatives and/or low molecular weight (LMW) derivatives, changes in purity, changes in isoelectric point, etc. In certain embodiments, the pH value of the pharmaceutical preparation provided herein does not change by more than about 10% (e.g., no more than about 9%, no more than about 8%, no more than about 7%, no more than about 6%, no more than about 5%, no more than about 4%, no more than about 3%, no more than about 2%, no more than about 1%, etc.) after being placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months). For example, after the pharmaceutical preparation provided by the present invention is placed for at least 1 month (for example, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), the pH value remains in the range of 6.4-6.7. In certain embodiments, after the pharmaceutical preparation provided by the present invention is placed for at least 1 month, the pH value changes by no more than about 3%. In certain embodiments, after the pharmaceutical preparation provided by the present invention is placed for at least 1 month, the pH value changes by no more than 3%.

In certain embodiments, the concentration of the GLP-1 fusion protein does not change by more than about 10% (e.g., no more than about 9%, no more than about 8%, no more than about 7%, no more than about 6%, no more than about

5%, no more than about 4%, no more than about 3%, no more than about 2%, no more than about 1%, etc.) after the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months). For example, after the pharmaceutical preparation provided by the present invention is placed for at least 1 month, the concentration of the GLP-1 fusion protein changes within the range of ±0.2 mg/mL. In certain embodiments, after the pharmaceutical preparation provided by the present invention is placed for at least 1 month, the concentration of the GLP-1 fusion protein does not change by more than about 3%. In certain embodiments, after the pharmaceutical preparation provided by the present invention is placed for at least 1 month, the concentration of the GLP-1 fusion protein does not change by more than 3%. A person skilled in the art can determine the concentration of the GLP-1 fusion protein in the pharmaceutical preparation by methods commonly used in the art, such as the method described in Example 12.1.3 or Example 13.2.9.

In certain embodiments, the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), and the content of high molecular weight (HMW) derivatives or low molecular weight (LMW) derivatives does not exceed about 10% (e.g., not more than about 9%, not more than about 8%, not more than about 7%, not more than about 6%, not more than about 5%, not more than about 4%, not more than about 3%, not more than about 2%, not more than about 1%, etc.).

In certain embodiments, the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), and the content of high molecular weight (HMW) derivatives and low molecular weight (LMW) derivatives does not exceed about 10% (e.g., not more than about 9%, not more than about 8%, not more than about 7%, not more than about 6%, not more than about 5%, not more than about 4%, not more than about 3%, not more than about 2%, not more than about 1%, etc.).

In certain embodiments, the content of the HMW derivative is no more than about 5% after the pharmaceutical preparation provided herein is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months). In certain embodiments, the content of the LMW derivative is no more than about 5% after the pharmaceutical preparation provided herein is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months). In certain embodiments, the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), the content of the HMW derivative does not exceed about 5%, and the content of the LMW derivative does not exceed about 5%.

In certain embodiments, the content of the HMW derivative does not exceed about 4% after the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months). In certain embodiments, the content of the HMW derivative does not exceed 4% after the pharmaceutical preparation provided by the present invention is placed for at least 1 month. In certain embodiments, the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), and the content of the LMW derivative does not exceed about 1%. In certain embodiments, the pharmaceutical preparation provided by the present invention is placed for at least 1 month, and the content of the LMW derivative does not

exceed 1%.

Those skilled in the art can determine the content of HMW derivatives and/or LMW derivatives in the pharmaceutical preparation by methods commonly used in the art. In certain embodiments, the content of the HMW derivatives and/or LMW derivatives is determined by size exclusion chromatography (SEC) or reverse phase liquid chromatography (RP-LC). Those skilled in the art can adjust the assay conditions based on experience or needs. In certain embodiments, the content of the HMW derivative and/or LMW derivative is determined by the method described in Examples 12.1.5, 12.1.6, 13.2.6 or 13.2.7 of the present application.

In certain embodiments, the pharmaceutical preparation provided by the present invention is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), and the purity changes by no more than about 5% (e.g., no more than about 4%, no more than about 3%, no more than about 2%, no more than about 1%, etc.). In certain embodiments, the purity of the pharmaceutical preparation provided by the present invention does not change by more than about 2% after being placed for at least 1 month.

The "purity" of the pharmaceutical preparation provided in the present application refers to the relative content of the active ingredient in the preparation, that is, the GLP-1 fusion protein (e.g., YN-011). For example, when the purity is determined by SEC-HPLC, the SEC monomer peak percentage calculated by the peak area normalization method is the protein purity. For example, when the purity is determined by SEC-HPLC, "the purity changes by no more than about 2%" means that after a specific period of time, the change in the SEC monomer peak percentage does not exceed about 2%.

Those skilled in the art can determine the purity of the pharmaceutical preparation by methods commonly used in the art, for example, by SDS-capillary electrophoresis (e.g., non-reducing SDS-capillary electrophoresis). In certain embodiments, the purity of the pharmaceutical preparation is determined by the method described in Examples 12.1.5, 12.1.7, 13.2.7 or 13.2.8 of the present application.

In certain embodiments, the pharmaceutical preparation provided herein is placed for at least 1 month (e.g., 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months or 36 months), and the isoelectric point changes by no more than about 5% (e.g., no more than about 4%, no more than about 3%, no more than about 2%, no more than about 1%, etc.). In certain embodiments, the pharmaceutical preparation provided herein is placed for at least 1 month, and the isoelectric point changes by no more than about 2%.

In certain embodiments, the isoelectric point of the pharmaceutical preparation provided by the present invention is determined by capillary focusing isoelectric electrophoresis (cIEF). In certain embodiments, the isoelectric point of the pharmaceutical preparation provided by the present invention is determined by the method described in Example 12.1.8 or 13.2.5 of the present application.

In certain embodiments, the pharmaceutical preparation provided by the present invention may also contain a suitable diluent or carrier.

In another preferred embodiment, the diluent is sterile. Diluents suitable for GLP-1 fusion protein and/or cells include, but are not limited to, saline solution, pH buffer solution, and diluents described herein, as well as glycerol solutions or other solutions suitable for freezing polypeptides and/or cells. Diluents suitable for nucleic acids and/or vectors include, but are not limited to, saline solution, pH buffer solution, and diluents described herein, as well as water, etc.

In another preferred embodiment, the carrier is a pharmaceutically acceptable carrier. Examples of pharmaceutically acceptable carriers for the pharmaceutical preparations provided by the present invention may include, for example, pharmaceutically acceptable liquids, gels, solid carriers, aqueous vehicles, non-aqueous vehicles, antimicrobial agents, isotonic agents, buffers, antioxidants, anesthetics, suspending/dispersing agents, sequestering/chelating agents, diluents, adjuvants, excipients or non-toxic auxiliary substances, other components known in the art, or various combinations thereof. Pharmaceutically acceptable carriers for the pharmaceutical preparations provided herein include, for example, aqueous vehicles such as sodium chloride injection, Ringer's injection, isotonic dextrose injection, sterile water injection, or dextrose and lactated Ringer's injection; non-aqueous vehicles such as non-volatile oils of plant origin, cottonseed oil, corn oil, sesame oil, or peanut oil; antimicrobial agents at bacteriostatic or fungistatic concentrations; isotonic agents such as sodium chloride or dextrose; buffers such as phosphate or citrate buffers; antioxidants such as sodium bisulfate; local anesthetics such as procaine hydrochloride; suspending and dispersing agents such as sodium carboxymethylcellulose, hydroxypropyl methylcellulose, or polyvinylpyrrolidone; emulsifiers such as polysorbate 80 (TWEEN-80); chelating agents such as ethylenediaminetetraacetic acid (EDTA) or ethylene glycol tetraacetic acid (EGTA), ethanol, polyethylene glycol, propylene glycol, sodium hydroxide, hydrochloric acid, citric acid, or lactic acid. Antimicrobial agents used as carriers can be added to the composition in the multi-dose container, and the antimicrobial agents include phenol or cresol, mercurials, benzyl alcohol, chlorobutanol, methyl and propyl parabens, thimerosal,

benzalkonium chloride and benzethonium chloride. Suitable excipients may include, for example, water, saline, dextrose, glycerol or ethanol. Suitable non-toxic auxiliary substances may include, for example, wetting agents or emulsifiers, pH buffers, stabilizers, solubility enhancers or agents such as sodium acetate, sorbitan monolaurate, triethanolamine oleate or cyclodextrin.

The pharmaceutical preparations provided by the present invention can be prepared into a variety of dosage forms. Suitable dosage forms may include, but are not limited to, solutions, suspensions, pills, tablets, lyophilized agents, etc. In certain embodiments, the pharmaceutical preparations provided by the present invention are formulated into injectable compositions. Injectable compositions can be prepared in any conventional form, such as liquid solutions, suspensions, emulsions, or solid forms suitable for producing liquid solutions, suspensions or emulsions. Injectable preparations may include sterile and/or pyrogen-free solutions ready for injection; sterile dry soluble products ready to be combined with solvents immediately prior to use, such as lyophilized powders, including subcutaneous tablets; sterile suspensions ready for injection; sterile dry insoluble products ready to be combined with a vehicle immediately prior to use; and sterile and/or pyrogen-free emulsions. Solutions may be aqueous or non-aqueous.

In certain embodiments, the pharmaceutical preparations provided herein are liquid preparations. In certain embodiments, the pharmaceutical preparations provided herein are aqueous solutions. The aqueous solution may contain, for example, at least 70% w/w, at least 75% w/w, at least 80% w/w, at least 85% w/w, at least 90% w/w, or at least 95% w/w of water. In certain embodiments, the pharmaceutical preparations provided herein are injectable injections. In certain embodiments, the pharmaceutical preparations provided herein are administered by intravenous, subcutaneous, or intramuscular injection. In certain embodiments, the pharmaceutical preparations provided herein are isotonic, i.e., liquid preparations having an osmotic pressure equal to that of plasma.

In certain embodiments, the sterile lyophilized powder is prepared by dissolving the GLP-1 fusion protein as disclosed herein in a suitable solvent. The solvent may contain excipients that improve the stability of the powder or the reconstituted solution prepared from the powder or other pharmacological components. The excipients that can be used include, but are not limited to, water, dextrose, sorbitol, fructose, corn syrup, xylitol, glycerol, glucose, sucrose or other suitable agents. The solvent may contain a buffer such as citrate, sodium phosphate or potassium phosphate, or other such buffers known to those skilled in the art, and in certain embodiments, the buffer is approximately at a neutral pH. The solution is then sterile filtered and then freeze-dried under standard conditions known to those skilled in the art to obtain the desired formulation. In certain embodiments, the resulting solution is dispensed into vials for freeze-drying. Each vial may contain a single dose or multiple doses of a fusion polypeptide, polypeptide complex or conjugate provided herein, or a composition thereof. It is acceptable to overfill the vial beyond the smaller amount (e.g., about 10%) required for a single dose or a group of doses in order to accurately draw samples and accurately administer the drug. The lyophilized powder can be stored under appropriate conditions, such as at about 4°C to room temperature. Reconstitution of the lyophilized powder with water for injection provides a formulation for parenteral administration. In certain embodiments, sterile and/or pyrogen-free water or other suitable liquid carrier is added to the lyophilized powder for reconstitution. The exact amount depends on the given selected therapy and can be determined empirically.

In certain embodiments, unit doses of parenteral formulations are packaged in ampoules, vials, or syringes with needles. All formulations for parenteral administration should be sterile and pyrogen-free, as known and practiced in the art.

Administration of the compositions described herein can be by any route known to be effective by a physician of ordinary skill. One embodiment is peripheral parenteral administration by a sterile syringe or some other mechanical device, such as an infusion pump. In certain embodiments, the peripheral parenteral route is an intravenous, intramuscular, subcutaneous, or intraperitoneal route of administration.

In certain embodiments, the GLP-1 fusion proteins described herein are formulated in a solid formulation, such as lyophilized or spray dried, which is then reconstituted in a suitable diluent solution prior to administration. Standard pharmaceutical preparation techniques may be employed, such as those described in Remington: The Science and Practice of Pharmacy (D.B. Troy, ed., 21st ed., Lippincott, Williams & Wilkins, 2006). The following describes in detail the active ingredient in the pharmaceutical preparation provided by the present invention, i.e., the GLP-1 fusion protein.

1. GLP-1 fusion protein

The pharmaceutical preparation provided by the present invention comprises a fusion protein formed by a stable GLP-1 and an IgG/Fc domain.

In certain embodiments, the GLP-1 fusion protein in the pharmaceutical preparation provided by the present invention comprises a GLP-1 polypeptide and an immunoglobulin Fc domain, wherein the GLP-1 polypeptide is covalently linked to the immunoglobulin Fc domain, wherein the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G; the immunoglobulin Fc domain comprises or is an IgG2-Fc domain, and the IgG2-Fc domain comprises one or more amino acid

substitutions selected from the following group: C222S, A330S and P331S.

GLP-1 polypeptide

Surprisingly, the yield and activity of the GLP-1 fusion protein in the pharmaceutical preparation provided by the present invention can be improved by increasing the hydroxylation level of the GLP-1 polypeptide at lysine 34 (K34) relative to native human GLP-1, or reducing the oxidation at tryptophan 31 (W31) relative to native human GLP-1 and/or introducing one or more site mutations of the GLP-1 polypeptide (e.g., one or more mutations in A8G, G22E, R36G) or site mutations of the IgG2-Fc domain (e.g., one or more mutations in C222S, A330S, P331S).

The unprocessed form of the native human GLP-1 polypeptide has 37 amino acids, and its amino acid sequence is shown in SEQ ID NO: 43, and is also commonly referred to as "GLP-1 (1-37)". The unprocessed form of the native human GLP-1 polypeptide is processed in the pancreas and small intestine to form GLP-1 (7-37) or GLP-1 (7-36) amide. Unless otherwise specified, the amino acid sites of the GLP-1 polypeptide mentioned in this application are the amino acid sites corresponding to SEQ ID NO: 43. For example, K34 of the GLP-1 polypeptide mentioned in this application corresponds to the 34th site of SEQ ID NO: 43. For another example, GLP-1 (7-36) amide refers to a GLP-1 polypeptide fragment formed by amino acids between positions 7 and 36 of SEQ ID NO: 43; GLP-1 (7-37) refers to a GLP-1 polypeptide fragment formed by amino acids between positions 7 and 37 of SEQ ID NO: 43. In some embodiments, the amino acid sequence of GLP-1 (7-36) amide is shown in SEQ ID NO: 1. In some embodiments, the amino acid sequence of GLP-1 (7-37) is shown in SEQ ID NO: 2.

Unless otherwise specified, the naming rules for amino acid mutations mentioned in this application are: amino acid name before mutation - amino acid site where mutation occurs - amino acid name after mutation. For example, the A8G mutation in the GLP-1 polypeptide refers to the mutation of alanine (A) at position 8 of SEQ ID NO: 43 to glycine (G).

The hydroxylation level of the GLP-1 fusion protein described in the present application can be determined by a variety of methods known in the prior art. For example, the mass spectrometry method described by Hou et al. (Hou, Y., et al., Nutrient Optimization Reduces Phosphorylation and Hydroxylation Level on an Fc-Fusion Protein in a CHO Fed-Batch Process. Biotechnol J, 2019. 14(3): p. e1700706.) can be used for measurement. Unless otherwise specified, the hydroxylation level described in the present application is calculated as a percentage of the number of molecules. For example, among 100g of the GLP-1 fusion protein of the present invention, if 10g of the GLP-1 fusion protein is hydroxylated at K34 of the GLP-1 polypeptide, and the remaining 90g of the GLP-1 fusion protein is not hydroxylated at K34 of the GLP-1 polypeptide, the hydroxylation level of the GLP-1 fusion protein is considered to be 10%.

The GLP-1 fusion protein of the present invention, in some embodiments, increases the half-life and/or yield in vivo during recombinant production. Therefore, the GLP-1 fusion protein and the reagents for preparing the GLP-1 fusion protein can be used to prepare drugs.

In some embodiments, the GLP-1 polypeptide has a certain hydroxylation level at lysine 34 (K34) relative to natural human GLP-1. K34 refers to the lysine residue at position 34 of the natural human GLP-1 polypeptide. Those skilled in the art know that the same lysine can correspond to different positions in other GLP-1 polypeptide sequences.

GLP-1 fusion protein may refer to multiple molecules, which may be hydroxylated at K34, or may not be hydroxylated, and the hydroxylation levels of the molecules may be different. The present invention also provides a composition comprising a GLP-1 fusion protein. In certain embodiments, the hydroxylation level of the GLP-1 fusion protein at K34 is about 10% to 100%, such as at least about 20%, at least about 26%, at least about 30%, at least about 40%, or at least about 50%. Any percentage or range between 10% and 100% is contemplated.

In certain embodiments, the hydroxylation level is between 10% and 100%, for example, greater than or equal to 10%, or greater than or equal to 15%, or greater than or equal to 20%, or greater than or equal to 26%, greater than or equal to 27%, greater than or equal to 28%, greater than or equal to 29%, or greater than or equal to 30%, or greater than or equal to 35%, or greater than or equal to 40%, or greater than or equal to 45%, or greater than or equal to 50%, or greater than or equal to 55%, or greater than or equal to 60%, or greater than or equal to 65%, or greater than or equal to 70%, or greater than or equal to 75%, or greater than or equal to 80%, or greater than or equal to 85%, or greater than or equal to 90%, or greater than or equal to 95%.

In certain embodiments, in the pharmaceutical preparation provided by the present invention, the GLP-1 fusion protein has greater than or equal to 10%, or greater than or equal to 15%, or at least greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90% of the GLP-1 polypeptide in the fusion protein is hydroxylated at K34 relative to natural human GLP-1.

As shown in the examples of the present application, the yield of the hydroxylated GLP-1 fusion protein is about 100 times to about 500 times higher than that of the GLP-1 fusion protein in which hydroxylation is not detected at K34 of GLP-1.

The present invention also found that the GLP-1 polypeptide in the GLP-1 fusion protein has a lower oxidation level at tryptophan 31 (W31) relative to natural human GLP-1, and the lower oxidation level may be beneficial to the stability

and/or activity of the GLP-1 fusion protein. In certain embodiments, the GLP-1 polypeptide in the GLP-1 fusion protein is substantially unoxidized at tryptophan (W31) at position 31 relative to native human GLP-**1. In** certain embodiments, the oxidation level of the GLP-1 polypeptide in the GLP-1 fusion protein at W31 relative to native human GLP-1 is less than 0.5% (e.g., less than 0.4%, less than 0.3%, less than 0.2%, or less than 0.1%) or undetectable. W31 refers to the tryptophan residue at position 31 of the native human GLP-1 polypeptide. Those skilled in the art will appreciate that the same tryptophan may correspond to different positions in other GLP-1 polypeptides.

It can be detected by the methods known in the embodiments of the present application or in the prior art (for example, the mass spectrometry method described in WO2002046227A2 or the protein oxidation determination method of Bettinger et al. (Bettinger, J.Q., et al., Quantitative Analysis of in Vivo Methionine Oxidation of the Human Proteome. J Proteome Res, 2020. 19(2): p. 624-633.). Unless otherwise specified, the oxidation level described in the present application is calculated as a percentage of the number of molecules. For example, in 100g of the GLP-1 fusion protein described in the present invention, if 10g of the GLP-1 fusion protein is oxidized at W31 of GLP-1 and the remaining 90g of the GLP-1 fusion protein is not oxidized at W31 of GLP-1, the oxidation level of the GLP-1 fusion protein is considered to be 10%.

The GLP-1 polypeptide of the fusion protein disclosed herein can be human GLP-1. In certain embodiments, the GLP-1 polypeptide is selected from human GLP-1 (7-37), human GLP-1 (7-36) amide, and DPP-IV resistant human GLP-1, and comprises A8G and G22E substitutions relative to native human GLP-1.

In certain embodiments, the GLP-1 polypeptide is selected from human GLP-1 (7-37), human GLP-1 (7-36) amide, and DPP-IV resistant human GLP-1, and comprises A8G, G22E, and R36G substitutions relative to native human GLP-1.

In certain embodiments, the GLP-1 polypeptide is GLP-1 (7-37). In certain embodiments, the GLP-1 polypeptide is GLP-1 (7-36) amide. In certain embodiments, the GLP-1 polypeptide is DPP-IV resistant GLP-1. In certain embodiments, the GLP-1 polypeptide may comprise amino acid substitutions, such as one, two, or three mutations of A8G, G22E, and R36G.

In certain embodiments, the GLP-1 polypeptide in the GLP-1 fusion protein described herein has at least about 70%, at least about 75%, at least about 80%, at least about 85%, at least about 90%, or at least about 95% sequence identity compared to the amino acid sequence shown in SEQ ID NO: 1, SEQ **ID** NO: 2, or SEQ ID NO: 3, and comprises one or more amino acid substitutions selected from the group consisting of A8G, G22E, and R36G relative to native human GLP-1. In certain embodiments, the GLP-1 polypeptide in the GLP-1 fusion protein described herein has at least about 70%, at least about 75%, at least about 80%, at least about 85%, at least about 90%, or at least about 95% sequence identity compared to the amino acid sequence shown in SEQ ID NO: 1, SEQ ID NO: 2, or SEQ ID NO: 3, and stimulates β cells to secrete insulin in a glucose-dependent manner. In certain embodiments, the GLP-1 polypeptide in the GLP-1 fusion protein described herein has at least about 70%, at least about 75%, at least about 80%, at least about 85%, at least about 90% or at least about 95% sequence identity compared to the amino acid sequence shown in SEQ ID NO: 1, SEQ ID NO: 2 or SEQ ID NO: 3, and comprises one or more amino acid substitutions selected from the following group: A8G, G22E and R36G relative to native human GLP-1, and stimulates β cells to secrete insulin in a glucose-dependent manner.

| NAME | Amino Acid Sequence | SEQ ID NO: |
|---|---|---|
| GLP-1 (1-37) | HDEFERHAEGTFTSDVSSYLEGQAAKEFIAWLVKGR G | 43 |
| GLP-1 (7-36) amide | HAEGTFTSDVSSYLEGQAAKEFIAWLVKGR | 1 |
| GLP-1 (7-37) | HAEGTFTSDVSSYLEGQAAKEFIAWLVKGRG | 2 |
| GLP-1 mutations (A8G, G22E, R36G) | HGEGTFTSDVSSYLEEQAAKEFIAWLVKGGG | 3 |

In certain embodiments, the GLP-1 polypeptide is human GLP-1 (7-37) and comprises A8G, G22E and R36G substitutions relative to native human GLP-1.

In certain embodiments, the amino acid sequence of the GLP-1 polypeptide comprises the amino acid sequence as shown in SEQ ID NO: 3. In certain embodiments, the amino acid sequence of the GLP-1 polypeptide is as shown in SEQ ID NO: 3.

In certain embodiments, the GLP-1 polypeptide is DPP-IV resistant human GLP-1. Native GLP-1 has a short circulation half-life (t1/2 < 2 minutes), which is mainly due to rapid enzymatic inactivation including dipeptidyl peptidase IV

(DPP-IV) and/or renal clearance. Therefore, when native GLP-1 is used, continuous subcutaneous infusion by a pump is necessary to maintain the effect of GLP-1 in vivo. Therefore, pharmaceutical long-acting degradation-resistant GLP-1 mimetic peptides have been developed. For example, dulaglutide is a DPP-IV protected GLP-1 analog fused to an IgG4/Fc fragment with a half-life of 4.7-5.5 days (Geiser, J.S., et al., Clinical Pharmacokinetics of Dulaglutide in Patients with Type 2 Diabetes: Analyses of Data from Clinical Trials. Clin Pharmacokinet, 2016. 55(5): p. 625-34.).

Fc domain

In certain embodiments, the IgG2-Fc domain in the GLP-1 fusion protein described herein is an Fc domain from human IgG2.

In this application, "IgG2-Fc" and "IgG2/Fc" are used interchangeably and both refer to the Fc domain of immunoglobulin IgG2.

In certain embodiments, the IgG2-Fc domain described herein has a certain level of oxidation at methionine 253 (M253) corresponding to SEQ ID NO: 7. In certain embodiments, the IgG2-Fc domain described herein has a reduced level of oxidation at methionine 253 (M253) corresponding to SEQ ID NO: 7. In certain embodiments, the IgG2-Fc domain described herein is not oxidized at methionine 253 (M253) corresponding to SEQ ID NO: 7. In the present application, M253 refers to the methionine residue at IgG2 at position 253 corresponding to SEQ ID NO: 7. Those skilled in the art will appreciate that the same methionine may correspond to different positions in other IgG polypeptides.

In certain embodiments, the IgG2-Fc domain has a level of oxidation at M253 corresponding to SEQ ID NO: 7 of less than or equal to about 15%, less than or equal to about 10%, less than or equal to about 9%, less than or equal to about 8%, less than or equal to about 7%, less than or equal to about 6%, less than or equal to about 5%, less than or equal to about 4%, less than or equal to about 3%, less than or equal to about 2%, less than or equal to about 1%, less than or equal to about 0.5% or undetectable.

As mentioned above, the GLP-1 fusion protein may refer to a plurality of molecules, which may be oxidized or unoxidized at one or both of the positions M253 of the IgG2-Fc domain and/or W31 of the GLP-1 polypeptide, and the oxidation levels of the molecules may be different. The oxidation occurs at the rectangular box in the following amino acid structure:

| The M253 position of the IgG2-Fc domain | The W31 position of the GLP-1 peptide |
|---|---|
| 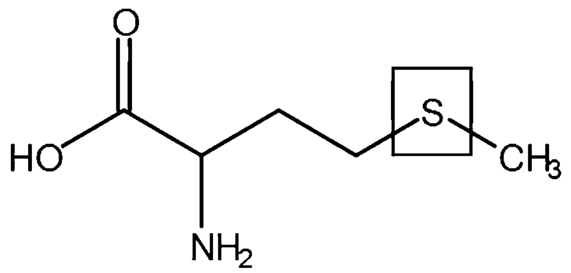 | 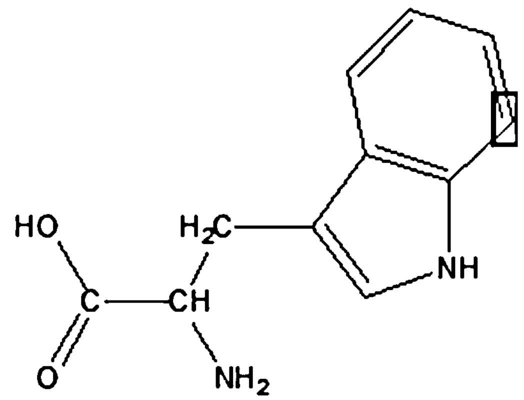 |

In certain embodiments, the GLP-1 fusion protein of the present invention has no detectable oxidation on W31 of the GLP-1 polypeptide relative to native human GLP-1 and about 2% oxidation level at M253 of the IgG2-Fc domain. Other GLP-1 analog fusion proteins have oxidation levels of 2-8% on W31 (for the GLP-1 polypeptide) and/or M253 (for the IgG2-Fc domain).

The GLP-1 polypeptide in the fusion protein is covalently linked (directly or through a linker peptide) to the Fc portion of the immunoglobulin. In certain embodiments, the immunoglobulin is IgG. The IgG-Fc of the fusion protein disclosed herein can be IgG2-Fc. In certain embodiments, the IgG2-Fc domain comprises a C222S substitution. The C222S substitution of IgG2-Fc can increase the flexibility of the N-terminal hinge region by removing the disulfide bond between the two monomers of the homodimer. The increased flexibility of the N-terminal hinge region can reduce the binding affinity of Fcγ receptors, thereby reducing antibody-dependent cellular cytotoxicity (ADCC) and complement-dependent cytotoxicity (CDC).

In another embodiment, the IgG2-Fc domain comprises A330S and P331S substitutions. The A330S and P331S substitutions reduce the affinity for Fcγ receptors and C1q complement proteins.

In certain embodiments, the IgG2-Fc domain comprises C222S, A330S and P331S substitutions.

Except for the amino acid position of M253, which corresponds to the amino acid position of SEQ ID NO: 7, the other amino acid residue positions of the IgG2-Fc domain described in the present application, including, for example, A330, P331 and C222, correspond to the positions of human IgG2 as shown in Genbank Accession No. QRG33935.1. The IgG2-Fc portion can be 228 amino acids as shown in SEQ ID NO: 5, corresponding to amino acids 219 to 445 of human IgG2, for example, as shown in Genbank accession number QRG33935.1. A person skilled in the art will readily recognize the residue positions of amino acids, such as residue positions of A330, P331 and C222 in the short or long Fc fragments shown in the reference sequence SEQ ID NO: 5 or SEQ ID NO: 6.

In certain embodiments, the IgG2-Fc domain described in the present application has at least about 70%, at least about 75%, at least about 80%, at least about 85%, at least about 90% or at least about 95% sequence identity compared to the amino acid sequence shown in SEQ ID NO: 5 or SEQ ID NO: 6, and comprises one or more amino acid substitutions selected from the group consisting of C222S, A330S and P331S. Wherein the fusion protein has an improved half-life compared to a GLP-1 polypeptide without IgG/Fc domain fusion or fused to an IgG4/Fc domain.

In certain embodiments, the IgG2-Fc domain has at least about 70%, at least about 75%, at least about 80%, at least about 85%, at least about 90%, or at least about 95% sequence identity compared to the amino acid sequence shown in SEQ ID NO: 5 or SEQ ID NO: 6, and comprises one or more amino acid substitutions selected from the group consisting of C222S, A330S, and P331S.

In certain embodiments, the IgG2-Fc domain has at least 90% sequence identity compared to the amino acid sequence shown in SEQ ID NO: 5 or SEQ ID NO: 6, and comprises A330S and P331S substitutions. Preferably, the IgG2-Fc domain further comprises a C222S substitution. More preferably, the amino acid sequence of the IgG2-Fc domain is as shown in SEQ ID NO: 6.

In certain embodiments, in the GLP-1 fusion protein described herein, the amino acid sequence of the GLP-1 polypeptide is as shown in SEQ ID NO: 3, and the amino acid sequence of the immunoglobulin Fc domain is as shown in SEQ ID NO: 6.

| NAME | Amino Acid Sequence | SEQ ID NO: |
|---|---|---|
| IgG2/Fc domain (human IgG2 heavy chain Fc region residues 219-445) | ERKCCVECPPCPAPPVAGPSVFLFPPKPKDTLMISRTPE VTCVVVDVSHEDPEVQFNWYVDGVEVHNAKTKPRE EQFNSTFRVVSVLTVVHQDWLNGKEYKCKVSNKGLP APIEKTISKTKGQPREPQVYTLPPSREEMTKNQVSLTC LVKGFYPSDIAVEWESNGQPENNYKTTPPMLDSDGSF FLYSKLTVDKSRWQQGNVFSCSVMHEALHNHYTQKS LSLSPG | 5 |
| IgG2/Fc domain mutation (human IgG2 heavy chain Fc region residues 219-445, including C222S, A330S, and P331S site mutations) | ERKSCVECPPCPAPPVAGPSVFLFPPKPKDTLMISRTPE VTCVVVDVSHEDPEVQFNWYVDGVEVHNAKTKPRE EQFNSTFRVVSVLTVVHQDWLNGKEYKCKVSNKGLP SSIEKTISKTKGQPREPQVYTLPPSREEMTKNQVSLTCL VKGFYPSDIAVEWESNGQPENNYKTTPPMLDSDGSFF LYSKLTVDKSRWQQGNVFSCSVMHEALHNHYTQKSL SLSPG | 6 |

The positions of the sites where the A8G, G22E, R36G substitutions and K34 hydroxylation of the GLP-1 polypeptide mentioned in the present application occur in SEQ ID NO: 3 and in SEQ ID NO: 7 are shown in the following

table. In addition, the positions of the sites where the C222S, A330S, P331S substitutions and M253 oxidation on the IgG2-Fc domain mentioned in the present application occur relative to the positions in QRG33935.1 and in SEQ ID NO: 7 are also shown in the following table.

| Modified Sites | Position in SEQ ID NO: 3 | Position in SEQ ID NO: 6 of IgG2 (219-445) Relative to the sequence shown in QRG33935.1 | Position in SEQ ID NO: 7 |
|---|---|---|---|
| A8G | AA2 | | AA2 |
| G22E | AA16 | | AA16 |
| R36G | AA30 | | AA30 |
| Hydroxylation of K34 | AA28 | | AA28 |
| Oxidation at W31 relative to native human GLP-1 | AA25 | | AA25 |
| Oxidation at M253 | | AA427 | AA253 |
| C222S | | AA222 | AA48 |
| A330S | | AA330 | AA155 |
| P331S | | AA331 | AA156 |

In certain embodiments, the GLP-1 polypeptide is located at the N-terminus of the immunoglobulin Fc domain. In certain embodiments, the GLP-1 polypeptide is located at the C-terminus of the immunoglobulin Fc domain.

Linker

In certain embodiments, in the GLP-1 fusion protein described in the present application, the GLP-1 polypeptide is directly covalently linked to the immunoglobulin Fc domain.

In certain embodiments, in the GLP-1 fusion protein described in the present application, the GLP-1 polypeptide is covalently linked to the immunoglobulin Fc domain through a linker.

In certain embodiments, the linker is selected from the group consisting of a cleavable linker, a non-cleavable linker, a flexible linker, a rigid linker, a helical linker, and a non-helical linker.

In certain embodiments, the linker includes a linker peptide.

The GLP-1 polypeptide and the IgG-Fc domain, such as the IgG2-Fc domain, of the fusion protein disclosed herein may be connected by a linker peptide. As used herein, the term "linker peptide" refers to any portion that connects different functional domains of a polypeptide together. The linker peptide may have any suitable length and structure.

In the present invention, the term "cleavable linker" refers to a linker that is sensitive to factors such as proteases, pH or chemicals in the body and is easily cleaved in the presence of the above factors.

In the present invention, the term "non-cleavable linker" refers to a linker that is stable to factors such as proteases, pH or chemicals in the body and is not easily cleaved.

In the present invention, the term "flexible linker" refers to a linker that can increase spatial extensibility when connecting different protein components, so that the spatial folding and conformation of the protein components are not affected by each other as much as possible. In the present invention, the term "rigid linker" refers to a linker that can maintain a fixed distance between protein components when connecting different protein components.

In the present invention, the term "helical linker" refers to a linker in which the rigid unit can form a helix (such as an $\alpha$-helix) within itself or with the same adjacent sequence, so that the formed fusion protein has a relatively stable three-dimensional conformation.

In the present invention, the term "non-helical linker" refers to a linker that cannot form a helical structure.

In some embodiments, the linker peptide includes a linker containing glycine and serine. Preferably, the linker comprises one, two, three, four or more repeats as shown in SEQ ID NO: 39 (GGGS), SEQ ID NO: 40 (GGGGS), SEQ ID NO: 41 (GGGGGS) or SEQ ID NO: 42 (GGGGGGGS).

In certain embodiments, the linker peptide comprises an amino acid sequence selected from the group consisting of SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19.

| Name | Amino acid sequence | SEQ ID NO: |
|---|---|---|
| Linker peptide | SSGAPPPSGGGGS | 9 |
| Linker peptide | GGGGSGGGGSGGGGS | 10 |
| Linker peptide | GGGGSGGGGSGGGGSGGGGS | 11 |
| Linker peptide | GSTGGGGSGKPGSGEGGGGS | 12 |
| Linker peptide | ESGRSGGGGSGGGGS | 13 |
| Linker peptide | EGKSSGSGSESKST | 14 |
| Linker peptide | EGKSSGSGSESKSTQ | 15 |
| Linker peptide | EGKSSGSGSESKVD | 16 |
| Linker peptide | GSTSGSGKSSEGKG | 17 |
| Linker peptide | KESGSVSSEQLAQFRSLD | 18 |
| Linker peptide | ESGSVSSEELAFRSLD | 19 |
| Linker peptide | GGGS | 39 |
| Linker peptide | GGGGS | 40 |
| Linker peptide | GGGGGS | 41 |
| Linker peptide | GGGGGGGS | 42 |

In certain embodiments, the linker peptide connecting the GLP-1 polypeptide and the IgG2/Fc domain may have at least about 70%, at least about 75%, at least about 80%, at least about 85%, at least about 90% or at least about 95% sequence identity with the amino acid sequence shown in SEQ ID NO: 9. In certain embodiments, the linker peptide comprises the amino acid sequence shown in SEQ ID NO: 9. In certain embodiments, the amino acid sequence of the linker peptide is shown in SEQ ID NO: 9.

In certain embodiments, in the GLP-1 fusion protein described in the present application, the amino acid sequence of the GLP-1 polypeptide is shown in SEQ ID NO: 3, the amino acid sequence of the immunoglobulin Fc domain is shown in SEQ ID NO: 6, and the amino acid sequence of the linker is shown in SEQ ID NO: 9.

Fusion protein

In certain embodiments, the GLP-1 fusion protein described in the present application has at least about 70%, at least about 75%, at least about 80%, at least about 85%, at least about 90% or at least about 95% sequence identity with the amino acid sequence shown in SEQ ID NO: 7. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 7, and the GLP-1 polypeptide comprises A8G, G22E, R36G substitutions, and the IgG2-Fc domain comprises C222S, A330S, and P331S substitutions. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 7, and stimulates β cells to secrete insulin in a glucose-dependent manner. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 7, and the GLP-1 polypeptide comprises A8G, G22E, R36G substitutions, the IgG2-Fc domain comprises C222S, A330S, and P331S substitutions, and stimulates β cells to secrete insulin in a glucose-dependent manner.

In certain embodiments, the GLP-1 fusion protein described herein has an amino acid sequence as shown in SEQ ID NO: 7 or an amino acid sequence having at least 80% (e.g., at least about 85%, at least about 90%, or at least about 95%) sequence identity with SEQ ID NO: 7.

In certain embodiments, the GLP-1 fusion protein described herein has an amino acid sequence as shown in SEQ ID NO: 7 or an amino acid sequence having at least about 90% sequence identity with SEQ ID NO: 7.

In certain embodiments, the GLP-1 fusion protein described herein has an amino acid sequence as shown in SEQ ID NO: 7. In certain embodiments, the amino acid sequence of the GLP-1 fusion protein described herein is as shown in SEQ ID NO: 7.

| Name | Amino acid sequence | SEQ ID NO: |
|---|---|---|
| GLP-1-IgG2/Fc fusion protein (comprising GLP-1, a linker peptide, and IgG2/Fc domain) | HGEGTFTSDVSSYLEEQAAKEFIAWLVKGGGSSGAPPPSGGG GSERKSCVECPPCPAPPVAGPSVFLFPPKPKDTLMISRTPEVT CVVVDVSHEDPEVQFNWYVDGVEVHNAKTKPREEQFNSTF RVVSVLTVVHQDWLNGKEYKCKVSNKGLPSSIEKTISKTKG QPREPQVYTLPPSREEMTKNQVSLTCLVKGFYPSDIAVEWES NGQPENNYKTTPPMLDSDGSFFLYSKLTVDKSRWQQGNVFS CSVMHEALHNHYTQKSLSLSPG | 7 |

As demonstrated in the examples, the GLP-1 fusion protein disclosed herein has a longer half-life after sequence modification, for example, the GLP-1 fusion protein YN-011 has a half-life of about 8.6 days (207 h).

In the preparation process of the GLP-1 fusion protein of the present invention, the GLP-1 fusion protein may also contain a signal peptide.

As referred to herein, the term "signal peptide" refers to a polypeptide that causes the fusion protein to be secreted into the extracellular medium. Such a polypeptide may also be referred to as a "leader peptide", "polypeptide precursor", "propeptide", etc. The use of signal peptides to direct the secretion of proteins is known in the art (e.g., U.S. Patent No. 8,658,174, the contents of which are incorporated herein by reference in their entirety). Examples of signal peptides include, but are not limited to, human CD33 signal peptide, human growth hormone-releasing hormone (GHRH) signal peptide, human alpha-1-microglobulin/bikunin precursor (AMBP) signal peptide, Gaussia luciferase signal peptide, mouse immunoglobulin heavy chain signal peptide, mouse immunoglobulin kappa light chain signal peptide. The signal peptide is cleaved during the secretion process. In some embodiments, cleavage of the signal peptide generates an active histidine residue at the N-terminus of the GLP-1 polypeptide.

In certain embodiments, the signal peptide is a human CD33 signal peptide.

In certain embodiments, the signal peptide has at least about 70%, at least about 75%, at least about 80%, at least about 85%, at least about 90%, or at least about 95% sequence identity with the amino acid sequence shown in SEQ ID NO: 4 (MPLLLLLPLLWAGALA) and allows secretion of the GLP-1 fusion protein. In certain embodiments, the signal peptide has an amino acid sequence as shown in SEQ ID NO: 4 or an amino acid sequence having at least 90% sequence identity with SEQ ID NO: 4. In certain embodiments, the signal peptide has an amino acid sequence as shown in SEQ ID NO: 4.

In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 8. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 8, and the GLP-1 polypeptide comprises A8G, G22E, R36G substitutions, and the IgG2-Fc domain comprises C222S, A330S, and P331S substitutions. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 8, and stimulates β cells to secrete insulin in a glucose-dependent manner. In certain embodiments, the GLP-1 fusion protein described herein has at least about 70%, at least about 75%, at least about 80%, at least about 85%, at least about 90%, or at least about 95% sequence identity with the amino acid sequence shown in SEQ ID NO: 8, and the GLP-1 polypeptide comprises A8G, G22E, and R36G substitutions, the IgG2-Fc domain comprises C222S, A330S, and P331S substitutions, and stimulates β cells to secrete insulin in a glucose-dependent manner.

In certain embodiments, the GLP-1 fusion protein described herein has an amino acid sequence as shown in SEQ ID NO: 8 or an amino acid sequence having at least 80% (e.g., at least about 85%, at least about 90%, or at least about 95%) sequence identity with SEQ ID NO: 8.

In certain embodiments, the GLP-1 fusion protein described herein has an amino acid sequence as shown in SEQ ID NO: 8 or an amino acid sequence having at least about 90% sequence identity with SEQ ID NO: 8.

In certain embodiments, the GLP-1 fusion protein described herein has an amino acid sequence as shown in SEQ ID NO: 8. In certain embodiments, the amino acid sequence of the GLP-1 fusion protein described herein is as shown

in SEQ ID NO: 8.

| Name | Amino acid sequence | SEQ ID NO: |
|---|---|---|
| GLP-1-IgG2/Fc fusion protein (including signal peptide, GLP-1, linker peptide, and IgG2/Fc domain) | MPLLLLLPLLWAGALAHGEGTFTSDVSSYLEEQAAKEFIAWL VKGGGSSGAPPPSGGGGSERKSCVECPPCPAPPVAGPSVFLFP PKPKDTLMISRTPEVTCVVVDVSHEDPEVQFNWYVDGVEV HNAKTKPREEQFNSTFRVVSVLTVVHQDWLNGKEYKCKVS NKGLPSSIEKTISKTKGQPREPQVYTLPPSREEMTKNQVSLTC LVKGFYPSDIAVEWESNGQPENNYKTTPPMLDSDGSFFLYSK LTVDKSRWQQGNVFSCSVMHEALHNHYTQKSLSLSPG | 8 |

In certain embodiments, the half-life of the GLP-1 fusion protein described herein in a subject (e.g., a human subject) is at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days.
In another preferred embodiment, the GLP-1 fusion protein described herein is pharmaceutical grade.

2. Nucleic acid, recombinant vector and cell

The GLP-1 fusion protein in the pharmaceutical preparation provided by the present invention can be prepared by a variety of ways. For example, it is prepared using recombinant cells, including, for example, Chinese hamster ovary cells that recombinantly express the GLP-1 fusion protein, and the recombinant cells contain a polynucleotide encoding the GLP-1 fusion protein.
The term "nucleic acid" or "nucleotide" used in this application refers to deoxyribonucleic acid (DNA) or ribonucleic acid (RNA) in single-stranded or double-stranded form and polymers thereof. Unless otherwise indicated, a particular nucleotide sequence also implicitly encompasses conservatively modified variants thereof (e.g., degenerate codon substitutions), alleles, orthologs, SNPs, and complementary sequences as well as explicitly indicated sequences. Specifically, degenerate codon substitutions can be achieved by generating sequences in which the third position of one or more selected (or all) codons is substituted with mixed bases and/or deoxyinosine residues (see Batzer et al., Nucleic Acid Res. 19:5081 (1991); Ohtsuka et al., J. Biol. Chem. 260:2605-2608 (1985) and Rossolini et al., Mol. Cell. Probes 8:91-98 (1994)).
In certain embodiments, the polynucleotides described herein are codon-optimized, for example, optimized for humans. The nucleic acid molecules can be used in the methods described herein.
In certain embodiments, the polynucleotide encoding the GLP-1 fusion protein described in the present application comprises a polynucleotide sequence as shown in SEQ ID NO: 26 or as shown in SEQ ID NO: 27, or a polynucleotide sequence having at least 70% sequence identity with SEQ ID NO: 26 or SEQ ID NO: 27.
In certain embodiments, the polynucleotide encoding the GLP-1 fusion protein described in the present application comprises a polynucleotide sequence having at least about 70%, at least about 75%, at least about 80%, at least about 85%, at least about 90%, at least about 95%, at least about 96%, at least about 97%, at least about 98% or at least about 99% sequence identity with the polynucleotide sequence as shown in SEQ ID NO: 26. In another embodiment, the polynucleotide encoding the GLP-1 fusion protein described in the present application is a polynucleotide sequence as shown in SEQ ID NO: 26.
In certain embodiments, the polynucleotide encoding the GLP-1 fusion protein described in the present application comprises a polynucleotide sequence having at least about 70%, at least about 75%, at least about 80%, at least about 85%, at least about 90%, at least about 95%, at least about 96%, at least about 97%, at least about 98% or at least about 99% sequence identity with the polynucleotide sequence shown in SEQ ID NO: 27. In another embodiment, the polynucleotide encoding the GLP-1 fusion protein described in the present application is a polynucleotide sequence as shown in SEQ ID NO: 27.
In certain embodiments, the polynucleotide encoding the GLP-1 polypeptide described in the present application comprises a polynucleotide sequence as shown in any one of SEQ ID NOs: 20 to 22 or a polynucleotide sequence having

at least 70% (e.g., at least about 70%, at least about 75%, at least about 80%, at least about 85%, at least about 90%, at least about 95%, at least about 96%, at least about 97%, at least about 98% or at least about 99%) sequence identity with the polynucleotide sequence shown in SEQ ID NOs: 20 to 22.

In certain embodiments, the polynucleotide encoding the IgG2-Fc domain in the GLP-1 fusion protein described herein comprises a polynucleotide sequence as shown in SEQ ID NO: 24 or SEQ ID NO: 25, or a polynucleotide sequence having at least 70% (e.g., at least about 70%, at least about 75%, at least about 80%, at least about 85%, at least about 90%, at least about 95%, at least about 96%, at least about 97%, at least about 98%, or at least about 99%) sequence identity with the polynucleotide sequence as shown in SEQ ID NO: 24 or SEQ ID NO: 25.

In certain embodiments, the polynucleotide encoding the signal peptide described herein comprises a polynucleotide sequence as shown in SEQ ID NO: 23, or a polynucleotide sequence having at least 70% (e.g., at least about 70%, at least about 75%, at least about 80%, at least about 85%, at least about 90%, at least about 95%, at least about 96%, at least about 97%, at least about 98%, or at least about 99%) sequence identity with the polynucleotide sequence as shown in SEQ ID NO: 23.

In certain embodiments, the polynucleotide encoding the linker described in the present application comprises a polynucleotide sequence as shown in any one of SEQ ID NOs: 28 to 38 or a polynucleotide sequence having at least 70% (e.g., at least about 70%, at least about 75%, at least about 80%, at least about 85%, at least about 90%, at least about 95%, at least about 96%, at least about 97%, at least about 98% or at least about 99%) sequence identity with the sequence shown in any one of SEQ ID NOs: 28 to 38.

The sequences of SEQ ID NOs: 20 to 38 are shown in the following table.

| Name | Nucleotide sequences | SEQ ID NO |
|---|---|---|
| GLP-1(7-36) | CACGCCGAGGGCACCTTCACCAGCGACGTGAGCAGCTACCTGG<br>AGGGCCAGGCCGCCAAG<br>GAGTTCATCGCCTGGCTGGTGAAGGGCAGG | 20 |
| GLP-1(7-37) | CACGCCGAGGGCACCTTCACCAGCGACGTGAGCAGCTACCTGG<br>AGGGCCAGGCCGCCAAG<br>GAGTTCATCGCCTGGCTGGTGAAGGGCAGGGGC | 21 |
| GLP-1 mutations (A8G, G22E, R36G) | CACGGCGAGGGCACCTTCACCAGCGACGTGAGCAGCTACCTGG<br>AGGAGCAGGCCGCCAAG<br>GAGTTCATCGCCTGGCTGGTGAAGGGCGGCGGC | 22 |
| CD33 signal peptide | ATGCCCCTGCTGCTGCTGCTGCCCCTGCTGTGGGCCGGCGCCCT<br>GGCC | 23 |
| IgG2/Fc domain (human IgG2 heavy chain Fc region residues 219-445) | GAGAGGAAGTGCTGCGTGGAGTGCCCCCCCTGCCCCGCCCCCC<br>CCGTGGCCGGCCCCAGC | 24 |

(continued)

| Name | Nucleotide sequences | SEQ ID NO |
|---|---|---|
| | GTGTTCCTGTTCCCCCCCAAGCCCAAGGACACCCTGATGATCAG CAGGACCCCCGAGGTG ACCTGCGTGGTGGTGGACGTGAGCCACGAGGACCCCGAGGTG CAGTTCAACTGGTACGTG GACGGCGTGGAGGTGCACAACGCCAAGACCAAGCCCAGGGAG GAGCAGTTCAACAGCACC TTCAGGGTGGTGAGCGTGCTGACCGTGGTGCACCAGGACTGGC TGAACGGCAAGGAGTAC AAGTGCAAGGTGAGCAACAAGGGCCTGCCCGCCCCCATCGAG AAGACCATCAGCAAGACC AAGGGCCAGCCCAGGGAGCCCCAGGTGTACACCCTGCCCCCCA GCAGGGAGGAGATGACC AAGAACCAGGTGAGCCTGACCTGCCTGGTGAAGGGCTTCTACC CCAGCGACATCGCCGTG GAGTGGGAGAGCAACGGCCAGCCCGAGAACAACTACAAGACC ACCCCCCCCATGCTGGAC AGCGACGGCAGCTTCTTCCTGTACAGCAAGCTGACCGTGGACA AGAGCAGGTGGCAGCAG GGCAACGTGTTCAGCTGCAGCGTGATGCACGAGGCCCTGCACA ACCACTACACCCAGAAG AGCCTGAGCCTGAGCCCCGGC | |

(continued)

| Name | Nucleotide sequences | SEQ ID NO |
|---|---|---|
| IgG2/Fc domain mutation (human IgG2 heavy chain Fc region residues 219-445, including mutations at positions C222S, A330S, and P331S) | GAGAGGAAGAGCTGCGTGGAGTGCCCCCCCTGCCCCGCCCCCC CCGTGGCCGGCCCCAGC GTGTTCCTGTTCCCCCCCAAGCCCAAGGACACCCTGATGATCAG CAGGACCCCCGAGGTG ACCTGCGTGGTGGTGGACGTGAGCCACGAGGACCCCGAGGTG CAGTTCAACTGGTACGTG GACGGCGTGGAGGTGCACAACGCCAAGACCAAGCCCAGGGAG GAGCAGTTCAACAGCACC TTCAGGGTGGTGAGCGTGCTGACCGTGGTGCACCAGGACTGGC TGAACGGCAAGGAGTAC AAGTGCAAGGTGAGCAACAAGGGCCTGCCCAGCAGCATCGAG AAGACCATCAGCAAGACC AAGGGCCAGCCCAGGGAGCCCCAGGTGTACACCCTGCCCCCCA GCAGGGAGGAGATGACC AAGAACCAGGTGAGCCTGACCTGCCTGGTGAAGGGCTTCTACC CCAGCGACATCGCCGTG GAGTGGGAGAGCAACGGCCAGCCCGAGAACAACTACAAGACC ACCCCCCCCATGCTGGAC AGCGACGGCAGCTTCTTCCTGTACAGCAAGCTGACCGTGGACA AGAGCAGGTGGCAGCAG GGCAACGTGTTCAGCTGCAGCGTGATGCACGAGGCCCTGCACA ACCACTACACCCAGAAG AGCCTGAGCCTGAGCCCCGGC | 25 |

(continued)

| Name | Nucleotide sequences | SEQ ID NO |
|---|---|---|
| GLP-1-IgG2/Fc fusion protein (containing GLP-1, linker peptide, and IgG2/Fc domain) | CACGGCGAGGGCACCTTCACCAGCGACGTGAGCAGCTACCTGG AGGAGCAGGCCGCCAAG GAGTTCATCGCCTGGCTGGTGAAGGGCGGCGGCAGCAGCGGCG CCCCCCCCCCCAGCGGC GGCGGCGGCAGCGAGAGGAAGAGCTGCGTGGAGTGCCCCCCC TGCCCCGCCCCCCCCGTG GCCGGCCCCAGCGTGTTCCTGTTCCCCCCCAAGCCCAAGGACA CCCTGATGATCAGCAGG ACCCCCGAGGTGACCTGCGTGGTGGTGGACGTGAGCCACGAG GACCCCGAGGTGCAGTTC AACTGGTACGTGGACGGCGTGGAGGTGCACAACGCCAAGACC AAGCCCAGGGAGGAGCAG TTCAACAGCACCTTCAGGGTGGTGAGCGTGCTGACCGTGGTGC ACCAGGACTGGCTGAAC GGCAAGGAGTACAAGTGCAAGGTGAGCAACAAGGGCCTGCCC AGCAGCATCGAGAAGACC ATCAGCAAGACCAAGGGCCAGCCCAGGGAGCCCCAGGTGTAC ACCCTGCCCCCCAGCAGG GAGGAGATGACCAAGAACCAGGTGAGCCTGACCTGCCTGGTG AAGGGCTTCTACCCCAGC GACATCGCCGTGGAGTGGGAGAGCAACGGCCAGCCCGAGAAC AACTACAAGACCACCCCC CCCATGCTGGACAGCGACGGCAGCTTCTTCCTGTACAGCAAGC TGACCGTGGACAAGAGC AGGTGGCAGCAGGGCAACGTGTTCAGCTGCAGCGTGATGCACG AGGCCCTGCACAACCAC TACACCCAGAAGAGCCTGAGCCTGAGCCCCGGC | 26 |

(continued)

| Name | Nucleotide sequences | SEQ ID NO |
|---|---|---|
| GLP-1-IgG2/Fc fusion protein (containing signal peptide, GLP-1, linker peptide, and IgG2/Fc domain) | ATGCCCCTGCTGCTGCTGCTGCCCCTGCTGTGGGCCGGCGCCCT GGCCCACGGCGAGGGC ACCTTCACCAGCGACGTGAGCAGCTACCTGGAGGAGCAGGCCG CCAAGGAGTTCATCGCC TGGCTGGTGAAGGGCGGCGGCAGCAGCGGCGCCCCCCCCCC AGCGGCGGCGGCGGCAGC GAGAGGAAGAGCTGCGTGGAGTGCCCCCCCTGCCCCGCCCCCC CCGTGGCCGGCCCCAGC GTGTTCCTGTTCCCCCCCAAGCCCAAGGACACCCTGATGATCAG CAGGACCCCCGAGGTG ACCTGCGTGGTGGTGGACGTGAGCCACGAGGACCCCGAGGTG CAGTTCAACTGGTACGTG GACGGCGTGGAGGTGCACAACGCCAAGACCAAGCCCAGGGAG GAGCAGTTCAACAGCACC TTCAGGGTGGTGAGCGTGCTGACCGTGGTGCACCAGGACTGGC TGAACGGCAAGGAGTAC AAGTGCAAGGTGAGCAACAAGGGCCTGCCCAGCAGCATCGAG AAGACCATCAGCAAGACC AAGGGCCAGCCCAGGGAGCCCCAGGTGTACACCCTGCCCCCCA GCAGGGAGGAGATGACC AAGAACCAGGTGAGCCTGACCTGCCTGGTGAAGGGCTTCTACC CCAGCGACATCGCCGTG GAGTGGGAGAGCAACGGCCAGCCCGAGAACAACTACAAGACC ACCCCCCCCATGCTGGAC AGCGACGGCAGCTTCTTCCTGTACAGCAAGCTGACCGTGGACA AGAGCAGGTGGCAGCAG GGCAACGTGTTCAGCTGCAGCGTGATGCACGAGGCCCTGCACA ACCACTACACCCAGAAG AGCCTGAGCCTGAGCCCCGGC | 27 |
| Linker peptide | AGCAGCGGCGCCCCCCCCCCAGCGGCGGCGGCGGCAGC | 28 |

(continued)

| Name | Nucleotide sequences | SEQ ID NO |
|---|---|---|
| Linker peptide | (GGCGGCGGCGGCAGC)3 | 29 |
| Linker peptide | (GGCGGCGGCGGCAGC)4 | 30 |
| Linker peptide | GGCAGCACCGGCGGCGGCGGCAGCGGCAAGCCCGGCAGCGGC GAGGGCGGCGGCGGCAGC | 31 |
| Linker peptide | GAGAGCGGCAGGAGCGGCGGCGGCGGCAGCGGCGGCGGCGGC AGC | 32 |
| Linker peptide | GAGGGCAAGAGCAGCGGCAGCGGCAGCGAGAGCAAGAGCAC C | 33 |
| Linker peptide | GAGGGCAAGAGCAGCGGCAGCGGCAGCGAGAGCAAGAGCAC CCAG | 34 |
| Linker peptide | GAGGGCAAGAGCAGCGGCAGCGGCAGCGAGAGCAAGGTGGA C | 35 |
| Linker peptide | GGCAGCACCAGCGGCAGCGGCAAGAGCAGCGAGGGCAAGGGC | 36 |
| Linker peptide | AAGGAGAGCGGCAGCGTGAGCAGCGAGCAGCTGGCCCAGTTC AGGAGCCTGGAC | 37 |
| Linker peptide | GAGAGCGGCAGCGTGAGCAGCGAGGAGCTGGCCTTCAGGAGC CTGGAC | 38 |

Peptides and fusion proteins can be synthesized using standard protein chemistry techniques (Bodanszky, M., Principles of peptide synthesis. 2nd rev. ed. Springer laboratory. 1993, Berlin; New York: Springer-Verlag. xii, 329 p.). In addition, automated peptide synthesizers are commercially available (e.g., Advanced ChemTech Model 1396; Milligen/Biosearch 9600). Alternatively, the peptides, polypeptides, or fragments or variants thereof described herein can be recombinantly produced using various expression systems well known in the art.

Any vector suitable for the intended use can be used. For example, some vectors can be introduced into expression systems, such as mammalian, insect, or bacterial expression systems, for expression and purification of the expressed protein. Some vectors can be used to produce viruses. These different vectors are well known in the art. Suitable vectors include, but are not limited to, pMPGCR5 and pAV0243.

In some embodiments, the vector is used together with an in vitro expression system to produce a fusion protein. Expression and purification of the fusion protein can be performed by any suitable method known in the art.

Possible expression vectors include, but are not limited to, plasmids or modified viruses (e.g., replication-deficient retroviruses, including lentiviral vectors, adenoviruses, and adeno-associated viruses, etc.). In one embodiment, an expression vector that can be used with the GLP-1 fusion protein of the present invention is pKN012, which can be obtained commercially (Beijing Kohnoor Science & Technology Co., Ltd).

The vector may contain suitable regulatory sequences and components. Suitable regulatory sequences may be selected from a variety of sources, including bacterial, fungal, viral, mammalian, or insect genes. Examples of such regulatory sequences include: transcription promoters and enhancers or RNA polymerase binding sequences, ribosome binding sequences, including translation initiation signals. In addition, other sequences, such as replication origins, additional DNA restriction sites, enhancers, and sequences that confer transcriptional induction ability, may be incorporated into the expression vector, depending on the cells to be transfected/infected/transduced and the vector used. In one embodiment, the regulatory sequence directs or increases expression in neural tissue and/or cells. In one embodiment, the vector is a viral vector. The recombinant expression vector may also contain a marker gene that helps select host cells transformed, infected, or transfected with the vector for expressing the antibodies described herein. The recombinant

expression vector may also contain other expression cassettes encoding, for example, fusion moieties that can aid in detection (e.g., for producing antibody "fusion proteins"), including, for example, tags and markers described herein.

In one embodiment, the vector comprises one or more, optionally as shown in FIG. 1 . For example, in one embodiment, the vector comprising a polynucleotide encoding a GLP-1 fusion protein is pKN012-GLP1-IgG2.

A variety of methods for transducing cells can be used, including viral vectors, "naked" DNA, DNA in lipids or other nanoparticles, adjuvant-assisted DNA, gene guns, and the like. For example, retroviral vectors such as lentiviral vectors can also be used to transduce cells in vivo. Other vector systems that can be used to implement the present invention include adenovirus and adeno-associated virus-based vectors.

In another aspect of the present invention, a recombinant cell is provided, the cell comprising a polynucleotide encoding the GLP-1 fusion protein, or comprising a vector described herein.

Stably expressing recombinant cells can be prepared, for example, by transforming, transfecting or transducing a recombinant cell with a vector comprising a polynucleotide, preferably any polynucleotide described herein.

In one embodiment, in the cells of the present invention, the cell also recombinantly expresses lysyl hydroxylase, or naturally expresses lysyl hydroxylase.

In one embodiment, the cell of the present invention expresses a lysyl hydroxylase level or activity that is higher than the lysine hydroxylase level or activity expressed by COS-7 cells. In other words, the cell has an increased level of lysyl hydroxylase activity compared to COS-7 cells. For example, the cell can be a modified cell to increase expression of lysine hydroxylase (e.g., by preparing a cell that recombinantly expresses lysine hydroxylase), or can be a cell with a naturally increased level of lysine hydroxylase compared to, for example, COS-7 cells. For example, the cell described in the present application expresses a lysyl hydroxylase level or activity that is at least 5%, at least 10%, at least 15%, at least 20%, at least 25%, at least 30%, at least 35%, at least 40%, at least 45%, or at least 50% higher than the lysine hydroxylase level or activity expressed by COS-7 cells.

In one embodiment, the cell described in the present application is a eukaryotic cell. In certain embodiments, the eukaryotic cell is a mammalian cell. In one embodiment, the mammalian cell is a cell derived from a human. For example, the mammalian cell is a human embryonic kidney cell 293 (HEK293 cell), for example, a HEK293T cell, a HEK293S cell or a HEK293F cell. In one embodiment, the mammalian cell is a Chinese hamster ovary (CHO) cell, for example, a CHO-K1 cell, a CHO-S cell or a CHO-DG44 cell.

In one embodiment, the recombinant cell of the present invention is obtained by suspension domestication of a Chinese hamster ovary cell, with CHOK1 cells as an example.

Therefore, the present invention also provides a method for preparing a GLP-1 fusion protein, the method comprising culturing a recombinant cell expressing a GLP-1 fusion protein, wherein the culturing comprises one or more process steps or materials described in the embodiments. For example, the method may comprise one or more process steps as described in Table 2 and/or one or more materials as described in Table 3 or 4.

III. Methods and uses for treating and preventing diseases

As shown herein, the pharmaceutical preparation provided by the present invention is long-acting, stable, has few side effects, has a simple preparation process, a clear route, is safe and reliable, has low adverse reactions, is easy to use, and can be used for corresponding drug treatment or prevention purposes.

In another aspect of the present invention, the pharmaceutical preparation comprising the GLP-1 fusion protein disclosed herein can be used in a subject to treat or prevent the onset of a disease or condition or slow down its progression.

In another aspect of the present invention, a pharmaceutical preparation comprising the GLP-1 fusion protein is provided for use as a GLP-1 receptor agonist.

In another aspect of the present invention, a pharmaceutical preparation comprising the GLP-1 fusion protein is provided for treating, preventing or slowing down the progression of a disease or condition.

Another aspect of the present invention provides a pharmaceutical preparation containing a GLP-1 fusion protein for preparing a drug for treating, preventing or slowing down the progression of a disease or condition.

Another aspect of the present invention provides a method for treating, preventing or slowing down the progression of a disease or condition by administering a therapeutically effective amount of the pharmaceutical preparation containing a GLP-1 fusion protein to a subject in need thereof.

Another aspect of the present invention provides the use of the pharmaceutical preparation containing a GLP-1 fusion protein described herein in preparing a drug for treating or preventing a disease.

Another aspect of the present invention provides the use of the pharmaceutical preparation containing a GLP-1 fusion protein described herein for treating or preventing a disease.

Another aspect of the present invention provides a method for treating or preventing a disease, the method comprising: administering a therapeutically effective amount of the pharmaceutical preparation containing a GLP-1 fusion protein described herein to a subject. In certain embodiments, the pharmaceutical preparation containing a GLP-1 fusion

protein is administered in an amount of about 0.2 mg to about 20 mg (per person per time) of the fusion protein. In certain embodiments, the amount of the fusion protein is about 1 mg to about 10 mg. In certain embodiments, the amount of the fusion protein is about 1 mg to about 5 mg. In certain embodiments, the amount of the fusion protein is about 0.2 mg, 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 11 mg, about 12 mg, about 13 mg, about 14 mg, about 15 mg, about 16 mg, about 17 mg, about 18 mg, about 19 mg, or about 20 mg.

In certain embodiments, the pharmaceutical preparation comprising the GLP-1 fusion protein is administered at a therapeutically effective dose of 0.01 mg/kg to about 100 mg/kg of the fusion protein (e.g., about 0.01 mg/kg, about 0.5 mg/kg, about 1 mg/kg, about 2 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, or about 100 mg/kg). In some of these embodiments, the pharmaceutical preparation provided herein comprising a GLP-1 fusion protein are administered at a dose of about 50 mg/kg or less of the fusion protein, and in some of these embodiments, the dose is 10 mg/kg or less, 5 mg/kg or less, 1 mg/kg or less, 0.5 mg/kg or less, or 0.1 mg/kg or less. In some embodiments, the administered dose may change during treatment. For example, in some embodiments, the initial administered dose may be higher than the subsequent administered dose. In some embodiments, the administered dose may change during treatment depending on the subject's response. In some embodiments, the pharmaceutical preparations provided herein are administered to a subject (e.g., a human) at a dosing schedule of no more than once a day, once every 3 days, or once a week, once every two weeks, once every three weeks, or once a month. In some embodiments, the pharmaceutical preparations provided herein may be administered to a subject (e.g., a human) at a dosing interval of twice a week, once a week, once every two weeks, once every three weeks, once a month, or once every two months. The efficacy at low dosing frequency has the potential to improve patient compliance and long-term treatment success. Currently available treatment semaglutide is administered once a week.

In certain embodiments, the pharmaceutical preparations comprising the GLP-1 fusion protein provided by the present invention are used for administration, for example, parenterally, intravenously, subcutaneously or intramuscularly. In certain embodiments, the pharmaceutical preparations comprising the GLP-1 fusion protein provided by the present invention can be administered parenterally or formulated for parenteral administration.

In certain embodiments, the pharmaceutical preparations comprising the GLP-1 fusion protein provided by the present invention can be administered subcutaneously or formulated for subcutaneous administration.

In certain embodiments, the pharmaceutical preparations comprising the GLP-1 fusion protein provided by the present invention can be administered intravenously or formulated for intravenous administration.

In certain embodiments, the pharmaceutical preparations comprising the GLP-1 fusion protein provided by the present invention can be administered intramuscularly or formulated for intramuscular administration.

In certain embodiments, the treatment regimen may include multiple administrations.

In certain embodiments, the pharmaceutical preparations comprising the GLP-1 fusion protein provided by the present invention are administered once every 3 days, or once a week, or once every two weeks.

In certain embodiments, the administration regimen of the pharmaceutical preparations comprising the GLP-1 fusion protein provided by the present invention is one week followed by discontinuation of the administration and subsequent administration once a week. In another preferred embodiment, the withdrawal period is 2 weeks.

In certain embodiments, the pharmaceutical preparation comprising the GLP-1 fusion protein provided by the present invention can be discontinued for two weeks after a single administration, and then continuously administered 4 times a week. In another preferred embodiment, the treatment further comprises administering an initial dose of 1 mg one week before the start of the dosing regimen.

In certain embodiments, the disease is selected from the following group: metabolic diseases related to disorders of glucose metabolism and/or lipid metabolism, complications of metabolic diseases, and neurological diseases and other related diseases.

In certain embodiments, the disease or condition is a metabolic disease related to disorders of glucose metabolism and/or lipid metabolism.

In certain embodiments, the metabolic disease related to disorders of glucose metabolism and/or lipid metabolism is selected from the following group: diabetes, non-alcoholic steatohepatitis (NASH), non-alcoholic fatty liver disease (NAFLD), obesity and metabolic syndrome.

In certain embodiments, the metabolic disease related to disorders of glucose metabolism and/or lipid metabolism is or includes diabetes. In certain embodiments, the metabolic disease related to disorders of glucose metabolism and/or lipid metabolism is type 2 diabetes. In certain embodiments, the metabolic disease associated with disorders of glucose metabolism and/or lipid metabolism is type 2 diabetes with poor glycemic control after diet and exercise intervention.

In another embodiment, the metabolic disease associated with disorders of glucose metabolism and/or lipid metabolism is or includes obesity. In another embodiment, the metabolic disease associated with disorders of glucose metabolism and/or lipid metabolism is or includes non-alcoholic fatty liver disease (NAFLD). In another embodiment, the metabolic disease associated with disorders of glucose metabolism and/or lipid metabolism is non-alcoholic liver fibrosis (NASH).

In certain embodiments, the subject is newly diagnosed or has been previously diagnosed with diabetes (e.g., type 2 diabetes). Diabetes can be diagnosed in a variety of ways, such as fasting blood glucose (FPG). According to the American Diabetes Association, diabetes is diagnosed when fasting blood glucose is greater than or equal to 126 mg/dl.

In certain embodiments, the subject has an increased likelihood of developing diabetes (e.g., type 2 diabetes). For example, the subject may be susceptible to diabetes because the subject is obese or the subject has a genetic susceptibility, such as when the subject has a family history of diabetes.

In certain embodiments, the subject is an obese patient. Obesity can be defined by reference to a body mass index (BMI). For example, the World Health Organization (WHO) defines obesity as having a BMI equal to or greater than 30. In another embodiment, the subject has a BMI of at least about 20 kg/m2. In another embodiment, the subject may have blood glucose higher than the average level of peers of comparable weight, but not enough to be diagnosed as diabetes. In another embodiment, the embodiment subject may also be an individual with a family history of diabetes.

In certain embodiments, the subject is a patient newly diagnosed or previously diagnosed with NAFLD or NASH. In another embodiment, the subject has an increased likelihood of developing NAFLD or NASH. For example, the subject may have a genetic susceptibility to NAFLD or NASH.

In certain embodiments, the complications of the metabolic disease include cardiovascular complications (e.g., coronary heart disease, sudden cardiac death, heart failure, etc.), renal complications (e.g., acute kidney injury, diabetic nephropathy), or liver complications caused by the metabolic disease.

In certain embodiments, the disease or condition is a neurological disease. In certain embodiments, the neurological disease is a neurodegenerative disease. In certain embodiments, the neurodegenerative disease is selected from the group consisting of Alzheimer’s disease (AD), motor neuron disease, Huntington’s disease, and Parkinson’s disease (PD).

In certain embodiments, the neurodegenerative disease is Alzheimer’s disease. In another embodiment, the neurodegenerative disease is Parkinson’s disease. In another embodiment, the neurodegenerative disease is motor neuron disease. In another embodiment, the neurodegenerative disease is Huntington’s disease.

In certain embodiments, the subject is newly diagnosed or has been previously diagnosed with Alzheimer’s disease. In another embodiment, the subject has an increased likelihood of developing Alzheimer’s disease. For example, the subject may be a subject who is susceptible to Alzheimer’s disease due to the subject’s genetics, such as when the subject has a family history of Alzheimer’s disease or a Tau or APP mutation associated with Alzheimer’s disease.

In certain embodiments, the subject is newly diagnosed or has been previously diagnosed with Parkinson’s disease. In another embodiment, the subject has an increased likelihood of developing Parkinson’s disease. For example, the subject may be a subject who is susceptible to Parkinson’s disease due to the subject’s genetics, such as when the subject has a family history of Parkinson’s disease.

In certain embodiments, the pharmaceutical preparation containing GLP-1 fusion protein provided by the present invention can be combined with any other known drug or therapy for treating or preventing a disease.

In one aspect, the present invention provides the use of the pharmaceutical preparation containing GLP-1 fusion protein according to the present invention and an additional therapeutic agent in the preparation of a drug for treating or preventing a disease.

In another aspect, the present invention provides the use of the pharmaceutical preparation containing GLP-1 fusion protein according to the present invention and an additional therapeutic agent for treating or preventing a disease.

In another aspect, the present invention provides a method for treating or preventing a disease, the method comprising: administering to a subject a therapeutically effective amount of the pharmaceutical preparation containing GLP-1 fusion protein described herein and an additional therapeutic agent.

In certain embodiments, the additional therapeutic agent is selected from the group consisting of insulin, metformin, sulfonylurea drugs (e.g., glimepiride, glibenclamide, gliclazide, gliquidone), $\alpha$-glucosidase inhibitors (e.g., acarbose) and $\gamma$-aminobutyric acid. In certain embodiments, the additional therapeutic agent is metformin. In certain embodiments, the additional therapeutic agent is $\gamma$-aminobutyric acid.

In certain embodiments, the disease is selected from the group consisting of metabolic diseases associated with disorders of glucose metabolism and/or lipid metabolism, complications of metabolic diseases, and neurological diseases and other related diseases. In certain embodiments, the metabolic diseases associated with disorders of glucose metabolism and/or lipid metabolism are selected from the group consisting of diabetes, non-alcoholic steatohepatitis (NASH), non-alcoholic fatty liver disease (NAFLD), obesity, and metabolic syndrome. In certain embodiments, the metabolic disease associated with disorders of glucose metabolism and/or lipid metabolism is diabetes (e.g., type 2 diabetes, type 2 diabetes with poor blood glucose control after diet and exercise intervention).

In certain embodiments, complications of the metabolic disease include cardiovascular complications (e.g., coronary heart disease, sudden cardiac death, heart failure, etc.), renal complications (e.g., acute kidney injury, diabetic nephropathy), or liver complications caused by metabolic diseases.

In certain embodiments, the disease or condition is a neurological disease. In certain embodiments, the neurological disease is a neurodegenerative disease. In certain embodiments, the neurodegenerative disease is selected from the group consisting of Alzheimer's disease (AD), motor neuron disease, Huntington's disease, and Parkinson's disease (PD).

In certain embodiments, the pharmaceutical preparation comprising the GLP-1 fusion protein disclosed herein can be administered in combination with a drug for treating diabetes, which can be a drug currently on the market, such as insulin, metformin, sulfonylureas mainly including glimepiride, glibenclamide, gliclazide, gliquidone, etc., $\alpha$-glucosidase inhibitors such as acarbose, etc., and other drugs for treating diabetes that are already on the market and under development. In certain embodiments, the diabetes drug is metformin or insulin. In certain embodiments, the diabetes drug is metformin or insulin.

Therefore, in one aspect, the present invention provides the use of the pharmaceutical preparation comprising the GLP-1 fusion protein according to the present invention and metformin in the preparation of a drug for treating diabetes (e.g., type 2 diabetes). In certain embodiments, the amino acid sequence of the GLP-1 fusion protein in the pharmaceutical preparation is as shown in SEQ ID NO: 7.

In another aspect, the present invention provides the use of the pharmaceutical preparation comprising the GLP-1 fusion protein according to the present invention and metformin for treating diabetes (e.g., type 2 diabetes). In certain embodiments, the amino acid sequence of the GLP-1 fusion protein in the pharmaceutical preparation is as shown in SEQ ID NO: 7.

In another aspect, the present invention provides a method for treating diabetes (e.g., type 2 diabetes), the method comprising: administering to a subject a therapeutically effective amount of a pharmaceutical preparation comprising a GLP-1 fusion protein as described herein and metformin. In certain embodiments, the amino acid sequence of the GLP-1 fusion protein in the pharmaceutical preparation is as shown in SEQ ID NO: 7.

In certain embodiments, the pharmaceutical preparation comprising a GLP-1 fusion protein disclosed herein can be administered in combination with an Alzheimer's disease drug and/or a non-drug intervention such as cognitive therapy. In certain embodiments, the pharmaceutical preparation comprising a GLP-1 fusion protein disclosed herein is administered in combination with gamma-aminobutyric acid for the treatment of neurodegenerative diseases. **In** certain embodiments, the pharmaceutical preparation comprising a GLP-1 fusion protein disclosed herein is administered in combination with gamma-aminobutyric acid for the treatment of Alzheimer's disease.

As used herein, "combined administration" includes simultaneous administration as part of the same pharmaceutical composition, simultaneous administration as separate compositions, or administration at different times as separate compositions. When the phrase "combination" is used herein, a composition administered before or after another agent is considered to be administered "in combination" with the agent, even if the composition and the second agent are administered by different routes. Where possible, the additional therapeutic agent administered in combination with the fusion polypeptide, polypeptide complex or conjugate provided herein is administered according to the schedule listed in the product information sheet of the additional therapeutic agent, or according to the Physicians' Desk Reference (Physicians' Desk Reference, 70th Edition (2016)) or a regimen well known in the art.

In certain embodiments, the pharmaceutical preparations comprising the GLP-1 fusion protein disclosed herein are used in combination with gamma-aminobutyric acid to inhibit the effect of TNF-$\alpha$ in reducing the viability of SH-SY5Y cells.

In certain embodiments, the pharmaceutical preparations comprising the GLP-1 fusion protein disclosed herein are used in combination with gamma-aminobutyric acid to reduce TNF-$\alpha$-induced apoptosis of neuronal cells SH-SY5Y.

In certain embodiments, the pharmaceutical preparation comprising the GLP-1 fusion protein disclosed herein, when used in combination with $\gamma$-aminobutyric acid, can have a protective effect on neuronal cells damaged by TNF-$\alpha$.

In certain embodiments, the pharmaceutical preparation comprising the GLP-1 fusion protein disclosed herein is used in combination with $\gamma$-aminobutyric acid to reduce TNF-$\alpha$ -induced apoptosis of neuronal cells.

In certain embodiments, the pharmaceutical preparation comprising the GLP-1 fusion protein disclosed herein is used in combination with $\gamma$-aminobutyric acid to reduce the mRNA expression of inflammatory factors (e.g., TNF-$\alpha$, IL-6) induced by A$\beta$1-42 oligomers in HMC3 microglia.

Therefore, in another aspect, the present invention provides the use of the pharmaceutical preparation comprising the GLP-1 fusion protein according to the present invention and $\gamma$ -aminobutyric acid in the preparation of a drug for treating neurodegenerative diseases (e.g., Alzheimer's disease, motor neuron disease, Huntington's disease and Parkinson's disease). **In** certain embodiments, the amino acid sequence of the GLP-1 fusion protein in the pharmaceutical preparation is as shown in SEQ ID NO: 7.

In another aspect, the present invention provides a use of a pharmaceutical preparation comprising a GLP-1 fusion protein according to the present invention and $\gamma$-aminobutyric acid for treating neurodegenerative diseases (e.g.,

Alzheimer's disease, motor neuron disease, Huntington's disease, and Parkinson's disease). In certain embodiments, the amino acid sequence of the GLP-1 fusion protein in the pharmaceutical preparation is as shown in SEQ ID NO: 7.

In another aspect, the present invention provides a method for treating a neurodegenerative disease (e.g., Alzheimer's disease, motor neuron disease, Huntington's disease, and Parkinson's disease), the method comprising: administering to a subject a therapeutically effective amount of a pharmaceutical preparation comprising a GLP-1 fusion protein as described herein and γ-aminobutyric acid. In certain embodiments, the amino acid sequence of the GLP-1 fusion protein in the pharmaceutical preparation is as shown in SEQ ID NO: 7.

In another aspect, the present invention also provides a pharmaceutical combination comprising a pharmaceutical preparation comprising a GLP-1 fusion protein according to the present invention, and an additional therapeutic agent. In certain embodiments, the additional therapeutic agent is a drug for treating diabetes or a drug for treating a neurodegenerative disease. In certain embodiments, the additional therapeutic agent is selected from the group consisting of insulin, metformin, sulfonylureas (e.g., glimepiride, glibenclamide, gliclazide, gliquidone), alpha glucosidase inhibitors (e.g., acarbose), and gamma-aminobutyric acid. In certain embodiments, the additional therapeutic agent is metformin. In certain embodiments, the additional therapeutic agent is gamma-aminobutyric acid.

IV. Kits

The present invention also provides kits for practicing the methods described herein. Such kits may include a pharmaceutical preparation as described herein, which may be provided in a sterile container. Optionally, instructions on how to use the provided pharmaceutical preparation to treat or prevent a disease may also be included, or made available to a patient or healthcare provider.

In one aspect, the kit comprises (a) a pharmaceutical preparation comprising a GLP-1 fusion protein according to the present invention; and (b) one or more containers for the pharmaceutical preparation. Such kits may also include instructions for their use, which may be customized according to the precise disease to be treated or prevented. The instructions may describe the uses and properties of the materials provided in the kit. In certain embodiments, the kit includes instructions for administration to a patient to treat or prevent a disease, wherein the disease is selected from the group consisting of metabolic diseases associated with disorders of glucose metabolism and/or lipid metabolism, complications of metabolic diseases, and neurological diseases, as well as other related diseases.

The instructions may be printed on a substrate (such as paper or plastic, etc.), and may be present in the kit as a package insert, in a label of a container of the kit or a component thereof (i.e., associated with the package), etc. In other embodiments, the instructions are present as an electronically stored data file on a suitable computer-readable storage medium (e.g., CD-ROM, floppy disk, etc.). In yet another embodiment, the actual instructions are not present in the kit, but a means for obtaining the instructions from a remote source such as via the Internet is provided. An example of this embodiment is a kit including a website where the instructions can be viewed and/or downloaded from the website. It is generally desirable to package some or all of the components of the kit in suitable packaging to maintain sterility. The components of the kit may be packaged in a kit containing element to make a single, easily disposable unit, wherein the kit containing element (e.g., a box or similar structure) may or may not be an airtight container, for example, to further maintain the sterility of some or all of the components of the kit.

The present invention also provides the following implementation scheme.

Embodiment 1. A pharmaceutical preparation comprising a GLP-1 fusion protein, comprising: (a) a GLP-1 fusion protein, the fusion protein comprising a GLP-1 polypeptide and an immunoglobulin Fc domain, wherein the GLP-1 polypeptide is selected from human GLP-1 (7-37), human GLP-1 (7-36) amide and DPPIV-resistant human GLP-1, the GLP-1 polypeptide is covalently linked to the immunoglobulin Fc domain, wherein the GLP-1 polypeptide comprises A8G and/or G22E and/or R36G substitutions, the immunoglobulin Fc domain comprises or is an IgG2/Fc domain, the IgG2/Fc domain comprises a C222S substitution, and/or the IgG2/Fc domain further comprises one or two selected from A330S substitution and P331S substitution; and (b) a buffer; wherein the pH value of the pharmaceutical preparation is in the range of 6.0-7.0, preferably in the range of 6.5-7.0, and more preferably 6.7.

Embodiment 2. The pharmaceutical preparation containing GLP-1 fusion protein according to Embodiment 1, wherein the buffer is one or more selected from phosphate buffer, citrate buffer and borate buffer, preferably phosphate buffer, more preferably disodium hydrogen phosphate/sodium dihydrogen phosphate buffer, still more preferably 5-15 mM disodium hydrogen phosphate/sodium dihydrogen phosphate buffer, more preferably 10 mM disodium hydrogen phosphate/sodium dihydrogen phosphate buffer.

Embodiment 3. A pharmaceutical preparation comprising a GLP-1 fusion protein according to Embodiment 1 or 2, wherein the pharmaceutical preparation further comprises a carbohydrate as an excipient, preferably, the carbohydrate is selected from mannitol, sorbitol, maltitol, erythritol, arabitol, xylitol, sucrose, lactose, trehalose, dextran or a mixture thereof, more preferably, the carbohydrate is selected from one or more of mannitol, sucrose and sorbitol, still more preferably, the carbohydrate is selected from one or two of mannitol and sucrose, still more preferably, the carbohydrate is mannitol, and the mannitol concentration is preferably 1-10% (w/v), and still more preferably, the mannitol concentration is

preferably 4.6% (w/v).

Embodiment 4. A pharmaceutical preparation containing a GLP-1 fusion protein according to any one of Embodiments 1-3, wherein the pharmaceutical preparation further contains a surfactant, preferably, the surfactant is selected from one or more of Tween 80 (polysorbate 80), polyoxyethylene castor oil derivatives, poloxamer, lecithin, polyethylene glycol 15-hydroxystearate, and cyclodextrins, more preferably, the surfactant is Tween 80, and the concentration of Tween 80 is preferably 0.01% (w/v) - 0.04% (w/v), more preferably 0.02% (w/v).

Embodiment 5. A pharmaceutical preparation containing a GLP-1 fusion protein according to any one of Embodiments 1-4, wherein the pharmaceutical preparation contains a GLP-1 fusion protein, disodium hydrogen phosphate/sodium dihydrogen phosphate buffer, mannitol, pH 6.5-7.0.

Embodiment 6. A pharmaceutical preparation containing a GLP-1 fusion protein according to any one of Embodiments 1-4, wherein the pharmaceutical preparation further contains Tween 80.

Embodiment 7. A pharmaceutical preparation comprising a GLP-1 fusion protein according to any one of Embodiments 1-6, wherein the pharmaceutical preparation comprises a GLP-1 fusion protein, 10 mM disodium hydrogen phosphate/sodium dihydrogen phosphate buffer, 4.6% (w/v) mannitol, 0.02% (w/v) Tween 80, pH 6.5-7.0.

Embodiment 8. A pharmaceutical preparation comprising a GLP-1 fusion protein according to any one of Embodiments 1-7, wherein the concentration of the GLP-1 fusion protein is 0.2-20 mg/ml, preferably 1-5 mg/ml, and more preferably 3 mg/ml.

Embodiment 9. Use of a pharmaceutical preparation containing a GLP-1 fusion protein as described in any one of Embodiments 1-7 in the preparation of a drug, preferably, the drug is a GLP-1 receptor agonist, or preferably, the drug is a drug for treating or preventing a glycolipid metabolic disease or a neurodegenerative disease; preferably, the glycolipid metabolic disease is selected from diabetes, non-alcoholic steatohepatitis (NASH), non-alcoholic fatty liver disease (NAFLD), obesity and metabolic syndrome; preferably, the neurodegenerative disease is selected from Alzheimer's disease, motor neuron disease, Huntington's disease or Parkinson's disease; more preferably, the glycolipid metabolic disease is diabetes, more preferably type 2 diabetes.

The above disclosure generally describes the present application. A more complete understanding can be obtained by referring to the following specific examples. These examples are described for illustrative purposes only and are not intended to limit the scope of the present application. Changes in form and substitutions of equivalents may be considered when circumstances may suggest or become convenient. Although specific terms are used herein, these terms are for descriptive purposes rather than for limiting purposes. The following non-limiting examples are used to describe the present invention.

## Examples

Unless otherwise specified, the experimental methods used in the following examples are conventional methods.

Unless otherwise specified, materials and reagents used in the following examples can be obtained from commercial sources.

### Example 1: Fusion Protein Expression and Activity Assay

#### 1.1 Plasmid Construction

A vector encoding the GLP-1 fusion protein was constructed. The fusion protein consists of human GLP-1 (7-37) and human IgG2/Fc (including the hinge region, CH2, and CH3 of human IgG2 heavy chain). The signal peptide sequence of human CD33 (hCD33) was fused with the GLP-1 sequence to guide the secretion of the peptide into the culture medium. The cDNA fragment encoding the fusion protein hCD33-GLP-1-IgG2/Fc (hinge-ch2-ch3) was chemically synthesized (as shown in SEQ ID NO: 27) and inserted into the Ncol and Hindlll sites of the pKN012 vector, generating pKN012-GLP-1-IgG2/Fc.

The pKN012-GLP-1-IgG2/$F_C$ stable expression vector was transformed into Escherichia coli DH5$\alpha$ competent cells. Plasmid DNA was extracted from the bacterial strain containing pKN012-GLP-1-IgG2/Fc and digested with Pvul. After electrophoresis, a target band with the correct molecular weight was observed. The linearized plasmid was quantified after ethanol precipitation and used for stable transfection.

To establish CHO-K1 cells (Lot#58995535/ATCC) stably expressing GLP-1-IgG2/Fc, linearized pKN012-GLP-1-IgG2/Fc (2 $\mu$g) was transfected into CHO-K1 cells grown in a 6-well plate (2.5 x 105 cells/well) using electroporation (electroporation-mediated transfection). After 24 hours of transfection, the cells were dispersed and cultured in CD-CHO medium containing MSX (Methionine Sulfoximine, 100 $\mu$M/L), selecting cells that had stably integrated the recombinant plasmid into the genome. The culture medium was changed every 3 days until colonies formed. Individual colonies were isolated and expanded into stable cell lines. The GLP-1 fusion protein in the culture supernatant of the cell lines grown in a

24-well plate was tested using a Rat GLP-1 RIA kit (YN-011). Cells capable of secreting the fusion protein were selected for further identification. The amino acid sequence of the prepared GLP-1 fusion protein is shown in SEQ ID NO: 7 (referred to as "YN-011" in this application).

1.2 Assessment of YN-011 Activity by Inducing cAMP Levels

Natural GLP-1 stimulates insulin secretion from beta cells in a glucose-dependent manner. To evaluate whether purified YN-011 possesses the functionality of natural GLP-1, its effect on insulin secretion from clonal insulin-secreting INS-1 cells was measured. INS-1 cells were serum-starved and glucose-starved, then treated with different amounts of purified YN-011 in the presence of 0, 5, or 20 mM glucose as indicated. In the absence of glucose, YN-011 did not stimulate insulin secretion from beta cells. However, in the presence of 5 mM or 20 mM glucose, YN-011 dose-dependently stimulated insulin secretion from beta cells. The data indicate that the GLP-1-IgG2/Fc fusion protein YN-011 possesses biological activity and can stimulate insulin secretion in an glucose-dependent manner in INS-1 cells.

In the absence of glucose, INS-1 cells treated with YN-011 (120 nM) maintained basal levels of cAMP. However, in the presence of 5 mM glucose, the cAMP levels in YN-011-treated cells significantly increased, reaching levels comparable to those induced by exendin-4.

Example 2: Increased GLP-1 K34 Hydroxylation Level Enhances Protein Yield and Activity

2.1 Determination of Modification Level by LC-MS/MS Peptide Mapping

Mass spectrometry analysis was performed using Lys-C digestion for identification. The YN-011 sample was denatured, reduced, and alkylated, followed by Lys-C enzyme hydrolysis to generate peptides. Online LC-MS/MS analysis was conducted using a Thermo LTQ Velos Orbitrap instrument, and the data were processed and analyzed using Mascot software. It was observed that the peptide segment 21-28: EFIAWLVK exhibited a molecular weight increase of 16 Daltons (Da) due to modification. The primary and secondary mass spectra of peptides without the +16 Da modification and peptides with the +16 Da modification in the YN-011 sample are shown in Figure 2. The mass measurement error of the primary spectra was within 10 ppm. By analyzing the mass-to-charge ratio of the secondary fragment ions, the site of the +16 Da modification was determined to be K28. K28 is also referred to as K34 since the active GLP-1 (e.g., amino acids 7-37 of GLP-1) lacks the first six residues. The peptide 21-28, also known as 27-34 peptide, can be found in natural human GLP-1 as well.

The modification level at the K34 site in YN-011 was determined using UV280 and EIC (Extracted Ion Chromatography) methods, as shown in Table 1 and Figures 3A and 3B. The calculation method for UV280 is as follows:

$$k = \frac{\frac{k\varepsilon_w}{k\varepsilon_w + \varepsilon_Y} A_1}{\frac{k\varepsilon_w}{k\varepsilon_w + \varepsilon_Y} A_1 + A_o} \qquad \varepsilon_w = 5500 \quad \varepsilon_w = 1490$$

Where:

K: The ratio of modified peptides with +16 Da to the total amount of peptides in the amino acids 21-28 segment (sum of unmodified peptides and +16 Da modified peptides).

A1: Absorbance value at 280 nm for the amino acids 21-28 peptide segment without the +16 Da modification.

A0: Absorbance value at 280 nm for the amino acids 21-28 peptide segment with the +16 Da modification.

$\varepsilon_W$ = 5500: The extinction coefficient of tryptophan (W) is 5500;

$\varepsilon_Y$ = 1490: The extinction coefficient of tyrosine (Y) is 1490.

Table 1: Detection of K34 modification levels in YN-011 using UV280 and EIC methods.

| Sample Name | Sequence Position | Amino Acid Sequence | Translated Modification (PTMs) | UV280 Ratio (%) | EIC* Ratio (%) |
|---|---|---|---|---|---|
| YN-011 Sample 1 | 21-28 | EFIAWLVK | / | / | / |
| | | EFIAWLVK | +16Da | 29.6% | 31.8% |
| YN-011 Sample 2 | 21-28 | EFIAWLVK | / | / | / |
| | | EFIAWLVK | +16Da | 37.0% | 31.4% |
| YN-011 Sample 3 | 21-28 | EFIAWLVK | / | / | / |
| | | EFIAWLVK | +16Da | 30.0% | 31.1% |
| Dulaglutide | 21-28 | EFIAWLVK | / | / | / |
| | | EFIAWLVK | +16Da | 8.2% | 4.4% |
| *Note: UV280 and EIC methods were used to detect the K34 modification levels in YN-011. | | | | | |

2.2 K34 Hydroxylation Level in YN-011

After conducting mass spectrometry analysis and confirming that the +16Da modification occurs at the 34th position of lysine (Lys), considering that +16Da corresponds to the molecular weight of an oxygen atom, it suggests the possibility of an oxidation reaction. In fact, there are two enzymes in cells that catalyze the modification of lysine side chains. One is called lysyl oxidase (Lysyl oxidase, see reference https://en.wikipedia.org/wiki/Lysyl_oxidase), and the other is lysyl hydroxylase (Lysyl hydroxylase, see reference https://en.wikipedia.org/wiki/Lysyl_hydroxylase). Lysyl oxidase can oxidize the amino group of the lysine side chain at the 6th carbon, forming an aldehyde group. However, this modification only results in a 1Da difference compared to the unmodified form, which does not match the observed +16Da modification. On the other hand, lysyl hydroxylase adds a hydroxyl group to the carbon ($\gamma$ position) of the lysine side chain, forming a stable hydroxylated lysine (hydroxylysine). Hydroxylation of lysine increases the molecular weight by 16Da, as it adds a hydroxyl group and reduces a hydrogen atom on the side chain. Therefore, the observed +16Da modification is consistent with lysine hydroxylation. The hydroxylation site at position K34 is shown below:

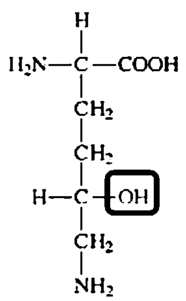

hydroxylysine (Hylys or Lys-OH)

In conclusion, the observed +16Da modification at K34 in YN-011 is highly likely to be due to lysine hydroxylation. Therefore, adding hydroxylation reaction inhibitors during cell culture allows for the verification of the +16Da modification as lysine hydroxylation. It also enables the collection of proteins with different proportions of the +16Da modification to study the impact of the degree of +16Da modification on protein biological activity.

The cell culture process of stable CHOK1 cells capable of secreting the fusion protein YN-011, as described in Implementation Example 1, is shown in Table 2. The relevant materials and cell culture media required for cell culture are listed in Tables 3 and 4, respectively. Mass spectrometry analysis, as described earlier, is used to detect the modification levels of the fusion protein. The inventors found that the fusion protein is indeed hydroxylated at K34, with a hydroxylation level ranging from 15% to 40%.

To investigate the biological effects of K34 hydroxylation, 8 shake flasks (SF1-SF8) were set up for culturing stable CHOK1 cells expressing the fusion protein YN-011. SF1-SF8 were used to evaluate various conditions. Hydroxylation reaction inhibitors were added during the cell culture process to collect proteins with different hydroxylation modification proportions and study the impact of the degree of hydroxylation on protein biological activity. Minoxidil is an inhibitor of lysyl hydroxylase that suppresses lysine hydroxylation, while Zn2+ ions competitively inhibit hydroxylation

enzyme activity by competing with Fe2+ ions during the reaction. SF1 was used as a control without the addition of any inhibitors. Minoxidil used in this experiment was dissolved in a 0.1N HCl solution. HCl was added to SF2 as the HCl control. Different concentrations of minoxidil were added to SF3-SF5 at concentrations of 0.1 mM, 0.5 mM, and 1.0 mM, respectively. SF6 and SF7 were experimental groups where Zn2+ was added. Zn2+ reagent used was ZnSO4, and the concentrations of ZnSO4 used were 200 μM and 400 μM, respectively. SF8 included both minoxidil and Zn2+ (provided by ZnSO4) as inhibitors, with total concentrations of minoxidil and Zn2+ being 0.5 mM and 200 μM, respectively.

Table 2. Cell Culture Process

| Basal Medium | Cell Density | Additive | Time | Percentage | Temperature |
|---|---|---|---|---|---|
| Dynamis (0.5% ACA) | 0.5($\times 10^6$ cell/ml) | Feed C(15g/L FM012), FAA02 | Day 3, 6, 8, 10 | 7.7%, 11%, 11%, 8.8% | 36.5°C→30°C (Day 4) |

Table 3. Materials Used in the Cell Culture Process

| Name | Abbreviations | Company | Item number |
|---|---|---|---|
| DynamisTM AGT medium | Dynamis | Gibco | A26175 |
| CD Efficient Feed C AGT | Feed C | Gibco | A1327501 |
| Sheff-CHO PLUS PF ACF | FM012 | Sheffield | 5X00483 |
| L-Cysteine | Cys | Applichem | A3671 |
| L-Tyrosine | Tyr | Applichem | A3401 |
| L-Glutamine | Gln | J.T.Baker | 2078-06 |
| Anhydrous glucose | Glucose | J.T.Baker | 1919-09 |
| HCl, 6.0N solution | HCl | J.T.Baker | 0327-02 |
| NaOH, 10N solution | NaOH | J.T.Baker | 5000-02 |
| Minoxidil | Minoxidil | Sigma | M4145 |
| $ZnSO_4$ | $Zn^{2+}$ | Sigma | Z0251 |
| Anticoagulant | ACA | Gibco | 0010057 |

Table 4 cell culture medium and its composition

| Cell Culture Medium | Composition |
|---|---|
| Dynamis ( 0.5% ACA ) | IL Dynamis (adding 5ml ACA) |
| Feed C(including 15g/L FM012) | 1L Feed C dissolving 15g FM012 |
| FAA02 | IL FAA02(including 7.95g L-Cysteine and 4.75g L-Tyrosine) |

2.3 Increasing the hydroxylation level of K34 in GLP-1 can enhance protein yield and activity.

The results of protein yield and activity are shown in Table 5. The hydroxylation levels of the blank control SF1 and SF2, which only received HCl addition, were in the range of 15.7% to 16.0%. From SF3 to SF5, the hydroxylation proportion gradually decreased with increasing concentrations of minoxidil (one of the hydroxylation inhibitors). When the dosage of minoxidil reached 0.5 mM, the hydroxylation proportion was 8.7%. When the minoxidil concentration was increased to 1 mM, the proportion was 9.4%. Cell growth and protein expression were significantly inhibited, with a decrease of approximately 17% in peak cell density and a decrease in protein yield of 57.0% to 67.9%. Additionally, when 400 μM of Zn2+ (another hydroxylation inhibitor) was added to the cell culture, the hydroxylation level also decreased to 10.7%. In the experiment where a combination of inhibitors was added (minoxidil at 0.5 mM and Zn2+ at 200 μM) in SF8, the hydroxylation level decreased to 9.6%.

In the presence of hydroxylation inhibitors such as minoxidil, the hydroxylation proportion gradually decreases with increasing concentration. Cell growth and protein expression are significantly inhibited, with a decrease of approximately 17% in peak cell density and a gradual decrease in yield ranging from 57.0% to 67.9%. Similarly, in the presence of

the hydroxylation inhibitor Zn2+, the hydroxylation level also decreases with increasing Zn2+ concentration, resulting in a decrease in protein yield of 22.9% to 34.2%. When the hydroxylation level reaches 15.7% to 16.0%, the yield increases, doubling compared to the low hydroxylation level. Therefore, higher hydroxylation levels result in higher yields. SUPA-1 is a GLP-1-IgG2/Fc fusion protein disclosed in the US patent US8658174. No linking peptide is used between the GLP-1 peptide and IgG2/Fc. Apart from the A8G substitution in the GLP-1 peptide, no other site mutations are present on the GLP-1 peptide and IgG2/Fc. SUPA-1 was not detected to be hydroxylated or undergo any other modifications, and under the same conditions, the protein yield is 22 mg/L. In contrast, when the hydroxylation level of K34 in YN-011 is 8.7% to 9.6%, the yield can reach 0.77 g/L to 1.03 g/L, which is over 100 times higher than that of SUPA-1. When the hydroxylation degree of K34 exceeds 15%, the yield rate of YN-011 can be over 500 times that of SUPA-1.

Furthermore, the blank control (SF1) and the two samples with the lowest hydroxylation levels (SF4 and SF5) were tested for the biological activity of YN-011 using the method described in Example 1. The results showed that SF1 had an activity of 88%, while SF4 and SF5 had activities of 83% and 68%, respectively. Thus, higher hydroxylation levels tend to increase the biological activity.

In summary, it is confirmed that K34 in the fusion protein is indeed hydroxylated, and this modification greatly enhances the yield of YN-011 and shows an increasing trend in biological activity.

Furthermore, by scaling up the cell culture to a 15 L and three 200 L reactors, YN-011 obtained from these batches exhibited 20% to 30% hydroxylation at position 34, with a yield of approximately 2.7 g/L.

Table 5. Summary of Yield and Protein Activity Results

| No. | Test conditions | Hydroxylation level by UV280 detection | Initial cell inoculation density (×106 cells/mL) | Cumulative live cell inoculation density (×106 cells/day/mL) | Time (Days) | Protein Yield (g/L) | Single cell yield (pg/cell/day) |
|---|---|---|---|---|---|---|---|
| SF1 | Control group 1 | 15.7 | 0.5 | 221.9 | 14 | 2.4 | 10.81 |
| SF2 | Control group 2, 0.1N HCl | 16.3 | 0.5 | 213.3 | 14 | 2.28 | 10.69 |
| SF3 | Minoxidil, 0.1 mM | 12.7 | 0.5 | 215.4 | 14 | 2.1 | 9.75 |
| SF4 | Minoxidil, 0.5 mM | 8.7 | 0.5 | 179.8 | 14 | 1.03 | 5.73 |
| SF5 | Minoxidil, 1.0 mM | 9.4 | 0.5 | 170.5 | 14 | 0.77 | 4.52 |
| SF6 | Zn2+, 200uM | 14.6 | 0.5 | 203.6 | 14 | 1.85 | 9.09 |
| SF7 | Zn2+, 400uM | 10.7 | 0.5 | 206.5 | 14 | 1.58 | 7.65 |
| SF8 | Minoxidil, 0.5 mM; $Zn^{2+}$, 200uM | 9.6 | 0.5 | 174.9 | 14 | 0.92 | 5.26 |

Example 3 Determination of Oxidation Levels by LC-MS/MS

Following the protein oxidation measurement method described in Example 2 or by referring to Bettinger et al. ( Bettinger, J.Q., et al., Quantitative Analysis of in Vivo Methionine Oxidation of the Human Proteome. J Proteome Res, 2020. 19(2): p. 624-633. ), the oxidation levels were determined. It was observed that the GLP-1 fusion protein YN-011 of the present invention was not oxidized at position W31, whereas the oxidation level of Degludec at the same position exceeded 5%. The oxidation occurred at the location indicated by the rectangular box in the structure of W31.

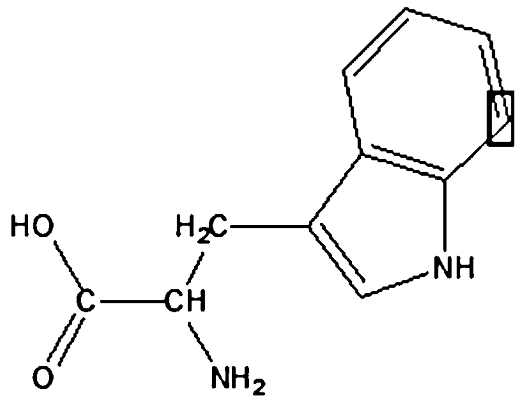

Additionally, the GLP-1 fusion protein YN-011 of the present invention exhibited an oxidation level of approximately 2%-4% at position M253, whereas Degludec showed an oxidation level exceeding 5% at the same position.

Example 4: Prevention and Therapeutic Effects of YN-011 in db/db Mice (Type 2 Diabetes Model)

The impact of multiple subcutaneous injections of YN-011 on reducing blood glucose levels was evaluated in db/db mice (Jackson Laboratories, 000642). The mice were kept under normal lighting conditions (12 hours light/12 hours dark) and at room temperature, with free access to food (normal rodent chow) and water. Db/db mice lack leptin receptors and spontaneously develop obesity, hyperglycemia, and pancreatic β-cell atrophy by 4 weeks of age.

Sixty affected db/db mice (33-45g) were divided into six groups of 10 mice each (5 males and 5 females) and administered 0 (PBS buffer control), 0.15, 0.3, 0.6, or 1.2 mg/kg of YN-011, or 0.3 mg/kg of Degludec via subcutaneous injection. Another group of wild-type mice (17-22g) with the same genetic background served as the normal control. The administration frequency was once every 3 days (Q3D), with a total of 9 doses administered over a period of 26 days.

As shown in Figure 6, throughout the entire experiment, the random plasma glucose (RPG) levels of the db/db mice in the model control group remained consistently high and significantly higher than those of the normal control mice ($P < 0.001$). Following the first administration, YN-011 significantly reduced the RPG concentration in db/db mice, with the therapeutic effect observed as early as 3 hours after administration at a dose as low as 0.15 mg/kg. The duration of the hypoglycemic effect ranged from 34 to 72 hours within the dose range of 0.15-1.2 mg/kg. YN-011 also significantly promoted insulin secretion, with an increase in serum insulin levels observed 3 hours after administration.

After the 9th administration of YN-011, the RPG levels in YN-011-treated mice were significantly lower than those in the control group at all dose groups and measurement time points, except for the 0.15 mg/kg group after the 4th administration (day 10), where the difference was not significant ($P = 0.07$). To gain a more intuitive understanding of the blood glucose reduction in each group of mice, the average rate of decline in random blood glucose levels was calculated at each measurement time point throughout the experiment. The results showed that the average rates of decline in random blood glucose levels at 72 hours after the 7th administration were 31.8%, 46.2%, and 50.8% in the 0.15, 0.3, and 0.6 mg/kg YN-011 groups, respectively, reaching as high as 54.3% in the 1.2 mg/kg YN-011 treatment group. The hypoglycemic effect of YN-011 was sustained for 72 hours after repeated administration.

Long-term administration of YN-011 also significantly reduced fasting blood glucose levels in db/db mice. Throughout the entire experiment, the fasting blood glucose levels of db/db mice remained consistently high and significantly higher than those of the normal control mice ($P < 0.001$). After subcutaneous injections of YN-011 at different doses every 3 days, the fasting blood glucose levels of mice in all YN-011 groups showed a significant decrease compared to the model control group ($P < 0.001$). The rates of fasting blood glucose reduction at 54 hours (day 21) after the 7th administration were 46.8%, 55.6%, 59.5%, and 64.7% in the 0.15, 0.3, 0.6, and 1.2 mg/kg YN-011 groups, respectively. This suggests that the hypoglycemic effect of YN-011 at doses of 0.15, 0.3, 0.6, and 1.2 mg/kg, administered every 3 days, can be maintained for at least 54 hours (3 days after administration). The positive control group treated with 0.3 mg/kg Degludec also showed a significant decrease in fasting blood glucose levels at all measurement time points.

To provide a more intuitive understanding of the blood glucose reduction in each group of mice, the average rate of decline in fasting blood glucose levels at each measurement time point throughout the experiment was calculated. The average rates of fasting blood glucose reduction in the 0.15, 0.3, 0.6, and 1.2 mg/kg YN-011 groups were 55.0%, 61.8%, 63.7%, and 66.1%, respectively. The average rate of fasting blood glucose reduction in the positive control group treated with 0.3 mg/kg Degludec was 63.6%. Therefore, multiple subcutaneous injections of YN-011 every 3 days significantly reduced fasting blood glucose levels in type 2 diabetic db/db mice. The effect was evident at a dose of 0.15 mg/kg, and the hypoglycemic effect of YN-011 at doses of 0.15, 0.3, 0.6, and 1.2 mg/kg could be maintained for at least 54 hours (3 days after administration). Subcutaneous injections of YN-011 at doses of 0.15-0.6 mg/kg in db/db mice showed a decreasing

trend in serum fructosamine levels, while the 1.2 mg/kg YN-011 treatment group exhibited a significant reduction in serum fructosamine levels.

In the model control group, the random and fasting body weights of db/db mice increased continuously throughout the experiment, while the YN-011 treatment groups at doses of 0.3, 0.6, and 1.2 mg/kg showed significant decreases in random and fasting body weights ($P < 0.05$, $P < 0.01$, $P < 0.001$). In contrast, the SUPA-1 fusion protein disclosed in U.S. Patent US8658174 did not have a significant effect on the body weight of db/db mice. This indicates that the GLP-1 fusion protein of the present application performs better.

Compared to the solvent control group, the YN-011 treatment groups showed a significant decrease in epididymal fat content and its ratio to body weight. The scapular fat content was significantly reduced in the 0.15, 0.3, and 0.6 mg/kg YN-011 groups. The subcutaneous fat content, inguinal fat content, and their ratios to body weight were significantly reduced in the 0.3, 0.6, and 1.2 mg/kg YN-011 groups. The perirenal fat content was significantly reduced in the 1.2 mg/kg YN-011 group.

After the last administration (9th dose, day 26), YN-011 dose-dependently increased fasting serum insulin levels in db/db mice, accompanied by a significant increase in pancreatic β-cell mass.

Compared to the solvent control group, multiple subcutaneous injections of YN-011 significantly decreased serum triglyceride levels, and 0.6 mg/kg YN-011 significantly reduced serum free fatty acid levels in db/db mice.

In summary, multiple subcutaneous injections of YN-011 demonstrated significant therapeutic effects in db/db mice. It not only improved glucose metabolism but also had significant therapeutic effects on abnormal lipid metabolism.

Example 5: Phase IIa Clinical Trial of YN-011

5.1 Study Design

This phase IIa clinical trial is a double-blind, placebo-controlled study conducted in subjects with type 2 diabetes mellitus (T2DM) to evaluate the efficacy and safety of subcutaneous administration of YN-011 at doses of 1 mg, 2 mg, 3 mg, and 4 mg. The design of this study is shown in Figure 5.

Key inclusion criteria for the phase IIa study:

- Patients with T2DM (WHO 1999) who have not taken metformin for at least 1 week and have not taken any other oral antidiabetic drugs for at least 2 weeks.
- HbA1c levels at screening: 7.0% ≤ HbA1c ≤ 10.0%.
- Age between 18 and 65 years at screening.
- BMI ≥ 20 kg/m2 and ≤ 40 kg/m2.

Key exclusion criteria for the phase **IIa** study:

- Type 1 diabetes.
- Fasting C-peptide < 0.81 ng/mL.
- Laboratory parameters meeting one of the following criteria: alanine aminotransferase (ALT) levels ≥ 2.5 x **ULN,** and/or aspartate aminotransferase (AST) levels ≥ 2.5 x **ULN,** fasting triglycerides > 5.6 mmol/L; estimated glomerular filtration rate (eGFR) calculated by the CKD-EPI (EPI-(Scr)) equation < 45 mL/min/1.73 m2 ,
- Known susceptibility to familial (first-degree relatives) or personal history of type 2 multiple endocrine neoplasia or medullary thyroid carcinoma.
- Uncontrolled hypertension.
- Known history of pancreatitis, pancreatic cancer, or serum amylase > 1.2 x ULN, or high-risk factors for pancreatitis at screening.
- Patients with uncontrolled hypothyroidism.
- Suspicion of active infection.
- Positive hepatitis B surface antigen (HBsAg), hepatitis C antibody (HCV-Ab), human immunodeficiency virus antibody (HIV-Ab), or Treponema pallidum antibody (TPAb).
- Treatment with GLP-1 receptor agonists, DPP-4 inhibitors, or insulin in the 3 months prior to randomization.
- History of grade 3-4 allergy to any protein drug as per CTCAE.
- Blood donation or blood loss exceeding 450 mL in the 3 months prior to screening.
- Any significant endocrine, immune, coagulation, urinary reproductive tract abnormality, or hematological disease.
- Clinically significant gastric emptying disorder (e.g., gastric outlet obstruction), severe chronic gastrointestinal disease (e.g., active ulcer within 6 months), long-term use of drugs directly affecting gastrointestinal motility, or previous gastrointestinal surgery.
- Any other condition that the investigator or primary physician deems unsuitable for participation in the study.

Subjects were randomized in a 4:1 ratio to receive YN-011 or placebo. YN-011 was administered at doses of 1 mg, 2 mg, and 3 mg, with a dosing regimen of a single dose followed by a 2-week rest period, then once-weekly dosing for 4 consecutive weeks. Subjects in the 4 mg group had the same dosing regimen, except they received an initial dose of 1 mg one week before dosing. All subjects received a total of 5 randomized doses.

The primary endpoint was the safety and tolerability of YN-011 in subjects with T2DM. Secondary endpoints included changes in fasting blood glucose from baseline on a weekly basis, changes in HbA1c from baseline at week 4 and week 7, changes in glycated albumin from baseline at week 4 and week 7, changes in glucose tolerance, and changes in pancreatic beta-cell function assessed by oral glucose tolerance test. Blood samples were collected from all subjects for pharmacokinetic testing. Safety assessment included adverse events, laboratory tests, vital signs, 12-lead electrocardiograms, physical examinations, and evaluation of anti-drug antibodies (ADAs).

5.2 YN-011 Single or Multiple Dose Pharmacokinetics

Within the dose range of 1.0 mg to 4.0 mg, YN-011 exhibited an increasing trend. After a single dose, the half-life (T1/2) of YN-011 was approximately 207 hours (8.6 days), with a median Tmax of 60-84 hours (Figure 4). Following the first dose of YN-011 after randomization, dosing occurred once every week, with the fourth dose being administered consecutively. The plasma concentration of YN-011 reached a steady state after the fourth dose (Figure 4). The fusion protein of this application showed a significantly extended half-life compared to SUPA-1 after amino acid substitution. Additionally, compared to the marketed drugs Dulaglutide with an elimination half-life of 4.7-5.5 days ( Geiser, J.S., et al., Clinical Pharmacokinetics of Dulaglutide in Patients with Type 2 Diabetes: Analyses of Data from Clinical Trials. Clin Pharmacokinet, 2016. 55(5): p. 625-34 ) and Semaglutide with an elimination half-life of 5.7-6.7 days ( Pratley, R.E., et al., Semaglutide versus dulaglutide once weekly in patients with type 2 diabetes (SUSTAIN 7): a randomised, open-label, phase 3b trial. Lancet Diabetes Endocrinol, 2018. 6(4): p. 275-286 ) , YN-011 also exhibited a significantly longer half-life than Dulaglutide and Semaglutide.

5.3 Efficacy of YN-011 in Type 2 Diabetes Mellitus

YN-011 was administered subcutaneously at formal doses of 1 mg, 2 mg, 3 mg, and 4 mg (as per the dosing scheme shown in Figure 5) for the treatment of type 2 diabetes mellitus.

A total of 40 subjects received at least one dose of YN-011 or placebo and were included in the safety analysis. The mean age of the subjects was 51.7 ± 10.32 years, with a mean BMI of 25.80 ± 2.875 kg/m2 and a mean weight of 71.91 ± 12.360 kg. 40% of the subjects were female. Among the 40 subjects, 38 (95%) had comorbidities, including 32 (80%) with confirmed fatty liver by ultrasound, 25 (62.5%) with hyperlipidemia, 19 (47.5%) with hypertension, 10 (22.5%) with atherosclerosis, and 10 (22.5%) with lung lesions. Throughout the entire trial, none of the 40 subjects received concomitant medication. The baseline characteristics of the subjects are summarized in the following table (Table 6).

Table 6. Baseline Characteristics of T2DM Subjects

| | 1 mg n=8 | 2 mg n=8 | 3 mg n=8 | 4 mg n=8 | Placebo n = 8 |
|---|---|---|---|---|---|
| Overweight N (%) | 2 (25%) | 7 (87.5%) | 6 (75%) | 4 (50%) | 4 (50%) |
| Obesity N (%) | 0 (0%) | 1 (12.5%) | 0 (0%) | 0 (0%) | 1 (12.5%) |
| Presence of fatty liver on ultrasound , N (%) | 4 (50.0%) | 6 (75.0%) | 6 (75.0) | 8 (100%) | 8 (100%) |
| AST/ALT/Total Bilirubin > ULN Baseline N (%) | 1 (12.5%) | 0 (0%) | 0 (0%) | 0 (0%) | 1 (12.5%) |
| Dyslipidemia N (%) | 3 (37.5%) | 5 (62.5%) | 7 (87.5%) | 5 (62.5%) | 5 (62.5%) |
| High blood pressure N (%) | 2 (25.0%) | 3 (37.5%) | 5 (62.5%) | 5 (62.5%) | 4 (50.0%) |
| Atherosclerosis N (%) | 0 (0%) | 3 (37.5%) | 4 (50.0%) | 2 (25.0%) | 0 (0%) |
| Lung swelling N (%) | 2 (25.0%) | 3 (37.5%) | 1 (12.5%) | 3 (37.5%) | 0 (0%) |

YN-011's effects on fasting blood glucose at different doses are shown in Figure 6. Multiple subcutaneous administrations of YN-011 at 3 mg or 4 mg resulted in a sustained and significantly improved clinical and statistically significant improvement in fasting blood glucose compared to placebo.

The impact of different doses of YN-011 on HbA1c is illustrated in Figure 7. Multiple subcutaneous administrations of YN-011 at doses of 1 mg, 3 mg, or 4 mg demonstrated a persistent improvement in HbA1c levels, which was clinically and statistically significant compared to placebo. No significant side effects or safety risks were observed.
Example 6: Preventive and Therapeutic Effects of YN-011 in Obesity

6.1 Effects of YN-011 in High-Fat Diet-Induced Obese Mice

The effects of multiple administrations of YN-011 on glucose tolerance, insulin sensitivity, metabolism, and weight reduction were evaluated in high-fat diet (HFD)-induced obese mice. Male C57BL/6 mice, 5 months old (obtained from commercial sources such as Shanghai Slake Experimental Animals Co., Ltd., under standard housing conditions), were fed with an HFD (60% of total calories from fat, 20% from carbohydrates) for 6 months to establish the diet-induced obesity (DIO) mouse model. The DIO mice (weighing >50 g) were then divided into two groups (5 animals per group), with the experimental group receiving subcutaneous injections of 0.3 mg/kg of YN-011 twice a week (BIW) and the control group receiving phosphate-buffered saline (PBS) injections. The treatment was administered for 4 weeks.

The experimental results showed that BIW injections of YN-011 for 4 weeks significantly reduced body weight in DIO mice. Compared to the PBS control group, a significant decrease in visceral fat, particularly epididymal fat content, was observed in the YN-011-treated mice. YN-011 had no impact on the weight of other weight-related tissues, including brown adipose tissue (BAT), white adipose tissue (WAT) in the inguinal region, pancreas, or calf muscle.

In comparison to the control group, BIW injections of YN-011 for 4 weeks significantly reduced ectopic lipid accumulation and hepatic triglycerides, as well as serum ALT levels (YN-011 vs. Ctrl = 34.2 $\pm$ 7.7 vs. 153.4 $\pm$ 18.7, $P < 0.01$) and AST levels (YN-011 vs. Ctrl = 72.20 $\pm$ 19.29 vs. 145.6 $\pm$ 16.8, $P < 0.05$). After 4 weeks of BIW treatment, YN-011 also significantly improved the lipid profile, with a 30% reduction in total cholesterol (TC) ($P < 0.001$), a 68% reduction in triglycerides (TG) ($P < 0.001$), and a 57% reduction in non-esterified fatty acids (NEFA) ($P < 0.001$).

In comparison to the control group, DIO mice receiving multiple administrations of YN-011 showed a significant decrease in food consumption. YN-011 treatment showed an increasing trend in metabolic rate (VO2 and VCO2) and energy expenditure (EE), but without statistical significance. When normalized to body weight, the mice treated with YN-011 exhibited significantly increased VO2, VCO2, and EE during the night. YN-011 had no effect on UcP1 expression in BAT and epididymal WAT but significantly upregulated Ucp1 expression in inguinal WAT, indicating that YN-011 did not enhance BAT thermogenesis but promoted browning of inguinal white adipose tissue. Therefore, YN-011-treated DIO mice exhibited higher core body temperature at room temperature and maintained higher rectal temperature when exposed to a cold environment, indicating increased thermogenesis compared to the control group. These results suggest that YN-011 enhances the adaptation of obese mice to cold environments by generating more calories.

Through glucose fluctuation, intraperitoneal glucose tolerance testing, and insulin tolerance experiments, YN-011 significantly reduced blood glucose ($P < 0.01$) and improved insulin sensitivity ($P < 0.01$) in DIO mice after 4 weeks of treatment.

In conclusion, YN-011 effectively reduced body weight in obese mice and improved obesity-related metabolic disorders, including hyperglycemia, hyperlipidemia, and hepatic steatosis. The beneficial effects of YN-011 on metabolism were associated with the inhibition of food consumption and browning and remodeling of WAT.

6.2 Therapeutic effect of YN-011 on obese rhesus monkeys

The obese rhesus monkeys used in this study were sourced from Sichuan Primate Biotechnology Co., Ltd. Fifteen male obese rhesus monkeys, aged 8-21 years (equivalent to 30-60 years in humans), with a body weight of 9.25-15.70 kg, fasting plasma glucose (FPG) levels between 5.50-8.58 mmol/L, and HbA1c levels between 4.5-5.0%, were selected. These monkeys were within the first year of disease onset and had not received any medication. Their liver and kidney functions were normal. The animals were stratified based on FPG levels and randomly assigned to different groups. The selected animals underwent an intravenous glucose tolerance test (IVGTT) to measure blood glucose and insulin levels before the administration of the test compounds. The experimental groups included a placebo group and two YN-011 treatment groups: YN-011 50 μg/kg and YN-011 25 μg/kg, with 5 animals in each group. The treatments were administered via subcutaneous injections (SC) once a week for 4 consecutive weeks, specifically on Day 0 (D0), Day 7 (D7), Day 14 (D14), and Day 21 (D21).

The effects of YN-011 on body weight (BW) in obese rhesus monkeys are shown in Table 7 and Figure 8. In the placebo group, there were no significant changes in body weight observed throughout the entire study period, indicating the stability of the model. In the YN-011 25 μg/kg group, compared to the baseline, body weight consistently decreased (2.60%-6.71%, $P < 0.05$ or $P < 0.01$) from D7 to D28 with a 6.17% decrease at D28. Compared to the placebo group, the BW in the YN-011 25 μg/kg group significantly decreased on D14 and D21 ($P < 0.05$). In the YN-011 50 μg/kg group, compared to the baseline, body weight consistently decreased (4.06%-7.32%, $P < 0.05$) from D7 to D28 with a significant 7.32% decrease at D28 ($P < 0.05$). Compared to the placebo group, the BW in the YN-011 50 μg/kg group significantly or extremely significantly decreased ($P < 0.05$ or $P < 0.01$) from D7 to D28.

Table 7. Effects of repeated subcutaneous injections of YN-011 on body weight (BW) in obese rhesus monkeys.

| Group and Time points | BW (kg) | | | | | | |
|---|---|---|---|---|---|---|---|
| | D1 SC1[a] Baseline value | D7 SC2 | D14 SC3 | D21 SC4 | D28 | D35 | D42 |
| Placebo Group N = 5 | 10.75 + 1.08 | 10.66 ± 1.10 | 10.64 + 1.11 | 10.64 + 1.12 | 10.47 + 1.13 | 10.52 + 1.11 | 10.44 + 1.05 |
| YN-011 Group 25 μg/kg N = 5 | 12.72 + 1.82 | 12.39 ± 1.76# | 12.16 + 1.61## | 12.06 + 1.59## | 11.84 + 1.46## | 12.05 + 1.56## | 12.03 + 1.51# |
| YN-011 Group 50 μg/kg N = 5 | 10.29 + 1.01 | 9.85 + 0.80# | 9.64 + 0.77# | 9.63 + 0.69# | 9.52 + 0.76# | 9.79 + 0.83# | 9.81 + 0.91# |
| Note : "a" represents the measurement before administration; # compared to baseline, $P < 0.05$; ## compared to baseline, $P < 0.01$. SC refers to subcutaneous injection. | | | | | | | |

Example 7: Preventive and Therapeutic Effects of YN-011 in Non-Alcoholic Steatohepatitis (NASH) Model of Rhesus Monkeys

7.1 Experimental Methods

7.1.1 Liver Biopsy under GE Ultrasound Guidance

Number of animals: 15

Amount of specimens collected: ≤ 2 needles, each needle with a specimen length ≤ 1.5 cm, and the total cumulative specimen length ≤ 3 cm.

Approximately 2 cm of the collected tissue was immediately placed in 4% buffered formalin at room temperature and fixed for at least 24 hours. After appropriate modifications, dehydration, embedding, sectioning, HE staining, and Masson staining were performed.

Equipment: GE Vivid S5 ultrasound system was used for ultrasound, LEICA RM2135 microtome was used for sectioning, OLYMPUS BX43 microscope was used for slide examination, and OLYMPUS DP22-CU camera was used for photomicroscopy.

Tissue sections were stained with H&E and Masson stains. Pathological slides were evaluated by a pathologist according to the diagnostic and treatment guidelines for non-alcoholic fatty liver disease (NAFLD) jointly drafted by the American Association for the Study of Liver Diseases (AASLD), the American College of Gastroenterology (ACG), and the American Gastroenterological Association (AGA). The diagnostic criteria can be found in Table 2 and Table 3.

7.1.2 Tissue Processing, Embedding, and Sectioning

After adequate fixation of the liver biopsy specimens, they were dehydrated, infiltrated with paraffin, embedded, and sectioned (5 μm) directly.

7.1.3 H&E Staining

Routine deparaffinization of the sections was performed. The sections were sequentially immersed in 100% ethanol I, 100% ethanol II, 95%, 85%, and 75% ethanol for 3 minutes each. They were then rinsed with tap water for 3 minutes, stained with hematoxylin-eosin (HE) staining solution for 12 minutes, rinsed with tap water for 5 minutes, and stained with eosin Y solution (aqueous) for 3 minutes. Dehydration was carried out rapidly with 95% ethanol, followed by two rounds of dehydration with absolute ethanol for 2-5 minutes each. The sections were then cleared with xylene twice for 10 minutes each. Neutral mounting medium was applied, and microscopic examination was performed.

7.1.4 Masson Staining

Routine deparaffinization of the sections was performed. The sections were stained with Weigert's iron hematoxylin for 5-7 minutes, and simultaneously, weak acid working solution was prepared by mixing distilled water and weak acid solution in a ratio of 2:1. The sections were rinsed with weak acid working solution for 1 minute. After rinsing with phosphomolybdic acid solution for 1-2 minutes, the sections were directly placed in aniline blue staining solution for 1-2 minutes, followed by rinsing with the prepared weak acid working solution for 1 minute. Dehydration was carried out rapidly with 95% ethanol, followed by two rounds of dehydration with absolute ethanol for 2-5 minutes each. The sections were then cleared with xylene twice for 10 minutes each. Neutral mounting medium was applied, and microscopic examination was performed.

7.1.5 Histopathological Examination of Tissues

The sections were observed under a microscope (with a digital imaging system), and pathological diagnosis and photography were performed. Based on the histopathological diagnosis, the HE and Masson results were scored according to the scoring criteria for non-alcoholic fatty liver disease activity score (NAS, Table 8) and liver fibrosis staging (Table 9).

Table 8 NAS Scoring Criteria

| Scoring / Feature | 0 | 1 | 2 | 3 |
|---|---|---|---|---|
| Steatosis | < 5% | 5%~33% | 34%~66% | > 66% |
| Interlobular inflammation (200x field of view) | No foci of inflammation were seen | Few to 2 foci of inflammation | 2-4 foci of inflammation | More than 4 foci of inflammation |
| Balloon-like degeneration | No balloon-like degeneration was seen | Few cells balloon-like degeneration | Massive balloon-like degeneration of cells | / |

Note: NAS score = steatosis score + interlobular inflammation score + balloon-like degeneration score

Table 9 Liver fibrosis grading scale

| Level | Grade Description |
|---|---|
| 0 | No fibrosis observed |
| 1a | Mild perisinus fibrosis in zone 3 |
| 1b | Moderate sinus fibrosis in zone 3 |
| 1c | Periportal fibrosis |
| 2 | Perisinus and periportal fibrosis |
| 3 | Bridging fibrosis |
| 4 | Cirrhosis |

7.1.6 GE 3.0T MRI Quantitative Analysis of Hepatic Steatosis

The GE 3.0T MRI scanner (750W3T MRI, GE Healthcare) was used for liver imaging in this study. The IDEAL-IQ sequence was employed, which combines the IDEAL (Iterative Decomposition of water and fat with Echo Asymmetry and Least-squares estimation) technique with fast three-dimensional multi-echo imaging. This technique includes multi-echo water-fat separation, region-growing algorithm, as well as various imaging methods for tissue fat quantification and R2* relaxation rate. Six contrast images, including in-phase, opposed-phase, water-only, fat-only, fat fraction maps, and R2* maps, were acquired through a single breath-hold scan by collecting signals from six different echo time (TE) values. The images were reconstructed using computer algorithms to obtain the aforementioned contrast images. The IDEAL-IQ hybrid water-fat separation algorithm consisted of two steps. The first step involved complex domain reconstruction to obtain water and fat images, as well as the T2* map. The second step generated an additional set of estimated water and fat images. These two sets of images were then combined using a hybrid algorithm to generate the final water and fat images.

Prior to conducting the experiment, a Phantom test was performed. Five standard fat solutions with known fat content were prepared by modifying the body phantom preparation method used by Clare P. et al. ( Elbassuoni, E.A. and R.F. Ahmed, Mechanism of the neuroprotective effect of GLP-1 in a rat model of Parkinson's with pre-existing diabetes. Neurochem Int, 2019. 131: p. 104583 ) . Pure water was used as 0% fat. Sodium dodecyl sulfate (60 mmol) was dissolved in 1 L of deionized water, heated to 50°C, and then 40 g of gelatin was added and mixed thoroughly. Soybean oil (96, 108, 114, 117, and 120 ml, obtained from China National Pharmaceutical Group Chemical Reagent Co., Ltd.) was mixed with 0, 24, 12, 6, and 3 ml of the aforementioned solution, respectively, to prepare solutions with fat contents of 0%, 2.5%, 5%, 10%, 20%, and 100%. These solutions were filled into six 100 ml EP tubes for subsequent scanning procedures.

Phantom validation experiments were performed before each scan. All animals underwent a baseline scan and one additional scan after the completion of the drug administration, totaling two scans. The scans were conducted in the 3.0T MRI room, and the scan parameters are provided in Table 10. The anesthesia procedure was as follows: intramuscular injection of 10 mg/kg ketamine hydrochloride, endotracheal intubation, and maintenance of anesthesia with isoflurane and oxygen using a ventilator. During the scanning process, veterinary monitoring of electrocardiography, blood oxygenation, respiratory rate, etc., was performed, and veterinary supervision was provided until the animals fully recovered consciousness and resumed spontaneous respiration.

Table 10: MRI scan parameters of the liver

| Features | No. | Sequence Name | Required level |
|---|---|---|---|
| Positioning like | 1 | 3-PI Loc SSFSE | NA |
| Water-fat separation | 2 | BH LAVA-Flex | Liver cross-section |
| Fat content | 3 | BH IDEAL IQ | Liver cross-section |

Image Post-processing and Analysis: The collected and stored Hepatic steatosis MRI images of Rhesus monkeys were analyzed using the 750W 3T MRI workstation. The liver MRI images were selected by choosing the planes with the maximum exposure of the right hepatic lobe area (layers 1-3), avoiding large blood vessels, bile ducts, and gallbladder to prevent volume effects. Regions of interest (ROI) were selected, with each ROI having an area of 90-110 $mm^2$. Hepatic steatosis was defined as MRI-PDFF% (Magnetic Resonance Imaging Proton Density Fat Fraction) > 6% (consistent with clinical standards).

7.2 Experimental Results

The in vivo pharmacological study of YN-011 was conducted in NASH Rhesus monkeys.

The evaluated clinical parameters included liver lipid content assessed by MRI-PDFF%, NAFLD Activity Score (NAS), liver fibrosis stage assessed by liver histology, body weight, body mass index (BMI), blood lipid profile, fructose metabolism, other biochemical parameters, and food consumption.

The selected Rhesus monkeys were 11-23 years old, which corresponds to 30-70 years in humans. All Rhesus monkeys were males, with weights ranging from 13.63 to 22.85 kg. They had abnormal lipid metabolism for more than 2 years, MRI-PDFF% ranging from 7.8% to 11.9%, NAS scores of 3 or higher, and fibrosis scores of 0-1c within 6 months. These animals clinically met the definition of NASH.

Fifteen Rhesus monkeys were divided into 3 groups, with 5 monkeys in each group. They received subcutaneous injections of 0 (solvent control, placebo control group), 0.050, or 0.150 mg/kg of YN-011 once a week for 13 weeks (QW). The 0.150 mg/kg YN-011 group received adaptive dosing (0.1 mg/kg for the first dose, followed by 0.150 mg/kg for the remaining 12 doses).

During the study, all Rhesus monkeys received designated treatments, and none of them missed the scheduled biopsy or discontinued the treatment. No significant adverse reactions were observed in any of the treatment groups during the study.

As shown in Table 11, the liver lipid content decreased by approximately 40% after 13 weeks of weekly YN-011 treatment compared to the placebo control group.

To investigate whether YN-011 treatment led to histological improvements in the liver, liver biopsy specimens were collected from all Rhesus monkeys before the first injection and on day 89 after the first injection. Liver slices were stained with HE and Masson stains for histological analysis. The degrees of steatosis, inflammation, hepatocyte ballooning, and fibrosis were scored according to the standards for NAFLD activity and NASH progression. Liver biopsy results showed a reduction in both NAS and liver fibrosis scores in Rhesus monkeys treated with YN-011, with no significant progression of NASH (Table 12).

MRI-PDFF detected liver fat decreased from 9.0% ± 0.9% to 5.0% ± 0.2% in the 50 μ g/kg YN-011 group and from 9.4% ± 1.5% to 5.6% ± 1.5% in the 150 μg/kg YN-011 group. Compared to baseline, the percentage reductions were 43.8% and 39.7%, respectively, at the end of the

treatment. There was no significant difference in MRI-PDFF changes between the 50 μ g/kg and 150 μg/kg YN-011 groups.

Histological analysis showed that in the 50 μg/kg YN-011 group, the average NAS decreased from 3.6 ± 0.5 at baseline to 1.6 ± 0.5 ($P = 0.003$). In the 150 μg/kg YN-011 group, the average NAS decreased from 3.6 ± 0.5 at baseline to 1.4 ± 0.5 ($P < 0.001$). There was no significant difference in NAS between the 50 μg/kg and 150 μg/kg YN-011 groups.

Regarding metabolic measurements, including liver injury biomarkers, lipid profiles, and body weight, YN-011 treatment demonstrated excellent improvements in both the 50 μ g/kg and 150 μg/kg groups.

Table 11: Effects of repeated subcutaneous administration of YN-011 on liver lipid content percentage change in NASH Rhesus monkeys after 13 weeks.

| **Group** | **Average ROI Baseline** (%) | **Mean ROI at day 80 of dosing** (%) | **Change rate (%)** |
|---|---|---|---|
| Placebo | 8.9 ± 1.0 | 9.0 ± 1.1 | **1.9** ± 7.9 |
| YN-011 (0.05 mg/kg) | 9.0 ± 0.9 | 5.0 ± 0.2** | -43.8 ± 7.4## |
| YN-011 (0.15 mg/kg) | 9.4 ± 1.5 | 5.6 ± 1.5** | -39.7 ± 14.2## |
| Note: ROI stands for Region of Interest; ** Compared to baseline, P < 0.01; ## Compared to placebo group, P < 0.01; Change rate = (Current value - Baseline value) / Baseline value × 100%. | | | |

Table 12: Effects of repeated subcutaneous administration of YN-011 on liver NAS and fibrosis scores in NASH Rhesus monkeys.

| Group | Animal number | Baseline | | | | | Day 89 post-injection | | | | |
|---|---|---|---|---|---|---|---|---|---|---|---|
| | | Steatosis | Inflammation | Balloon-like degeneration | NAS Score | Fibrosis score | Steatosis | Inflammation | Balloon-like degeneration | NAS Score | Fibrosis score |
| YN-011 Group 150 μg/kg (n = 5) | 4801 | 2 | 1 | 0 | 3 | 0~1a | 0 | 1 | 0 | 1 | 0~1a |
| | 1261 | 3 | 1 | 0 | 4 | 1b | 0 | 1 | 0 | 1 | 0~1a |
| | 4283 | 2 | 1 | 0 | 3 | 1a | 1 | 0 | 0 | 1 | 1a |
| | 4985 | 2 | 1 | 1 | 4 | 1a | 1 | 1 | 0 | 2 | 1a |
| | 2065 | 2 | 1 | 1 | 4 | 1b | 1 | 1 | 0 | 2 | 1b |
| | Mean + SD | / | / | / | 3.6+ 0.5 | / | / | / | / | 1.4 ± 0.5** | / |

(continued)

| Group | Animal number | Baseline | | | | | Day 89 post-injection | | | | |
|---|---|---|---|---|---|---|---|---|---|---|---|
| | | Steatosis | Inflammation | Balloon-like degeneration | NAS Score | Fibrosis scor e | Steatosis | Inflammation | Balloon-like degeneration | NAS Score | Fibrosis score |
| YN-011 Group 50 μg/kg (n = 5) | 4821 | 1 | 1 | 1 | 3 | 1a | 0 | 1 | 1 | 2 | 1a |
| | 5091 | 1 | 1 | 1 | 3 | 1a | 0 | 1 | 0 | 1 | 0~1 a |
| | 1545 | 1 | 2 | 1 | 4 | 1c | 0 | 1 | 1 | 2 | 0~1 a |
| | 1919 | 2 | 2 | 0 | 4 | 0-1a | 1 | 1 | 0 | 2 | 0~1 a |
| | 157 | 1 | 3 | 0 | 4 | 1c | 0 | 1 | 0 | 1 | 1a |
| | Mean + SD | / | / | / | 3.6 ± 0.5 | / | / | / | / | 1.6 ± 0.5 | / |
| Placebo group (n = 5) | 1477 | 1 | 2 | 0 | 4 | 1a | 2 | 1 | 0 | 4 | 1a |
| | 1493 | 1 | 2 | 0 | 3 | 1a | 1 | 2 | 0 | 3 | 1c |
| | 4779 | 3 | 1 | 0 | 3 | 1a | 3 | 1 | 0 | 3 | 1a |
| | 2043 | 1 | 2 | 0 | 3 | 1b | 1 | 2 | 0 | 3 | 1c |
| | 3581 | 2 | 1 | 0 | 3 | 1c | 2 | 1 | 0 | 3 | 1c |
| | Mean + SD | / | / | / | 3.2 ± 0.4 | / | / | / | / | 3.2 ± 0.4 | / |

Furthermore, compared to baseline, the YN-011 treatment group (Day 7- Day 84) showed a significant decrease in body weight and BMI in NASH Rhesus monkeys. YN-011 treatment also significantly reduced serum low-density lipoprotein cholesterol (LDL-c), total cholesterol (TC), and total triglycerides (TG) at various measurement time points during the treatment period, with 0.15 mg/kg YN-011 displaying more consistent lipid-lowering effects. The levels of high-density lipoprotein cholesterol (HDL-c) in the YN-011 treatment group showed a decreasing trend compared to baseline. These changes in serum lipid profile were attributed to the reduced food consumption in YN-011 treated animals. Throughout the study, plasma fasting plasma glucose (FPG) levels fluctuated within the normal range, and no significant differences were detected between the YN-011 and placebo groups after YN-011 and placebo administration. Plasma fructosamine (FRA), a validated biomarker reflecting average glycemic control over the past 2-3 weeks, showed a decreasing trend in the YN-011 group one week after the first injection and a significant reduction of 4.3% after 12 weeks of treatment with 150 μg/kg YN-011. These results indicate that YN-011 has glucose control efficacy in NASH Rhesus monkeys. The dose-dependent reduction in food consumption observed in animals treated with YN-011 is a normal pharmacological effect of YN-011. In conclusion, these data demonstrate that multiple subcutaneous injections of YN-011 provide significant therapeutic benefits for NASH Rhesus monkeys.

Example 8: In vitro experiments on neurodegenerative diseases

8.1 TNF-α concentration-dependent decrease in the viability of SH-SY5Y neuronal cells

SH-SY5Y neuronal cells were digested using 0.25% trypsin to prepare a single-cell suspension. The single-cell suspension was seeded in a 96-well plate at a density of 10,000 cells per well in 100 μl of medium. The plate was placed in a CO2 incubator overnight (37°C, 5% CO2) to allow cell adhesion. The medium was replaced with medium containing 10% FBS or 5% FBS, and cells were stimulated with 20, 40, 60, 80, or 100 ng/ml TNF-α for 48 hours. Each drug treatment was performed in six replicate wells. After 48 hours of drug treatment, 10 μl of CCK-8 solution was added to each well, and the plate was gently shaken to mix the reagents. The plate was then incubated in a CO2 incubator for 1-4 hours. The absorbance at 450 nm was measured using a microplate reader, and cell viability was calculated using the following

formula:

$$\text{Cell viability (\%)} = [A\ (\text{treated}) - A\ (\text{blank})] / [A\ (\text{control}) - A\ (\text{blank})] \times 100$$

A (treated): OD value of wells containing cells, CCK-8 solution, and drug solution
A (control): OD value of wells containing cells and CCK-8 solution without drug solution
A (blank): OD value of wells without cells

The experimental results (as shown in Figure 9) demonstrated a significant decrease in the viability of SH-SY5Y neurons with increasing concentrations of TNF-α.

8.2 Inhibitory effects of YN-011 and γ-Aminobutyric acid (GABA) on TNF-α-induced decrease in SH-SY5Y cell viability

Please refer to section 8.1 for the experimental procedure, but the drug treatment in this experiment is different from section 8.1. Specifically, the drug treatment in this experiment is as follows: using medium containing 5% FBS, cells were treated with 60 ng/ml TNF-α alone or co-treated with 10, 100, or 500 nM YN-011, or co-treated with 1, 10, or 100 μM GABA for 48 hours. Each drug treatment was performed in six replicate wells.
The experimental results (as shown in Figure 10) revealed a significant decrease in SH-SY5Y cell viability in the presence of 60 ng/ml TNF-α, while treatment with 10, 100, and 500 nM YN-011 or 100 μM GABA significantly enhanced cell viability.

8.3 Significant increase in SH-SY5Y cell viability with the combined use of YN-011 and GABA

Please refer to section 8.1 for the experimental procedure, but the drug treatment in this experiment is different from section 8.1. Specifically, the drug treatment in this experiment is as follows: using medium containing 5% FBS, cells were treated with 60 ng/ml TNF-α alone or co-treated with 100 nM YN-011 and/or 100 μM GABA for 48 hours. Each drug treatment was performed in six replicate wells.
The experimental results (as shown in Figure 11) demonstrated a significant decrease in the viability of SH-SY5Y neurons in the presence of 60 ng/ml TNF-α, while co-treatment with 100 nM YN-011 and 100 μM GABA significantly enhanced cell viability. The combined use of YN-011 and GABA exhibited stronger potency in enhancing cell viability compared to individual use.

8.4 YN-011 reduces TNF-α-induced apoptosis in SH-SY5Y neuronal cells

The experimental procedure is as follows:

1) Digest SH-SY5Y neuronal cells in a 10 cm culture dish using 0.25% trypsin and seed them into a 12-well plate.
2) Place the culture plate in a CO2 incubator for overnight pre-culture (37°C, 5% CO2) to allow cell adhesion.
3) Using medium containing 5% FBS, treat the cells in each well with 60 ng/ml TNF-α alone or co-treated with 10, 100, or 500 nM YN-011. Incubate the cells at 37°C for 48 hours. Each drug treatment is performed in three replicate wells.
4) After 48 hours, discard the supernatant, gently wash the cells once with PBS, and place the cell culture plate on ice. Add 120 μl of RIPA cell lysis buffer (Beyotime P0013B) to each well, including protease and phosphatase inhibitors, and lyse the cells for 15 minutes.
5) Transfer the cell lysate to a 1.5 ml EP tube and centrifuge at 14,000 rpm for 30 minutes.
6) Carefully aspirate the supernatant into a new EP tube, add 5x loading buffer containing β-mercaptoethanol, and boil the samples at 100°C for 10 minutes to denature the proteins.
7) Perform SDS-PAGE gel electrophoresis with 10 μg of protein loaded per lane. Position the gel vertically against the power rack in the electrophoresis tank, ensuring that the concave side of the gel faces the power rack. Two gels can share one power rack. Fix the gel and power rack in the electrophoresis tank as required. Add the electrophoresis buffer to create separate chambers between the two gels and the buffer in the electrophoresis tank. Gently remove the comb from the gel.
8) Electrophoresis: Connect the electrophoresis tank to the power supply using two electrodes, ensuring that the red and black electrode plugs match the corresponding ports. During electrophoresis, use low voltage constant voltage electrophoresis for the upper gel. Turn on the power and adjust the voltage to 80 V (usually takes about 15 minutes). When the bromophenol blue dye enters the lower gel, switch to high voltage constant voltage electrophoresis and

adjust the voltage to 120 V until the bromophenol blue dye reaches the bottom of the gel.
9) Perform wet transfer. The wet transfer sandwich arrangement is as follows:

sponge/filter paper/gel/membrane/filter paper/sponge, tightly arranged. There should be no air bubbles between the gel and membrane, and the orientation of the sandwich should be correct, with the negative electrode toward the protein side of the gel,
migrating toward the positive electrode (membrane). After SDS-PAGE is complete, gently separate the two glass plates of the gel using a razor blade, allowing the gel to rest on one of the glass plates. Use a blade to cut the gel at the boundary between the separating gel and the stacking gel, and cut off a small corner in the upper left corner of the separating gel to mark the sample order. Then carefully transfer the gel into transfer buffer. Cut a PVDF membrane of the same size as the gel, soak it in methanol for 5 seconds. Cut 6 pieces of filter paper of the same size and equilibrate them with the transfer buffer at the same time as the PVDF membrane and the gel for 15 minutes. Place a sponge pad, filter paper, gel, membrane, filter paper, and sponge pad (from bottom to top) on the transfer apparatus, ensuring the exclusion of air bubbles, especially between the membrane and filter paper, gel and membrane, and filter paper and gel. Set the transfer current to a constant current of 200 mA, and the transfer time is approximately 90 minutes.

10) Remove the transferred PVDF membrane, rinse it briefly with TBST, discard TBST, and add 5% milk blocking solution to cover the PVDF membrane. Incubate at room temperature on a rocking shaker for 1 hour (30 rpm).
11) Incubate with primary antibodies. Remove the blocking solution, wash the membrane with TBST three times for 5 minutes each time, and cut the membrane according to the molecular weight. Add the appropriate dilution of primary antibodies (Bcl-2, CST #3498S, 1:1000; Cleaved-caspase3, CST #9661S, 1:1000; Caspase3, CST #9662S, 1:1000; HSP90, Proteintech #13171-1-AP, 1:10000) and incubate overnight at 4°C on a rocking shaker.
12) Wash the membrane. Take out the PVDF membrane incubated overnight and store the antibodies at -20°C. Place the PVDF membrane in TBST solution and quickly rinse it three times, each time for 15 minutes.
13) Incubate with secondary antibodies. Add the corresponding species-specific secondary antibodies (Jackson Lab, 1:10000) prepared in blocking solution to the membrane and incubate at room temperature for 1 hour with slow shaking.
14) Wash the membrane. Remove the secondary antibodies and place the PVDF membrane in TBSTsolution. Quickly rinse it three times, each time for 15 minutes.
15) Perform ECL chemiluminescence. Under dark conditions, prepare fresh developing solution (A solution:B solution = 1:1) according to the instructions of the ECL chemiluminescence kit (Millipore #WBKLS0500). Add the developing solution evenly to the membrane and place it in an imaging instrument for image acquisition.
16) Protein expression analysis. Use Image J software to analyze the grayscale values of western blot bands, and normalize the values to the grayscale value of the reference protein HSP90 to calculate the relative expression level of the target protein.

The experimental results (shown in Figure 12) demonstrate that YN-011 significantly reduces TNF-α-induced apoptosis in SH-SY5Y neuronal cells.

8.5 GABA reduces TNF-α-induced apoptosis in SH-SY5Y neuronal cells

Please refer to section 8.4 for the experimental procedure, but the drug treatment in step 3) of this experiment is different from that in section 8.4. In this experiment, the drug treatment in step 3) is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.
The experimental results (shown in Figure 13) demonstrate that GABA reduces TNF-α-induced apoptosis in SH-SY5Y neuronal cells.

8.6 Reducing TNF-α-induced apoptosis in SH-SY5Y neuronal cells by combined use of YN-011 and GABA

Please refer to section 8.4 for the experimental procedure, but the drug treatment in step 3) of this experiment is different from that in section 8.4. In this experiment, the drug treatment in step 3) is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells. The experimental results (shown in Figure 14) demonstrate that the combined use of YN-011 and GABA significantly reduces TNF-α-induced apoptosis in SH-SY5Y neuronal cells.

8.7 TNF-α promotes damage in SH-SY5Y neuronal cells

Digest the SH-SY5Y neuronal cells in a 10 cm culture dish using 0.25% trypsin to prepare a single-cell suspension. Seed the single-cell suspension into a 24-well plate. Place the culture plate in a CO2 incubator and pre-culture overnight at 37°C with 5% CO2 to allow the cells to adhere. Treat the cells in each well with 20, 40, 60, or 80 ng/ml TNF-α using medium containing 5% FBS at 37°C for 48 hours. Each drug treatment is performed in three replicate wells. Add the fluorescent dye Hoechst 33342 (Beyotime) at a dilution of 1:1000 to the wells, and incubate the plate at 37°C for 10 minutes. Remove the culture medium, gently wash twice with PBS, and immediately take the plate to a fluorescence microscope for imaging (20x magnification). Capture 10 fields of view for each cell well and count the number of blue-stained positive cells in each field of view. The experimental results (shown in Figure 15) demonstrate that TNF-α promotes damage in SH-SY5Y neuronal cells.

8.8 Protective effect of YN-011 on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 10, 100, or 500 nM YN-011 at 37°C for 48 hours. Each drug treatment is performed in three replicate wells.

The experimental results (shown in Figure 16) demonstrate that YN-011 has a protective effect on TNF-α-induced damage in neuronal cells.

8.9 Protective effect of GABA on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.

The experimental results (shown in Figure 17) demonstrate that GABA has a protective effect on TNF-α-induced damage in neuronal cells.

8.10 Protective effect of combined use of YN-011 and GABA on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.

The experimental results (shown in Figure 18) demonstrate that the combined use of YN-011 and GABA has a protective effect on TNF-α-induced damage in neuronal cells.

8.11 YN-011 reduces apoptosis in TNF-α-induced neuronal cells

The experimental procedure is as follows:

1) Digest the SH-SY5Y neuronal cells in a 10 cm culture dish using 0.25% trypsin to prepare a single-cell suspension. Seed the single-cell suspension into a 24-well plate, placing a sterile coverslip in each well.
2) Place the culture plate in a CO2 incubator and pre-culture overnight at 37°C with 5% CO2 to allow the cells to adhere.
3) Using medium containing 5% FBS, treat the cells in each well with 60 ng/ml TNF-α alone or co-treated with 10, 100, or 500 nM YN-011 at 37°C for 48 hours. Each drug treatment is performed in three replicate wells.
4) After 48 hours, remove the culture medium and add 400 μl of 4% paraformaldehyde (PFA) to each well for fixation at room temperature for 15 minutes.
5) Wash twice with PBS for 5 minutes each.
6) Add 0.1% Triton X-100 for membrane permeabilization at room temperature for 10 minutes.
7) Wash twice with PBS for 5 minutes each.
8) Add 10% goat serum (GS) and incubate at room temperature for 30 minutes for blocking.
9) Remove the 10% GS and add the primary antibody Cleaved-caspase 3 (CST 9661S) diluted in 1% GS at a dilution ratio of 1:400, with 30 μl of antibody solution per coverslip.
10) Place in a refrigerator at 4°C overnight.

11) On the following day, remove the primary antibody, wash three times with PBS for 5 minutes each.
12) Add the fluorescent secondary antibody (Alexa Fluor® 488 Conjugate) and incubate at room temperature in the dark for 1 hour.
13) Wash three times with PBS for 5 minutes each.
14) Stain with DAPI for 5 minutes, then wash three times with PBS for 5 minutes each.
15) Apply mounting medium onto a clean glass slide, gently place the coverslip with the cells upside down on the mounting medium, and let it dry at room temperature in a cool, ventilated area overnight.
16) Capture images under a fluorescence microscope (40x magnification).
17) Capture 10 fields of view for each treatment group and count the number of green-stained positive cells in each field of view.

The experimental results (shown in Figure 19) demonstrate that YN-011 significantly reduces apoptosis in TNF-α-induced neuronal cells.

8.12 GABA reduces apoptosis in TNF-α-induced neuronal cells

Please refer to section 8.11 for the experimental procedure, but the drug treatment in this experiment is different from that in section 8.11. In this experiment, the drug treatment in step 3) is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.
The experimental results (shown in Figure 20) demonstrate that GABA reduces apoptosis in TNF-α-induced neuronal cells.

8.13 Combined use of YN-011 and GABA reduces apoptosis in TNF-α-induced neuronal cells

Please refer to section 8.11 for the experimental procedure, but the drug treatment in this experiment is different from that in section 8.11. In this experiment, the drug treatment in step 3) is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.
The experimental results (shown in Figure 21) demonstrate that the combined use of YN-011 and GABA significantly reduces apoptosis in TNF-α-induced neuronal cells.

8.14 YN-011 reduces apoptosis in TNF-α-induced neuronal cells

Digest the SH-SY5Y neuronal cells in a 10 cm culture dish using 0.25% trypsin to prepare a single-cell suspension. Seed the single-cell suspension into a 12-well plate.
Place the culture plate in a CO2 incubator and pre-culture overnight at 37°C with 5% CO2 to allow the cells to adhere. Using medium containing 5% FBS, treat the cells in each well with 60 ng/ml TNF-α alone or co-treated with 10, 100, or 500 nM YN-011 at 37°C for 48 hours. Each drug treatment is performed in three replicate wells. Collect the cells and digest the cells with 0.25% trypsin without EDTA for 1 minute. Transfer the cell suspension to a 1.5 ml EP tube, centrifuge at 1000 rpm for 5 minutes, and pellet the cells at the bottom of the tube. Resuspend 105 cells in 100 μl Binding Buffer, add 5 μl of PI and 5 μl of Annexin V-FITC solution, and incubate at room temperature in the dark for 15 minutes. Add 400 μl of Binding Buffer to each tube, and detect using a flow cytometer (Annexin V-FITC excitation wavelength: 488 nm, emission wavelength: 520 nm; PI excitation wavelength: 535 nm, emission wavelength: 617 nm). Count the number of Annexin V-FITC positive cells and PI negative cells in each sample.
The experimental results (shown in Figure 22) demonstrate that YN-011 significantly reduces apoptosis in TNF-α-induced neuronal cells.

8.15 GABA reduces apoptosis in TNF-α-induced neuronal cells

Please refer to section 8.14 for the experimental procedure, but the drug treatment in this experiment is different from that in section 8.14. In this experiment, the drug treatment is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.
The experimental results (shown in Figure 23) demonstrate that GABA reduces apoptosis in TNF-α-induced neuronal cells.

8.16 Combined use of YN-011 and GABA reduces apoptosis in TNF-α-induced neuronal cells

Please refer to section 8.14 for the experimental procedure, but the drug treatment in this experiment is different from that in section 8.14. In this experiment, the drug treatment is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.

The experimental results (shown in Figure 24) demonstrate that the combined use of YN-011 and GABA significantly reduces apoptosis in TNF-α-induced neuronal cells.

8.17 GABA reduces mRNA expression of inflammatory factors in HMC3 microglial cells induced by Aβ1-42 oligomers

Under sterile conditions, dissolve the synthesized Aβ1-42 peptide (AnaSpec #AS-20276) in hexafluoroisopropanol (Mecklin #H811026) to obtain a 1 mM solution. Divide the solution into 1.5 ml sterile centrifuge tubes and evaporate the hexafluoroisopropanol in a vacuum evaporator. The dried peptide forms a film in the tubes and is stored at -20°C for later use. Freshly prepare Aβ1-42 oligomers from the dried peptide film as follows:
dissolve the dried peptide film in anhydrous DMSO to obtain a 5 mM solution, then dilute with cold cell culture basal medium to obtain a 100 μM stock solution. After incubating at 4°C for 24 hours, use the stock solution for cell treatment.

Seed HMC3 human microglial cells into a 24-well plate. After overnight incubation at 37° C with 5% CO2, treat the cells in each well with 10 μM Aβ1-42 oligomers alone or co-treated with 1, 10, or 100 μM GABA for 48 hours. Each drug treatment is performed in three replicate wells. After 48 hours of treatment, isolate total RNA from the cells in each well using 1 ml of Trizol (Invitrogen #15596026) following the manufacturer's instructions. Dissolve the RNA pellet in 20 μl of DEPC-treated water. Measure the RNA concentration and purity using a NanoDrop 2000. Convert RNA into cDNA using a reverse transcription kit (Yeasen #11141ES60). Prepare the PCR reaction as follows: 5 μl of PCR mix (Yeasen #10108ES03), 0.2 μl of forward primer, 0.2 μl of reverse primer, 1 μl of cDNA, and 3.6 μl of water. Perform qPCR on an Applied Biosystems 7500 real-time PCR system using the following thermal program: initial denaturation at 95°C for 3 minutes, followed by 40 cycles of denaturation at 95°C for 30 seconds, annealing at 60°C for 30 seconds, and extension at 72°C for 1 minute. Compare the relative mRNA expression of TNF-α and IL-6 between treated and untreated cells using the ΔΔCt method, normalized to the expression of the housekeeping gene RPLP0.

The experimental results (shown in Figure 25) demonstrate that in the presence of 10 μM Aβ1-42 oligomers, the mRNA expression of TNF-α and IL-6 in HMC3 cells is significantly increased, while 100 μM GABA significantly reduces the mRNA expression of TNF-α, and both 10 μM and 100 μM GABA significantly reduce the mRNA expression of IL-6. 8.18 YN-011 reduces mRNA expression of inflammatory factors in HMC3 microglial cells induced by Aβ1-42 oligomers.

Please refer to section 8.17 for the experimental procedure, but the drug treatment in this experiment is different from that in section 8.17. In this experiment, the drug treatment is as follows: treat the cells in each well with 10 μM Aβ1-42 oligomers alone or co-treated with 10, 100, or 500 nM YN-011 for 48 hours. Each drug treatment is performed in three replicate wells.

The experimental results (shown in Figure 26) demonstrate that in the presence of 10 μM Aβ1-42 oligomers, the mRNA expression of TNF-α and IL-6 in HMC3 cells is significantly increased, while 100 nM and 500 nM YN-011 significantly reduce the mRNA expression of TNF-α and IL-6.

8.19 Combined use of YN-011 and GABA reduces mRNA expression of inflammatory factors in HMC3 microglial cells induced by Aβ1-42 oligomers.

Please refer to section 8.17 for the experimental procedure, but the drug treatment in this experiment is different from that in section 8.17. In this experiment, the drug treatment is as follows: treat the cells in each well with 10 μM Aβ1-42 oligomers alone or co-treated with 100 μM GABA and/or 100 nM YN-011 for 48 hours. Each drug treatment is performed in three replicate wells.

The experimental results (shown in Figure 27) demonstrate that compared to the individual treatment of GABA or YN-011, the combined use of 100 μM GABA and 100 nM YN-011 exhibits significantly stronger inhibitory effects on the TNF-α mRNA expression induced by 10 μM Aβ1-42 oligomers.

8.20 GABA reduces inflammatory factor expression in HMC3 microglial cells induced by Aβ1-42 oligomers.

Seed HMC3 human microglial cells into a 12-well plate. After overnight incubation at 37° C with 5% CO2, treat the cells in each well with 10 μM Aβ1-42 oligomers alone or co-treated with 1, 10, or 100 μM GABA for 48 hours. Each drug treatment is performed in three replicate wells. After 48 hours of treatment, wash the cells with PBS and lyse the cells in RIPA buffer supplemented with protease inhibitor and phosphatase inhibitor on ice for 15 minutes. Centrifuge the cell lysate at 14,000 rpm for 30 minutes. Collect the supernatant, mix with 5x loading buffer supplemented with β-mercaptoethanol, and boil at 100°C for 10 minutes. Separate denatured proteins by SDS-PAGE. Briefly, load 10 μg of protein onto precast mini polyacrylamide gels (SurePAGE, GenScript #M00657) and run at 120 V voltage. Subsequently, transfer the gel onto a PVDF membrane (0.22 μm pore size) at 200 mA for 90 minutes. After unblocking, incubate the membrane at room temperature with primary antibodies diluted in 5% milk solution (TNF-α, Proteintech #60291-Ig; IL-6, Proteintech #21865-1-AP; HSP90, Proteintech #13171-1-AP) overnight at 4°C. After washing, incubate the membrane with corresponding secondary antibodies (Jackson Lab, 1:10000) at room temperature for 1 hour. After washing, visualize the antibody signals on the membrane using an ECL kit (Millipore #WBKLS0500) following the manufacturer's instructions. Calculate the relative protein levels normalized to the endogenous protein HSP90 using ImageJ.

The results (shown in Figure 28) demonstrate that in the presence of 10 μM Aβ1-42 oligomers, the expression of TNF-α and IL-6 in HMC3 cells is significantly increased, while 100 μM GABA significantly reduces IL-6 expression, and both 10 μM and 100 μM GABA significantly reduce TNF-α expression.

8.21 YN-011 reduces inflammatory factor expression in HMC3 microglial cells induced by Aβ1-42 oligomers.

Please refer to section 8.20 for the experimental procedure, but the drug treatment in this experiment is different from that in section 8.20. In this experiment, the drug treatment is as follows: treat the cells in each well with 10 μM Aβ1-42 oligomers alone or co-treated with 10, 100, or 500 nM YN-011 for 48 hours. Each drug treatment is performed in three replicate wells.

The experimental results (shown in Figure 29) demonstrate that in the presence of 10 μM Aβ1-42 oligomers, the expression of TNF-α and IL-6 in HMC3 cells is significantly increased, while 100 nM and 500 nM YN-011 significantly reduce the expression of TNF-α and IL-6.

8.22 Combined use of YN-011 and GABA reduces inflammatory factor expression in HMC3 microglial cells induced by Aβ1-42 oligomers.

Please refer to section 8.20 for the experimental procedure, but the drug treatment in this experiment is different from that in section 8.20. In this experiment, the drug treatment is as follows: treat the cells in each well with 10 μM Aβ1-42 oligomers alone or co-treated with 100 μM GABA and/or 100 nM YN-011 for 48 hours. Each drug treatment is performed in three replicate wells.

The experimental results (shown in Figure 30) demonstrate that compared to the individual treatment of GABA or YN-011, the combined use of 100 μM GABA and 100 nM YN-011 exhibits significantly stronger inhibitory effects on the expression of TNF-α and IL-6 induced by 10 μM Aβ1-42 oligomers.

Example 9: Neurodegenerative In Vivo Disease

Neurodegenerative diseases such as Alzheimer's disease (AD), Parkinson's disease (PD), Huntington's disease (HD), etc., are central nervous system disorders characterized by progressive loss of function in central neurons or their myelin sheaths, resulting in gradual loss of neuronal cell structure and function, leading to symptoms such as dementia and movement disorders.

Among them, Alzheimer's disease (AD), also known as senile dementia, is characterized by progressive cognitive impairment and memory loss, with no effective treatment currently available. Studies have shown that AD patients' brains accumulate neurotoxic plaques formed by abnormal aggregation of beta-amyloid (Aβ). Aβ can cause the formation of senile plaques in the brain and apoptosis of nerve cells, making it a crucial factor in the development of AD. Aβ is a peptide consisting of 39-43 amino acids, and the most common subtypes of Aβ in the human body are Aβ40 and Aβ42, which are prone to aggregation, leading to the formation of Aβ deposits and subsequent neurotoxic effects.

Research has shown that GLP-1 has neuroprotective biological effects and can improve AD symptoms ( Zheng, J., et al., GLP-1 improves the supportive ability of astrocytes to neurons by promoting aerobic glycolysis in Alzheimer's disease. Mol Metab, 2021. 47: p. 101180 ; Park, J.S., et al., Blocking microglial activation of reactive astrocytes is neuroprotective in models of Alzheimer's disease. Acta Neuropathol Commun, 2021. 9(1): p. 78 ) . In animal models, GLP-1 improves cognitive function in AD mice, reduces Aβ deposition, alleviates glial cell overactivation induced by Aβ, and mitigates oxidative stress and inflammation in the brain, demonstrating the neuroprotective role of GLP-1 in AD. The present invention discloses a long-acting GLP-1 receptor agonist (GLP-1RA) that exerts the biological effect of protecting neurons and improving central nervous system function, thereby helping to improve AD symptoms.

The experiment uses model mice with Alzheimer's disease and wild-type control adult mice of the same genetic background. The animals are individually housed in cages, maintaining a 12/12-hour light/dark cycle (lights on at 08:00, lights off at 20:00), and the temperature is kept at 21.5 degrees Celsius. Food and water are available ad libitum. Prior to the behavioral experiments, the mice are intraperitoneally (i.p.) injected with YN-011 or a placebo for 16 weeks.

Morris Water Maze Setup

The maze is made of white opaque plastic with a diameter of 120 cm and wall height of 40 cm, and it is filled with water at 25°C to avoid excessive cooling of body temperature. A small escape platform (10 × 6.5 × 21.5 cm) is placed in a fixed position within a quadrant, 25 cm away from the surrounding walls, and hidden 1 cm below the water surface. There are several fixed visual cues on the walls of the room.

Spatial Memory

Four equidistant points (north, south, east, west) along the circumference of the pool serve as starting positions. The starting positions are randomly assigned for training in four trials per day. During training, the mice are trained to find a fixed safety platform submerged in the pool. After 24 hours following the end of the training period, the safety platform is removed from the pool, and the mice's swimming path length and time are recorded, starting from a random starting position (n = 12 per group). Data collected are analyzed using one-way ANOVA and two-way ANOVA to evaluate spatial memory, represented by the time and path length required to reach the original position of the safety platform, and spatial acuity, represented by the time spent searching in the area where the safety platform was located.

Detection of Aβ40 and Aβ42 Levels

Aβ40 and Aβ42 levels are measured using an Aβ detection kit. In brief, the hemispheres of the brains from AD model mice treated with YN-011 and control mice are homogenized in Tris-buffered saline (25 mM Tris HCl, pH 7.4; 150 mM NaCl) containing a proteinase inhibitor (Sigma, 250 ml/5 ml buffer). The brain homogenates are then centrifuged at 100,000 g and 4°C for 1 hour. The supernatant is then diluted 1:10, and ELISA is performed. The ELISA measures only soluble beta-amyloid oligomer levels and not monomers. Protein quantification is performed using the Bradford protein assay. The final Aβ values are determined after normalization to total protein levels (n = 6 per group).

Example 10: Phase IIb and III Clinical Trials of YN-011 (Monotherapy)

10.1 Trial Design

The Phase IIb and III clinical trials are multicenter, randomized, double-blind, placebo-controlled studies conducted to evaluate the efficacy and safety of YN-011 in patients with poorly controlled type 2 diabetes mellitus (T2DM) despite dietary and exercise interventions. The Phase IIb stage is an exploratory clinical trial to investigate dose response and safety assessment (divided into four groups: YN-011 1 mg group, YN-011 2 mg group, YN-011 3 mg group, and placebo control group), aiming to obtain the Recommended Phase 3 Dose (RP3D) of YN-011 for the Phase III stage. The Phase III stage is a confirmatory clinical trial of efficacy.. Key Inclusion Criteria for Phase IIb and III Studies:

- Age between 18 and 75 years at screening
- Diagnosed with type 2 diabetes for at least 8 weeks (according to WHO 2000 criteria) and not receiving any antihyperglycemic drug treatment in the 8 weeks before screening
- Glycated hemoglobin HbA1c: 7.5% ≤ HbA1c ≤ 11% at screening
- Glycated hemoglobin HbA1c: 7.5% ≤ HbA1c ≤ 10.5% before randomization
- Fasting plasma glucose (FPG) < 13.9 mmol/L at screening and before randomization
- BMI ≥ 18.5 kg/m2 and ≤ 40 kg/m2

Key Exclusion Criteria for Phase IIb and III Studies:

- Type 1 diabetes
- Use of any DPP-4 inhibitors and/or GLP-1 analogs in the 3 months before screening
- Continuous insulin treatment for more than 14 days in the year before screening (excluding gestational diabetes requiring insulin treatment)
- Fasting C-peptide < 0.3 nmol/mL
- Diabetic ketoacidosis, diabetic lactic acidosis, or hyperosmolar non-ketotic diabetic coma in the 6 months before screening
- Unstable or requiring treatment for proliferative retinopathy or maculopathy, severe diabetic neuropathy, intermittent claudication, or diabetic foot in the 6 months before screening
- Severe hypoglycemic event without apparent cause (Grade 3 hypoglycemia) in the 6 months before screening, or 3 or more hypoglycemic events (blood glucose < 3.9 mmol/L) in the month before screening, or recurrent symptoms

related to hypoglycemia

- Serious trauma, severe infection, or surgery within the month before screening that may affect glycemic control
- Blood donation or significant blood loss (>400 mL) or blood transfusion in the past 3 months
- Uncontrolled hypertension
- Patients with a history of acute or chronic pancreatitis, symptomatic gallbladder disease, pancreatic injury, or other high-risk factors for pancreatitis, or patients with amylase and/or lipase ≥ 1.5 times the upper limit of normal (ULN) at screening
- History of medullary thyroid carcinoma, multiple endocrine neoplasia (MEN) 2A or 2B syndrome, or relevant family history; or history of other malignant tumors
- Clinically significant gastric emptying abnormalities, severe chronic gastrointestinal disease, long-term use of medications directly affecting gastrointestinal motility, or gastrointestinal surgery within the 6 months before screening, as determined by the investigator
- Blood disorders or any condition causing hemolysis or red blood cell instability
- Uncontrolled hyperthyroidism or hypothyroidism
- Positive for hepatitis B surface antigen (HBsAg) with hepatitis B virus load (HBV-DNA) higher than the local laboratory detection limit, positive for hepatitis C antibody (HCV-Ab), human immunodeficiency virus antibody (HIV-Ab), treponema pallidum antibody (TP-Ab), or positive for novel coronavirus pneumonia (COVID-19) nucleic acid test
- Acute or chronic hepatitis or laboratory findings meeting any of the following criteria: alanine aminotransferase (ALT) level ≥ 2.5 x ULN and/or aspartate aminotransferase (AST) ≥ 2.5 x ULN, fasting triglycerides > 5.7 mmol/L, estimated glomerular filtration rate (eGFR) calculated by the CKD-EPI (EPI-(Scr)) equation < 60 mL/min/1.73 m2
- Any other condition that the investigator or primary physician deems unsuitable for participation in the study

The treatment regimen for the Phase IIb stage dose confirmation is as follows for enrolled subjects:

Subjects Enrolled in the Phase IIb Efficacy Confirmation Stage:

<table>
<tr><th rowspan="3">Group</th><th colspan="3">Treatment (subcutaneous abdominal injection, 1 time/week, 52 weeks of continuous dosing)</th></tr>
<tr><th>Double-blind treatment period (first 12 weeks)</th><th>Double-blind treatment period (post 12 weeks)</th><th rowspan="2">Open treatment period (28 weeks)</th></tr>
<tr><th colspan="2">24 weeks in total</th></tr>
<tr><td>Dose Group 1</td><td>YN-011 1mg</td><td>YN-011 1mg</td><td rowspan="3">Subjects entering open treatment prior to RP3D determination: YN-011 (1mg)<br>Subjects entering the open treatment period after RP3D determination:<br>· YN-011 dose group selected by IDMC (RP3D low dose group or high dose group): maintain the original dose treatment<br>· Other subjects were randomized twice in a 1:1 ratio to receive either low or high dose of YN-011 RP3D</td></tr>
<tr><td>Dose Group 2</td><td>YN-011 2mg</td><td>YN-011 2mg</td></tr>
<tr><td>Dose Group 3</td><td>YN-011 3mg</td><td>YN-011 3mg</td></tr>
<tr><td>Control Group</td><td>Placebo</td><td>Placebo</td><td>Subjects entering open treatment prior to RP3D determination: YN-011 (1mg)<br>Subjects entering the open treatment period after RP3D determination: secondary randomization in a 1:1 ratio to receive either low or high dose of YN-011 RP3D</td></tr>
</table>

Subjects Enrolled in the Phase III Efficacy Confirmation Stage:

| Group | Treatment (subcutaneous abdominal injection, 1 time/week, 52 weeks of continuous dosing) | |
|---|---|---|
| | Double-blind treatment period (24 weeks) | Open treatment period (28 weeks) |
| Dose Group 1 | YN-011 RP3D low dose, open treatment period to maintain the original dose treatment | |
| Dose Group 2 | YN-011 RP3D high dose, open treatment period to maintain the original dose treatment | |
| Control Group | Placebo | Secondary randomization in a 1:1 ratio to receive either low or high dose of YN-011 RP3D |

Primary efficacy endpoints included the comparison of YN-011 with placebo in terms of the change in HbA1c levels from baseline after 12 weeks (Phase IIb) or 24 weeks (Phase III) of double-blind administration in patients with T2DM. Secondary efficacy endpoints mainly included changes from baseline (at 12 weeks for Phase IIb and 24 weeks for Phase III) in FPG, fasting insulin, fasting C-peptide, fasting glucagon, fasting lipid profile, and fasting body weight. Other secondary endpoints included HbA1c target achievement rate (proportion of subjects with HbA1c <7.0% and <6.5%), glucose area under the curve during mixed meal tolerance test (MMTT), and insulin or C-peptide area under the curve during MMTT. Safety assessments included adverse events, laboratory tests, vital signs, and 12-lead electrocardiogram evaluation.

10.2 Treatment Effect of YN-011 in Type 2 Diabetes

Clinical trial results showed a reduction of 1.73% in HbA1c levels after 24 weeks of treatment with 1 mg YN-011. It has been reported that semaglutide at a dose of 1 mg reduced HbA1c levels by 1.55% after 30 weeks of treatment (Sorli et al., Lancet Diabetes Endocrinol 2017; 5: 251-60), and dulaglutide at a dose of 1.5 mg reduced HbA1c levels by 1.46% after 26 weeks of treatment (Shi et al., J Diabetes Investig 2020; 11: 142-150). urthermore, clinical trial results also showed that the incidence of hypoglycemia (<3.9 mmol/L) after 24 weeks of treatment with 1 mg and 3 mg YN-011 was 0.8% and 1.7%, respectively. It has been reported that the incidence of hypoglycemia after 26 weeks of treatment with dulaglutide at doses of 0.75 mg and 1.5 mg was 4.1% and 6.3%, respectively (Shi et al., J Diabetes Investig 2020; 11: 142-150).

Moreover, the incidence of nausea after 24 weeks of treatment with 1 mg and 3 mg YN-011 was 3.4% and 6.0%, respectively. It has been reported that the incidence of nausea after 30 weeks of treatment with semaglutide at doses of 0.5 mg and 1 mg was 20% and 24%, respectively (Sorli et al., Lancet Diabetes Endocrinol 2017; 5: 251-60), and the incidence of nausea after 26 weeks of treatment with dulaglutide at a dose of 1.5 mg was 9.5% (Shi et al., J Diabetes Investig 2020; 11: 142-150). For liraglutide, the incidence of nausea after 24 weeks of treatment with 1 mg and 3 mg doses was 5.6% and 10%, respectively (Shuai et al., Diabetes Obes Metab. 2021; 23(1):116-124).

Example 11: Phase IIb and III Clinical Trials of YN-011 in Combination with Metformin 11.1 Trial Design

The Phase IIb and III clinical trials were multicenter, randomized, double-blind, placebo-controlled studies evaluating the efficacy and safety of YN-011 in patients with T2DM who had inadequate glycemic control on metformin therapy. The Phase IIb trial aimed to explore dose response and evaluate safety (divided into three groups: YN-011 1 mg + metformin, YN-011 3 mg + metformin, and placebo + metformin), to determine the Recommended Phase 3 Dose (RP3D) for the Phase III trial, which aimed at efficacy confirmation..

The inclusion criteria for subjects in the Phase IIb and III studies of YN-011 in combination with metformin were primarily the same as those in Implementation Example 10, except for the following point:

- Diagnosed with type 2 diabetes for at least 8 weeks (WHO 2000), and met one of the following conditions:

    a) Received monotherapy with metformin for ≥8 weeks with a dose of ≥1500 mg/day or maximum tolerated dose (<1500 mg/day but ≥1000 mg/day) (eligible subjects could directly enter the run-in period).

    b) Received monotherapy with metformin for <8 weeks with a dose of ≥1500 mg/day or maximum tolerated dose (<1500 mg/day but ≥1000 mg/day) (eligible subjects needed to enter the metformin dose stabilization period).

    c) Received monotherapy with metformin at a dose <1500 mg/day and had not reached the maximum tolerated dose (eligible subjects needed to enter the metformin dose titration period and dose stabilization period).

Please refer to the exclusion criteria in Implementation Example 10 for the primary exclusion criteria for subjects in the Phase IIb and III studies of YN-011 in combination with metformin.

The treatment regimen for subjects enrolled in the Phase IIb dose confirmation stage is as follows:

Subjects enrolled in the Phase IIb dose confirmation stage:

| Group | Treatment (subcutaneous abdominal injection, 1 time/week, 52 weeks of continuous dosing) | | |
| --- | --- | --- | --- |
| | Double-blind treatment period (first 12 weeks) | Double-blind treatment period (post 12 weeks) | Open treatment period (28 weeks) |
| | 24 weeks in total | | |
| Dose Group 1 | YN-011 1mg | YN-011 1mg | Before the completion of the interim analysis: YN-011 1mg<br>After completion of interim analysis: treated with YN-011 RP3D dose |
| Dose Group 2 | YN-011 3mg | YN-011 3mg | |
| Control Group | Placebo | Placebo | |

Subjects enrolled in the Phase III dose confirmation stage:

| Group | Treatment (subcutaneous abdominal injection, 1 time/week, 52 weeks of continuous dosing) | |
| --- | --- | --- |
| | Double-blind treatment period (24 weeks) | Open treatment period (28 weeks) |
| YN-011 Group | YN-011 RP3D dose, open treatment period to maintain the original dose treatment | |
| Control Group | Placebo | YN-011 RP3Dose treatment |

Primary efficacy endpoints included the comparison of YN-011 in combination with metformin versus placebo in combination with metformin in terms of the change in HbA1c levels from baseline after 12 weeks (Phase IIb) or 24 weeks (Phase III) of double-blind administration in patients with T2DM who had inadequate glycemic control on metformin therapy. Please refer to Implementation Example 10 for the secondary efficacy endpoints and safety assessments.

11.2 Treatment Effect of YN-011 in Combination with Metformin in Type 2 Diabetes

Clinical trial results showed a reduction of 1.8% in HbA1c levels and 2.42% in FPG levels after 24 weeks of treatment with 3 mg YN-011 in combination with metformin. It has been reported that dulaglutide at a dose of 1.5 mg in combination with metformin reduced HbA1c levels by 1.42% and FPG levels by 1.93% after 40 weeks of treatment (Dungan et al., Lancet 2014; 384: 1349-57).

Furthermore, clinical trial results also showed that the incidence of hypoglycemia (<3.9 mmol/L) after 24 weeks of treatment with 3 mg YN-011 in combination with metformin was 1.8%, which is comparable to the incidence of hypoglycemia (1.7%) in the placebo group in combination with metformin. It has been reported that the incidence of hypoglycemia after 40 weeks of treatment with dulaglutide at a dose of 1.5 mg in combination with metformin was 9% (Dungan et al., Lancet 2014; 384: 1349-57). Moreover, the incidence of nausea after 24 weeks of treatment with 3 mg YN-011 in combination with metformin was 7.0%. It has been reported that the incidence of nausea after 40 weeks of treatment with dulaglutide at a dose of 1.5 mg in combination with metformin was 20% (Dungan et al., Lancet 2014; 384: 1349-57), and the incidence of nausea after 30 weeks of treatment with semaglutide at a dose of 1 mg in combination with metformin was 13.4% (Ji et al., Diabetes Obes Metab. 2021; 23:404-414).

Example 12: Screening of pharmaceutical preparation of GLP-1 fusion protein

In this example, the inventors purified the tissue culture supernatant containing GLP-1 fusion protein (YN-011) obtained in Example 1, and then prepared different formulations with excipients of different types and concentrations, and tested each formulation to screen out the preferred YN-011 formulation.

12.1 Detection method

12.1.1 Appearance

Using the visual method, wipe the sample bottle clean, and observe the color, clarity and visible particles of the sample in front of the black background and white background of the clarity detector under the light intensity of 1500 lx.

12.1.2 pH value

Using the potentiometric method, calibrate the pH meter with the standard solution, and take 50 to 100 μl of sample to determine the pH value.

12.1.3 Protein content

Using the ultraviolet method, use a pipette to take 2.5μl of sample, and then use Nano Drop 2000 to measure the sample absorption at a wavelength of 280 nm and calculate the sample concentration. Repeat the measurement twice and take the average as the final result.

12.1.4 Turbidity (A350)

Since protein has no absorption peak at 350nm, the absorbance value of the sample in this wavelength range can reflect the turbidity of the sample itself. Therefore, the A350 test result is used as the basis for turbidity comparison. Use a pipette to draw 100μl of sample into a 96-well plate, and then use a SpectraMax M5e microplate reader to detect the absorbance value of the sample at 350 nm.

12.1.5 Molecular Exclusion Chromatography Purity (SEC-HPLC)

Molecular sieve gel chromatography was used. During the formulation development experiment stage, this test method used the Agilent system, TSK G3000SWXL gel column (5 μm, 7.8 × 300 mm, 25°C), injection temperature of 5°C, column temperature of 25°C, and detection wavelength of 280 nm. The mobile phase composition is 50 mM sodium phosphate, 300 mM sodium chloride, pH 7.0 ± 0.2, isocratic elution, flow rate is 1.0 mL/min. The sample concentration is 3 mg/mL, and the standard is diluted to 3 mg/mL with the mobile phase, which is consistent with the sample concentration. The injection volume is 20 μL.

12.1.6 Reverse Phase Liquid Chromatography (RP-LC)

Reverse phase high performance liquid chromatography is based on the interaction strength between the protein molecules after reduction denaturation and the C8 reverse phase column for separation. The solvent on the surface of the stationary phase flows at a certain flow rate, and the molecules with high polarity are first to elute, while the molecules with low polarity interact more strongly with the C8 column and elute later. After separation, various substances are detected by UV detector.

12.1.7 Non-reducing chip gel electrophoresis (SDS_CALIPER_NR)

Use the sample buffer master solution in the kit (purchased from SCIEX), 10% SDS and 100 mM N-Ethylmaleimide in a volume ratio of 20:1:0.7 to prepare the denaturation solution (prepared and used immediately); at the same time, dilute the reference and sample with water to 1 mg/mL. Take 2μL of sample dilution and 7μL of denaturation solution to a new 1.5 mL EP tube, mix well and heat at 70°C for 10 mins (heat the ladder directly at 70°C without adding denaturation solution). After instant centrifugation, add 35μL of water (12μL ladder plus 120 μL of water), mix well with an oscillator, and the sample preparation is complete. Take 42μL of the prepared sample and transfer it to a 96-well plate, centrifuge at 4000 rpm for 20 mins. Then place the 96-well plate in the plate slot of the instrument (Note: there are two empty spaces in the plate slot for wash buffer and ladder respectively). The sample will complete the steps of sample absorption, staining, separation, decolorization and detection in the chip equipped with destain-gel, gel-dye and maker. The raw data is analyzed using LabChip GX Reviwer.

12.1.8 Capillary focusing isoelectric electrophoresis (cIEF)

In the ProteinSimple iCE3 full-column imaging capillary isoelectric focusing electrophoresis system, the charged

isomers in each sample are focused and separated according to their isoelectric points using fluorine-coated capillaries (ProteinSimple, FC coated cartridge, i.d. 100μm). The sample concentration in the loading solution is 0.3 mg/mL; the temperature of the autosampler is controlled at 15°C; the injection duration is 90 seconds. The voltage and time of the first stage of focusing were 1500 V, 1 min; the voltage and time of the second stage of focusing were 3000 V, 8 min; the detection wavelength was 280 nm. Two markers (pl maker) with known isoelectric points were added to the sample and focused simultaneously to calibrate the isoelectric point of the sample to be tested. Data analysis was performed using Empower (Empower@ 3) or Chrom Perfect Software. The peak area normalization method was used to obtain the main peak content, peak group A (acidic peak) content, and peak group B (alkaline peak) content.

12.2 Buffer system screening

12.2.1 Buffer system screening scheme

Four buffer systems were selected: 10 mM citrate, histidine, phosphate, and acetate, and 10 different buffer systems to be investigated were designed from pH 4.5 to 7.0. See Table 13 for details. The YN-011 stock solution was dialyzed to 10 mM citrate at pH 6.2, 10 mM histidine at pH 6.0, 10 mM phosphate at pH 6.5, and 10 mM acetate at pH 5.0. The pH was adjusted by adding acid or alkali (such as HCl/NaOH, etc.) to obtain the remaining 6 pH prescriptions, and then the protein concentration was adjusted to 3 mg/mL. Take 2 mL of sample and fill it into 5 mL neutral borosilicate glass tube injection bottle, stopper and cap it for investigation. Place it under two different investigation conditions of 25°C and 40°C for 4 weeks, take samples at different time points for various tests, and evaluate the protein stability in 10 buffer systems. The buffer system scheme is shown in Table 13.

Table 13. Buffer System Solutions

| Sample No. | Buffer system | pH |
|---|---|---|
| 01 | Citric acid/sodium citrate 10 mM | 6.2 |
| 02 | Histidine/Histidine HCl 10 mM | 5.5 |
| 03 | | 6.0 |
| 04 | | 6.5 |
| 05 | disodium hydrogen phosphate /sodium dihydrogen phosphate 10 mM | 6.0 |
| 06 | | 6.5 |
| 07 | | 7.0 |
| 08 | Acetic acid/sodium acetate 10 mM | 4.5 |
| 09 | | 5.0 |
| 10 | | 5.5 |

12.2.2 Appearance and pH test

Observe the appearance of YN-011 according to the buffer system scheme in Table 13. At 25°C, all YN-011 samples were colorless and slightly opalescent. After 2 weeks, a small amount of fine particles were observed in the samples of the citric acid/sodium citrate buffer system (pH6.2); a large amount of fine particles were observed after 4 weeks. The samples of the three pH values (pH5.5, 6.0, 6.5) of the histidine/histidine hydrochloride buffer system all showed different degrees of particles at 25°C. After 2 weeks, a small amount of fine particles were observed in the pH5.5 sample, while larger granular proteins appeared in the pH6.0 and 6.5 samples, and the number of particles increased over time. The samples of the disodium hydrogen phosphate/sodium dihydrogen phosphate buffer system (pH6.0, 6.5, 7.0) all showed a small amount of particles or floccules at 25°C. Samples of the three pH values (pH4.5, 5.0, 5.5) of the acetic acid/sodium acetate buffer system showed very small amounts of particles after being placed at 25°C for 4 weeks, and the number of particles increased slightly after 4 weeks.

At 40°C, all YN-011 samples were colorless and slightly opalescent. A small amount of flocculent particles were observed in the samples of the citric acid/sodium citrate buffer system (pH6.2) placed at 40°C for 1 week and 2 weeks, and a large amount of fine particles were observed in the samples placed at 4 weeks. Samples of the three pH values (pH5.5, 6.0, 6.5) of the histidine/histidine hydrochloride buffer system showed different degrees of particles at different sampling points at 40°C. A small amount of fine particles were observed in the pH5.5 samples at 40°C for one and two weeks, and in the pH6.0 and pH5.5 samples at 40°C for one week, and the number of particles increased over time. Samples in the

disodium hydrogen phosphate/sodium dihydrogen phosphate buffer system pH6.0 and pH6.5 showed a small amount of particles or floccules at different sampling points at 40°C, while samples in the pH7.0 of the system had no particles at 40°C for one and two weeks, but a small amount of floccules were observed at 4 weeks. Samples of the three pH values (pH4.5, 5.0, 5.5) of the acetic acid/sodium acetate buffer system showed no particles after one week at 40°C, very small amounts of particles appeared in samples of pH5.0 and pH5.5 after two weeks, and a small amount of floccules appeared in samples of the three pH values after four weeks.

According to the buffer system scheme in Table 13, the initial pH of the YN-011 formulation system and the pH stability at different time points were tested. As shown in Table 14, the test results of samples at three pH values (pH 6.0, 6.5, 7.0) of the disodium hydrogen phosphate/sodium dihydrogen phosphate buffer system and samples at three pH values (pH 4.5, 5.0, 5.5) of the acetic acid/sodium acetate buffer system at different sampling points were basically consistent, indicating that their pH was relatively stable. The samples in the citric acid/sodium citrate buffer system (pH 6.2) and the samples in the histidine/histidine hydrochloride buffer system at three pH values (pH 5.5, 6.0, 6.5) increased in pH to varying degrees over time at 25°C and 40°C, indicating that their pH was unstable.

Table 14. pH stability results of buffer system screening

| Sample No. | T0 | 25°C | | 40°C | | |
|---|---|---|---|---|---|---|
| | | 2W | 4W | 1W | 2W | 4W |
| 01 | 6.1 | 6.7 | 8.0 | 6.3 | 6.3 | 8.1 |
| 02 | 5.7 | 6.2 | 7.6 | 6.4 | 7.5 | 7.9 |
| 03 | 6.1 | 6.5 | 8.1 | 6.4 | 7.8 | 7.9 |
| 04 | 6.6 | 6.3 | 7.0 | 6.6 | 8.0 | 6.6 |
| 05 | 6.1 | 5.9 | 6.1 | 6.0 | 5.9 | 6.1 |
| 06 | 6.5 | 6.6 | 6.6 | 6.6 | 6.5 | 6.5 |
| 07 | 7.1 | 7.0 | 7.0 | 7.0 | 7.0 | 7.0 |
| 08 | 4.6 | 4.5 | 4.5 | 4.6 | 4.6 | 4.6 |
| 09 | 5.1 | 5.1 | 5.0 | 5.1 | 5.1 | 5.1 |
| 10 | 5.5 | 5.5 | 5.5 | 5.5 | 5.6 | 5.5 |
| Note: W = week, TO = starting time of the survey | | | | | | |

12.2.3 Protein content detection in different buffer systems

As shown in Table 15, the test results of samples in the citric acid/sodium citrate buffer system (pH 6.2), samples of three pH values (pH 6.0, 6.5, 7.0) of the disodium hydrogen phosphate/sodium dihydrogen phosphate buffer system, and samples of three pH values (pH 4.5, 5.0, 5.5) of the acetic acid/sodium acetate buffer system at different sampling points were basically consistent, indicating that their protein content was stable. The test results of samples of pH 5.5 and 6.0 in the histidine/histidine hydrochloride buffer system were basically consistent over time at 25°C, while the protein content of the sample of pH 6.5 increased after being placed at 25°C for four weeks. The samples of the three pH values (pH 5.5, 6.0, 6.5) of the buffer system were all detected to have increased protein content after being placed at 40°C for four weeks. This change may be due to the increase in UV280 absorption value caused by many particles observed in the aforementioned appearance results.

Table 15. Protein content of buffer system screening

| Sample No. | Protein content mg/mL | | | | | |
|---|---|---|---|---|---|---|
| | T0 | 25°C | | 40°C | | |
| | | 2W | 4W | 1W | 2W | 4W |
| 01 | 2.9 | 2.9 | 2.7 | 2.9 | 2.9 | 2.9 |
| 02 | 2.9 | 2.9 | 3.0 | 4.3 | 12.7 | 24.8 |
| 03 | 2.9 | 2.8 | 2.8 | 3.6 | 33.7 | 22.5 |
| 04 | 2.7 | 2.9 | 3.7 | 2.7 | 18.3 | 2.7 |

(continued)

| Sample No. | Protein content mg/mL | | | | | |
|---|---|---|---|---|---|---|
| | T0 | 25°C | | 40°C | | |
| | | 2W | 4W | 1W | 2W | 4W |
| 05 | 2.9 | 2.9 | 2.8 | 2.8 | 2.8 | 2.8 |
| 06 | 2.9 | 2.9 | 2.9 | 2.9 | 2.9 | 2.8 |
| 07 | 2.9 | 2.8 | 2.8 | 2.8 | 2.8 | 2.8 |
| 08 | 2.8 | 2.8 | 2.8 | 2.8 | 2.8 | 2.8 |
| 09 | 2.8 | 2.8 | 2.8 | 2.8 | 2.8 | 2.8 |
| 10 | 2.9 | 2.8 | 2.8 | 2.8 | 2.8 | 2.9 |
| Note: W = week, TO = starting time of the survey | | | | | | |

12.2.4 Turbidity (A350) test

As shown in Table 16, the test results of each buffer system under different test conditions and different sampling points were basically unchanged.

Table 16. Buffer system screening turbidity (A350) test results

| Sample No. | A350 | | | | | |
|---|---|---|---|---|---|---|
| | T0 | 25°C | | 40°C | | |
| | | 2W | 4W | 1W | 2W | 4W |
| 01 | 0.10 | 0.11 | 0.14 | 0.10 | 0.11 | 0.12 |
| 02 | 0.10 | 0.12 | 0.14 | 0.11 | 0.11 | 0.13 |
| 03 | 0.11 | 0.11 | 0.13 | 0.11 | 0.13 | 0.14 |
| 04 | 0.11 | 0.11 | 0.12 | 0.11 | 0.13 | 0.11 |
| 05 | 0.11 | 0.11 | 0.12 | 0.12 | 0.11 | 0.12 |
| 06 | 0.11 | 0.11 | 0.11 | 0.11 | 0.11 | 0.12 |
| 07 | 0.10 | 0.10 | 0.11 | 0.11 | 0.11 | 0.11 |
| 08 | 0.10 | 0.10 | 0.11 | 0.10 | 0.10 | 0.11 |
| 09 | 0.11 | 0.10 | 0.10 | 0.10 | 0.10 | 0.10 |
| 10 | 0.12 | 0.10 | 0.11 | 0.10 | 0.11 | 0.10 |
| Note: W = week, TO = starting time of the survey | | | | | | |

12.2.5 SEC-HPLC detection of protein degradation level

As shown in Table 17, under 25°C conditions, the results of SEC showed that after 4 weeks of observation, the purity of the main peak in the disodium hydrogen phosphate/sodium dihydrogen phosphate buffer system of pH 6.5 and pH 7.0 did not change; the main peak in the disodium hydrogen phosphate/sodium dihydrogen phosphate buffer system of pH 6.0 decreased by 38.0%. The main peaks of the samples in the citric acid/sodium citrate buffer system and histidine/-histidine hydrochloride buffer system were basically undetectable after 4 weeks, while the fragment peaks after protein degradation continued to increase. The main peaks of the samples in the acetic acid/sodium acetate buffer system of pH 4.5 and pH 5.0 were already lower than 40% at TO point and were basically undetectable after 4 weeks. The main peaks of the samples in the acetic acid/sodium acetate buffer system of pH 5.5 decreased by 70.5% after 4 weeks, and the fragment peaks after protein degradation also continued to increase.

At 40°C, the results of SEC showed that after 4 weeks of observation, the purity of the main peak in the disodium hydrogen phosphate/sodium dihydrogen phosphate buffer system at pH 6.5 and pH 7.0 did not change, and the fragment peak after protein degradation remained at a low level. The main peak of the sample in the citric acid/sodium citrate buffer

system decreased by 53.2% after 4 weeks. The main peaks of the samples in the histidine/histidine hydrochloride buffer system, the disodium hydrogen phosphate/sodium dihydrogen phosphate buffer system at pH 6.0, and the acetic acid/sodium acetate buffer system were basically undetectable after 4 weeks, while the fragment peak after protein degradation continued to increase.

The SEC results show that YN-011 has good stability in the disodium hydrogen phosphate/sodium dihydrogen phosphate buffer system at pH 6.5 and pH 7.0.

Table 17. Buffer system screening results of SEC_HPLC detection of protein degradation level

| Sample No. | SEC | TO | 25°C | | 40°C | | |
|---|---|---|---|---|---|---|---|
| | | | 2W | 4W | 1W | 2W | 4W |
| 01 | Main Peak % | 95.9 | 7.9 | ND | 59.8 | 51.7 | 42.7 |
| | Polymer Peak % | 4.1 | 3.0 | 0.6 | 2.7 | 2.5 | 2.1 |
| | Fragment peak % | ND | 89.1 | 99.4 | 37.5 | 45.8 | 55.2 |
| 02 | Main Peak % | 96.0 | ND | 0.5 | ND | ND | ND |
| | Polymer Peak % | 3.9 | 0.6 | ND | 1.7 | 1.5 | 1.1 |
| | Fragment peak % | 0.1 | 99.4 | 99.5 | 98.3 | 98.5 | 98.9 |
| 03 | Main Peak % | 96.8 | ND | ND | 34.7 | ND | ND |
| | Polymer Peak % | 3.2 | 1.0 | 0.5 | 1.8 | 0.6 | 0.9 |
| | Fragment peak % | 0.1 | 99.0 | 99.5 | 63.4 | 99.4 | 99.1 |
| 04 | Main Peak % | 96.4 | 26.6 | ND | 64.2 | ND | ND |
| | Polymer Peak % | 3.5 | 3.3 | 1.0 | 1.8 | 1.2 | 1.3 |
| | Fragment peak % | 0.1 | 70.1 | 99.0 | 34.0 | 98.8 | 98.7 |
| 05 | Main Peak % | 96.0 | 65.7 | 58.0 | 35.7 | ND | ND |
| | Polymer Peak % | 3.9 | 3.8 | 3.0 | 2.6 | 1.7 | 1.0 |
| | Fragment peak % | 0.1 | 30.5 | 39.0 | 61.7 | 98.3 | 99.0 |
| 06 | Main Peak % | 96.1 | 96.2 | 96.8 | 96.9 | 97.0 | 97.5 |
| | Polymer Peak % | 3.9 | 3.7 | 3.1 | 3.0 | 2.9 | 2.4 |
| | Fragment peak % | 0.1 | 0.1 | 0.2 | 0.1 | 0.1 | 0.2 |
| 07 | Main Peak % | 95.9 | 96.2 | 96.7 | 96.7 | 96.8 | 97.1 |
| | Polymer Peak % | 4.0 | 3.7 | 3.2 | 3.2 | 3.1 | 2.8 |
| | Fragment peak % | 0.1 | 0.1 | 0.2 | 0.1 | 0.1 | 0.2 |
| 08 | Main Peak % | 39.5 | ND | 0.7 | ND | ND | 0.4 |
| | Polymer Peak % | 4.4 | 1.4 | 0.3 | 4.4 | 4.0 | 2.0 |
| | Fragment peak % | 56.1 | 98.6 | 99.0 | 95.6 | 96.0 | 97.6 |
| 09 | Main Peak % | 31.1 | ND | ND | ND | ND | 0.4 |
| | Polymer Peak % | 68.7 | 2.4 | 1.0 | 1.5 | 1.5 | 0.9 |
| | Fragment peak % | 0.2 | 97.6 | 99.0 | 98.5 | 98.5 | 98.7 |
| 10 | Main Peak % | 95.6 | 40.7 | 25.1 | ND | ND | 0.7 |
| | Polymer Peak % | 4.3 | 3.7 | 2.1 | 1.7 | 1.3 | 0.4 |
| | Fragment peak % | 0.1 | 55.6 | 72.8 | 98.3 | 98.7 | 98.9 |
| Note: W = week, TO = starting time of the study; "ND" means not detected | | | | | | | |

12.2.6 RP-HPLC protein stability test

As shown in Table 18, under 25°C, the results of reverse phase HPLC showed that after 4 weeks of observation, the main peak purity in the disodium hydrogen phosphate/sodium dihydrogen phosphate buffer system at pH 6.5 and pH 7.0 decreased the least, by 3.5% and 1.9%, respectively, and the main peak in the disodium hydrogen phosphate/sodium dihydrogen phosphate buffer system at pH 6.0 decreased by 9.7%. The main peaks of the samples in the citric acid/sodium citrate buffer system and histidine/histidine hydrochloride buffer system were basically undetectable after 4 weeks. The main peaks of the samples in the acetic acid/sodium acetate buffer system at pH 4.5 were undetectable after 4 weeks, and the main peaks of the samples in the acetic acid/sodium acetate buffer system at pH 5.0 and 5.5 decreased by 51.6% and 25.3%, respectively, after 4 weeks.

At 40°C, the results of reversed-phase HPLC showed that after 4 weeks of observation, the purity of the main peak in the disodium hydrogen phosphate/sodium dihydrogen phosphate buffer system at pH 6.5 and pH 7.0 decreased the least, by 10.3% and 7.8% respectively; the main peak in the disodium hydrogen phosphate/sodium dihydrogen phosphate buffer system at pH 6.0 decreased by 50.6%. The main peak of the citric acid/sodium citrate buffer system sample decreased by 27.4% after 4 weeks. The main peak of the histidine/histidine hydrochloride buffer system samples at pH 5.5 and pH 6.0 was basically undetectable after 4 weeks, and the main peak of the histidine/histidine hydrochloride buffer system sample at pH 6.5 decreased by 27.4% after 4 weeks. The main peak of the sample in the acetic acid/sodium acetate buffer system was basically undetectable after 4 weeks.

The results of reversed-phase HPLC show that YN-011 has good stability in the disodium hydrogen phosphate/sodium dihydrogen phosphate buffer system at pH 6.5 and pH 7.0.

Table 18. RP_HPLC protein stability test results

| Sample No. | RP_HPLC | T0 | 25°C | | 40°C | | |
|---|---|---|---|---|---|---|---|
| | | | 2W | 4W | 1W | 2W | 4W |
| 01 | Main Peak % | 71.0 | 50.9 | ND | 64.8 | 60.0 | 43.6 |
| | Acromion % | 15.9 | 21.7 | ND | 14.7 | 13.6 | 13.0 |
| | Peak 2% | 4.0 | 17.3 | 98.9 | 11.5 | 17.5 | 28.8 |
| | Peak4% | 9.1 | 10.1 | 1.1 | 9.0 | 9.0 | 14.5 |
| 02 | Main Peak % | 71.6 | 18.3 | ND | 34.5 | 15.4 | ND |
| | Acromion % | 15.4 | 3.1 | ND | 7.1 | 6.1 | ND |
| | Peak 2% | 4.5 | 77.2 | 100.0 | 54.0 | 75.9 | 100.0 |
| | Peak4% | 8.5 | 1.4 | ND | 4.4 | 2.6 | ND |
| 03 | Main Peak % | 71.2 | 50.6 | ND | 54.8 | ND | ND |
| | Acromion % | 15.9 | 10.3 | ND | 11.0 | ND | ND |
| | Peak 2% | 3.9 | 33.9 | 100.0 | 26.8 | 100.0 | 100.0 |
| | Peak4% | 9.0 | 5.2 | ND | 7.3 | ND | ND |
| 04 | Main Peak % | 71.0 | 38.7 | ND | 63.4 | 37.4 | 43.6 |
| | Acromion % | 16.1 | 7.1 | ND | 13.9 | 11.7 | 11.6 |
| | Peak 2% | 3.7 | 49.4 | 99.4 | 13.8 | 46.2 | 37.8 |
| | Peak4% | 9.3 | 4.8 | 0.6 | 8.8 | 4.7 | 7.0 |
| 05 | Main Peak % | 71.1 | 67.6 | 61.4 | 55.1 | 40.0 | 20.5 |
| | Acromion % | 15.6 | 13.6 | 13.7 | 11.3 | 8.5 | 5.5 |
| | Peak 2% | 4.0 | 9.8 | 16.2 | 25.6 | 45.2 | 70.6 |
| | Peak 4% | 9.3 | 9.1 | 8.7 | 8.0 | 6.4 | 3.4 |

(continued)

| Sample No. | RP_HPLC | T0 | 25°C | | 40°C | | |
|---|---|---|---|---|---|---|---|
| | | | 2W | 4W | 1W | 2W | 4W |
| 06 | Main Peak % | 71.3 | 71.2 | 67.8 | 68.1 | 65.8 | 61.0 |
| | Acromion % | 15.9 | 14.5 | 16.5 | 15.1 | 14.7 | 16.3 |
| | Peak 2% | 3.7 | 4.7 | 5.9 | 7.2 | 9.7 | 12.7 |
| | Peak 4% | 9.0 | 9.7 | 9.9 | 9.8 | 9.8 | 10.0 |
| 07 | Main Peak % | 70.9 | 71.1 | 69.0 | 68.7 | 68.3 | 63.1 |
| | Acromion % | 16.4 | 15.1 | 16.7 | 16.2 | 14.9 | 19.1 |
| | Peak 2% | 3.5 | 3.9 | 4.1 | 4.8 | 6.2 | 7.2 |
| | Peak4% | 9.3 | 10.0 | 10.2 | 10.4 | 10.6 | 10.6 |
| 08 | Main Peak % | 69.0 | 0.5 | ND | 0.4 | 0.9 | ND |
| | Acromion % | 13.8 | ND | ND | ND | ND | ND |
| | Peak 2% | 8.7 | 99.6 | 99.3 | 97.6 | 99.1 | 98.7 |
| | Peak 4% | 8.5 | ND | 0.7 | 2.0 | ND | 1.3 |
| 09 | Main Peak % | 70.4 | 44.8 | 18.8 | 0.3 | 0.4 | ND |
| | Acromion % | 15.3 | 7.0 | ND | ND | ND | ND |
| | Peak 2% | 5.4 | 43.1 | 81.2 | 98.7 | 99.6 | 99.2 |
| | Peak 4% | 8.9 | 5.1 | ND | 1.0 | ND | 0.8 |
| 10 | Main Peak % | 71.0 | 58.8 | 45.7 | 30.1 | 3.4 | ND |
| | Acromion % | 15.2 | 10.5 | 8.5 | 6.2 | ND | ND |
| | Peak 2% | 4.9 | 23.1 | 39.8 | 59.4 | 96.4 | 100.0 |
| | Peak 4% | 9.0 | 7.6 | 5.9 | 4.2 | 0.2 | ND |
| Note: W = week, TO = starting time of the study; "ND" means "not detected" | | | | | | | |

12.2.7 cIEF protein stability test

As shown in Table 19, under 25°C, the results of cIEF showed that after 4 weeks of observation, the main peak, peak group A and peak group B in the disodium hydrogen phosphate/sodium dihydrogen phosphate buffer system at pH 6.5 and pH 7.0 did not change significantly; the main peak in the disodium hydrogen phosphate/sodium dihydrogen phosphate buffer system at pH 6.0 decreased by 10.6%, peak group A decreased by 13.3%, and peak group B increased by 24.0%. The main peak and peak group A of the samples in the citric acid/sodium citrate buffer system, histidine/histidine hydrochloride buffer system, and the acetic acid/sodium acetate buffer system at pH 4.5 and 5.0 decreased to 0, and peak group B reached 100%. The main peak of the samples in the acetic acid/sodium acetate buffer system at pH 5.5 decreased by 20.8%, peak group A decreased by 29.5%, and peak group B increased by 50.4%.

At 40°C, the results of cIEF showed that after 4 weeks of observation, the main peak, peak group A and peak group B in the disodium hydrogen phosphate/sodium dihydrogen phosphate buffer systems of pH 6.5 and pH 7.0 changed the least. After 4 weeks, the main peak and peak group A in the disodium hydrogen phosphate/sodium dihydrogen phosphate buffer system of pH 6.0 were close to 0, and peak group B reached 96.1%; the main peak in the disodium hydrogen phosphate/sodium dihydrogen phosphate buffer system of pH 6.5 decreased by 14.5%, peak group A decreased by 2.5%, and peak group B increased by 17.0%; the main peak in the disodium hydrogen phosphate/sodium dihydrogen phosphate buffer system of pH 7.0 decreased by 15.3%, peak group A increased by 7.6%, and peak group B increased by 7.7%. The main peak of the sample in the citric acid/sodium citrate buffer system decreased by 17.6%, peak group A decreased by 6.2%, and peak group B increased by 23.6%. The main peak of the samples in the histidine/histidine hydrochloride buffer system at pH 5.5 and pH 6.0 decreased to 0; the main peak of the samples in the histidine/histidine hydrochloride buffer system at pH 6.5 decreased by 28.9%, peak group A decreased by 14.4%, and peak group B increased by 43.2% after 4 weeks. The main peak and peak group A of the samples in the acetic acid/sodium acetate buffer system decreased to 0 after 4 weeks, and peak group B reached 100%.

The cIEF results show that YN-011 has good stability in the disodium hydrogen phosphate/sodium dihydrogen phosphate buffer system at pH 6.5 and pH 7.0.

Table 19. cIEF stability test results

| Sample No. | cIEF | T0 | 25°C | | 40°C | | |
|---|---|---|---|---|---|---|---|
| | | | 2W | 4W | 1W | 2W | 4W |
| 01 | Main peak isoelectric point | 6.1 | 6.2 | ND | 6.1 | 6.1 | 6.4 |
| | Main peak % | 33.8 | 11.0 | ND | 26.5 | 23.2 | 16.2 |
| | Peak Group A% | 37.9 | 17.4 | ND | 29.4 | 27.2 | 31.7 |
| | Peak Group B% | 28.5 | 71.6 | 100.0 | 44.1 | 49.6 | 52.1 |
| 02 | Main peak isoelectric point | 6.1 | ND | 6.5 | 6.1 | 6.1 | ND |
| | Main peak % | 35.0 | ND | 0.9 | 4.8 | 0.5 | ND |
| | Peak Group A% | 36.2 | 5.2 | 4.5 | 24.4 | 66.0 | 72.7 |
| | Peak Group B% | 28.9 | 94.8 | 94.6 | 70.9 | 33.6 | 27.3 |
| 03 | Main peak isoelectric point | 6.1 | 6.1 | 6.5 | 6.1 | ND | ND |
| | Main peak % | 34.8 | 1.3 | 0.6 | 17.1 | ND | ND |
| | Peak Group A% | 36.5 | 4.9 | 3.7 | 25.4 | 82.5 | 66.0 |
| | Peak Group B% | 28.7 | 93.9 | 95.7 | 57.5 | 17.5 | 34.0 |
| 04 | Main peak isoelectric point | 6.1 | 6.0 | ND | 6.1 | 6.1 | 6.5 |
| | Main peak % | 34.7 | 11.4 | ND | 26.4 | 2.7 | 5.8 |
| | Peak Group A% | 37.8 | 18.9 | ND | 28.6 | 72.3 | 23.4 |
| | Peak Group B% | 27.6 | 69.6 | 100.0 | 45.1 | 25.0 | 70.8 |
| 05 | Main peak isoelectric point | 6.1 | 6.1 | 6.5 | 6.1 | 6.1 | 6.5 |
| | Main peak % | 34.8 | 30.8 | 24.2 | 18.4 | 7.7 | 2.1 |
| | Peak Group A% | 37.0 | 30.3 | 23.7 | 18.9 | 8.9 | 1.8 |
| | Peak Group B% | 28.2 | 39.0 | 52.2 | 62.7 | 83.4 | 96.1 |
| 06 | Main peak isoelectric point | 6.1 | 6.1 | 6.5 | 6.1 | 6.1 | 6.4 |
| | Main peak % | 34.9 | 33.9 | 30.6 | 30.6 | 27.7 | 20.4 |
| | Peak Group A% | 37.7 | 35.4 | 34.1 | 34.7 | 36.4 | 35.2 |
| | Peak Group B% | 27.4 | 30.7 | 35.3 | 34.8 | 35.9 | 44.4 |
| 07 | Main peak isoelectric point | 6.1 | 6.1 | 6.5 | 6.1 | 6.1 | 6.5 |
| | Main peak % | 34.1 | 32.2 | 31.3 | 31.3 | 28.3 | 18.8 |
| | Peak Group A% | 38.0 | 39.6 | 37.0 | 41.8 | 43.2 | 45.6 |
| | Peak Group B% | 27.9 | 28.3 | 31.8 | 27.0 | 28.6 | 35.6 |
| 08 | Main peak isoelectric point | 6.1 | ND | ND | ND | ND | ND |
| | Main peak % | 32.1 | ND | ND | ND | ND | ND |
| | Peak Group A% | 31.5 | 0.0 | ND | 1.1 | 0.8 | ND |
| | Peak Group B% | 36.5 | 100.0 | 100.0 | 98.9 | 99.2 | 100.0 |
| 09 | Main peak isoelectric point | 6.1 | 6.1 | ND | ND | ND | ND |
| | Main peak % | 34.7 | 13.3 | ND | ND | ND | ND |
| | Peak Group A% | 35.0 | 7.7 | ND | 2.1 | 1.4 | ND |
| | Peak Group B% | 30.3 | 79.0 | 100.0 | 97.9 | 98.6 | 100.0 |

(continued)

| Sample No. | cIEF | T0 | 25°C | | 40°C | | |
|---|---|---|---|---|---|---|---|
| | | | 2W | 4W | 1W | 2W | 4W |
| 10 | Main peak isoelectric point | 6.1 | 6.2 | 6.4 | 6.1 | ND | ND |
| | Main peak % | 34.4 | 26.1 | 13.6 | 1.8 | ND | ND |
| | Peak Group A% | 36.3 | 19.1 | 6.8 | 3.4 | 2.2 | ND |
| | Peak Group B% | 29.3 | 54.9 | 79.7 | 94.8 | 97.8 | 100.0 |
| Note: W = week, TO = starting time of the study; "ND" means "not detected" | | | | | | | |

12.2.8 Summary

Taking the results of the above tests into consideration, YN-011 has good stability in the disodium hydrogen phosphate/sodium dihydrogen phosphate buffer system at pH 6.5 and pH 7.0. The results of reversed-phase HPLC show that the protein stability in the phosphate buffer system at pH 7.0 is slightly better than that at pH 6.5, but the difference between the two is not obvious. Therefore, the disodium hydrogen phosphate/sodium dihydrogen phosphate buffer system at pH 6.5-7.0 has a better effect.

12.3 Excipient screening

12.3.1 Excipient screening scheme

Select 10 mM disodium hydrogen phosphate/sodium dihydrogen phosphate buffer system at pH 6.7, and match it with five common pharmaceutical excipients (mannitol, sucrose, sorbitol, sodium chloride and glycine), and select 10 mM citric acid/sodium citrate buffer system at pH 6.5 and mannitol, and design 8 different prescriptions to investigate the thermal stability and stability under freeze-thaw conditions of YN-011, see Table 20 for details. The YN-011 stock solution was dialyzed to a 10 mM disodium hydrogen phosphate/sodium dihydrogen phosphate buffer system at pH 6.7 and a 10 mM citric acid/sodium citrate buffer system at pH 6.5. The corresponding excipients were added and the protein concentration was adjusted to 3 mg/mL. 2 mL of the sample was filled into a 5 mL neutral borosilicate glass injection bottle and capped. The sample was placed at 25°C for 2 weeks and 4 weeks, and at 40°C for 1 week, 2 weeks, and 4 weeks. The sample was subjected to two and five freeze-thaw cycles from -40°C to room temperature. Samples were taken at different time points for various tests to screen and evaluate the candidate excipients.

Table 20. Excipient screening plan

| **Sample No.** | **Composition** | **Excipients** |
|---|---|---|
| 01 | Citric acid/sodium citrate, 10 mM, pH 6.5 | / |
| 02 | | 4.6% Mannitol (w/v) |
| 03 | disodium hydrogen phosphate/sodium dihydrogen phosphate, 10 mM, pH 6.7 | / |
| 04 | | 4.6% Mannitol (w/v) |
| 05 | | 9% sucrose (w/v) |
| 06 | | 4.6% Sorbitol (w/v) |
| 07 | | 140 mM NaCl |
| 08 | | 2% Glycine (w/v) |

12.3.2 Appearance

In the appearance observation of thermal stability, all YN-011 samples were colorless. In the citric acid buffer system, the opalescence phenomenon of the samples placed at 25°C intensified, accompanied by the generation of particles. A small amount of particles was also observed in the samples placed at 40°C for 4 weeks. In the phosphate buffer system, the samples observed under various excipients and sampling points were slightly opalescent. In the formulation with sodium chloride, the particles increased, which was consistent with the control without excipients, while no particles

were observed in the phosphate formulation containing several other excipients.

In the appearance observation of the freeze-thaw experiment, all YN-011 samples were colorless and slightly opalescent. In the citric acid buffer system with mannitol added, a small amount of fine particles were generated in the samples after two freeze-thaw cycles, and the number of particles increased after five freeze-thaw cycles. In the phosphate buffer system, similar particles were observed in the formulations with mannitol and glycine added. In the phosphate formulation containing sucrose, sorbitol and sodium chloride, no particles were produced in the samples after two freeze-thaw cycles, and a small amount of fine particles were produced in the samples after five freeze-thaw cycles.

12.3.3 pH value and protein content test results

As shown in Tables 21 and 22, in the six formulations containing excipients, the protein content and pH value did not change significantly over time.

Table 21. Protein content detection of excipient screening

| Sample No. | Protein content mg/mL | | | | | | | |
|---|---|---|---|---|---|---|---|---|
| | T0 | 25°C | | 40°C | | | Freeze-thaw, -40°C to room temperature | |
| | | 2W | 4W | 1W | 2W | 4W | 2times | 5 times |
| 01 | 3.0 | 2.9 | 2.9 | 3.0 | 2.9 | 2.9 | 2.9 | 2.9 |
| 02 | 3.0 | 3.0 | 3.0 | 3.1 | 3.0 | 3.0 | 3.0 | 2.9 |
| 03 | 2.9 | 2.9 | 2.9 | 2.9 | 3.0 | 2.8 | 2.9 | 2.9 |
| 04 | 2.9 | 3.0 | 2.9 | 3.0 | 3.0 | 2.9 | 3.0 | 2.9 |
| 05 | 3.1 | 3.1 | 3.0 | 3.1 | 3.1 | 3.0 | 3.0 | 3.1 |
| 06 | 3.0 | 3.0 | 3.0 | 3.0 | 3.0 | 3.0 | 3.0 | 3.0 |
| 07 | 2.9 | 2.9 | 2.9 | 2.9 | 2.9 | 2.8 | 2.9 | 2.9 |
| 08 | 2.9 | 2.9 | 2.9 | 2.9 | 2.9 | 2.8 | 2.8 | 2.8 |
| Note: W = week, TO = starting time of the survey | | | | | | | | |

Table 22. Excipient screening pH test results

| Sample No. | pH | | | | | | | |
|---|---|---|---|---|---|---|---|---|
| | T0 | 25°C | | 40°C | | | Freeze-thaw, -40°C to room temperature | |
| | | 2W | 4W | 1W | 2W | 4W | 2times | 5 times |
| 01 | 6.7 | 6.7 | 7.4 | 6.6 | 6.6 | 6.6 | 6.7 | 6.6 |
| 02 | 6.6 | 6.6 | 6.5 | 6.6 | 6.6 | 6.6 | 6.6 | 6.6 |
| 03 | 6.7 | 6.6 | 6.7 | 6.7 | 6.8 | 6.7 | 6.7 | 6.7 |
| 04 | 6.7 | 6.7 | 6.6 | 6.6 | 6.7 | 6.6 | 6.6 | 6.6 |
| 05 | 6.6 | 6.6 | 6.6 | 6.6 | 6.6 | 6.6 | 6.6 | 6.6 |
| 06 | 6.7 | 6.7 | 6.6 | 6.6 | 6.7 | 6.7 | 6.6 | 6.6 |
| 07 | 6.7 | 6.7 | 6.7 | 6.7 | 6.7 | 6.7 | 6.6 | 6.7 |
| 08 | 6.6 | 6.6 | 6.6 | 6.6 | 6.6 | 6.6 | 6.6 | 6.6 |
| Note: W = week, TO = starting time of the survey | | | | | | | | |

12.3.4 SEC-HPLC detection of protein degradation level

As shown in Table 23, after 4 weeks of observation at 25°C, the main SEC peak in the phosphate buffer system containing different excipients did not decrease significantly; the least decrease was in the formulation containing sucrose, which decreased by 0.4%; the most decrease was in the formulation containing glycine, which decreased by 1.2%. The main SEC peak in the citric acid buffer system without excipients decreased by 0.2%, while the main SEC peak in the citric

acid buffer system containing mannitol decreased by 2.2%. After 4 weeks of observation at 40°C, the main SEC peak in the phosphate buffer system containing sorbitol and glycine decreased significantly, by 2.0% and 3.1%, respectively, and the main SEC peak in the phosphate buffer system containing mannitol decreased the least, by 0.9%. The main SEC peak in the citric acid buffer system without excipients decreased by 0.5%, while the main SEC peak in the citric acid buffer system containing mannitol decreased by 1.0%.

In the freeze-thaw experiment, after five freeze-thaw cycles, the SEC main peak of the sample in the phosphate buffer system containing sodium chloride decreased most significantly, by 2.6%; the SEC main peak decreased least in the phosphate formulation containing glycine, by 0.5%; the main peaks of the phosphate formulations containing sucrose, mannitol and sorbitol decreased by 0.8% after five freeze-thaw cycles. The SEC main peak in the citric acid buffer system without excipients decreased by 2.0% after five freeze-thaw cycles, while the SEC main peak in the citric acid buffer system containing mannitol decreased by 1.2%.

Table 23. Results of protein degradation level detected by SEC for excipient screening

| Sample No. | SEC | T0 | 25°C | | 40°C | | | Freeze-thaw, -40°C to room temperature | |
|---|---|---|---|---|---|---|---|---|---|
| | | | 2W | 4W | 1W | 2W | 4W | 2times | 5times |
| 01 | Main Peak % | 98.9 | 98.0 | 98.7 | 98.5 | 95.8 | 98.4 | 97.8 | 96.9 |
| | Polymer Peak % | 1.1 | 2.0 | 1.3 | 1.5 | 1.9 | 1.6 | 2.2 | 1.9 |
| | Fragment peak % | ND | ND | ND | ND | 2.3 | ND | ND | 1.2 |
| 02 | Main Peak % | 98.9 | 97.5 | 96.7 | 98.6 | 97.9 | 97.9 | 97.8 | 97.7 |
| | Polymer Peak % | 1.1 | 2.5 | 3.3 | 1.4 | 2.1 | 2.1 | 2.3 | 2.4 |
| | Fragment peak % | ND | ND | ND | ND | ND | ND | ND | ND |
| 03 | Main Peak % | 99.1 | 98.5 | 98.5 | 98.9 | 98.4 | 98.1 | 98.3 | 98.1 |
| | Polymer Peak % | 0.9 | 1.5 | 1.5 | 1.1 | 1.6 | 1.9 | 1.9 | 2.0 |
| | Fragment peak % | ND | ND | ND | ND | ND | ND | ND | ND |
| 04 | Main Peak % | 99.1 | 98.3 | 98.3 | 98.9 | 98.2 | 98.2 | 98.3 | 98.3 |
| | Polymer Peak % | 0.9 | 1.7 | 1.7 | 1.1 | 1.8 | 1.8 | 1.7 | 1.9 |
| | Fragment peak % | ND | ND | ND | ND | ND | ND | ND | ND |
| 05 | Main Peak % | 99.1 | 97.9 | 98.7 | 98.9 | 98.3 | 98.0 | 98.3 | 98.3 |
| | Polymer Peak % | 0.9 | 2.0 | 1.3 | 1.1 | 1.7 | 2.0 | 1.7 | 1.8 |
| | Fragment peak % | ND | 0.1 | ND | ND | ND | ND | ND | ND |
| 06 | Main Peak % | 99.1 | 98.2 | 98.1 | 98.6 | 97.6 | 97.1 | 98.4 | 98.3 |
| | Polymer Peak % | 1.0 | 1.8 | 1.9 | 1.4 | 2.4 | 2.9 | 1.7 | 1.9 |
| | Fragment peak % | ND | ND | ND | ND | ND | ND | ND | ND |
| 07 | Main Peak % | 99.0 | 98.2 | 98.3 | 98.4 | 97.9 | 97.8 | 96.9 | 96.4 |
| | Polymer Peak % | 1.0 | 1.8 | 1.7 | 1.6 | 2.1 | 2.2 | 3.6 | 3.7 |
| | Fragment peak % | ND | ND | ND | ND | ND | ND | ND | ND |
| 08 | Main Peak % | 99.1 | 97.9 | 97.9 | 98.4 | 96.8 | 96.0 | 98.4 | 98.6 |
| | Polymer Peak % | 0.9 | 2.0 | 2.1 | 1.6 | 3.2 | 4.0 | 1.4 | 1.4 |
| | Fragment peak % | ND | 0.1 | ND | ND | ND | ND | ND | ND |
| Note: W = week, TO = starting time of the study; "ND" means "not detected" | | | | | | | | | |

12.3.5 RP-HPLC protein stability test

As shown in Table 24, after 4 weeks of observation at 25°C, in the phosphate buffer system, the RP main peak of the formulation containing glycine decreased most significantly, by 3.9%; the formulation without excipients had the least

decrease, by 1.3%; the RP main peaks of the formulations with other excipients decreased by 1.5% for mannitol, 1.8% for sucrose, 1.8% for sorbitol, and 1.5% for sodium chloride. Compared with the phosphate buffer system, the RP main peak in the citrate buffer system decreased significantly, with the formulation without excipients decreasing by 72.6%, and the RP main peak in the citrate buffer system containing mannitol decreasing by 9.4%. After 4 weeks of observation at 40°C, in the phosphate buffer system, the RP main peak of the formulation containing glycine decreased most significantly, by 20.3%; the formulation without excipients had the least decrease, with a main peak decrease of 6.1%; the RP main peaks of the formulations with other excipients decreased by 7.6% for mannitol, 8.2% for sucrose, 9.3% for sorbitol, and 6.7% for sodium chloride. In the citric acid buffer system, the RP main peak of the formulation without excipients decreased by 6.1%, while the RP main peak of the citric acid buffer system containing mannitol decreased by 7.3%.

There was no significant change in the RP peaks of each formulation in the freeze-thaw experiment.

Table 24. Results of protein stability testing by RP_HPLC for excipient screening

| Sample No. | RP_HPLC | T0 | 25°C | | 40°C | | | Freeze-thaw, -40°C to room temperature | |
|---|---|---|---|---|---|---|---|---|---|
| | | | 2W | 4W | 1W | 2W | 4W | 2times | 5 times |
| 01 | Main Peak % | 73.9 | 73.1 | 1.3 | 72.1 | 70.2 | 67.8 | 74.2 | 74.4 |
| | Acromion % | 13.7 | 14.0 | 0.3 | 14.7 | 15.4 | 15.3 | 13.8 | 13.6 |
| | Peak 2% | 3.2 | 4.0 | 97.9 | 4.4 | 4.9 | 6.6 | 3.3 | 3.4 |
| | Peak4% | 9.2 | 9.0 | 0.6 | 8.9 | 9.6 | 10.4 | 8.8 | 8.6 |
| 02 | Main Peak % | 74.0 | 41.2 | 64.6 | 72.1 | 69.9 | 66.7 | 73.9 | 74.0 |
| | Acromion % | 13.7 | 12.0 | 12.3 | 14.3 | 15.1 | 15.5 | 13.9 | 13.9 |
| | Peak 2% | 3.2 | 39.5 | 11.3 | 4.7 | 5.6 | 7.6 | 3.4 | 3.3 |
| | Peak4% | 9.1 | 7.3 | 11.8 | 9.0 | 9.5 | 10.2 | 8.8 | 8.8 |
| 03 | Main Peak % | 75.5 | 75.0 | 74.2 | 73.8 | 72.4 | 69.4 | 76.1 | 75.6 |
| | Acromion % | 13.1 | 13.2 | 13.0 | 13.6 | 14.2 | 14.0 | 12.7 | 12.5 |
| | Peak 2% | 2.5 | 3.0 | 3.8 | 3.6 | 4.1 | 6.2 | 2.5 | 2.6 |
| | Peak4% | 9.0 | 8.7 | 9.0 | 9.1 | 9.3 | 10.4 | 8.7 | 9.3 |
| 04 | Main Peak % | 75.7 | 74.9 | 74.2 | 73.8 | 71.8 | 68.1 | 76.0 | 76.7 |
| | Acromion % | 12.6 | 13.1 | 12.7 | 13.3 | 14.0 | 14.2 | 12.7 | 12.4 |
| | Peak 2% | 2.4 | 3.2 | 3.9 | 3.9 | 4.9 | 7.4 | 2.5 | 2.4 |
| | Peak4% | 9.3 | 8.8 | 9.2 | 9.0 | 9.3 | 10.3 | 8.8 | 8.5 |
| 05 | Main Peak % | 75.5 | 74.8 | 73.7 | 73.3 | 71.3 | 67.3 | 76.1 | 75.9 |
| | Acromion % | 12.9 | 13.0 | 13.3 | 13.4 | 13.9 | 14.2 | 12.6 | 12.6 |
| | Peak 2% | 2.4 | 3.3 | 4.0 | 4.3 | 5.4 | 7.9 | 2.6 | 2.7 |
| | Peak4% | 9.1 | 8.9 | 9.0 | 9.2 | 9.5 | 10.6 | 8.7 | 8.8 |
| 06 | Main Peak % | 75.6 | 74.8 | 73.8 | 73.2 | 70.6 | 66.3 | 76.4 | 76.5 |
| | Acromion % | 12.9 | 12.7 | 12.8 | 13.6 | 14.6 | 15.2 | 12.4 | 12.4 |
| | Peak 2% | 2.5 | 3.1 | 4.0 | 3.8 | 4.9 | 7.6 | 2.4 | 2.5 |
| | Peak4% | 9.0 | 9.4 | 9.4 | 9.4 | 9.9 | 10.9 | 8.8 | 8.6 |
| 07 | Main Peak % | 75.7 | 74.5 | 74.2 | 73.7 | 72.2 | 69.0 | 74.9 | 75.7 |
| | Acromion % | 12.8 | 12.8 | 13.0 | 13.8 | 14.5 | 14.3 | 13.0 | 12.8 |
| | Peak 2% | 2.5 | 2.9 | 3.6 | 3.5 | 4.1 | 6.3 | 2.7 | 2.7 |
| | Peak4% | 9.0 | 9.8 | 9.1 | 9.0 | 9.2 | 10.3 | 9.3 | 8.8 |

(continued)

| Sample No. | RP_HPLC | T0 | 25°C | | 40°C | | | Freeze-thaw, -40°C to room temperature | |
|---|---|---|---|---|---|---|---|---|---|
| | | | 2W | 4W | 1W | 2W | 4W | 2times | 5 times |
| 08 | Main Peak % | 75.6 | 74.4 | 71.7 | 71.3 | 66.0 | 55.3 | 75.7 | 76.0 |
| | Acromion % | 13.1 | 13.5 | 13.9 | 14.9 | 16.7 | 16.8 | 13.0 | 12.9 |
| | Peak 2% | 2.4 | 3.5 | 5.1 | 4.8 | 7.3 | 13.9 | 2.7 | 2.6 |
| | Peak4% | 8.9 | 8.6 | 9.3 | 9.1 | 10.0 | 14.0 | 8.6 | 8.5 |
| Note: W = week, TO = starting time of the survey | | | | | | | | | |

12.3.6 SDS_CALIPER_NR purity (%) test results

As shown in Table 25, in the non-reducing SDS-Caliper test, after 4 weeks at 25°C, the purity of each formulation did not change significantly; after 4 weeks at 40°C, the purity of the phosphate formulation containing glycine and sodium chloride decreased most significantly, by 2.6% and 1.9% respectively, and the purity of the other formulations did not change significantly; in the freeze-thaw experiment, the purity of each formulation did not change significantly.

Table 25. Excipient screening SDS_CALIPER_NR purity (%) test results

| Sample No. | Purity ( % ) | | | | | | | |
|---|---|---|---|---|---|---|---|---|
| | T0 | 25°C | | 40°C | | | Freeze-thaw, -40°C to room temperature | |
| | | 2W | 4W | 1W | 2W | 4W | 2times | 5 times |
| 01 | 98.8 | 98.8 | 99.6 | 98.4 | 98.4 | 97.9 | 98.9 | 99.0 |
| 02 | 98.7 | 98.9 | 98.0 | 98.6 | 98.4 | 97.9 | 98.9 | 98.9 |
| 03 | 97.6 | 99.0 | 99.0 | 98.7 | 98.4 | 97.9 | 99.2 | 99.1 |
| 04 | 99.0 | 99.0 | 99.0 | 98.4 | 98.4 | 97.8 | 99.1 | 99.1 |
| 05 | 99.0 | 99.0 | 99.0 | 97.8 | 98.4 | 97.7 | 99.2 | 99.2 |
| 06 | 99.0 | 98.9 | 98.9 | 98.6 | 98.1 | 97.2 | 99.1 | 99.2 |
| 07 | 98.9 | 98.9 | 98.8 | 98.1 | 97.9 | 97.0 | 99.1 | 99.1 |
| 08 | 99.1 | 99.0 | 98.7 | 98.5 | 97.6 | 96.5 | 99.1 | 99.1 |
| Note: W = week, TO = starting time of the survey | | | | | | | | |

12.3.7 Stability of cIEF test

As shown in Table 26, after 4 weeks of observation at 25°C, the isoelectric points of each formulation did not change, the main peaks of the two formulations in the citric acid buffer system decreased significantly, and the main peaks of the other formulations did not change significantly. After 4 weeks of observation at 40°C, the isoelectric points of each formulation did not change, but the main peaks all decreased significantly, among which the main peak in the phosphate formulation containing glycine decreased most significantly, by 18.0%, and the main peak in the citric acid buffer system containing mannitol decreased the least, by 5.3%. There was no significant change in the main peaks of each formulation in the freeze-thaw experiment.

Table 26. Excipient screening cIEF stability test results

| Sample No. | cIEF | T0 | 25°C | 40°C | Freeze-thaw, -40°C to room temperature | |
|---|---|---|---|---|---|---|
| | | | 4W | 4W | 2times | 5 times |
| 01 | Main Peak % | 6.4 | ND | 6.5 | 6.4 | 6.4 |
| | Acromion % | 34.3 | ND | 27.1 | 35.7 | 36.6 |
| | Peak 2% | 27.4 | ND | 37.4 | 28.8 | 32.5 |
| | Peak4% | 38.3 | 100.0 | 35.6 | 35.5 | 30.9 |
| 02 | Main Peak % | 6.4 | 6.4 | 6.4 | 6.4 | 6.4 |
| | Acromion % | 33.8 | 26.8 | 28.5 | 36.8 | 35.1 |
| | Peak 2% | 27.8 | 33.0 | 36.4 | 29.8 | 31.1 |
| | Peak4% | 38.4 | 40.1 | 35.1 | 33.4 | 33.7 |
| 03 | Main Peak % | 6.5 | 6.4 | 6.4 | 6.4 | 6.4 |
| | Acromion % | 35.2 | 36.1 | 27.1 | 38.0 | 41.3 |
| | Peak 2% | 26.6 | 32.1 | 40.0 | 28.7 | 34.4 |
| | Peak4% | 38.2 | 31.8 | 32.9 | 33.3 | 24.3 |
| 04 | Main Peak % | 6.4 | 6.4 | 6.4 | 6.4 | 6.4 |
| | Acromion % | 36.8 | 38.1 | 27.7 | 38.6 | 38.0 |
| | Peak 2% | 26.3 | 35.7 | 38.2 | 27.7 | 29.4 |
| | Peak4% | 36.9 | 26.3 | 34.2 | 33.7 | 32.6 |
| 05 | Main Peak % | 6.4 | 6.4 | 6.4 | 6.4 | 6.4 |
| | Acromion % | 36.7 | 39.2 | 27.8 | 39.1 | 37.3 |
| | Peak 2% | 27.2 | 36.8 | 39.0 | 30.9 | 29.9 |
| | Peak4% | 36.2 | 24.0 | 33.3 | 30.0 | 32.8 |
| 06 | Main Peak % | 6.5 | 6.4 | 6.4 | 6.4 | 6.4 |
| | Acromion % | 36.3 | 38.6 | 26.7 | 37.7 | 36.4 |
| | Peak 2% | 27.5 | 35.2 | 41.4 | 29.5 | 29.1 |
| | Peak4% | 36.3 | 26.3 | 31.9 | 32.7 | 34.5 |
| 07 | Main Peak % | 6.5 | 6.5 | 6.5 | 6.5 | 6.5 |
| | Acromion % | 35.0 | 36.6 | 26.1 | 36.8 | 37.4 |
| | Peak 2% | 26.6 | 31.7 | 39.1 | 29.4 | 28.1 |
| | Peak4% | 38.4 | 31.8 | 34.8 | 33.9 | 34.5 |
| 08 | Main Peak % | 6.5 | 6.5 | 6.5 | 6.5 | 6.5 |
| | Acromion % | 35.9 | 35.2 | 17.9 | 42.0 | 39.9 |
| | Peak 2% | 27.0 | 35.5 | 47.0 | 30.2 | 30.0 |
| | Peak4% | 37.1 | 29.3 | 35.2 | 27.8 | 30.1 |
| Note: W = week, TO = starting time of the study; "ND" means "not detected" | | | | | | |

12.3.8 Summary

YN-011 is stable in a phosphate buffer system containing sucrose and mannitol, and other systems are stable.

12.4 Surfactant Screening

After screening, commonly used surfactants in injections such as Tween 80 (polysorbate 80), polyoxyethylene castor oil derivatives, poloxamer, lecithin, polyethylene glycol 15-hydroxystearate, cyclodextrins, etc. can be used in this preparation. As a surfactant, Tween 80 (polysorbate 80) is a stabilizer commonly used in injections. In this example, the concentration of Tween 80 is optimized.

12.4.1 Optimization design of Tween 80 concentration

Based on the results of buffer system screening, a 10 mM disodium hydrogen phosphate/sodium dihydrogen phosphate buffer system with a pH of 6.7 was selected, and matched with four different Tween 80 concentrations (0, 0.01%, 0.02%, and 0.04%) to select the optimal Tween 80 concentration for YN-011. For specific schemes, see Table 27. The YN-011 stock solution was dialyzed and replaced with a 10 mM disodium hydrogen phosphate/sodium dihydrogen phosphate buffer system with a pH of 6.7, and Tween 80 mother solution was added. The protein concentration was adjusted to 3 mg/mL by adding the corresponding phosphate buffer, and 2 mL of the sample was filled into a 5 mL neutral borosilicate glass injection bottle and capped. Four Tween 80 concentrations were screened and evaluated by placing the samples at 40°C for 1, 2, and 4 weeks, and shaking them in a shaker at 25°C and 300 rpm for 1 and 2 days. Samples were taken at different time points for various tests.

Table 27. Tween 80 (PS 80) concentration optimization

| Sample No. | Composition | Tween 80 ( w/v ) |
|---|---|---|
| 03 | disodium hydrogen phosphate /sodium dihydrogen phosphate, 10 mM, pH 6.7 | 0 |
| 09 | | 0.01% |
| 10 | | 0.02% |
| 11 | | 0.04% |

12.4.2 Appearance

In the appearance observation, all YN-011 samples were colorless and slightly opalescent. In the formulation without Tween 80, very small amounts of flocculent protein particles were observed under shaking conditions, and very small amounts of protein particles were observed after being placed at 40°C for 4 weeks. In the formulation containing 0.01% Tween 80, very small amounts of protein particles were observed after being placed at 40° C for 4 weeks, and no particles were generated under shaking conditions. In the formulations containing 0.02% and 0.04% Tween 80, the appearance did not change.

12.4.3 pH value and protein content

The pH value and protein content of the four formulations containing different concentrations of Tween 80 did not change significantly under 40°C and shaking conditions.

12.4.4 SEC-HPLC detection of protein degradation level

As shown in Table 28, after 2 days of shaking, the main peak purity of the formulation without Tween 80 decreased by 1.8%, and the aggregates and fragments increased by 0.9%, respectively, while the SEC purity of the three formulations containing Tween 80 did not change. Under the 40°C inspection condition, the SEC purity of each formulation decreased. After 4 weeks, the SEC purity of the formulations without Tween 80 and containing 0.01% Tween 80 decreased by 1.0% and 0.9%, respectively, and the SEC purity of the formulations containing 0.02% and 0.04% Tween 80 decreased by 0.8%.

Table 28. Results of SEC detection of protein degradation level by optimizing Tween 80 concentration

| Sample No. | Tween 80 concentration | SEC | T0 | Shake | | 40°C | | |
|---|---|---|---|---|---|---|---|---|
| | | | | 1D | 2D | 1W | 2W | 4W |
| 03 | 0 | Main Peak % | 99.1 | 97.3 | 97.3 | 98.9 | 98.4 | 98.1 |
| | | Polymer Peak% | 0.9 | 1.7 | 1.8 | 1.1 | 1.6 | 1.9 |
| | | Fragment peak% | ND | 1.0 | 0.9 | ND | ND | ND |
| 09 | 0.01% | Main Peak % | 99.0 | 98.9 | 98.8 | 98.8 | 98.2 | 98.1 |
| | | Polymer Peak% | 1.0 | 1.1 | 1.2 | 1.2 | 1.8 | 1.9 |
| | | Fragment peak% | ND | ND | ND | ND | ND | ND |
| 10 | 0.02% | Main Peak % | 98.9 | 98.8 | 98.8 | 98.8 | 98.2 | 98.1 |
| | | Polymer Peak% | 1.1 | 1.2 | 1.2 | 1.2 | 1.8 | 1.9 |
| | | Fragment peak% | ND | ND | ND | ND | ND | ND |
| 11 | 0.04% | Main Peak % | 98.8 | 98.8 | 98.8 | 98.8 | 97.8 | 98.0 |
| | | Polymer Peak% | 1.2 | 1.2 | 1.2 | 1.2 | 2.2 | 2.0 |
| | | Fragment peak% | ND | ND | ND | ND | ND | ND |
| Note: D = day, W = week, TO = starting time of investigation; "ND" means "not detected" | | | | | | | | |

12.4.5 SDS_CALIPER_NR purity test results

As shown in Table 29, under shaking conditions, the purity of the non-reduced SDS-Caliper of each formulation did not change significantly. After 4 weeks of observation at 40°C, the purity of the formulations containing 0.01%, 0.02% and 0.04% Tween 80 decreased by 1.2%, 1.2% and 1.3%, respectively.

Table 29. Tween 80 concentration optimization SDS_CALIPER_NR purity test results

| Sample No. | Tween 80concentration | Purity % | | | | | |
|---|---|---|---|---|---|---|---|
| | | T0 | Shake | | 40°C | | |
| | | | 1D | 2D | 1W | 2W | 4W |
| 03 | 0 | 97.6 | 99.1 | 99.1 | 98.7 | 98.4 | 97.9 |
| 09 | 0.01% | 99.0 | 98.9 | 98.4 | 98.8 | 98.3 | 97.8 |
| 10 | 0.02% | 99.0 | 99.1 | 98.9 | 98.7 | 98.4 | 97.8 |
| 11 | 0.04% | 99.0 | 99.0 | 98.2 | 98.6 | 98.4 | 97.7 |

12.4.6 RP-HPLC protein stability test

As shown in Table 30, under shaking conditions, the RP main peak of the formulation without Tween 80 decreased by 1.2%, while in the three formulations containing Tween 80, the RP main peak did not change. Under the 40°C test conditions, the RP main peak of each formulation decreased. After 4 weeks, the RP purity of the formulation without Tween 80 decreased by 6.1%, and the RP main peak of the formulations containing 0.01%, 0.02% and 0.04% Tween 80 decreased by 8.1%, 8.5% and 8.6%, respectively.

Table 30. Tween 80 concentration optimization RP_HPLC protein stability test results

| Sample No. | Tween 80 concentration | RP_HPLC | T0 | Shake | | 40°C | | |
|---|---|---|---|---|---|---|---|---|
| | | | | 1D | 2D | 1W | 2W | 4W |
| 03 | 0 | Peak 2% | 2.5 | 2.2 | 2.3 | 3.6 | 4.1 | 6.2 |
| | | Acromion % | 13.1 | 13.1 | 12.9 | 13.6 | 14.2 | 14.0 |
| | | Main Peak % | 75.5 | 74.3 | 74.3 | 73.8 | 72.4 | 69.4 |
| | | Peak4% | 9.0 | 10.4 | 10.6 | 9.1 | 9.3 | 10.4 |
| 09 | 0.01% | Peak 2% | 2.5 | 2.6 | 2.5 | 3.6 | 4.4 | 7.0 |
| | | Acromion % | 12.7 | 12.7 | 12.7 | 13.4 | 14.5 | 15.4 |
| | | Main Peak % | 75.6 | 75.7 | 75.8 | 74.1 | 72.0 | 67.5 |
| | | Peak4% | 9.2 | 9.1 | 9.0 | 8.9 | 9.1 | 10.2 |
| 10 | 0.02% | Peak 2% | 2.5 | 2.5 | 2.5 | 3.7 | 4.5 | 6.7 |
| | | Acromion % | 12.8 | 12.6 | 12.9 | 13.3 | 14.3 | 15.9 |
| | | Main Peak % | 75.8 | 76.0 | 75.8 | 73.9 | 71.8 | 67.3 |
| | | Peak4% | 8.9 | 8.9 | 8.8 | 9.2 | 9.3 | 10.1 |
| 11 | 0.04% | Peak 2% | 2.5 | 2.5 | 2.5 | 3.7 | 4.4 | 6.8 |
| | | Acromion % | 12.3 | 12.9 | 12.9 | 13.5 | 14.4 | 15.4 |
| | | Main Peak % | 76.2 | 75.5 | 75.8 | 73.7 | 72.0 | 67.6 |
| | | Peak4% | 9.0 | 9.1 | 8.8 | 9.1 | 9.2 | 10.2 |
| Note: D = day, W = week, T0 = starting time of the survey | | | | | | | | |

12.4.7 Stability of cIEF test

As shown in Table 31, under shaking conditions, the components of cIEF did not change significantly. Under 40°C conditions, the main peak and peak group B of each formulation decreased, while peak group A increased, and there was no significant difference between the formulations.

Table 31. Tween 80 concentration optimization cIEF test stability results

| Sample No. | Tween 80 concentration | cIEF | T0 | Shake 2D | 40°C 4W |
|---|---|---|---|---|---|
| 03 | 0 | Main peak isoelectric point | 6.5 | / | 6.4 |
| | | Main peak % | 35.2 | / | 27.1 |
| | | Peak Group A% | 26.6 | / | 40.0 |
| | | Peak Group B% | 38.2 | / | 32.9 |
| 09 | 0.01% | Main peak isoelectric point | 6.5 | 6.5 | 6.4 |
| | | Main peak % | 36.0 | 33.5 | 26.3 |
| | | Peak Group A% | 27.1 | 27.2 | 41.0 |
| | | Peak Group B% | 37.0 | 39.2 | 32.7 |
| 10 | 0.02% | Main peak isoelectric point | 6.5 | 6.5 | 6.4 |
| | | Main peak % | 36.0 | 34.4 | 27.2 |
| | | Peak Group A% | 27.2 | 25.5 | 40.6 |
| | | Peak Group B% | 36.9 | 40.1 | 32.2 |

(continued)

| Sample No. | Tween 80 concentration | cIEF | T0 | Shake 2D | 40°C 4W |
|---|---|---|---|---|---|
| 11 | 0.04% | Main peak isoelectric point | 6.5 | 6.5 | 6.4 |
| | | Main peak % | 36.0 | 33.9 | 27.2 |
| | | Peak Group A% | 27.0 | 25.5 | 40.2 |
| | | Peak Group B% | 37.0 | 40.6 | 32.7 |
| Note: D = day, W = week, T0 = starting time of the study; "/" means no such test | | | | | |

12.4.8 Summary of Tween 80 concentration optimization

The results of the Tween 80 concentration optimization experiment showed that Tween 80 should be added to the formulation to improve the stability of the protein under shaking conditions. There was no significant difference in the effect of 0.01%, 0.02% and 0.04% Tween 80 concentrations on the stability of the protein. Considering the possible errors in the future production process, the Tween 80 concentration of 0.02% was selected.

Example 13: Stability test of drug preparations

13.1 Preparation preparation method

YN-011 preparations with a specification of 0.5 mL/tube were prepared according to the excipient composition shown in Table 32, wherein the YN-011 protein content was 1 mg, 2 mg and 3 mg respectively (as shown in Table 34). YN-011 preparations with a specification of 0.75 mL/tube were prepared according to the excipient composition shown in Table 33, wherein the YN-011 protein content was 4 mg, 5 mg, 7.5 mg and 9 mg respectively (as shown in Table 34). The long-term stability test was performed on each of the prepared preparations according to the detection method described in Example 13.2.

Table 32. Composition of excipients in the 0.5 mL/vial formulation

| Excipients | Content (mg/syringe) | Final concentration | Main Function |
|---|---|---|---|
| Disodium hydrogen phosphate dodecahydrate | 0.58 | 10 mM | pH Adjusters |
| Sodium dihydrogen phosphate monohydrate | 0.465 | | pH Adjusters |
| Mannitol | 23.20 | 4.6% (w/v) | Osmotic pressure regulator |
| Polysorbate 80 | 0.10 | 0.02% (w/v) | Protein Stabilizer |
| Water for injection | Add to 0.5 mL | | Solvents |

Table 33. Composition of excipients in the formulation of 0.75 mL/vial

| Excipients | Content (mg/syringe) | Final concentration | Main Function |
|---|---|---|---|
| Disodium hydrogen phosphate dodecahydrate | 0.87 | 10 mM | pH Adjusters |
| Sodium dihydrogen phosphate monohydrate | 0.6975 | | pH Adjusters |
| Mannitol | 34.8 | 4.6% (w/v) | Osmotic pressure regulator |
| Polysorbate 80 | 0.15 | 0.02% (w/v) | Protein Stabilizer |
| Water for injection | Add to 0.5 mL | | Solvents |

Table 34. Preparation samples of different strengths

| No. | YN-011 protein content | Loading amount | Protein concentration |
|---|---|---|---|
| 1mg-1 | 1 mg | 0.5 mL | 2.0±0.2 mg/mL |
| 1mg-2 | | | |
| 1mg-3 | | | |
| 1mg-4 | | | |
| 1mg-5 | | | |
| 2mg-1 | 2 mg | 0.5 mL | 4.0±0.4 mg/mL |
| 2mg-2 | | | |
| 3mg-1 | 3 mg | 0.5 mL | 6.0±0.6 mg/mL |
| 3mg-2 | | | |
| 3mg-3 | | | |
| 3mg-4 | | | |
| 3mg-5 | | | |
| 4mg-1 | 4 mg | 0.75 mL | 5.3±0.5 mg/mL |
| 4mg-2 | | | |
| 5mg-1 | 5 mg | 0.75 mL | 6.7±0.6 mg/mL |
| 5mg-2 | | | |
| 7.5mg-1 | 7.5 mg | 0.75 mL | 10.0±1.0 mg/mL |
| 7.5mg-2 | | | |
| 9mg-1 | 9mg | 0.75 mL | 12.0±1.2 mg/mL |

The preparation methods of each preparation are briefly described as follows:

(1) Preparation of buffer solution: weigh disodium hydrogen phosphate dodecahydrate, sodium dihydrogen phosphate monohydrate, mannitol and polysorbate 80 according to the contents listed in Table 32 and Table 33, and dissolve the weighed excipient components in water for injection and sterilize and filter;
(2) Thawing of stock solution: thaw the unthawed stock solution in a water bath at 18°C-26° C in the dark;
(3) Dilution and mixing of stock solution: stir and mix the thawed stock solution in step (2) and the buffer solution prepared in step (1) in a certain proportion;
(4) Sterilization and filtration: sterilize and filter the drug solution prepared in step (3) into a sterile container;
(5) Packaging: pack the drug solution prepared in step (4) according to the filling amount corresponding to the target specification.

13.2 Detection method

13.2.1 Appearance

Take about 1 ml of the sample and place it in a clean colorimetric tube, and take about 1 ml of ultrapure water and place it in a clean colorimetric tube as a control. Under ordinary laboratory lighting, place the sample and ultrapure water on a white background and compare the color in the horizontal direction; under a clarity meter, adjust the illumination to 1000 lx, place the sample and ultrapure water on a black background and compare the clarity in the horizontal direction; use the visual method to detect visible particles.

13.2.2 Light blocking method

Use an insoluble particle detector to measure the insoluble particles of the sample according to the operating procedures of the detector. The measurement should be at least 4 times, and each sample should be no less than 5 ml. Discard the first measurement data, take the average of the subsequent 3 measurement data as the measurement result,

and calculate the number of particles contained in each container based on the sampling volume and the volume of the marking device of each container.

13.2.3 pH value

Use the potentiometric method, calibrate the pH meter with the standard solution, take 0.2 ml of sample, measure three times in parallel and record the measured pH value, and report the test result as the average value.

13.2.4 Osmotic pressure

Use the freezing point osmometer, calibrate the instrument, and measure the osmotic pressure molar concentration of the sample according to the operating procedures. Mix the sample before testing, check the integrity of the sample tube, use a pipette to take 50μl of the sample into a clean and dry sample tube, and measure its osmotic pressure molar concentration. Each sample is tested three times in parallel, and the test result is reported as the average value.

13.2.5 icIEF method

In the Protein Simple Maurice C capillary electrophoresis system, the charged isomers in each sample are focused and separated according to their different isoelectric points. Set the instrument parameters as follows: pre-focusing: 1500 V, 1 minute; focusing: 3000 V, 8 minutes; injection time: 90 seconds; UV detection wavelength: 280 nm; sample plate temperature: 15°C. Add two markers with known isoelectric points (pl maker) to the sample and focus them at the same time to calibrate the isoelectric point of the sample to be tested. Export the spectrum, report the isoelectric point of the main peak of the sample, report the peak area percentage of the main peak, Group A and Group B, and use the peak area normalization method to obtain the main peak content, Group A content and Group B content.

13.2.6 SEC-HPLC method

Thermo UltiMate 3000 high performance liquid chromatograph was used to detect the purity of YN-011 protein by molecular exclusion chromatography. The instrument parameters were set as follows: TSKgelG3000 column (5 μm, 7.8 × 300 mm); flow rate: 0.5 ml/min; injection volume: 25μL; column temperature: 25±3°C; injection plate temperature: 5±3°C; detection wavelength: 280 nm; sampling time: 35 min; elution method: isocratic elution. Mobile phase A: 50mmol/L phosphate buffer, 150mmol/L NaCl, 500mmol/L L-arginine hydrochloride, 10%IPA, pH 6.3±0.1.

13.2.7 Non-reduced CE-SDS method (non-reduced sodium dodecyl sulfate capillary electrophoresis)

The purity of YN-011 protein was detected by non-reduced sodium dodecyl sulfate capillary electrophoresis using Beckman PA800 Plus capillary electrophoresis instrument, SDS-MW Gel Buffer and Uncoated Capillary purchased from SCIEX. The instrument was set up according to the standard operating procedures. The output spectrum was integrated and the purity of the protein was calculated according to the peak area normalization method. The protein purity of the non-reduced sample was the percentage of the main peak peak area, and the contents of LMW and HMW were reported separately, where the LMW content was the sum of the corrected peak area percentages on the left side of the main peak, and the HMW content was the sum of all corrected peak area percentages on the right side of the main peak.

13.2.8 Reduced CE-SDS method (reduced sodium dodecyl sulfate capillary electrophoresis)

The reduced sodium dodecyl sulfate capillary electrophoresis method was used to detect the purity of YN-011 protein using Beckman PA800 Plus capillary electrophoresis instrument, SDS-MW Gel Buffer and Uncoated Capillary purchased from SCIEX. The instrument was set up according to the standard operating procedures. The output spectrum was integrated and the purity of the protein was calculated according to the peak area normalization method. The protein purity of the reduced sample was the percentage of the main peak area. The protein purity and the content of Others were reported, where Others was the sum of the percentage of the remaining corrected peak areas except the main peak.

13.2.9 UV method (ultraviolet spectrophotometry)

Use UV spectrophotometers (UV-1900, UV-1900i) purchased from Shimadzu to determine the YN-011 protein concentration by UV spectrophotometry, photometric and spectral determination, respectively, with a detection wavelength of 280nm. The reported protein content value is the average value of two parallel preparations and tests of the sample, and the reporting unit is mg/mL.

13.2.10 Biological activity detection

The biological activity of the sample is determined by detecting cAMP activity. Prepare cAMP stock solution and use cAMP-Gs Dynamic kit to measure the amount of cAMP produced in CHO K1 cells. The operation steps are briefly described as follows:

- · Sample processing: Add the diluted sample to the 96-well cell culture plate at 20 μl/well according to the experimental layout;
- · Cell processing: Use a spray gun to adjust the CHO K1 cell density to 7.5x105cells/ml and add the cell solution to columns 1 to 11 of the 96-well cell culture plate, 20 μl per well;
- · Cell and sample incubation: Centrifuge the reaction plate at 300rpm for 30s, then incubate at 25±2°C for 30±8 minutes;
- · Color development: Add 40 μl/well of the mixture of cAMP antibody, cAMP-d2 and cAMP lysis buffer to the 96-well cell culture plate for 60±9 minutes;
- · Read the plate;
- Data processing: Relative activity (%) = (reference $EC_{50}$/test sample $EC_{50}$) X100%

13.3 Test results

13.3.1 Physical and chemical identification

The isoelectric point was determined by the icIEF method. In the long-term stability study at 2-8°C, the main peak isoelectric point of all preparations remained within 6.2±0.2 within 24 months after the start of the test, which was consistent with the working reference and in compliance with regulations.

In the appearance observation, all preparation samples were colorless and clear liquids. The visible foreign matter detection method was used to detect visible foreign matter, and all preparations met the regulations. The light obstruction method was used to detect insoluble particles, and all preparations met the regulations.

During the sampling time, the pH value of all samples was stable in the range of 6.7±0.3, and the pH stability of the preparations was good.

The osmotic pressure molar concentration of the samples was determined by the osmotic pressure molar concentration determination method. During the sampling time, the osmotic pressure molar concentration of all samples was stable in the range of 290±50 mOsmol/kg, and the osmotic pressure of the preparations was relatively stable.

13.3.2 Purity and Charge Variant Determination

Tables 35, 36 and 37 show the protein degradation levels of the preparations tested by SEC-HPLC, non-reduced CE-SDS and reduced CE-SDS respectively during the sampling time. All samples showed a main peak purity greater than 95%.

Table 35. Protein stability results determined by SEC-HPLC

| **Sample No.** | **SEC-HPLC** | Time (month) | | | | | | |
|---|---|---|---|---|---|---|---|---|
| | | **0** | **3** | **6** | **9** | **12** | **18** | **24** |
| 1mg-1 | Monomer % | 98.0 | 97.6 | 97.6 | 97.4 | 97.3 | 97.1 | 96.9 |
| | HMW% | 2.0 | 2.4 | 2.4 | 2.6 | 2.7 | 2.9 | 3.1 |
| | LMW% | 0.0 | 0.0 | 0.0 | 0.0 | 0.0 | 0.0 | 0.0 |
| 1mg-2 | Monomer % | 97.9 | 97.7 | 97.3 | 96.8 | | | |
| | HMW% | 2.1 | 2.3 | 2.7 | 3.2 | | | |
| | LMW% | 0.0 | 0.0 | 0.0 | 0.0 | | | |
| 1mg-3 | Monomer % | 98.2 | 98.2 | 98.2 | 98.1 | 97.8 | | |
| | HMW% | 1.8 | 1.8 | 1.8 | 1.9 | 2.2 | | |
| | LMW% | 0.0 | 0.0 | 0.0 | 0.0 | 0.0 | | |

(continued)

| Sample No. | SEC-HPLC | Time (month) | | | | | | |
|---|---|---|---|---|---|---|---|---|
| | | 0 | 3 | 6 | 9 | 12 | 18 | 24 |
| 1mg-4 | Monomer % | 98.3 | 98.2 | 98.2 | 98.1 | 97.9 | | |
| | HMW% | 1.7 | 1.8 | 1.8 | 1.9 | 2.1 | | |
| | LMW% | 0.0 | 0.0 | 0.0 | 0.0 | 0.0 | | |
| 1mg-5 | Monomer % | 98.3 | 98.4 | 98.0 | 98.0 | 97.9 | | |
| | HMW% | 1.7 | 1.6 | 2.0 | 2.0 | 2.1 | | |
| | LMW% | 0.0 | 0.0 | 0.0 | 0.0 | 0.0 | | |
| 2mg-1 | Monomer % | 98.0 | 97.4 | 97.5 | 97.1 | 97.1 | 96.8 | 96.9 |
| | HMW% | 2.0 | 2.6 | 2.5 | 2.9 | 2.9 | 3.2 | 3.2 |
| | LMW% | 0.0 | 0.0 | 0.0 | 0.0 | 0.0 | 0.0 | 0.0 |
| 2mg-2 | Monomer % | 97.8 | 97.6 | | | | | |
| | HMW% | 2.2 | 2.4 | | | | | |
| | LMW% | 0.0 | 0.0 | | | | | |
| 3mg-1 | Monomer % | 98.0 | 97.3 | 97.5 | 96.9 | 96.9 | 96.9 | 96.5 |
| | HMW% | 2.0 | 2.7 | 2.5 | 3.1 | 3.1 | 3.1 | 3.5 |
| | LMW% | 0.0 | 0.0 | 0.0 | 0.0 | 0.0 | 0.0 | 0.0 |
| 3mg-2 | Monomer % | 97.8 | 97.5 | 96.9 | 96.5 | | | |
| | HMW% | 2.2 | 2.5 | 3.1 | 3.5 | | | |
| | LMW% | 0.0 | 0.0 | 0.0 | 0.0 | | | |
| 3mg-3 | Monomer % | 98.1 | 98.0 | 97.9 | 97.9 | 97.6 | | |
| | HMW% | 1.9 | 2.0 | 2.1 | 2.1 | 2.4 | | |
| | LMW% | 0.0 | 0.0 | 0.0 | 0.0 | 0.0 | | |
| 3mg-4 | Monomer % | 98.2 | 98.1 | 98.0 | 98.0 | 97.8 | | |
| | HMW% | 1.8 | 1.9 | 2.0 | 2.0 | 2.2 | | |
| | LMW% | 0.0 | 0.0 | 0.0 | 0.0 | 0.0 | | |
| 3mg-5 | Monomer % | 98.3 | 98.2 | 97.9 | 98.0 | 97.8 | | |
| | HMW% | 1.7 | 1.8 | 2.1 | 2.0 | 2.2 | | |
| | LMW% | 0.0 | 0.0 | 0.0 | 0.0 | 0.0 | | |
| 4mg-1 | Monomer % | 98.0 | 97.1 | 97.0 | 97.0 | 96.8 | 96.8 | |
| | HMW% | 2.0 | 2.9 | 3.0 | 3.0 | 3.2 | 3.3 | |
| | LMW% | 0.0 | 0.0 | 0.0 | 0.0 | 0.0 | 0.0 | |
| 4mg-2 | Monomer % | 98.4 | 97.7 | 97.7 | 97.6 | 97.5 | 97.4 | |
| | HMW% | 1.6 | 2.3 | 2.3 | 2.4 | 2.5 | 2.6 | |
| | LMW% | 0.0 | 0.0 | 0.0 | 0.0 | 0.0 | 0.0 | |
| 5mg-1 | Monomer % | 98.3 | 97.6 | 97.6 | 97.5 | 97.3 | 97.1 | |
| | HMW% | 1.7 | 2.4 | 2.4 | 2.5 | 2.7 | 2.9 | |
| | LMW% | 0.0 | 0.0 | 0.0 | 0.0 | 0.0 | 0.0 | |

(continued)

| Sample No. | SEC-HPLC | Time (month) | | | | | | |
|---|---|---|---|---|---|---|---|---|
| | | 0 | 3 | 6 | 9 | 12 | 18 | 24 |
| 5mg-2 | Monomer % | 98.1 | 97.6 | 97.7 | 97.9 | 97.4 | 97.1 | |
| | HMW% | 1.9 | 2.4 | 2.3 | 2.1 | 2.6 | 2.9 | |
| | LMW% | 0.0 | 0.0 | 0.0 | 0.0 | 0.0 | 0.0 | |
| 7.5mg-1 | Monomer % | 98.1 | 97.5 | 97.6 | 97.4 | 97.4 | 97.2 | |
| | HMW% | 1.9 | 2.5 | 2.4 | 2.6 | 2.6 | 2.8 | |
| | LMW% | 0.0 | 0.0 | 0.0 | 0.0 | 0.0 | 0.0 | |
| 7.5mg-2 | Monomer % | 97.9 | 97.7 | 97.5 | 98.0 | 97.2 | 96.9 | |
| | HMW% | 2.1 | 2.3 | 2.5 | 2.0 | 2.8 | 3.1 | |
| | LMW% | 0.0 | 0.0 | 0.0 | 0.0 | 0.0 | 0.0 | |
| 9mg-1 | Monomer % | 98.1 | 97.5 | | | | | |
| | HMW% | N/A | 2.5 | | | | | |
| | LMW% | N/A | 0.0 | | | | | |

Table 36. Protein stability results determined by non-reduced CE-SDS method

| Sample No. | Non-reduced CE-SDS | Time (month) | | | | | | |
|---|---|---|---|---|---|---|---|---|
| | | 0 | 3 | 6 | 9 | 12 | 18 | 24 |
| 1mg-1 | Main Peak % | 98.8 | 98.7 | 98.2 | 98.3 | 97.1 | 97.7 | 97.4 |
| | HMW% | 0.9 | 0.9 | 1.5 | 1.4 | 2.5 | 1.8 | 2.1 |
| | LMW% | 0.2 | 0.4 | 0.3 | 0.3 | 0.4 | 0.5 | 0.6 |
| 1mg-2 | Main Peak % | 98.7 | 98.5 | 98.1 | 97.1 | | | |
| | HMW% | 1.1 | 1.2 | 1.5 | 2.4 | | | |
| | LMW% | 0.3 | 0.3 | 0.4 | 0.5 | | | |
| 1mg-3 | Main Peak % | 99.3 | 98.8 | 98.8 | 98.7 | 98.4 | | |
| | HMW% | 0.6 | 1.1 | 1.0 | 1.1 | 1.4 | | |
| | LMW% | 0.2 | 0.2 | 0.2 | 0.3 | 0.2 | | |
| 1mg-4 | Main Peak % | 98.8 | 98.9 | 98.8 | 98.7 | 98.6 | | |
| | HMW% | 1.0 | 0.9 | 1.0 | 1 | 1.1 | | |
| | LMW% | 0.1 | 0.2 | 0.2 | 0.3 | 0.2 | | |
| 1mg-5 | Main Peak % | 99.2 | 99.5 | 98.8 | 98.8 | 98.5 | | |
| | HMW% | 0.7 | 0.4 | 1.0 | 1.0 | 1.2 | | |
| | LMW% | 0.1 | 0.2 | 0.2 | 0.2 | 0.4 | | |
| 2mg-1 | Main Peak % | 98.5 | 98.6 | 98.1 | 98.2 | 97.2 | 97.4 | 97.4 |
| | HMW% | 1.1 | 1.0 | 1.5 | 1.5 | 2.4 | 2.0 | 2.0 |
| | LMW% | 0.3 | 0.4 | 0.3 | 0.4 | 0.4 | 0.6 | 0.6 |
| 2mg-2 | Main Peak % | 98.4 | 98.3 | | | | | |
| | HMW% | 1.5 | 1.5 | | | | | |
| | LMW% | 0.2 | 0.3 | | | | | |

(continued)

| Sample No. | Non-reduced CE-SDS | Time (month) | | | | | | |
|---|---|---|---|---|---|---|---|---|
| | | 0 | 3 | 6 | 9 | 12 | 18 | 24 |
| 3mg-1 | Main Peak % | 98.6 | 97.3 | 97.8 | 98.0 | 97.1 | 97.2 | 97.2 |
| | HMW% | 1.1 | 2.3 | 1.8 | 1.7 | 2.5 | 2.2 | 2.3 |
| | LMW% | 0.3 | 0.3 | 0.4 | 0.4 | 0.5 | 0.5 | 0.6 |
| 3mg-2 | Main Peak % | 98.1 | 98.1 | 97.7 | 96.8 | | | |
| | HMW% | 1.6 | 1.5 | 1.9 | 2.7 | | | |
| | LMW% | 0.3 | 0.3 | 0.4 | 0.5 | | | |
| 3mg-3 | Main Peak % | 99.4 | 98.7 | 98.5 | 98.4 | 98.4 | | |
| | HMW% | 0.5 | 1.1 | 1.3 | 1.4 | 1.3 | | |
| | LMW% | 0.2 | 0.2 | 0.2 | 0.3 | 0.2 | | |
| 3mg-4 | Main Peak % | 99.1 | 98.5 | 98.6 | 98.6 | 98.5 | | |
| | HMW% | 0.7 | 1.3 | 1.1 | 1.1 | 1.2 | | |
| | LMW% | 0.1 | 0.2 | 0.3 | 0.2 | 0.3 | | |
| 3mg-5 | Main Peak % | 99.2 | 99.0 | 98.5 | 98.6 | 98.0 | | |
| | HMW% | 0.6 | 0.9 | 1.3 | 1.2 | 1.6 | | |
| | LMW% | 0.1 | 0.2 | 0.2 | 0.3 | 0.4 | | |
| 4mg-1 | Main Peak % | 98.8 | 98.2 | 96.9 | 98.1 | 97.7 | 96.9 | |
| | HMW% | 0.9 | 1.4 | 2.6 | 1.5 | 1.9 | 2.6 | |
| | LMW% | 0.3 | 0.4 | 0.4 | 0.4 | 0.5 | 0.5 | |
| 4mg-2 | Main Peak % | 99.1 | 98.9 | 98.1 | 98.6 | 98.2 | 97.6 | |
| | HMW% | 0.8 | 0.9 | 1.7 | 1.2 | 1.4 | 1.9 | |
| | LMW% | 0.2 | 0.2 | 0.3 | 0.3 | 0.3 | 0.4 | |
| 5mg-1 | Main Peak % | 99.0 | 99.0 | 98.1 | 98.3 | 98.1 | 97.4 | |
| | HMW% | 0.8 | 0.8 | 1.7 | 1.4 | 1.6 | 2.2 | |
| | LMW% | 0.2 | 0.1 | 0.2 | 0.3 | 0.3 | 0.4 | |
| 5mg-2 | Main Peak % | 98.9 | 98.8 | 98.2 | 97.5 | 98.2 | 97.8 | |
| | HMW% | 0.9 | 1.0 | 1.4 | 2.2 | 1.5 | 1.8 | |
| | LMW% | 0.2 | 0.2 | 0.3 | 0.3 | 0.3 | 0.4 | |
| 7.5mg-1 | Main Peak % | 99.0 | 98.9 | 98.0 | 98.3 | 98.1 | 97.6 | |
| | HMW% | 0.8 | 0.9 | 1.8 | 1.4 | 1.5 | 2.0 | |
| | LMW% | 0.2 | 0.2 | 0.2 | 0.3 | 0.3 | 0.4 | |
| 7.5mg-2 | Main Peak % | 98.9 | 97.8 | 98.3 | 99.1 | 97.9 | 97.5 | |
| | HMW% | 1 | 2.0 | 1.5 | 0.7 | 1.8 | 2.1 | |
| | LMW% | 0.2 | 0.2 | 0.2 | 0.2 | 0.3 | 0.4 | |
| 9mg-1 | Main Peak % | 98.6 | 98.4 | | | | | |
| | HMW% | N/A | 1.4 | | | | | |
| | LMW% | N/A | 0.2 | | | | | |

Table 37. Protein stability results determined by reduced CE-SDS method

| Sample No. | Reduced CE-SDS | Time (month) | | | | | | |
|---|---|---|---|---|---|---|---|---|
| | | 0 | 3 | 6 | 9 | 12 | 18 | 24 |
| 1mg-1 | Main Peak % | 96.4 | 96.3 | 96.3 | 95.8 | 96.2 | 96.4 | 95.9 |
| | Others% | 3.6 | 3.7 | 3.7 | 4.2 | 3.8 | 3.6 | 4.1 |
| 1mg-2 | Main Peak % | 96.8 | 96.6 | 96.4 | 95.8 | | | |
| | Others% | 3.2 | 3.4 | 3.6 | 4.2 | | | |
| 1mg-3 | Main Peak % | 96.6 | 96.6 | 96.7 | 96.5 | 96.5 | | |
| | Others% | 3.4 | 3.4 | 3.3 | 3.5 | 3.5 | | |
| 1mg-4 | Main Peak % | 97.1 | 96.5 | 96.8 | 96.5 | 96.5 | | |
| | Others% | 2.9 | 3.5 | 3.2 | 3.5 | 3.5 | | |
| 1mg-5 | Main Peak % | 97.3 | 97.1 | 97.1 | 97.1 | 96.8 | | |
| | Others% | 2.7 | 2.9 | 2.9 | 2.9 | 3.2 | | |
| 2mg-1 | Main Peak % | 96.5 | 96.3 | 96.0 | 95.5 | 95.9 | 96.2 | 95.9 |
| | Others% | 3.5 | 3.7 | 4.1 | 4.6 | 4.1 | 3.8 | 4.1 |
| 2mg-2 | Main Peak % | 97.1 | 96.7 | | | | | |
| | Others% | 2.9 | 3.3 | | | | | |
| 3mg-1 | Main Peak % | 96.6 | 96.3 | 96.2 | 95.3 | 96.1 | 96.3 | 95.7 |
| | Others% | 3.4 | 3.7 | 3.8 | 4.7 | 3.9 | 3.7 | 4.3 |
| 3mg-2 | Main Peak % | 96.9 | 96.7 | 96.2 | 95.5 | | | |
| | Others% | 3.1 | 3.3 | 3.8 | 4.5 | | | |
| 3mg-3 | Main Peak % | 96.8 | 97.3 | 96.8 | 96.2 | 96.3 | | |
| | Others% | 3.2 | 2.7 | 3.2 | 3.8 | 3.7 | | |
| 3mg-4 | Main Peak % | 97.1 | 97.3 | 97.1 | 96.7 | 96.4 | | |
| | Others% | 2.9 | 2.7 | 2.9 | 3.3 | 3.6 | | |
| 3mg-5 | Main Peak % | 97.1 | 97.1 | 97.1 | 96.8 | 96.6 | | |
| | Others% | 2.9 | 2.9 | 2.9 | 3.2 | 3.4 | | |
| 4mg-1 | Main Peak % | 96.5 | 95.8 | 96.2 | 95.9 | 95.9 | 95.6 | |
| | Others% | 2.8 | 4.2 | 3.8 | 4.1 | 4.1 | 4.4 | |
| 4mg-2 | Main Peak % | 96.7 | 96.7 | 96.6 | 96.5 | 96.3 | 95.9 | |
| | Others% | 3.3 | 3.3 | 3.4 | 3.5 | 3.7 | 4.1 | |
| 5mg-1 | Main Peak % | 96.8 | 96.5 | 96.6 | 96.5 | 96.3 | 95.7 | |
| | Others% | 3.2 | 3.5 | 3.4 | 3.5 | 3.7 | 4.3 | |
| 5mg-2 | Main Peak % | 97.1 | 97.1 | 96.5 | 96.6 | 96.9 | 96.4 | |
| | Others% | 2.9 | 2.9 | 3.5 | 3.4 | 3.1 | 3.6 | |
| 7.5mg-1 | Main Peak % | 96.3 | 96.1 | 96.7 | 96.2 | 95.9 | 95.8 | |
| | Others% | 3.7 | 3.9 | 3.3 | 3.8 | 4.1 | 4.2 | |
| 7.5mg-2 | Main Peak % | 97.6 | 97.2 | 96.5 | 96.8 | 96.7 | 96.4 | |
| | Others% | 2.4 | 2.8 | 3.5 | 3.2 | 3.3 | 3.6 | |
| 9mg-1 | Main Peak % | 97.0 | 96.7 | | | | | |
| | Others% | N/A | 3.3 | | | | | |

The proportion of the main peak measured by the icIEF method for all preparations was greater than 46%, and varied little within the sampling interval, as shown in Table 38.

Table 38. Protein stability results of icIEF assay

| No. | icIEF | Time (month) | | | | | | |
|---|---|---|---|---|---|---|---|---|
| | | **0** | **3** | **6** | **9** | **12** | **18** | **24** |
| 1mg-1 | Main Peak % | 52.7 | 49.6 | 50.2 | 47.8 | 49.2 | 48.7 | 47.0 |
| | Group A%, Group B% | A:41.7 B:5.6 | A:43.8 B:6.6 | A:44.2 B:5.8 | A:44.6 B:7.6 | A:45.1 B:5.8 | A:45.7 B:5.7 | A:46.8 B:6.2 |
| 1mg-2 | Main Peak % | 52.7 | 50.6 | 49.7 | 46.8 | | | |
| | Group A%, Group B% | A:42.1 B:5.3 | A:43.2 B:6.4 | A:44.7 B:5.8 | A:46.7 B:6.5 | | | |
| 1mg-3 | Main Peak % | 58.6 | 57.0 | 56.2 | 55.6 | 55.0 | | |
| | Group A%, Group B% | A:36.7 B:4.8 | A:38.6 B:4.6 | A:39.3 B:4.4 | A:39.9 B:4.6 | A:40.2 B:4.8 | | |
| 1mg-4 | Main Peak % | 58.3 | 57.2 | 56.5 | 55.1 | 55.1 | | |
| | Group A%, Group B% | A:37.1 B:4.7 | A:38.5 B:4.4 | A:39.3 B:4.3 | A:40.4 B:4.6 | A:40.2 B:4.7 | | |
| 1mg-5 | Main Peak % | 58.7 | 57.1 | 56.2 | 55.7 | 54.7 | | |
| | Group A%, Group B% | A:37.0 B:4.3 | A:38.7 B:4.2 | A:39.5 B:4.3 | A:39.8 B:4.5 | A:40.5 B:4.8 | | |
| 2mg-1 | Main Peak % | 52.5 | 49.3 | 50.2 | 48.7 | 49.6 | 48.1 | 48.1 |
| | Group A%, Group B% | A:41.8 B:5.8 | A:44.7 B:6.1 | A:44.3 B:5.6 | A:44.7 B:6.7 | A:44.8 B:5.7 | A:46.3 B:5.7 | A:45.9 B:6.0 |
| 2mg-2 | Main Peak % | 52.0 | 50.4 | | | | | |
| | Group A%, Group B% | A:42.6 B:5.5 | A:43.5 B:6.1 | | | | | |
| 3mg-1 | Main Peak % | 52.8 | 49.1 | 50.4 | 48.6 | 49.7 | 49.0 | 48.2 |
| | Group A%, Group B% | A:41.5 B:5.7 | A:45.0 B:5.9 | A:44.3 B:5.4 | A:44.1 B:7.5 | A:44.2 B:6.1 | A:45.6 B:5.5 | A:46.0 B:5.8 |
| 3mg-2 | Main Peak % | 51.9 | 50.7 | 49.9 | 48.7 | | | |
| | Group A%, Group B% | A:42.7 B:5.5 | A:43.4 B:5.9 | A:44.9 B:5.4 | A:45.1 B:6.2 | | | |
| 3mg-3 | Main Peak % | 58.4 | 57.5 | 56.6 | 56.0 | 55.6 | | |
| | Group A%, Group B% | A:36.8 B:4.9 | A:38.3 B:4.3 | A:38.9 B:4.5 | A:39.6 B:4.5 | A:39.8 B:4.7 | | |
| 3mg-4 | Main Peak % | 58.1 | 57.2 | 56.5 | 56.9 | 56.0 | | |
| | Group A%, Group B% | A:37.5 B:4.5 | A:38.7 B:4.3 | A:39.2 B:4.4 | A:39.3 B:3.9 | A:39.5 B:4.5 | | |
| 3mg-5 | Main Peak % | 58.1 | 57.4 | 56.2 | 56.1 | 55.9 | | |
| | Group A%, Group B% | A:37.6 B:4.5 | A:38.4 B:4.2 | A:39.6 B:4.4 | A:39.5 B:4.3 | A:39.5 B:4.6 | | |
| 4mg-1 | Main Peak % | 52.7 | 49.9 | 51.1 | 49.7 | 49.8 | 49.1 | |
| | Group A%, Group B% | A:41.6 B:5.7 | A:42.4 B:7.8 | A:42.8 B:6.1 | A:43.2 B:7.0 | A:43.8 B:6.4 | A:43.9 B:7.0 | |

(continued)

| No. | icIEF | Time (month) | | | | | | |
|---|---|---|---|---|---|---|---|---|
| | | 0 | 3 | 6 | 9 | 12 | 18 | 24 |
| 4mg-2 | Main Peak % | 58.1 | 56.1 | *55.5* | 55.2 | 55.3 | 53.9 | |
| | Group A%, Group B% | A:37.4 B:4.5 | A:38.5 B:5.5 | A:38.8 B:5.7 | A:39.2 B:5.7 | A:39.9 B:4.9 | A:40.3 B:5.8 | |
| 5mg-1 | Main Peak % | 57.0 | 56.0 | 56.0 | 54.8 | 55.0 | 54.0 | |
| | Group A%, Group B% | A:38.4 B:4.7 | A:38.9 B:5.3 | A:38.9 B:5.2 | A:39.5 B:5.9 | A:40.0 B:5.0 | A:40.5 B:5.6 | |
| 5mg-2 | Main Peak % | 57.1 | 54.7 | 56.6 | 55.8 | 55.2 | 54.8 | |
| | Group A%, Group B% | A:38.8 B:4.2 | A:39.1 B:6.3 | A:39.0 B:4.5 | A:39.8 B:4.5 | A:40.5 B:4.5 | A:40.7 B:5.4 | |
| 7.5mg-1 | Main Peak % | 56.1 | 55.9 | 56.7 | 55.7 | 56.0 | 55.0 | |
| | Group A%, Group B% | A:39.2 B:4.9 | A:39.2 B:5.1 | A:38.3 B:5.2 | A:39.3 B:5.1 | A:40.0 B:4.2 | A:40.1 B:4.9 | |
| 7.5mg-2 | Main Peak % | 57.8 | 56.3 | 56.5 | 55.8 | 55.4 | 53.4 | |
| | Group A%, Group B% | A:38.3 B:4.0 | A:39.2 B:4.6 | A:39.4 B:4.2 | A:40.3 B:4.1 | A:40.6 B:4.1 | A:40.3 B:6.4 | |
| 9mg-1 | Main Peak % | 58.6 | 57.3 | | | | | |
| | Group A%, Group B% | A:37.2 B:4.2 | A:38.0 B:4.7 | | | | | |

13.3.3 Biological potency

As shown in Table 39, the relative biological activity was determined by detecting cAMP activity. All tested samples showed a relative biological activity of 85%-121 %, which met the relevant quality standards.

Table 39. Cell-based cAMP assay results

| No. | | Time (month) | | | | | | |
|---|---|---|---|---|---|---|---|---|
| | | 0 | 3 | 6 | 9 | 12 | 18 | 24 |
| 1mg-1 | Relative activity (%) | 102 | 86 | 100 | 101 | 100 | 97 | 100 |
| 1mg-2 | Relative activity (%) | 116 | 95 | 99 | 93 | | | |
| 1mg-3 | Relative activity (%) | 93 | 91 | 99 | 90 | 87 | | |
| 1mg-4 | Relative activity (%) | 98 | 94 | 99 | 99 | 110 | | |
| 1mg-5 | Relative activity (%) | 97 | 90 | 92 | 100 | 104 | | |
| 2mg-1 | Relative activity (%) | 93 | 95 | 119 | 110 | 93 | 115 | 103 |
| 2mg-2 | Relative activity (%) | 119 | 89 | | | | | |
| 3mg-1 | Relative activity (%) | 107 | 117 | 120 | 107 | 91 | 108 | 105 |
| 3mg-2 | Relative activity (%) | 85 | 84 | 82 | 95 | | | |
| 3mg-3 | Relative activity (%) | 93 | 104 | 94 | 108 | 105 | | |
| 3mg-4 | Relative activity (%) | 86 | 100 | 106 | 102 | 123 | | |
| 3mg-5 | Relative activity (%) | 104 | 101 | 93 | 127 | 95 | | |
| 4mg-1 | Relative activity (%) | 93 | 112 | 88 | 103 | 107 | 105 | |
| 4mg-2 | Relative activity (%) | 98 | 104 | 93 | 101 | 104 | 99 | |

(continued)

| No. | | Time (month) | | | | | | |
|---|---|---|---|---|---|---|---|---|
| | | 0 | 3 | 6 | 9 | 12 | 18 | 24 |
| 5mg-1 | Relative activity (%) | 104 | 90 | 97 | 105 | 106 | 111 | |
| 5mg-2 | Relative activity (%) | 102 | 99 | 102 | 102 | 96 | | |
| 7.5mg-1 | Relative activity (%) | 94 | 94 | 93 | 96 | 104 | 106 | |
| 7.5mg-2 | Relative activity (%) | 121 | 85 | 101 | 102 | 102 | | |
| 9mg-1 | Relative activity (%) | 105 | | | | | | |

13.3.4 Protein concentration

As shown in Table 40, the protein concentration was determined by UV spectroscopy. The concentrations of all tested samples were within the standard range and had good stability.

Table 40. Variation of protein concentration in formulations

| No. | Protein concentration | Time (month) | | | | | | |
|---|---|---|---|---|---|---|---|---|
| | | 0 | 3 | 6 | 9 | 12 | 18 | 24 |
| 1mg-1 | 2.0±0.2 mg/mL | 2.2 | 2.1 | 2.2 | 2.2 | 2.2 | 2.2 | 2.2 |
| 1mg-2 | | 2.2 | 2.2 | 2.2 | 2.2 | | | |
| 1mg-3 | | 2.1 | 2.1 | 2.1 | 2.1 | 2.1 | | |
| 1mg-4 | | 2.1 | 2.1 | 2.1 | 2.1 | 2.1 | | |
| 1mg-5 | | 2.1 | 2.1 | 2.1 | 2.1 | 2.1 | | |
| 2mg-1 | 4.0±0.4 mg/mL | 4.2 | 4.1 | 4.1 | 4.2 | 4.1 | 4.1 | 4.1 |
| 2mg-2 | | 4.2 | 4.2 | | | | | |
| 3mg-1 | 6.0±0.6 mg/mL | 6.1 | 6.1 | 6.1 | 6.2 | 6.1 | 6.1 | 6.1 |
| 3mg-2 | | 6.1 | 6.2 | 6.2 | 6.2 | | | |
| 3mg-3 | | 6.2 | 6.2 | 6.2 | 6.2 | 6.2 | | |
| 3mg-4 | | 6.2 | 6.2 | 6.2 | 6.2 | 6.2 | | |
| 3mg-5 | | 6.2 | 6.2 | 6.2 | 6.2 | 6.2 | | |
| 4mg-1 | 5.3±0.5 mg/mL | 5.6 | 5.5 | 5.5 | 5.4 | 5.4 | 5.5 | |
| 4mg-2 | | 5.6 | 5.7 | 5.7 | 5.7 | 5.7 | 5.7 | |
| 5mg-1 | 6.7±0.6 mg/mL | 6.7 | 6.9 | 6.8 | 6.7 | 6.7 | 6.8 | |
| 5mg-2 | | 6.6 | 6.7 | 6.7 | 6.7 | 6.7 | 6.7 | |
| 7.5mg-1 | 10.0±1.0 mg/mL | 10.0 | 10.0 | 10.0 | 10.1 | 10.1 | 10.1 | |
| 7.5mg-2 | | 10.0 | 10.0 | 10.1 | 10.0 | 10.0 | 10.0 | |
| 9mg-1 | 12.0±1.2 mg/mL | 12.1 | 12.1 | | | | | |

The above examples list what is currently considered to be the preferred examples of the present application, but it should be understood that the present application is not limited to the disclosed examples. On the contrary, the present application is intended to cover various modifications and equivalent examples included in the spirit and scope of the appended claims.

All publications, patents and patent applications are incorporated herein by reference in their entirety. Specifically, the sequences associated with each accession number provided herein, including, for example, accession numbers and/or biomarker sequences (e.g., proteins and/or polynucleotides) provided in the table or elsewhere, are incorporated by reference in their entirety.

The scope of the claims should not be limited by the preferred examples and embodiments, but should be interpreted as the broadest interpretation consistent with the specification.

## Claims

1. A pharmaceutical preparation comprising:

   (a) a GLP-1 fusion protein, the GLP-1 fusion protein comprising a GLP-1 polypeptide and an immunoglobulin Fc domain, wherein the GLP-1 polypeptide is covalently linked to the immunoglobulin Fc domain, wherein,

      the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the group consisting of A8G, G22E and R36G relative to native human GLP-1;
      the immunoglobulin Fc domain comprises or is an IgG2-Fc domain, the IgG2-Fc domain comprises one or more amino acid substitutions selected from the group consisting of C222S, A330S and P331S;
      and

   (b) a buffer;

   wherein, the pH value of the pharmaceutical preparation is in the range of about 6.0 to about 7.0.

2. A pharmaceutical preparation comprising:

   (a) a GLP-1 fusion protein, the fusion protein comprising a GLP-1 polypeptide and an immunoglobulin Fc domain, wherein the GLP-1 polypeptide is covalently linked to the immunoglobulin Fc domain, wherein,

      the GLP-1 polypeptide is selected from human GLP-1 (7-37), human GLP-1 (7-36) amide and DPP-IV resistant human GLP-1, and the GLP-1 polypeptide comprises one or more amino acid substitutions selected from the following group relative to native human GLP-1: A8G, G22E and R36G;
      the immunoglobulin Fc domain comprises or is an IgG2-Fc domain, the IgG2-Fc domain comprises one or more amino acid substitutions selected from the following group: C222S, A330S and P331S;
      and

   (b) a buffer, the buffer being selected from the following group: phosphate buffer, citrate buffer, borate buffer, histidine buffer and acetate buffer.

3. The pharmaceutical preparation according to claim 1 or 2, wherein the buffer is a phosphate buffer.

4. The pharmaceutical preparation according to claim 2 or 3, wherein the phosphate buffer comprises phosphate dibasic (e.g., disodium hydrogen phosphate, dipotassium hydrogen phosphate, etc.), dihydrogen phosphate (e.g., sodium dihydrogen phosphate, potassium dihydrogen phosphate, etc.) or a combination thereof.

5. The pharmaceutical preparation according to any one of claims 2 to 4, wherein the phosphate buffer is disodium hydrogen phosphate/sodium dihydrogen phosphate buffer.

6. The pharmaceutical preparation according to any one of claims 2 to 5, wherein the phosphate buffer is prepared from disodium hydrogen phosphate hydrate and sodium dihydrogen phosphate hydrate.

7. The pharmaceutical preparation according to any one of claims 2 to 6, wherein the phosphate buffer is prepared from disodium hydrogen phosphate dodecahydrate and sodium dihydrogen phosphate monohydrate.

8. The pharmaceutical preparation according to any one of claims 2 to 7, wherein the concentration of the phosphate buffer in the pharmaceutical preparation is about 5 mM to about 15 mM (e.g., about 10 mM).

9. The pharmaceutical preparation according to any one of the preceding claims, wherein the pharmaceutical preparation further comprises carbohydrates.

10. The pharmaceutical preparation according to claim 9, wherein the carbohydrates are selected from the group consisting of mannitol, sorbitol, maltitol, erythritol, arabitol, xylitol, sucrose, lactose, trehalose, dextran, and combinations thereof.

11. The pharmaceutical preparation according to any one of claims 9 to 10, wherein the carbohydrates are one or more selected from mannitol, sucrose, sorbitol, for example, the carbohydrates are mannitol.

12. The pharmaceutical preparation according to any one of claims 9 to 11, wherein the concentration of the carbohydrates in the pharmaceutical preparation is 1-10% (w/v), for example, about 4.6% (w/v).

13. The pharmaceutical preparation according to any one of the preceding claims, further comprising a surfactant.

14. The pharmaceutical preparation according to claim 13, wherein the surfactant is selected from the group consisting of polysorbates (e.g., polysorbate 80), polyoxyethylene castor oil derivatives, poloxamers (e.g., poloxamer 188), lecithin, polyethylene glycol 15-hydroxystearate, cyclodextrins, and combinations thereof.

15. The pharmaceutical preparation according to any one of claims 13 to 14, wherein the surfactant is polysorbate 80.

16. The pharmaceutical preparation according to any one of claims 13 to 15, wherein the concentration of the surfactant in the pharmaceutical preparation is 0.01% (w/v) - 0.04% (w/v), e.g., about 0.02% (w/v).

17. The pharmaceutical preparation according to any one of the preceding claims, wherein the pharmaceutical preparation comprises the GLP-1 fusion protein, a phosphate buffer, a carbohydrate, and a surfactant.

18. The pharmaceutical preparation according to any one of the preceding claims, wherein the pharmaceutical preparation comprises the GLP-1 fusion protein, a phosphate buffer (e.g., disodium hydrogen phosphate/sodium dihydrogen phosphate buffer), mannitol, and polysorbate.

19. The pharmaceutical preparation according to any one of the preceding claims, wherein the GLP-1 polypeptide in the GLP-1 fusion protein has a certain level of hydroxylation at lysine 34 (K34) relative to native human GLP-1.

20. The pharmaceutical preparation according to claim 19, wherein the hydroxylation level is between 10% and 100%, for example, greater than or equal to 10%, or greater than or equal to 15%, or greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90%.

21. The pharmaceutical preparation according to any of the preceding claims, wherein the GLP-1 polypeptide in the GLP-1 fusion protein is substantially unoxidized at tryptophan position 31 (W31) relative to native human GLP-1.

22. The pharmaceutical preparation according to any of the preceding claims, wherein the oxidation level of the GLP-1 polypeptide in the GLP-1 fusion protein at W31 relative to native human GLP-1 is less than 0.5% or undetectable.

23. The pharmaceutical preparation according to any of the preceding claims, wherein the GLP-1 polypeptide in the GLP-1 fusion protein has at least 90% sequence identity compared to the amino acid sequence shown in SEQ ID NO: 1, SEQ **ID** NO: 2 or SEQ ID NO: 3, and comprises one or more amino acid substitutions selected from the group consisting of A8G, G22E and R36G relative to native human GLP-1.

24. The pharmaceutical preparation according to any one of the preceding claims, wherein the GLP-1 polypeptide in the GLP-1 fusion protein is selected from human GLP-1 (7-37), human GLP-1 (7-36) amide and DPP-IV resistant human GLP-1, and comprises A8G and G22E substitutions relative to native human GLP-1.

25. The pharmaceutical preparation according to any one of the preceding claims, wherein the GLP-1 polypeptide in the GLP-1 fusion protein is selected from human GLP-1 (7-37), human GLP-1 (7-36) amide and DPP-IV resistant human GLP-1, and comprises A8G, G22E and R36G substitutions relative to native human GLP-1.

26. The pharmaceutical preparation according to any one of the preceding claims, wherein the GLP-1 polypeptide in the GLP-1 fusion protein is human GLP-1 (7-37), and comprises A8G, G22E and R36G substitutions relative to native

human GLP-1.

**27.** The pharmaceutical preparation according to any one of the preceding claims, wherein the amino acid sequence of the GLP-1 polypeptide in the GLP-1 fusion protein is as shown in SEQ ID NO: 3.

**28.** The pharmaceutical preparation according to any one of the preceding claims, wherein the IgG2-Fc domain in the GLP-1 fusion protein is an Fc domain from human IgG2.

**29.** The pharmaceutical preparation according to any one of the preceding claims, wherein the IgG2-Fc domain in the GLP-1 fusion protein has at least 90% sequence identity compared to the amino acid sequence shown in SEQ **ID** NO: 5 or SEQ **ID** NO: 6 and comprises one or more amino acid substitutions selected from the group consisting of C222S, A330S, and P331S.

**30.** The pharmaceutical preparation according to any one of the preceding claims, wherein the IgG2-Fc domain in the GLP-1 fusion protein has at least 90% sequence identity compared to the amino acid sequence shown in SEQ **ID** NO: 5 or SEQ **ID** NO: 6 and comprises A330S and P331S substitutions.

**31.** The pharmaceutical preparation according to any of the preceding claims, wherein the IgG2-Fc domain in the GLP-1 fusion protein further comprises a C222S substitution.

**32.** The pharmaceutical preparation according to any of the preceding claims, wherein the amino acid sequence of the IgG2-Fc domain in the GLP-1 fusion protein is as shown in SEQ **ID** NO: 6.

**33.** The pharmaceutical preparation according to any of the preceding claims, wherein the amino acid sequence of the GLP-1 polypeptide in the GLP-1 fusion protein is as shown in SEQ ID NO: 3, and the amino acid sequence of the immunoglobulin Fc domain is as shown in SEQ **ID** NO: 6.

**34.** The pharmaceutical preparation according to any of the preceding claims, wherein, in the GLP-1 fusion protein, the GLP-1 polypeptide is located at the N-terminus or C-terminus of the immunoglobulin Fc domain.

**35.** The pharmaceutical preparation according to any of the preceding claims, wherein, in the GLP-1 fusion protein, the GLP-1 polypeptide is directly covalently linked to the immunoglobulin Fc domain.

**36.** The pharmaceutical preparation according to any one of claims 1 to 35, wherein, in the GLP-1 fusion protein, the GLP-1 polypeptide is covalently linked to the immunoglobulin Fc domain via a linker.

**37.** The pharmaceutical preparation according to claim 36, wherein the linker is selected from the group consisting of a cleavable linker, a non-cleavable linker, a flexible linker, a rigid linker, a helical linker, and a non-helical linker.

**38.** The pharmaceutical preparation according to any one of claims 36 to 37, wherein the linker comprises a linker peptide connecting the GLP-1 polypeptide and the IgG2-Fc domain.

**39.** The pharmaceutical preparation according to claim 38, wherein the linker peptide comprises a linker containing glycine and serine.

**40.** The pharmaceutical preparation according to claim 39, wherein the linker containing glycine and serine comprises one, two, three, four or more repeats as shown in SEQ **ID** NO: 39 (GGGS), SEQ **ID** NO: 40 (GGGGS), SEQ **ID** NO: 41 (GGGGGS) or SEQ **ID** NO: 42 (GGGGGGGS).

**41.** The pharmaceutical preparation according to any one of claims 36 to 40, wherein the linker comprises an amino acid sequence selected from the group consisting of SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19.

**42.** The pharmaceutical preparation according to claim 41, wherein the linker comprises an amino acid sequence as shown in SEQ ID NO: 9.

**43.** The pharmaceutical preparation according to any one of claims 36 to 42, wherein the amino acid sequence of the GLP-1 polypeptide is as shown in SEQ ID NO: 3, the amino acid sequence of the immunoglobulin Fc domain is as

shown in SEQ ID NO: 6, and the amino acid sequence of the linker is as shown in SEQ ID NO: 9.

**44.** The pharmaceutical preparation according to any one of the preceding claims, wherein the GLP-1 fusion protein has an amino acid sequence as shown in SEQ ID NO: 7 or an amino acid sequence having at least 80% sequence identity with SEQ ID NO: 7.

**45.** The pharmaceutical preparation according to any one of the preceding claims, wherein the oxidation level of the IgG2-Fc domain at M253 corresponding to SEQ ID NO: 7 is less than or equal to 5%.

**46.** The pharmaceutical preparation according to any of the preceding claims, wherein the half-life of the GLP-1 fusion protein in a human subject is at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days.

**47.** The pharmaceutical preparation according to any of the preceding claims, wherein in the GLP-1 fusion protein, greater than or equal to 10%, or greater than or equal to 15%, or at least greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90% of the GLP-1 polypeptide in the fusion protein is hydroxylated at K34 relative to native human GLP-1.

**48.** The pharmaceutical preparation according to any one of the preceding claims, wherein the concentration of the GLP-1 fusion protein in the pharmaceutical preparation is 0.2-20 mg/mL (e.g., 1-5 mg/mL).

**49.** The pharmaceutical preparation according to any one of the preceding claims, wherein the pharmaceutical preparation comprises:

(a) a GLP-1 fusion protein, wherein the GLP-1 fusion protein has an amino acid sequence as shown in SEQ ID NO: 7;
(b) disodium hydrogen phosphate/sodium dihydrogen phosphate buffer;
(c) mannitol; and
(d) polysorbate 80.

**50.** The pharmaceutical preparation according to any one of the preceding claims, wherein the pharmaceutical preparation comprises:

(a) 0.2-20 mg/mL GLP-1 fusion protein, wherein the GLP-1 fusion protein has an amino acid sequence as shown in SEQ ID NO: 7;
(b) 5-15 mM disodium hydrogen phosphate/sodium dihydrogen phosphate buffer;
(c) 1-10% (w/v) mannitol; and
(d) 0.01% (w/v) - 0.04% (w/v) polysorbate 80.

**51.** The pharmaceutical preparation according to any of the preceding claims, wherein the pharmaceutical preparation comprises:

(a) about 2 mg/mL, about 4 mg/mL or about 6 mg/mL of a GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and
(d) about 0.02% (w/v) polysorbate 80.

**52.** The pharmaceutical preparation according to any of the preceding claims, wherein the pharmaceutical preparation comprises:

(a) about 5.3 mg/mL, about 6.67 mg/mL, about 10 mg/mL, about 12 mg/mL, about 13.3 mg/mL or about 20 mg/mL of a GLP-1 fusion protein, wherein the amino acid sequence of the GLP-1 fusion protein is as shown in SEQ ID NO: 7;
(b) about 10 mM disodium hydrogen phosphate/sodium dihydrogen phosphate buffer;
(c) about 4.6% (w/v) mannitol; and

(d) about 0.02% (w/v) polysorbate 80.

**53.** The pharmaceutical preparation according to any of the preceding claims, wherein the unit dose of the pharmaceutical preparation is about 0.5 mL and comprises:

(a) about 1 mg, about 2 mg or about 3 mg of a GLP-1 fusion protein, wherein the amino acid sequence of the GLP-1 fusion protein is as shown in SEQ ID NO: 7;
(b) about 0.58 mg of disodium hydrogen phosphate dodecahydrate;
(c) about 0.465 mg of sodium dihydrogen phosphate monohydrate;
(d) about 23.2 mg of mannitol; and
(e) about 0.1 mg of polysorbate 80,

the remainder being water for injection.

**54.** The pharmaceutical preparation according to any of the preceding claims, wherein the unit dose of the pharmaceutical preparation is about 0.75 mL and comprises:

(a) about 4 mg, about 5 mg, about 7.5 mg, about 9 mg, about 10 mg or about 15 mg of a GLP-1 fusion protein, wherein the amino acid sequence of the GLP-1 fusion protein is as shown in SEQ ID NO: 7;
(b) about 0.87 mg of disodium hydrogen phosphate dodecahydrate;
(c) about 0.6975 mg of sodium dihydrogen phosphate monohydrate;
(d) about 34.8 mg of mannitol; and
(e) about 0.15 mg of polysorbate 80,

with the remainder being water for injection.

**55.** The pharmaceutical preparation according to any of the preceding claims, having a pH value in the range of 6.4-7.0.

**56.** The pharmaceutical preparation according to any of the preceding claims, wherein the preparation is isotonic.

**57.** The pharmaceutical preparation according to any of the preceding claims, which is a liquid preparation.

**58.** The pharmaceutical preparation according to any of the preceding claims, wherein the pharmaceutical preparation is administered by intravenous, subcutaneous or intramuscular injection.

**59.** The pharmaceutical preparation according to any of the preceding claims, wherein the preparation remains stable for at least 3 months at 25±2°C, or at least 2 weeks at 40±2°C, or at least 24 months at 2-8°C.

**60.** The pharmaceutical preparation according to any of the preceding claims, wherein the pH value of the preparation changes by no more than about 10% (e.g., no more than about 5%, no more than about 4%, no more than about 3%, no more than about 2%, no more than about 1%) after the preparation is placed for at least 1 month.

**61.** The pharmaceutical preparation according to any of the preceding claims, wherein the concentration of the GLP-1 fusion protein changes by no more than about 10% (e.g., no more than about 5%, no more than about 4%, no more than about 3%, no more than about 2%, no more than about 1%) after the preparation is placed for at least 1 month.

**62.** The pharmaceutical preparation according to any of the preceding claims, wherein the content of high molecular weight (HMW) derivatives or low molecular weight (LMW) derivatives does not exceed about 10% (e.g., not more than about 5%, not more than about 4%, not more than about 3%, not more than about 2%, not more than about 1%, etc.) after the preparation is placed for at least 1 month.

**63.** The pharmaceutical preparation according to any of the preceding claims, wherein the amount of the HMW derivatives and/or LMW derivatives is determined by size exclusion chromatography (SEC) or reverse phase liquid chromatography (RP-LC).

**64.** The pharmaceutical preparation according to any of the preceding claims, wherein the purity of the preparation does not change by more than about 5% after the preparation is placed for at least 1 month.

**65.** The pharmaceutical preparation according to any of the preceding claims, wherein the purity of the preparation is determined by SDS-capillary electrophoresis (e.g., non-reducing SDS-capillary electrophoresis).

**66.** The pharmaceutical preparation according to any of the preceding claims, wherein the isoelectric point of the preparation does not change by more than about 5% (e.g., no more than about 4%, no more than about 3%, no more than about 2%, no more than about 1%, etc.) after the preparation is placed for at least 1 month.

**67.** The pharmaceutical preparation according to any of the preceding claims, wherein the isoelectric point of the preparation is determined by capillary focusing isoelectric electrophoresis (cIEF).

**68.** Use of a pharmaceutical preparation according to any of the preceding claims in the preparation of a medicament for the treatment or prevention of a disease.

**69.** The use according to claim 68, wherein the disease is selected from the group consisting of metabolic diseases associated with disorders of glucose metabolism and/or lipid metabolism, complications of metabolic diseases, and neurological diseases and other related diseases.

**70.** The use according to claim 69, wherein the metabolic disease associated with disorders of glucose metabolism and/or lipid metabolism is selected from the group consisting of diabetes, non-alcoholic steatohepatitis (NASH), non-alcoholic fatty liver disease (NAFLD), obesity and metabolic syndrome.

**71.** The use according to claim 69 or 70, wherein the metabolic disease associated with disorders of glucose metabolism and/or lipid metabolism is diabetes (e.g., type 2 diabetes, type 2 diabetes with poor glycemic control after diet and exercise intervention).

**72.** The use according to claim 69, wherein the complications of the metabolic disease include cardiovascular complications, renal complications or liver complications caused by the metabolic disease.

**73.** The use according to claim 69, wherein the nervous system disease is a neurodegenerative disease.

**74.** The use according to claim 73, wherein the neurodegenerative disease is selected from the group consisting of Alzheimer's disease, motor neuron disease, Huntington's disease and Parkinson's disease.

**75.** The pharmaceutical preparation according to any one of claims 1 to 67, and the use of an additional therapeutic agent in the preparation of a medicament for treating or preventing a disease.

**76.** The use according to claim 75, wherein the disease is selected from the group consisting of metabolic diseases related to disorders of glucose metabolism and/or lipid metabolism, complications of metabolic diseases, and nervous system diseases and other related diseases.

**77.** The use according to claim 76, wherein the metabolic diseases related to disorders of glucose metabolism and/or lipid metabolism are selected from the group consisting of diabetes, non-alcoholic steatohepatitis (NASH), non-alcoholic fatty liver disease (NAFLD), obesity, and metabolic syndrome.

**78.** The use according to claim 76 or 77, wherein the metabolic disease related to disorders of glucose metabolism and/or lipid metabolism is diabetes (e.g., type 2 diabetes, type 2 diabetes with poor blood glucose control after diet and exercise intervention).

**79.** The use according to claim 76, wherein the complications of the metabolic disease include cardiovascular complications, renal complications, or liver complications caused by the metabolic disease.

**80.** The use according to claim 76, wherein the nervous system disease is a neurodegenerative disease.

**81.** The use according to claim 80, wherein the neurodegenerative disease is selected from the group consisting of Alzheimer's disease, motor neuron disease, Huntington's disease and Parkinson's disease.

**82.** The use according to any one of claims 75 to 81, wherein the additional therapeutic agent is selected from the group consisting of insulin, metformin, sulfonylureas (e.g., glimepiride, glibenclamide, gliclazide, gliquidone), alpha glucosidase inhibitors (e.g., acarbose) and gamma-aminobutyric acid.

**83.** The use according to claim 82, wherein the additional therapeutic agent is metformin.

**84.** The use according to claim 82, wherein the additional therapeutic agent is gamma-aminobutyric acid.

**85.** The use of a pharmaceutical preparation according to any one of claims 49 to 54 and metformin in the preparation of a medicament for treating diabetes.

**86.** The use according to claim 85, wherein the diabetes is type 2 diabetes.

**87.** The use according to claim 85 or 86, wherein the amino acid sequence of the GLP-1 fusion protein in the pharmaceutical preparation is as shown in SEQ **ID** NO: 7.

**88.** The use of the pharmaceutical preparation according to any one of claims 49 to 54 and γ-aminobutyric acid in the preparation of a drug for treating a neurodegenerative disease.

**89.** The use according to claim 88, wherein the neurodegenerative disease is selected from the group consisting of Alzheimer's disease, motor neuron disease, Huntington's disease and Parkinson's disease.

**90.** The use according to claim 88 or 89, wherein the amino acid sequence of the GLP-1 fusion protein in the pharmaceutical preparation is as shown in SEQ ID NO: 7.

**91.** A drug combination comprising the pharmaceutical preparation according to any one of claims 1 to 67, and an additional therapeutic agent.

**92.** The drug combination according to claim 91, wherein the additional therapeutic agent is a drug for treating diabetes or a drug for treating a neurodegenerative disease.

**93.** The drug combination according to claim 91 or 92, wherein the additional therapeutic agent is selected from the group consisting of insulin, metformin, sulfonylureas (e.g., glimepiride, glibenclamide, gliclazide, gliquidone), alpha glucosidase inhibitors (e.g., acarbose), and gamma-aminobutyric acid.

**94.** The drug combination according to claim 93, wherein the additional therapeutic agent is metformin.

**95.** The drug combination according to claim 93, wherein the additional therapeutic agent is gamma-aminobutyric acid.

FIG. 1

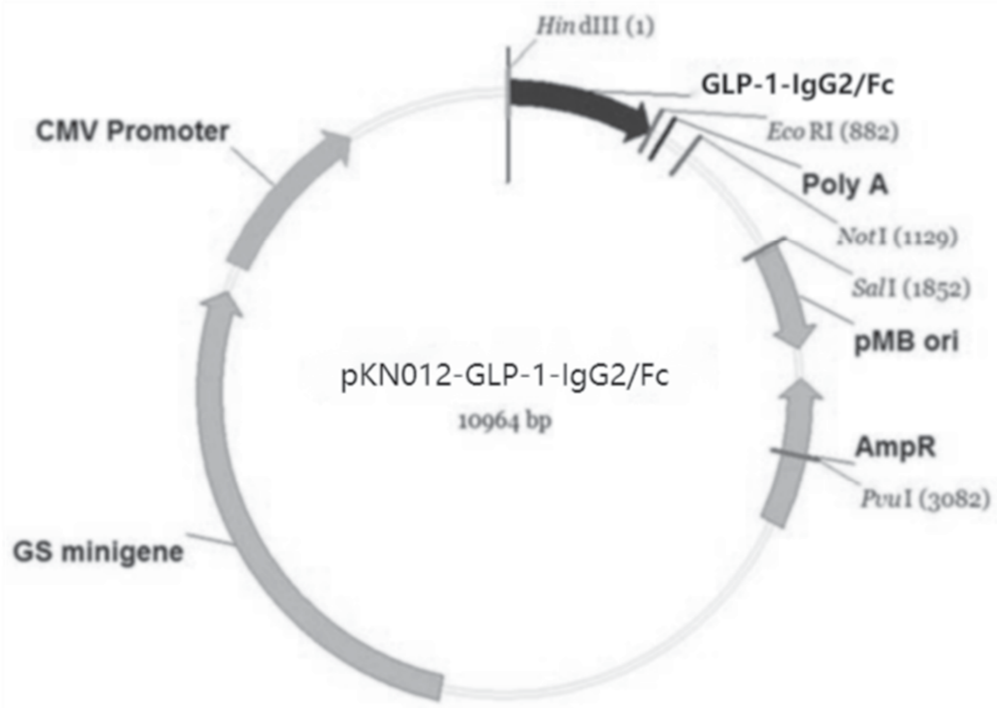
HindIII (1)
GLP-1-IgG2/Fc
EcoRI (882)
Poly A
NotI (1129)
SalI (1852)
pMB ori
AmpR
PvuI (3082)
CMV Promoter
GS minigene
pKN012-GLP-1-IgG2/Fc
10964 bp

FIG. 2

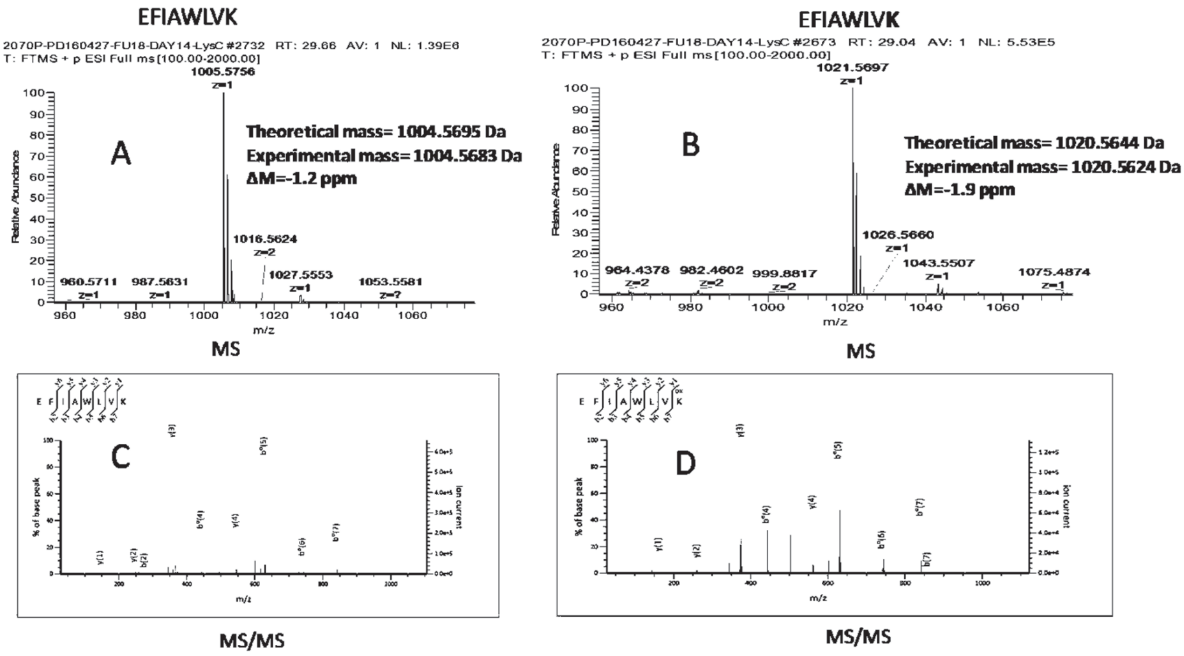
EFIAWLVK
2070P-PD160427-FU18-DAY14-LysC #2732 RT: 29.66 AV: 1 NL: 1.39E6
T: FTMS + p ESI Full ms [100.00-2000.00]
A
Theoretical mass= 1004.5695 Da
Experimental mass= 1004.5683 Da
ΔM=-1.2 ppm
1005.5756 z=1
1016.5624 z=2
1027.5553 z=1
960.5711 z=1
987.5631 z=1
1053.5581 z=?
Relative Abundance
m/z
MS
EFIAWLVK
2070P-PD160427-FU18-DAY14-LysC #2673 RT: 29.04 AV: 1 NL: 5.53E5
T: FTMS + p ESI Full ms [100.00-2000.00]
B
Theoretical mass= 1020.5644 Da
Experimental mass= 1020.5624 Da
ΔM=-1.9 ppm
1021.5697 z=1
1026.5660 z=1
1043.5507 z=1
964.4378 z=2
982.4602 z=2
999.8817 z=2
1075.4874 z=1
Relative Abundance
m/z
MS
C
% of base peak
Ion current
m/z
MS/MS
D
% of base peak
Ion current
m/z
MS/MS

# FIG. 3A

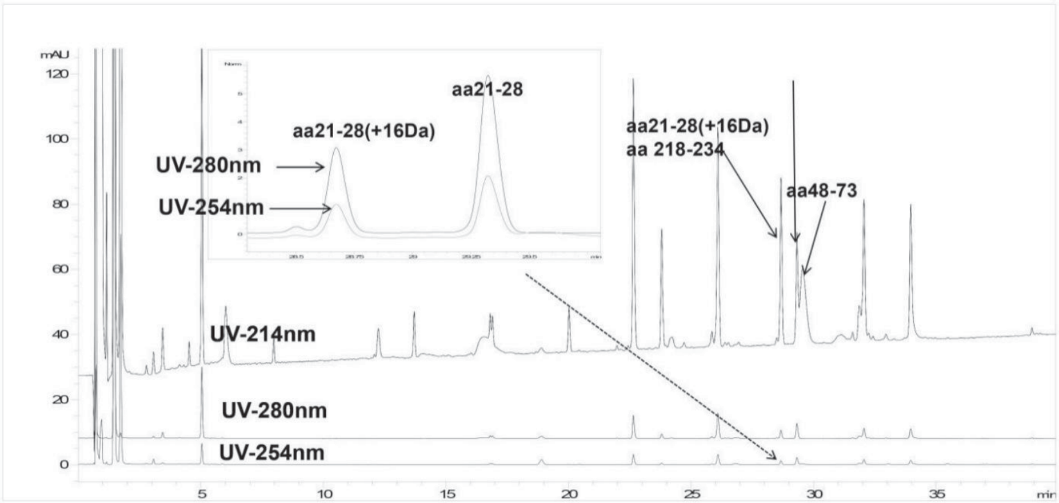


$\varepsilon_W = 5500$

$\varepsilon_Y = 1490$

| | |
|---|---|
| 21-28 | EFIA**W**LVK |
| 218-234 | TTPPMLDSDGSFFL**Y**SK |
| 48-73 | SCVECPPCPAPPVAGPSVFLFPPKPK |

# FIG. 3B

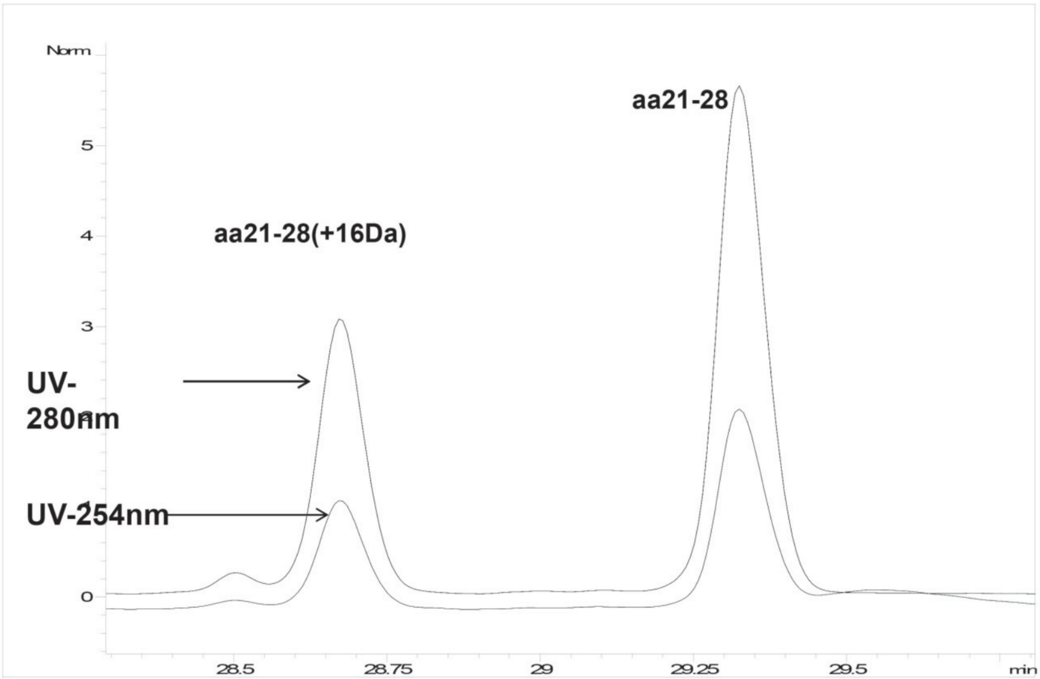
Norm
5
4
3
0
aa21-28
aa21-28(+16Da)
UV-280nm
UV-254nm
28.5
28.75
29
29.25
29.5
min

FIG. 4

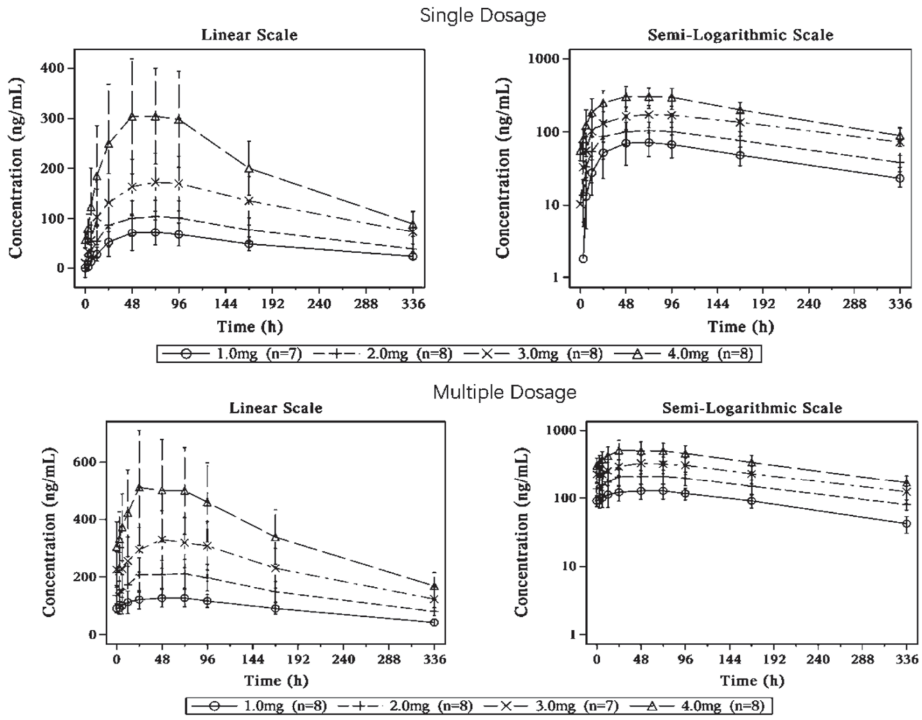
Single Dosage
Linear Scale
Semi-Logarithmic Scale
Concentration (ng/mL)
Time (h)
1.0mg (n=7)
2.0mg (n=8)
3.0mg (n=8)
4.0mg (n=8)
Multiple Dosage
Linear Scale
Semi-Logarithmic Scale
Concentration (ng/mL)
Time (h)
1.0mg (n=8)
2.0mg (n=8)
3.0mg (n=7)
4.0mg (n=8)

# FIG. 5

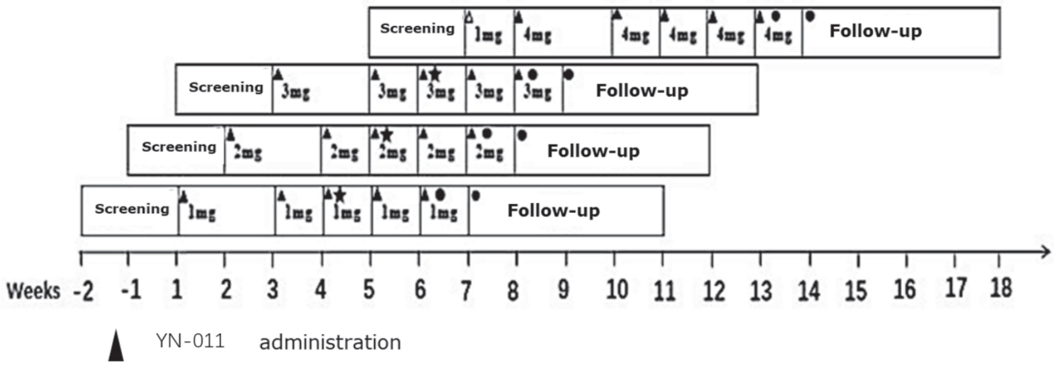
Screening
1mg
4mg
4mg
4mg
4mg
4mg
Follow-up
Screening
3mg
3mg
3mg
3mg
3mg
Follow-up
Screening
2mg
2mg
2mg
2mg
2mg
Follow-up
Screening
1mg
1mg
1mg
1mg
1mg
Follow-up
Weeks -2 -1 1 2 3 4 5 6 7 8 9 10 11 12 13 14 15 16 17 18
YN-011 administration

# FIG. 6

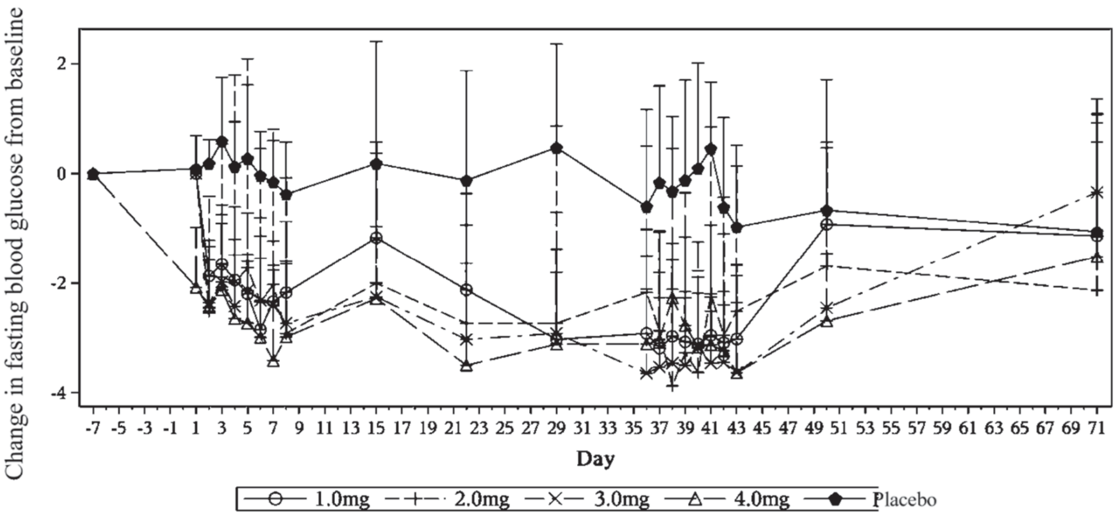
Change in fasting blood glucose from baseline
2
0
-2
-4
-7 -5 -3 -1 1 3 5 7 9 11 13 15 17 19 21 23 25 27 29 31 33 35 37 39 41 43 45 47 49 51 53 55 57 59 61 63 65 67 69 71
Day
1.0mg
2.0mg
3.0mg
4.0mg
Placebo

# FIG. 7

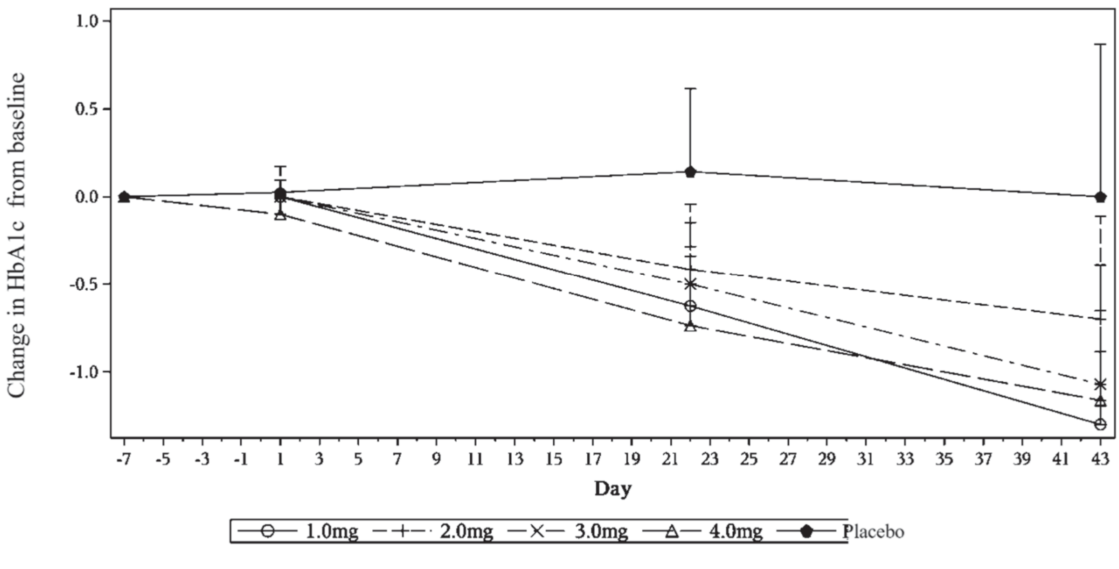
Change in HbA1c from baseline
1.0
0.5
0.0
-0.5
-1.0
-7 -5 -3 -1 1 3 5 7 9 11 13 15 17 19 21 23 25 27 29 31 33 35 37 39 41 43
Day
1.0mg
2.0mg
3.0mg
4.0mg
Placebo

# FIG. 8

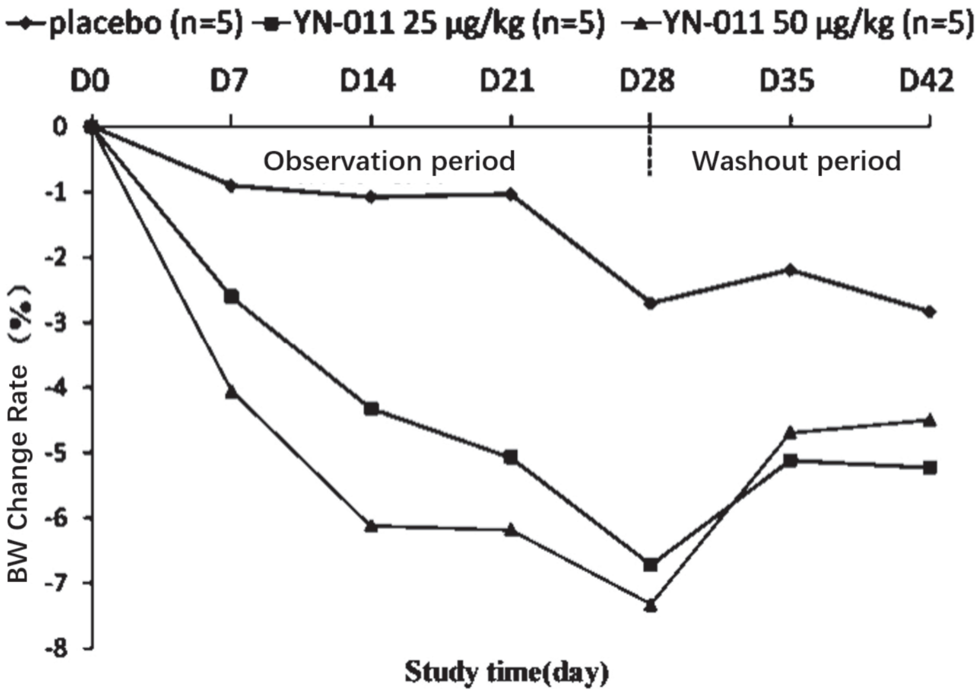
placebo (n=5)
YN-011 25 μg/kg (n=5)
YN-011 50 μg/kg (n=5)
D0
D7
D14
D21
D28
D35
D42
0
-1
-2
-3
-4
-5
-6
-7
-8
Observation period
Washout period
BW Change Rate (%)
Study time(day)

FIG. 9

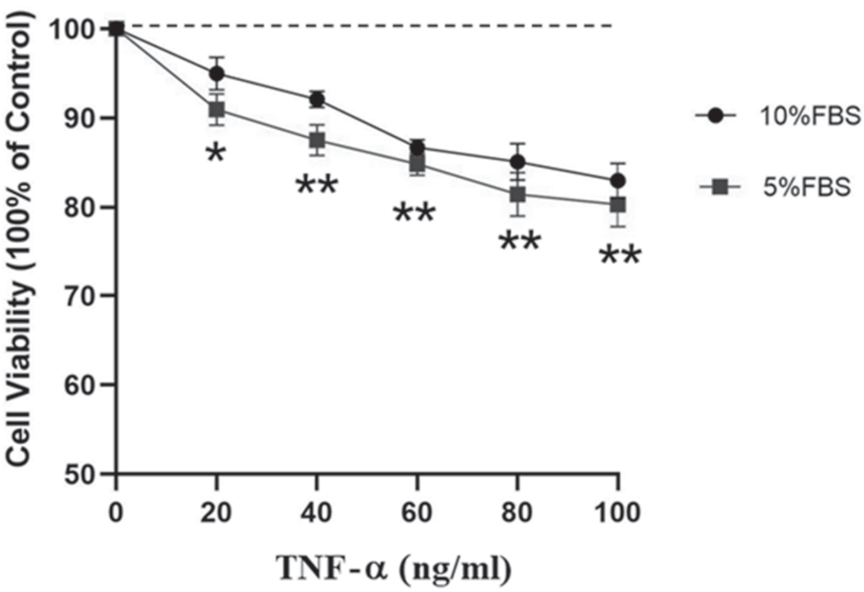
Cell Viability (100% of Control)
100
90
80
70
60
50
0
20
40
60
80
100
TNF-α (ng/ml)
*
**
**
**
**
10%FBS
5%FBS

# FIG. 10

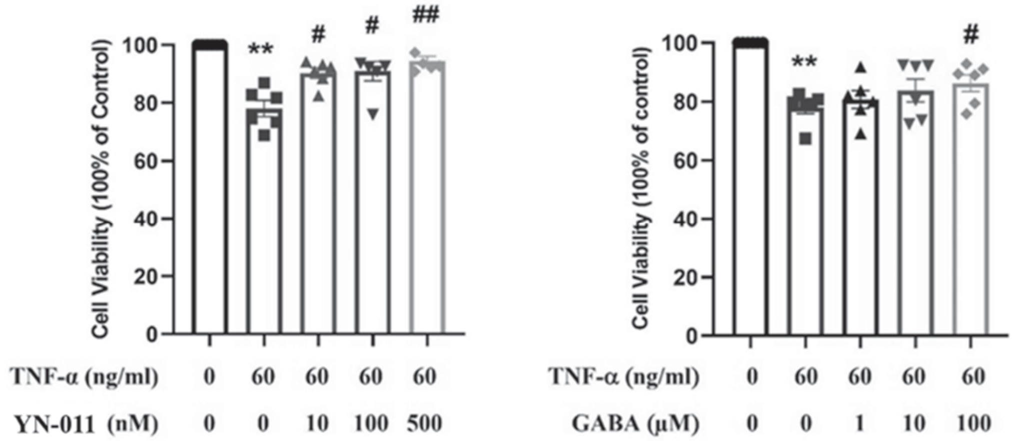
Cell Viability (100% of Control)
100
80
60
40
20
0
**
#
#
##
TNF-α (ng/ml) 0 60 60 60 60
YN-011 (nM) 0 0 10 100 500
Cell Viability (100% of control)
100
80
60
40
20
0
**
#
TNF-α (ng/ml) 0 60 60 60 60
GABA (μM) 0 0 1 10 100

FIG. 11

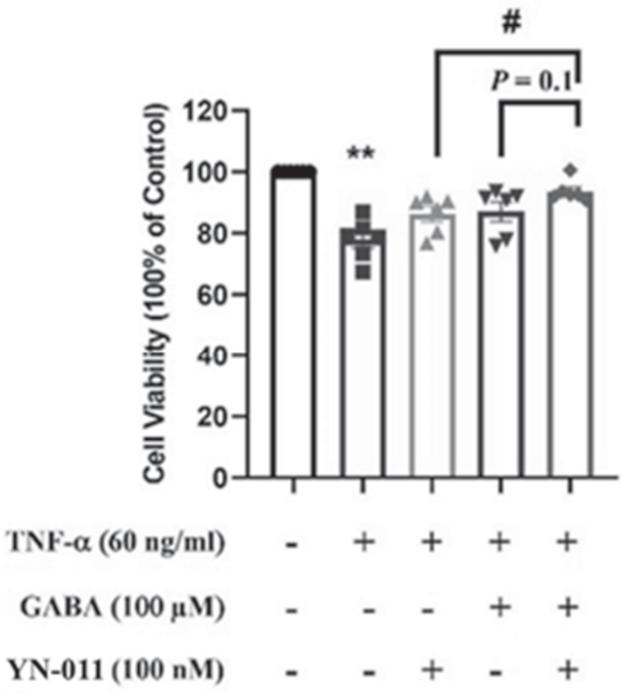
#
P = 0.1
**
Cell Viability (100% of Control)
120
100
80
60
40
20
0
TNF-α (60 ng/ml) - + + + +
GABA (100 μM) - - - + +
YN-011 (100 nM) - - + - +

FIG. 12

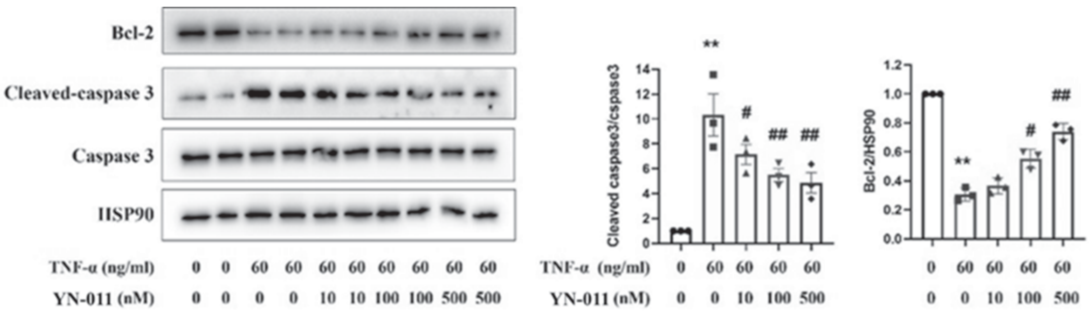
Bcl-2
Cleaved-caspase 3
Caspase 3
HSP90
TNF-α (ng/ml) 0 0 60 60 60 60 60 60 60 60
YN-011 (nM) 0 0 0 0 10 10 100 100 500 500
Cleaved caspase3/cspase3
**
#
##
##
TNF-α (ng/ml) 0 60 60 60 60
YN-011 (nM) 0 0 10 100 500
Bcl-2/HSP90
**
#
##
0 60 60 60 60
0 0 10 100 500

# FIG. 13

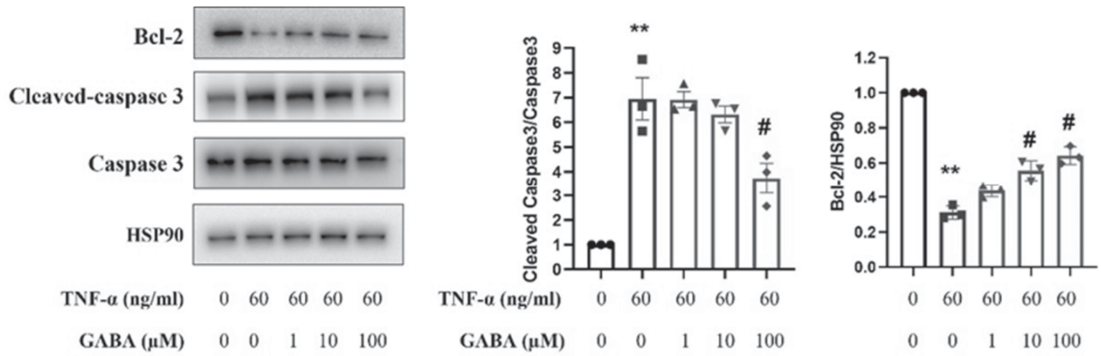
Bcl-2
Cleaved-caspase 3
Caspase 3
HSP90
TNF-α (ng/ml) 0 60 60 60 60
GABA (μM) 0 0 1 10 100
Cleaved Caspase3/Caspase3
**
#
Bcl-2/HSP90
**
#
#

FIG. 14

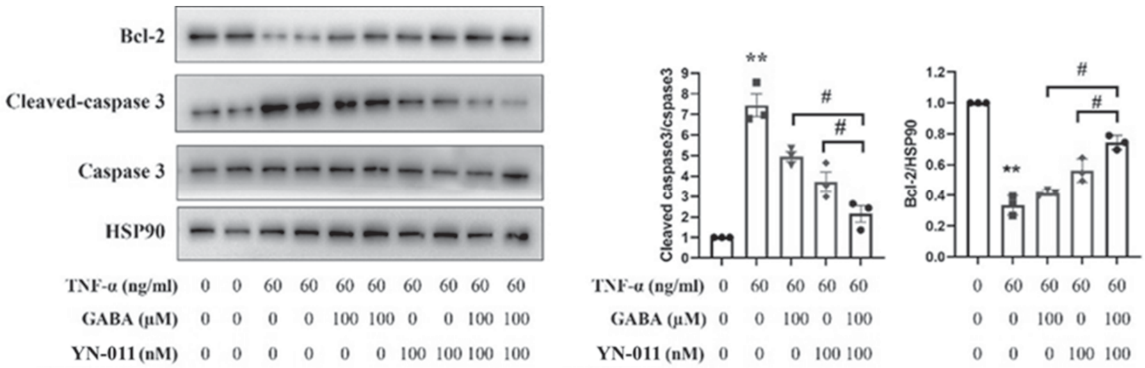

Bcl-2
Cleaved-caspase 3
Caspase 3
HSP90
TNF-α (ng/ml) 0 0 60 60 60 60 60 60 60 60
GABA (μM) 0 0 0 0 100 100 0 0 100 100
YN-011 (nM) 0 0 0 0 0 0 100 100 100 100
Cleaved caspase3/cspase3
**
#
#
TNF-α (ng/ml) 0 60 60 60 60
GABA (μM) 0 0 100 0 100
YN-011 (nM) 0 0 0 100 100
Bcl-2/HSP90
**
#
#
0 60 60 60 60
0 0 100 0 100
0 0 0 100 100

# FIG. 15

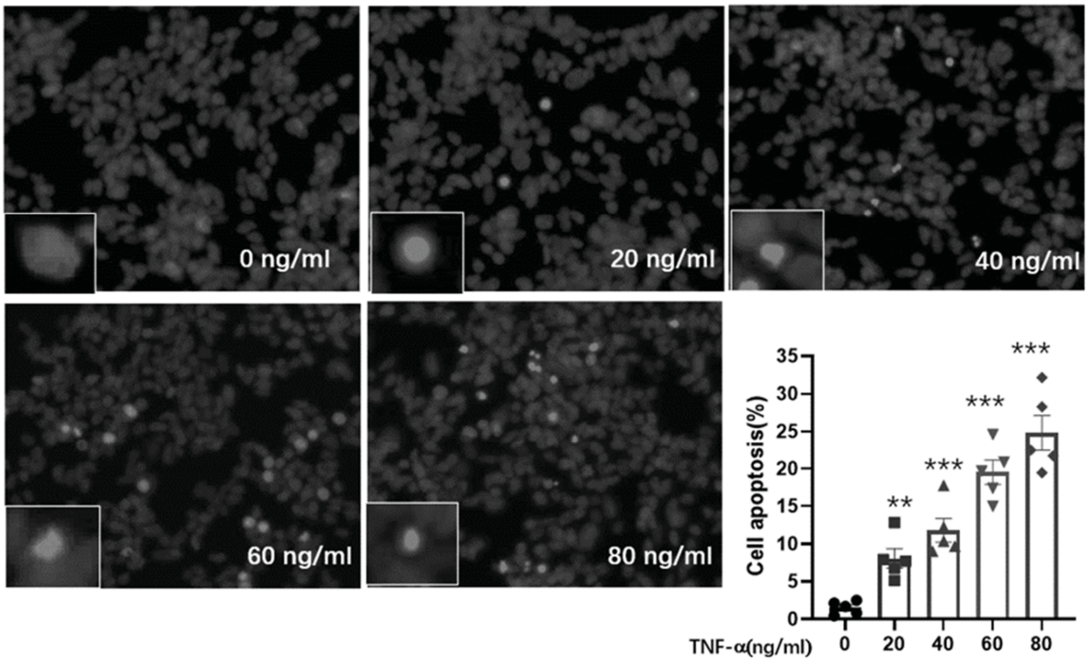
0 ng/ml
20 ng/ml
40 ng/ml
60 ng/ml
80 ng/ml
Cell apoptosis(%)
35
30
25
20
15
10
5
0
**
***
***
***
TNF-α(ng/ml)
0
20
40
60
80

# FIG. 16

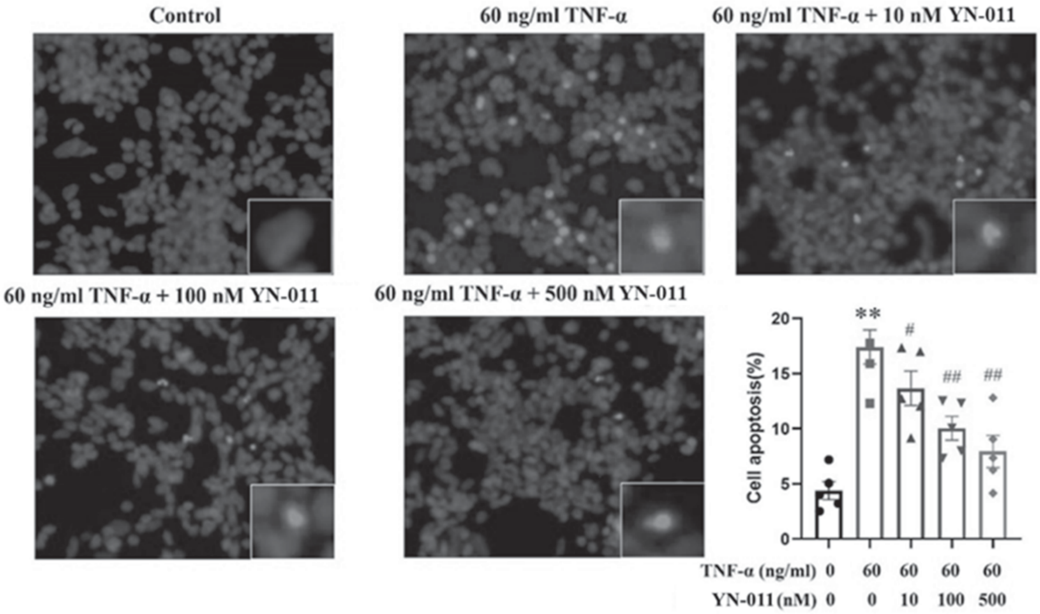
Control
60 ng/ml TNF-α
60 ng/ml TNF-α + 10 nM YN-011
60 ng/ml TNF-α + 100 nM YN-011
60 ng/ml TNF-α + 500 nM YN-011
Cell apoptosis(%)
20
15
10
5
0
**
#
##
##
TNF-α (ng/ml) 0 60 60 60 60
YN-011 (nM) 0 0 10 100 500

# FIG. 17

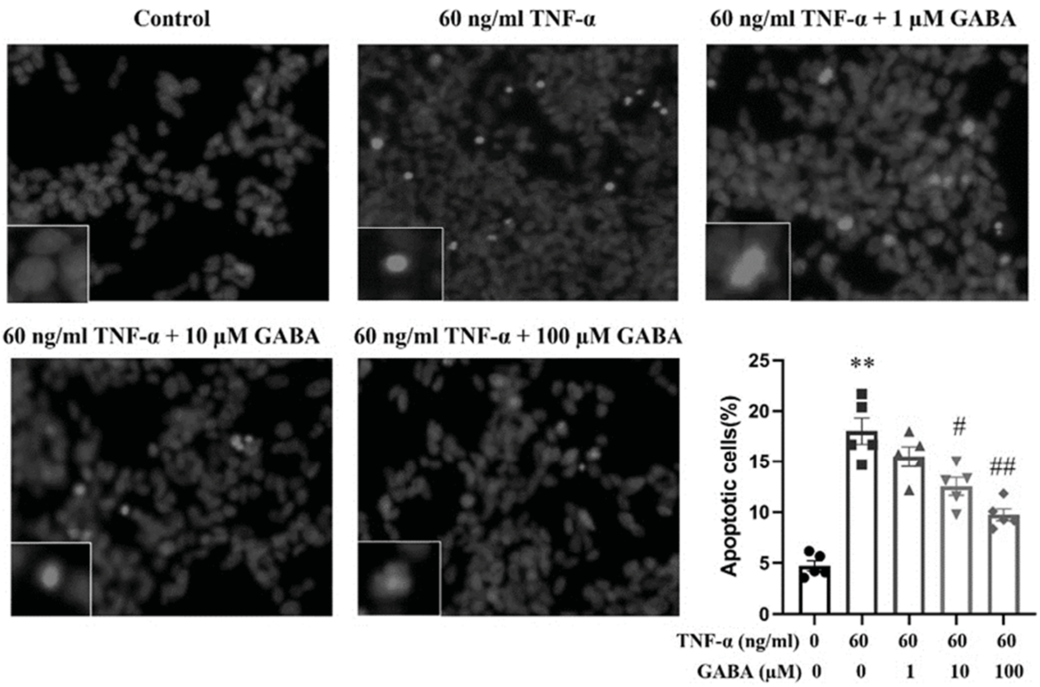
Control
60 ng/ml TNF-α
60 ng/ml TNF-α + 1 μM GABA
60 ng/ml TNF-α + 10 μM GABA
60 ng/ml TNF-α + 100 μM GABA
Apoptotic cells(%)
25
20
15
10
5
0
**
#
##
TNF-α (ng/ml) 0 60 60 60 60
GABA (μM) 0 0 1 10 100

FIG. 18

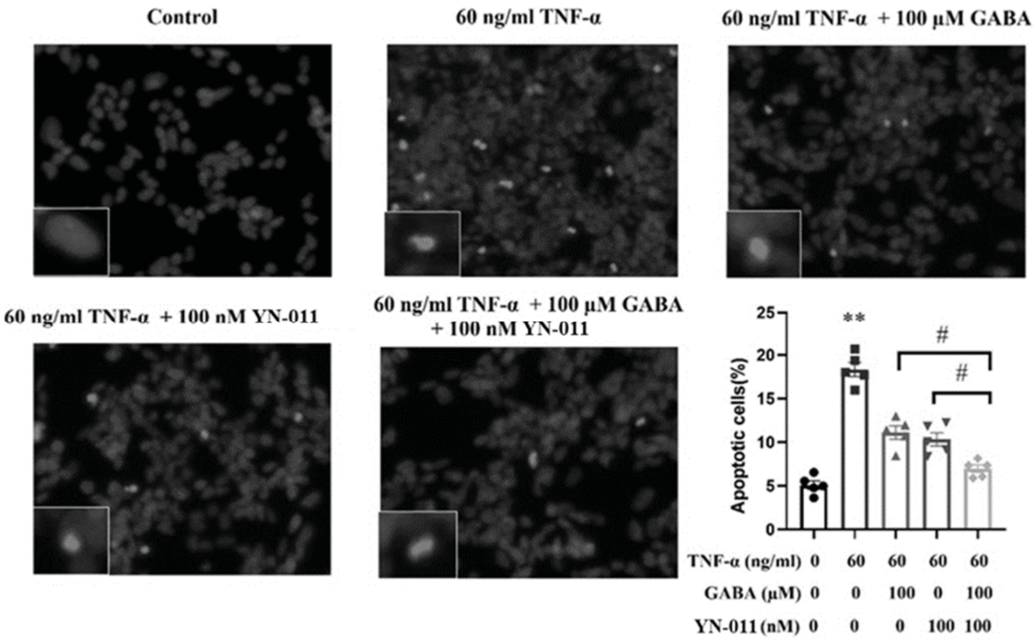
Control
60 ng/ml TNF-α
60 ng/ml TNF-α + 100 μM GABA
60 ng/ml TNF-α + 100 nM YN-011
60 ng/ml TNF-α + 100 μM GABA + 100 nM YN-011
Apoptotic cells(%)
25
20
15
10
5
0
**
#
#
TNF-α (ng/ml) 0 60 60 60 60
GABA (μM) 0 0 100 0 100
YN-011 (nM) 0 0 0 100 100

# FIG. 19

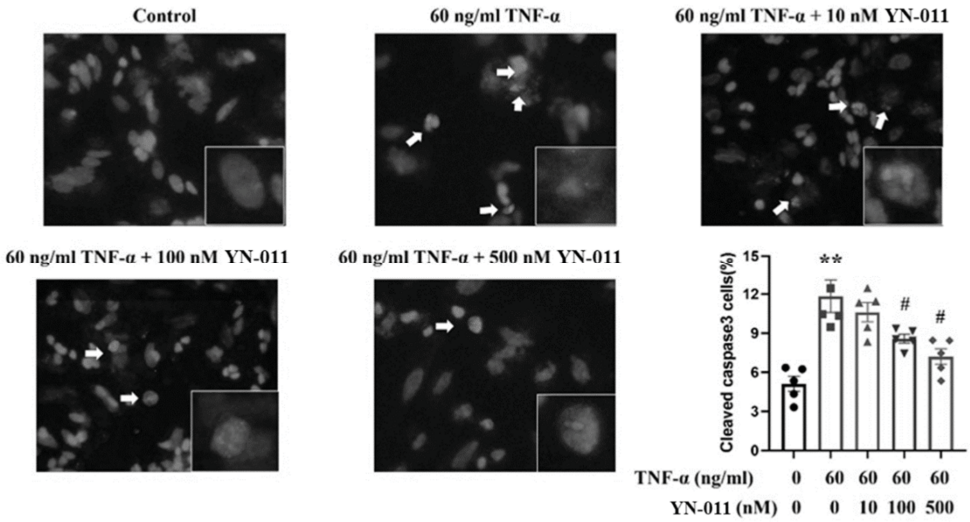
Control
60 ng/ml TNF-α
60 ng/ml TNF-α + 10 nM YN-011
60 ng/ml TNF-α + 100 nM YN-011
60 ng/ml TNF-α + 500 nM YN-011
Cleaved caspase3 cells(%)
15
12
9
6
3
0
**
#
#
TNF-α (ng/ml) 0 60 60 60 60
YN-011 (nM) 0 0 10 100 500

# FIG. 20

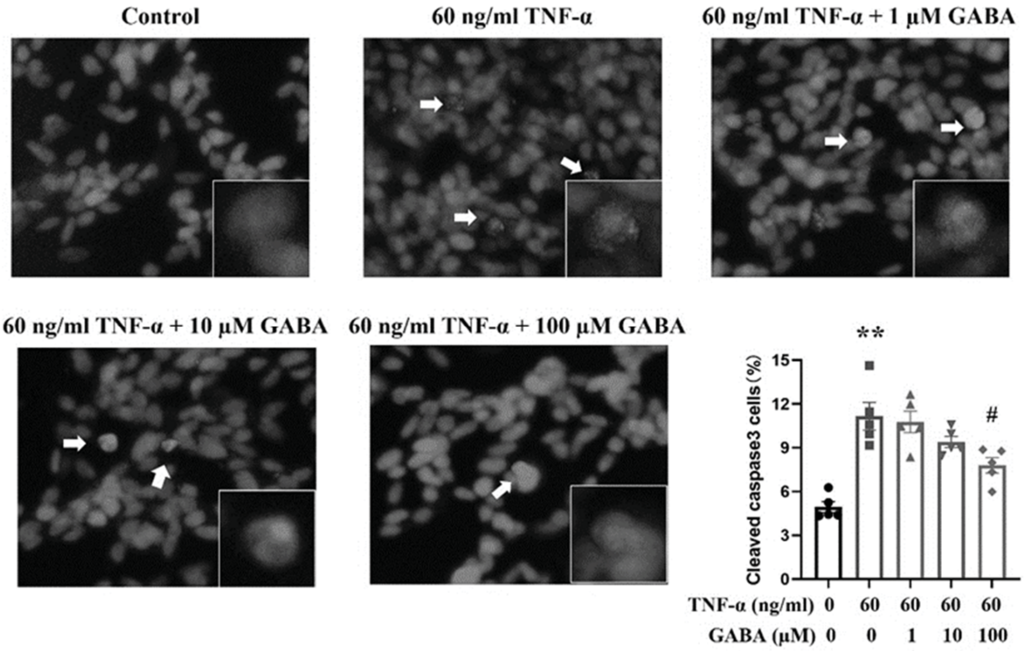
Control
60 ng/ml TNF-α
60 ng/ml TNF-α + 1 μM GABA
60 ng/ml TNF-α + 10 μM GABA
60 ng/ml TNF-α + 100 μM GABA
**
#
Cleaved caspase3 cells (%)
15
12
9
6
3
0
TNF-α (ng/ml) 0 60 60 60 60
GABA (μM) 0 0 1 10 100

# FIG. 21

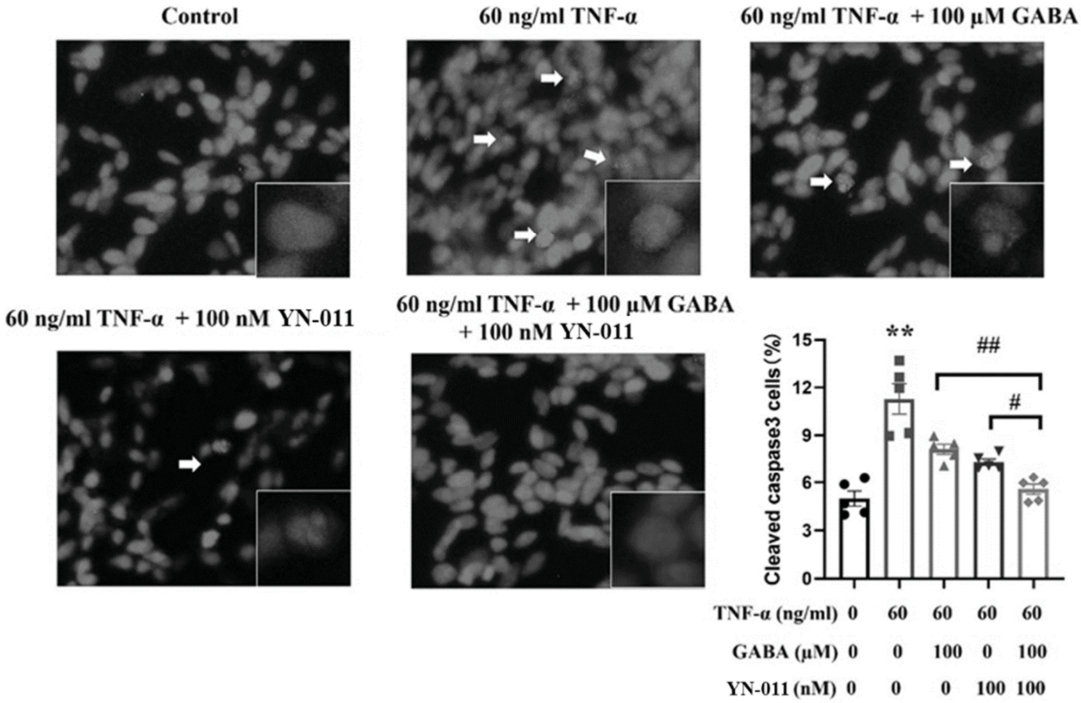

Control
60 ng/ml TNF-α
60 ng/ml TNF-α + 100 μM GABA
60 ng/ml TNF-α + 100 nM YN-011
60 ng/ml TNF-α + 100 μM GABA + 100 nM YN-011
Cleaved caspase3 cells (%)
0
3
6
9
12
15
**
##
#
TNF-α (ng/ml) 0 60 60 60 60
GABA (μM) 0 0 100 0 100
YN-011 (nM) 0 0 0 100 100

# FIG. 22

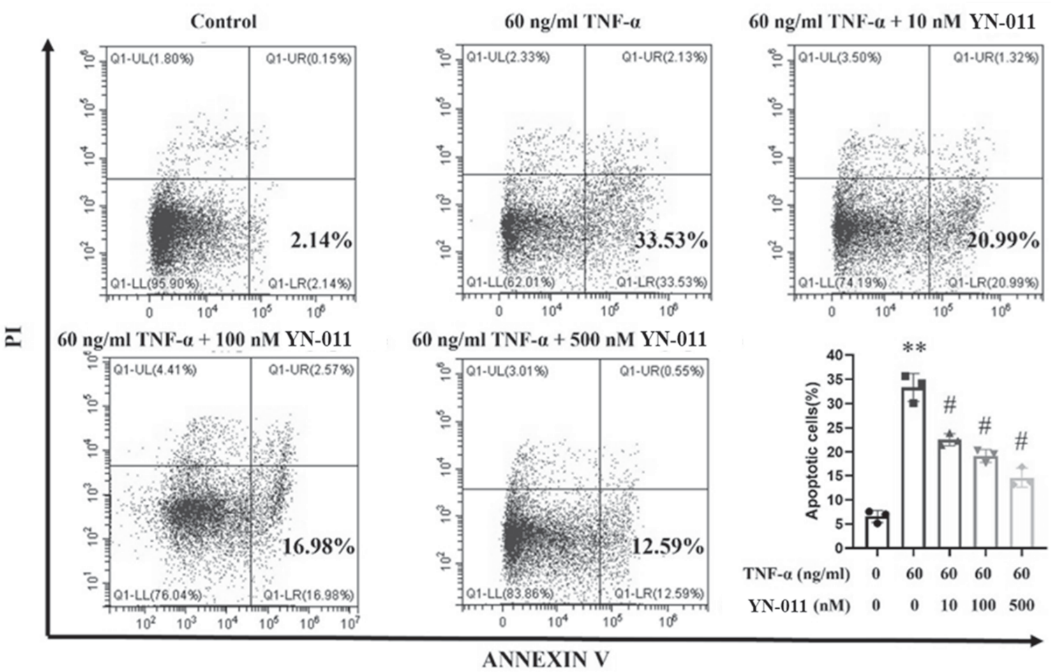
Control
60 ng/ml TNF-α
60 ng/ml TNF-α + 10 nM YN-011
60 ng/ml TNF-α + 100 nM YN-011
60 ng/ml TNF-α + 500 nM YN-011
Q1-UL(1.80%)
Q1-UR(0.15%)
Q1-LL(95.90%)
Q1-LR(2.14%)
2.14%
Q1-UL(2.33%)
Q1-UR(2.13%)
Q1-LL(62.01%)
Q1-LR(33.53%)
33.53%
Q1-UL(3.50%)
Q1-UR(1.32%)
Q1-LL(74.19%)
Q1-LR(20.99%)
20.99%
Q1-UL(4.41%)
Q1-UR(2.57%)
Q1-LL(76.04%)
Q1-LR(16.98%)
16.98%
Q1-UL(3.01%)
Q1-UR(0.55%)
Q1-LL(83.86%)
Q1-LR(12.59%)
12.59%
PI
ANNEXIN V
Apoptotic cells(%)
TNF-α (ng/ml) 0 60 60 60 60
YN-011 (nM) 0 0 10 100 500
**
#

# FIG. 23

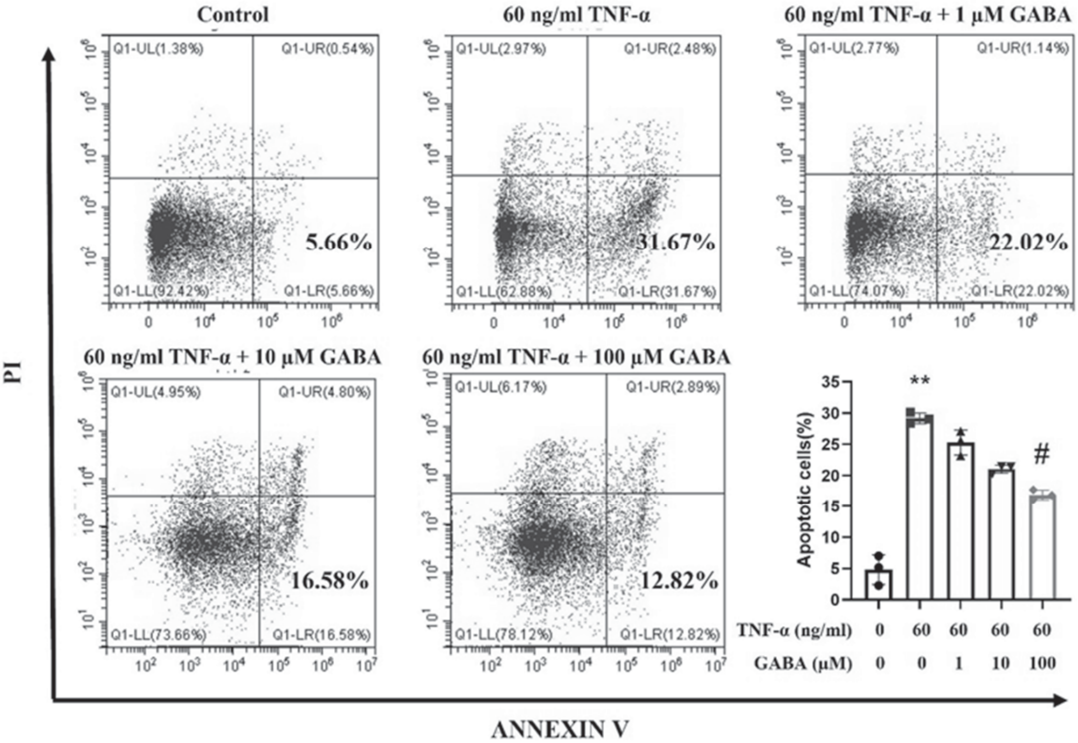
Control
60 ng/ml TNF-α
60 ng/ml TNF-α + 1 μM GABA
60 ng/ml TNF-α + 10 μM GABA
60 ng/ml TNF-α + 100 μM GABA
Q1-UL(1.38%)
Q1-UR(0.54%)
Q1-LL(92.42%)
Q1-LR(5.66%)
5.66%
Q1-UL(2.97%)
Q1-UR(2.48%)
Q1-LL(62.88%)
Q1-LR(31.67%)
31.67%
Q1-UL(2.77%)
Q1-UR(1.14%)
Q1-LL(74.07%)
Q1-LR(22.02%)
22.02%
Q1-UL(4.95%)
Q1-UR(4.80%)
Q1-LL(73.66%)
Q1-LR(16.58%)
16.58%
Q1-UL(6.17%)
Q1-UR(2.89%)
Q1-LL(78.12%)
Q1-LR(12.82%)
12.82%
PI
ANNEXIN V
Apoptotic cells(%)
**
#
TNF-α (ng/ml) 0 60 60 60 60
GABA (μM) 0 0 1 10 100

# FIG. 24

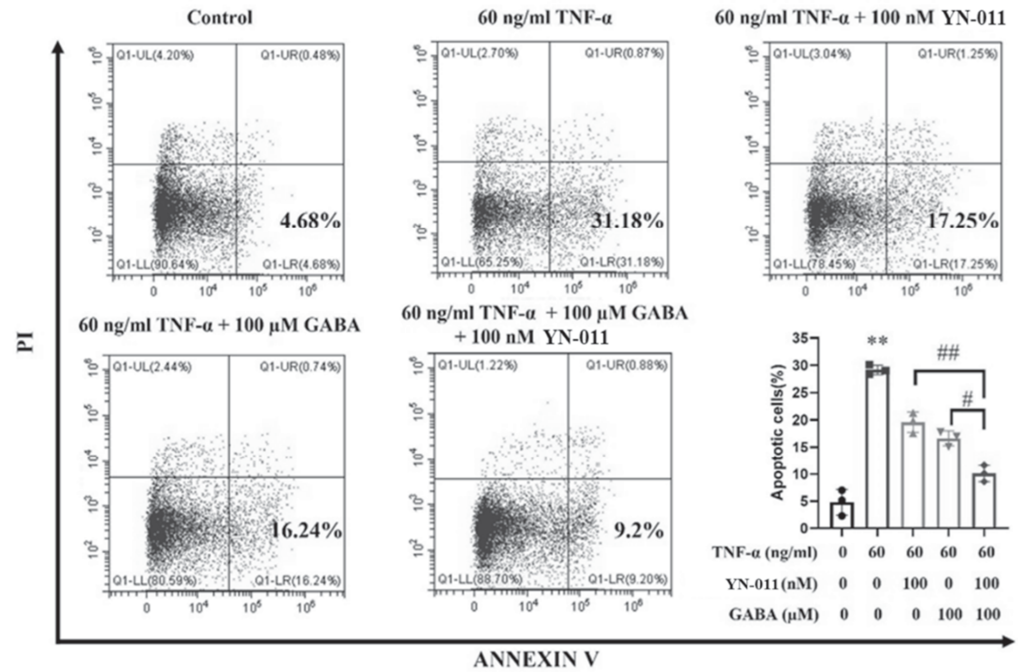
Control
60 ng/ml TNF-α
60 ng/ml TNF-α + 100 nM YN-011
60 ng/ml TNF-α + 100 μM GABA
60 ng/ml TNF-α + 100 μM GABA + 100 nM YN-011
Q1-UL(4.20%)
Q1-UR(0.48%)
Q1-LL(90.64%)
Q1-LR(4.68%)
4.68%
Q1-UL(2.70%)
Q1-UR(0.87%)
Q1-LL(65.25%)
Q1-LR(31.18%)
31.18%
Q1-UL(3.04%)
Q1-UR(1.25%)
Q1-LL(78.45%)
Q1-LR(17.25%)
17.25%
Q1-UL(2.44%)
Q1-UR(0.74%)
Q1-LL(80.59%)
Q1-LR(16.24%)
16.24%
Q1-UL(1.22%)
Q1-UR(0.88%)
Q1-LL(88.70%)
Q1-LR(9.20%)
9.2%
PI
ANNEXIN V
Apoptotic cells(%)
**
##
#
TNF-α (ng/ml) 0 60 60 60 60
YN-011(nM) 0 0 100 0 100
GABA (μM) 0 0 0 100 100

# FIG. 25

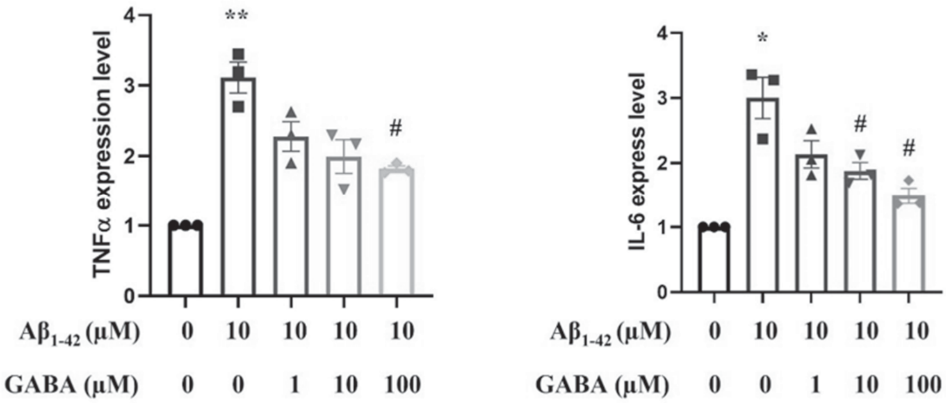
TNFα expression level
**
#
0
1
2
3
4
Aβ1-42 (μM) 0 10 10 10 10
GABA (μM) 0 0 1 10 100
IL-6 express level
*
#
#
0
1
2
3
4
Aβ1-42 (μM) 0 10 10 10 10
GABA (μM) 0 0 1 10 100

# FIG. 26

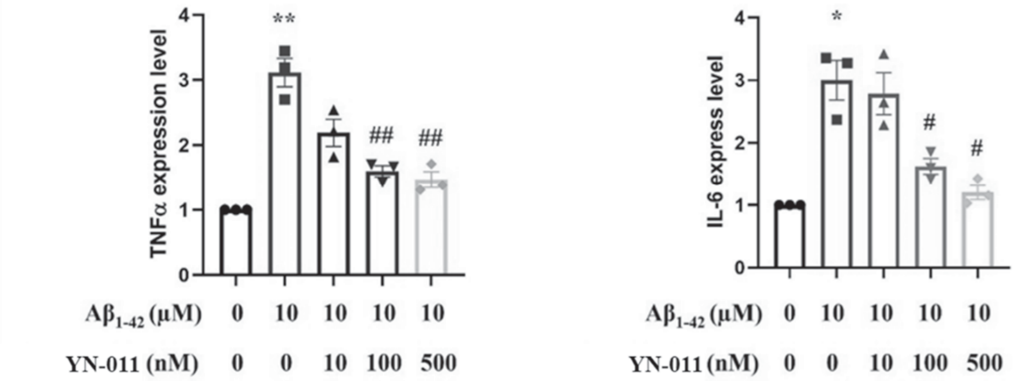
TNFα expression level
**
##
##
IL-6 express level
*
#
#
Aβ1-42 (μM) 0 10 10 10 10
YN-011 (nM) 0 0 10 100 500
Aβ1-42 (μM) 0 10 10 10 10
YN-011 (nM) 0 0 10 100 500

# FIG. 27

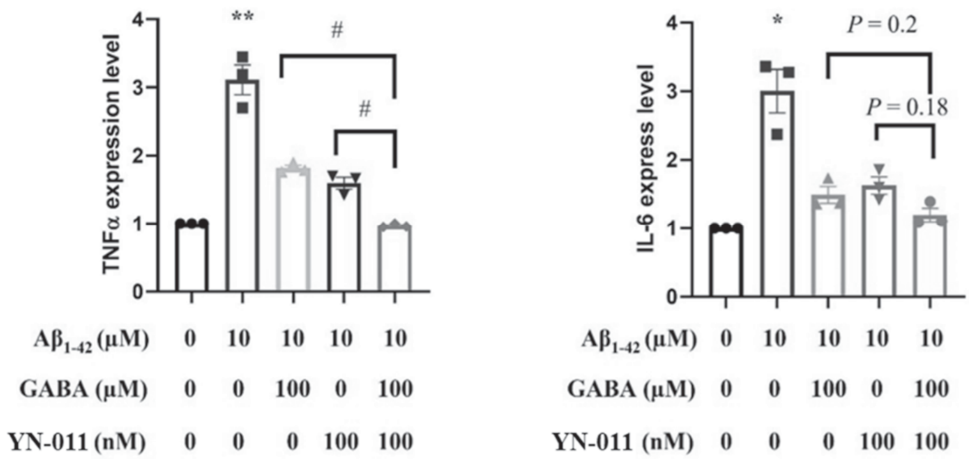
TNFα expression level
**
#
#
0
1
2
3
4
Aβ1-42 (μM) 0 10 10 10 10
GABA (μM) 0 0 100 0 100
YN-011 (nM) 0 0 0 100 100
IL-6 express level
*
P = 0.2
P = 0.18
0
1
2
3
4
Aβ1-42 (μM) 0 10 10 10 10
GABA (μM) 0 0 100 0 100
YN-011 (nM) 0 0 0 100 100

FIG. 28

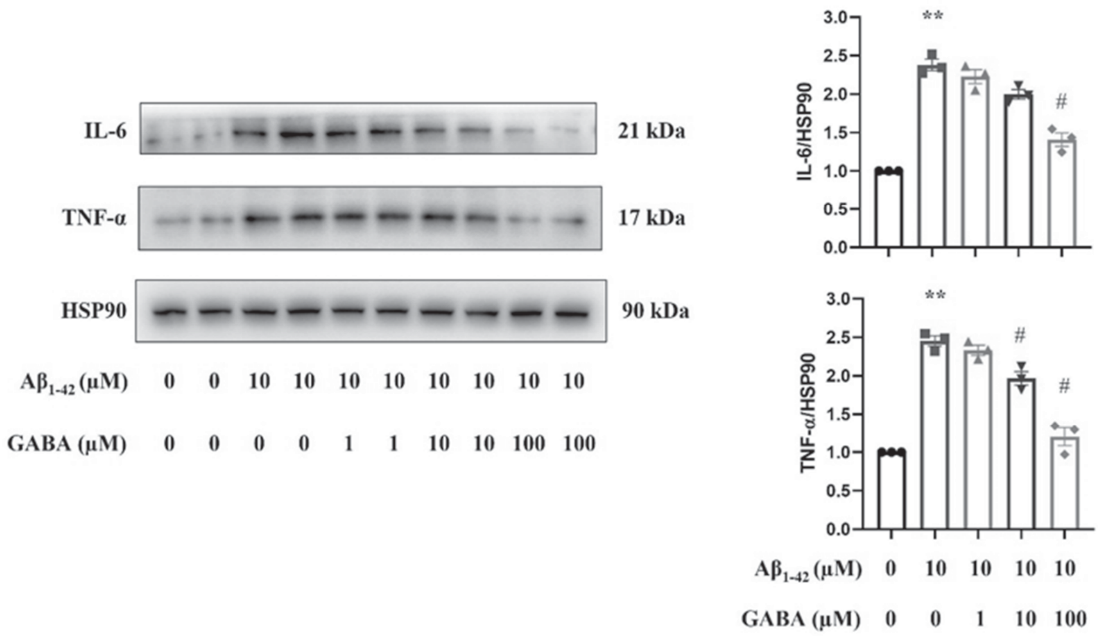
IL-6
21 kDa
TNF-α
17 kDa
HSP90
90 kDa
Aβ1-42 (μM) 0 0 10 10 10 10 10 10 10 10
GABA (μM) 0 0 0 0 1 1 10 10 100 100
IL-6/HSP90
TNF-α/HSP90
**
#
Aβ1-42 (μM) 0 10 10 10 10
GABA (μM) 0 0 1 10 100

# FIG. 29

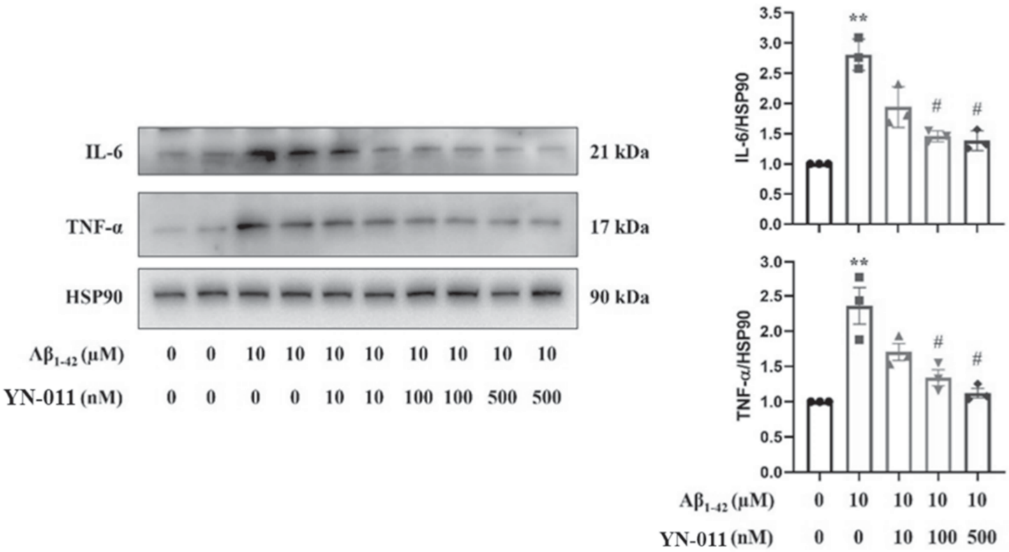
IL-6
21 kDa
TNF-α
17 kDa
HSP90
90 kDa
Aβ1-42 (μM) 0 0 10 10 10 10 10 10 10 10
YN-011 (nM) 0 0 0 0 10 10 100 100 500 500
IL-6/HSP90
TNF-α/HSP90
**
#
Aβ1-42 (μM) 0 10 10 10 10
YN-011 (nM) 0 0 10 100 500

# FIG. 30

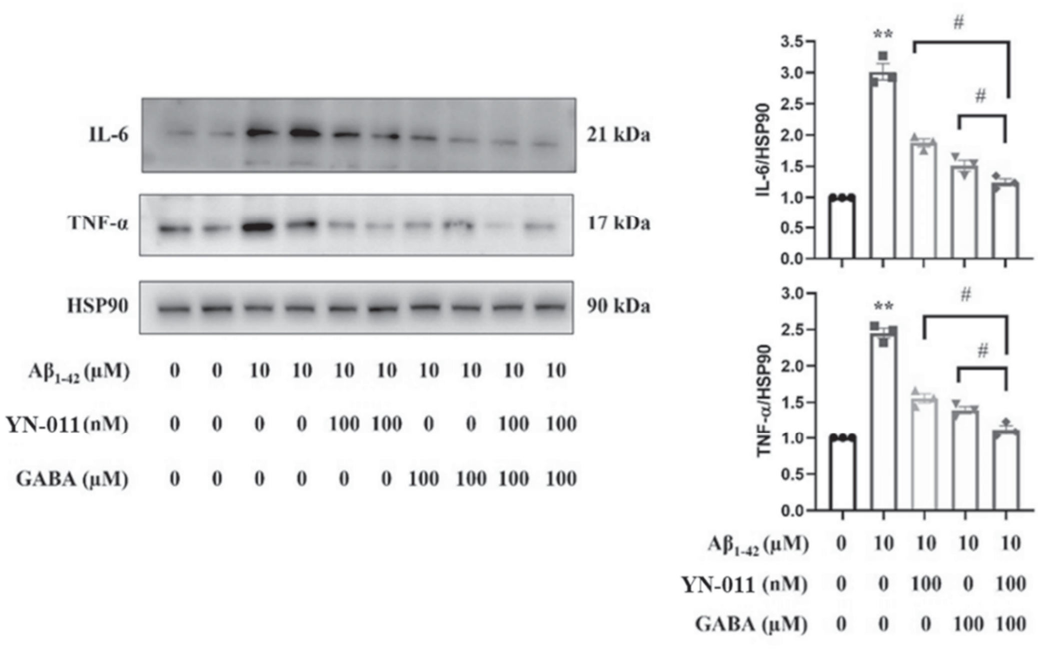
IL-6
21 kDa
TNF-α
17 kDa
HSP90
90 kDa
Aβ1-42 (μM) 0 0 10 10 10 10 10 10 10 10
YN-011 (nM) 0 0 0 0 100 100 0 0 100 100
GABA (μM) 0 0 0 0 0 0 100 100 100 100
IL-6/HSP90
TNF-α/HSP90
**
#
Aβ1-42 (μM) 0 10 10 10 10
YN-011 (nM) 0 0 100 0 100
GABA (μM) 0 0 0 100 100

## INTERNATIONAL SEARCH REPORT

International application No.
**PCT/CN2024/085034**

**A. CLASSIFICATION OF SUBJECT MATTER**

A61K 47/68(2017.01)i; C07K19/00(2006.01)i; A61K38/16(2006.01)i; A61P25/28(2006.01)i; A61P3/10(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CNTXT, CNABS, DWPI, VEN, ENTXT, CNKI, 万方, WANFANG, ISI Web of Knowledge, 中国专利生物序列检索系统, China Patents Biological Sequence Search System, Genbank: 广州银诺医药集团股份有限公司, 上海银诺医药技术有限公司, 上海银诺生物医药工程有限公司, 王庆华, 药物制剂, 半衰期, 融合蛋白, GLP-1, 免疫球蛋白Fc结构域, 氨基酸, 替换, 羟基化, 氧化, A8G, G22E, R36G, C222S, A330S, P331S, SEQ ID NO: 1-43, 缓冲液, 糖尿病, 神经退行性疾病, 二甲双胍, γ-氨基丁酸, pharmaceutical formulations, half-lives, fusion proteins, immunoglobulin Fc domain, amino acids, substitutions, hydroxylations, oxidations, buffers, diabetes, neurodegenerative diseases, metformin, γ-aminobutyric acid

**C. DOCUMENTS CONSIDERED TO BE RELEVANT**

| Category* | Citation of document, with indication, where appropriate, of the relevant passages | Relevant to claim No. |
|---|---|---|
| X | YANG, Yi et al. "Expression and Characterization of a Potent Long-Acting GLP-1 Receptor Agonist, GLP-1-IgG2σ-Fc" *PLOS ONE*, Vol. 11, No. 5, 27 May 2016 (2016-05-27), document number e0156449 | 1-95 |
| X | US 2016310575 A1 (INDIANA UNIVERSITY RESEARCH AND TECHNOLOGY CORPORATION et al.) 27 October 2016 (2016-10-27) claims 1-30, and description, paragraphs 11-266 | 1-95 |
| A | US 2009181912 A1 (WANG, Q. H. et al.) 16 July 2009 (2009-07-16) claims 62-87 | 1-95 |
| A | CN 104147611 A (GLAXO GROUP LTD.) 19 November 2014 (2014-11-19) claims 1-10 | 1-95 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 July 2024** | **08 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/085034**

**C. DOCUMENTS CONSIDERED TO BE RELEVANT**

| Category* | Citation of document, with indication, where appropriate, of the relevant passages | Relevant to claim No. |
|---|---|---|
| A | CN 114395597 A (FOSHAN HANTENG BIOTECHNOLOGY CO., LTD. et al.) 26 April 2022 (2022-04-26)<br>claims 1-12 | 1-95 |
| A | CN 106110325 A (SHANGHAI LANGAN BIOTECHNOLOGY CO., LTD.) 16 November 2016 (2016-11-16)<br>description, paragraphs 18-54 | 1-95 |
| A | CN 115212293 A (CSPC ZHONGQI PHARMACEUTICAL TECHNOLOGY (SHIJIAZHUANG) CO., LTD. et al.) 21 October 2022 (2022-10-21)<br>claims 1-10 | 1-95 |

Form PCT/ISA/210 (second sheet) (July 2022)

**INTERNATIONAL SEARCH REPORT**

International application No.
**PCT/CN2024/085034**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/085034**

| Patent document cited in search report | Publication date (day/month/year) | Patent family member(s) | Publication date (day/month/year) |
|---|---|---|---|
| US 2016310575 A1 | 27 October 2016 | WO 2015095684 A1 | 25 June 2015 |
| | | JP 2017504598 A | 09 February 2017 |
| | | US 10137170 B2 | 27 November 2018 |
| | | EP 3082847 A1 | 26 October 2016 |
| US 2009181912 A1 | 16 July 2009 | WO 2007012188 A1 | 01 February 2007 |
| | | CA 2658673 A1 | 01 February 2008 |
| | | EP 1920061 A1 | 14 May 2008 |
| | | EP 1920061 A4 | 13 May 2009 |
| | | US 8658174 B2 | 25 February 2014 |
| CN 104147611 A | 19 November 2014 | MX 2012003939 A | 30 July 2012 |
| | | US 2012276098 A1 | 01 November 2012 |
| | | WO 2011039096 A1 | 07 April 2011 |
| | | JP 2013506628 A | 28 February 2013 |
| | | KR 20120092611 A | 21 August 2012 |
| | | EP 2483308 A1 | 08 August 2012 |
| | | US 2014227264 A1 | 14 August 2014 |
| | | CA 2774552 A1 | 07 April 2011 |
| | | BR 112012007374 A2 | 24 September 2019 |
| | | SG 10201406063 XA | 27 November 2014 |
| | | AU 2010303112 A1 | 26 April 2012 |
| | | EA 201290123 A1 | 30 October 2012 |
| | | IL 218651 A0 | 31 May 2012 |
| CN 114395597 A | 26 April 2022 | None | |
| CN 106110325 A | 16 November 2016 | None | |
| CN 115212293 A | 21 October 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8658174 B
- WO 2021163972 A1
- CN 111217915 A
- WO 2011056713 A2
- WO 2000034332 A1
- WO 2002046227 A2


### Non-patent literature cited in the description


- **LEECH, C.A. et al.** Expression of cAMP-regulated guanine nucleotide exchange factors in pancreatic beta-cells.. *Biochem Biophys Res Commun*, 2000, vol. 278 (1), 44-7
- **SEE HOLLIGER et al.** *Proc. Natl. Acad. Sci. USA*, 1993, vol. 90, 6444-6448
- **POLJAK et al.** *Structure*, 1994, vol. 2, 1121-1123
- **HOU, Y. et al.** Nutrient Optimization Reduces Phosphorylation and Hydroxylation Level on an Fc-Fusion Protein in a CHO Fed-Batch Process.. *Biotechnol J*, 2019, vol. 14 (3), e1700706
- **BETTINGER, J.Q. et al.** Quantitative Analysis of in Vivo Methionine Oxidation of the Human Proteome.. *J Proteome Res*, 2020, vol. 19 (2), 624-633
- **ALLEN, L.V.** ; **JR., REMINGTON**. The Science and Practice of Pharmacy: from the past into the future.. *Int J Pharm Compd*, 2012, vol. 16 (5), 358-62
- **REMINGTON**. The Science and Practice of Pharmacy. Lippincott, Williams & Wilkins, 2006
- **GEISER, J.S. et al.** Clinical Pharmacokinetics of Dulaglutide in Patients with Type 2 Diabetes: Analyses of Data from Clinical Trials.. *Clin Pharmacokinet*, 2016, vol. 55 (5), 625-34
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98
- Principles of peptide synthesis. **BODANSZKY, M.** Springer laboratory. Springer-Verlag, 1993, 329
- **GEISER, J.S. et al.** Clinical Pharmacokinetics of Dulaglutide in Patients with Type 2 Diabetes: Analyses of Data from Clinical Trials. *Clin Pharmacokinet*, 2016, vol. 55 (5), 625-34
- **PRATLEY, R.E. et al.** Semaglutide versus dulaglutide once weekly in patients with type 2 diabetes (SUSTAIN 7): a randomised, open-label, phase 3b trial.. *Lancet Diabetes Endocrinol*, 2018, vol. 6 (4), 275-286
- **ELBASSUONI, E.A.** ; **R.F. AHMED**. Mechanism of the neuroprotective effect of GLP-1 in a rat model of Parkinson's with pre-existing diabetes.. *Neurochem Int*, 2019, vol. 131, 104583
- **ZHENG, J. et al.** GLP-1 improves the supportive ability of astrocytes to neurons by promoting aerobic glycolysis in Alzheimer's disease.. *Mol Metab*, 2021, vol. 47, 101180
- **PARK, J.S. et al.** Blocking microglial activation of reactive astrocytes is neuroprotective in models of Alzheimer's disease.. *Acta Neuropathol Commun*, 2021, vol. 9 (1), 78
- **SORLI et al.** *Lancet Diabetes Endocrinol*, 2017, vol. 5, 251-60
- **SHI et al.** *J Diabetes Investig*, 2020, vol. 11, 142-150
- **SHUAI et al.** *Diabetes Obes Metab.*, 2021, vol. 23 (1), 116-124
- **DUNGAN et al.** *Lancet*, 2014, vol. 384, 1349-57
- **JI et al.** *Diabetes Obes Metab*, 2021, vol. 23, 404-414